(19) Europäisches Patentamt / European Patent Office / Office européen des brevets

(11) **EP 2 797 946 B1**

(12) **EUROPÄISCHE PATENTSCHRIFT**

(45) Veröffentlichungstag und Bekanntmachung des Hinweises auf die Patenterteilung:
**04.05.2016 Patentblatt 2016/18**

(21) Anmeldenummer: **12815643.7**

(22) Anmeldetag: **21.12.2012**

(51) Int Cl.:
***C07H 15/04*** (2006.01) ***C07H 15/10*** (2006.01)
***A61K 31/7028*** (2006.01) ***A61P 31/04*** (2006.01)

(86) Internationale Anmeldenummer:
**PCT/EP2012/005360**

(87) Internationale Veröffentlichungsnummer:
**WO 2013/097942 (04.07.2013 Gazette 2013/27)**

(54) **SIALINSÄURE-DIMERE**

SIALIC ACID DIMERS

DIMÈRES D'ACIDE SIALIQUE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **30.12.2011 EP 11196220**

(43) Veröffentlichungstag der Anmeldung:
**05.11.2014 Patentblatt 2014/45**

(73) Patentinhaber:
- **Brossmer, Reinhard**
  **69120 Heidelberg (DE)**
- **Prescher, Horst**
  **4057 Basel (CH)**

(72) Erfinder:
- **Brossmer, Reinhard**
  **69120 Heidelberg (DE)**
- **Prescher, Horst**
  **4057 Basel (CH)**

(74) Vertreter: **Rippel, Hans Christoph**
**Isenbruck Bösl Hörschler LLP**
**Eastsite One**
**Seckenheimer Landstrasse 4**
**68163 Mannheim (DE)**

(56) Entgegenhaltungen:
**WO-A2-03/000709**

- **COLLINS B E ET AL: "High-affinity ligand probes of CD22 overcome the threshold set by cis ligands to allow for binding, endocytosis, and killing of B cells", JOURNAL OF IMMUNOLOGY, AMERICAN ASSOCIATION OF IMMUNOLOGISTS, US, Bd. 177, Nr. 5, 1. September 2006 (2006-09-01), Seiten 2994-3003, XP003013261, ISSN: 0022-1767 in der Anmeldung erwähnt**
- **HAJJAJ ABDU-ALLAH ET AL: "Design and Synthesis of a Multivalent Heterobifunctional CD22 Ligand as a Potential Immunomodulator", SYNTHESIS, Bd. 2011, Nr. 18, 1. September 2011 (2011-09-01), Seiten 2968-2974, XP055027695, ISSN: 0039-7881, DOI: 10.1055/s-0030-1260151**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 797 946 B1

Printed by Jouve, 75001 PARIS (FR)

## Beschreibung

Die Erfindung betrifft Abkömmlinge der Sialinsäure, Verfahren zu deren Herstellung, deren Verwendung, insbesondere als pharmazeutische Wirkstoffe, sowie pharmazeutische Wirkstoffzusammensetzungen, die solche Verbindungen enthalten.

"Sialinsäuren" ist der Oberbegriff für eine große Familie von unverzweigten Zuckern mit einem Grundgerüst aus neun Kohlenstoffatomen die sämtlich Derivate der Neuraminsäure (Neu) bzw. der Keto-desoxy-nonuloson-säure (KDN) darstellen.

Sialinsäuren spielen vielfältige Rollen in Säugetieren und im menschlichen Organismus (Schauer (2004) Zoology, 107, 49-64; Varki (2008) Trends in Mol Med, 14, 8, 351-360). Des Weiteren werden sie von vielen Pathogenen genutzt, um z.B. eine effiziente Infektion zu erreichen oder um dem Immunsystem des Wirtes zu entkommen (Glycoconjugate J. 2006, vol. 23, issue 1-2, alle Artikel). Viele solcher Funktionen werden über Sialinsäure erkennende Proteine reguliert (Lehmann et al. (2006) Cell. Mol. Life Sci. 63, 1331-1354). Durch Bindung von artifiziellen Liganden, insbesondere durch modifizierte Sialinsäuren, an diese Proteine kann ein therapeutisch vorteilhafter Effekt erzielt werden.

Eine Untergruppe solcher Proteine sind die Siglecs (Sialic acid recognizing Immunoglobulin like lectins). Sie sind Lektine vom Ig-Typ, die durch eine N-terminale V-Set Domäne gekennzeichnet sind, welche eine spezifische Erkennung von Sialinsäuren ermöglicht. Ein Überblick über die bis heute bekannt gewordenen Typen von Siglec-Proteinen und mit Siglec-Liganden potentiell behandelbaren Krankheiten findet sich in "Trends in Pharmacological Sciences" 2009, 30 (5), 240-248 und Referenzen darin. In dieser Untergruppe hat CD22 (Siglec-2) einen starken Einfluss auf die Entwicklung und Regulation der B-Zellen und deren Einfluss auf das Immunsystem. Liganden für Siglec-2 können insbesondere bei Krankheitsbildern im Zusammenhang mit B-Zellen eingesetzt werden (Tedder et al (2005) Advances in Immunology 88, 1-50).

Es ist beispielsweise aus der WO 03/000709 bekannt, dass bestimmte monomere Derivate der Sialinsäure als Liganden für so genannte Siglecs ("Sialinsäuren bindende Ig-artige Lektine")-Proteine wirken und eine potentielle Eignung als Arzneimittel aufweisen.

Weiter wurden bereits Antikörper und polymere Sialinsäuren als Liganden mit therapeutischer Eignung für Siglec's entwickelt (Courtney et al (2009) PNAS 106, 8, 2500-505; Collins et al (2006) Journal of Immunology 177, 2994-3003). Die beschriebenen Polymere führen aufgrund der erhöhten Anzahl von Liganden zu einer starken Steigerung der Affinität und besitzen gleichzeitig den Vorteil, an mehrere Bindungstaschen binden zu können. In diesen polymeren Liganden wurden Galactose enthaltende Trisaccharide benutzt, wodurch sie potentielle Liganden für den Asialoglycoprotein-Rezeptor und für Galektine sind (Steirer et al. (2009) J. Biol. Chem. 284, 6, 3777-3783). Die Polymeren haben weiter den Nachteil einer sehr hohen Molmasse, einer nicht definierbaren und nicht einheitlichen Größe und Zusammensetzung.

Obwohl die bekannten Siglec-Liganden bereits eine hohe Affinität zu bestimmten Siglec-Proteinen aufweisen, besteht doch ein breiter Raum für Verbesserungen, insbesondere was Affinität, Selektivität und Definierbarkeit des therapeutisch wirksamen Moleküls betrifft, aber auch im Hinblick auf pharmakologische Verträglichkeit und Verabreichungsformen sowie auf Stabilität in Plasma und Leber.

Aufgabe der Erfindung ist, Verbindungen bereitzustellen, mit denen zumindest in Teilbereichen Vorteile in den genannten Bereichen erzielt werden.

Es wurde gefunden, dass sich bestimmte dimere Sialinsäureabkömmlinge, mit einem in der 9-Position substituierten Stickstoff, in besonderer Weise als Siglec-Liganden, insbesondere für Siglec-2 (CD22) eignen. Durch die Kombination der Divalenz und der Substitution in der 9-Position wurde eine unerwartet hohe Affinität erzielt.

Gegenstand der Erfindung ist daher ein Sialinsäurederivat der Formel (I),

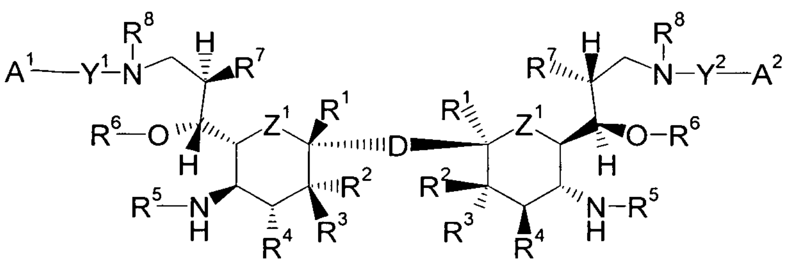

(I)

wobei die Symbole folgende Bedeutungen haben:

$A^1$, $A^2$ sind gleich oder verschieden eine Gruppe $D^1$-[$Y^3$-$D^2$-]$_m$-.

$D^1$, $D^2$ sind gleich oder verschieden ein mono- oder polycyclischer aromatischer, partiell ungesättigter oder gesättigter $C_3$-$C_{14}$-Kohlenwasserstoffrest oder ein aromatischer, partiell ungesättigter oder gesättigter drei- bis achtgliedriger heterocyclischer Rest, wobei die genannten Reste unsubstituiert oder ein- oder mehrfach durch eine Gruppe $X^1$ substituiert sind;

$X^1$ ist gleich oder verschieden Halogen, Cyano, Nitro, Hydroxy, Mercapto, Amino, Carboxyl, Hydroxylamino, Azido, $B(OH_2)$, SO, $SO_3M$, $OSO_3M$, $SO_2NH_2$, $SO_2CF_3$, $PO_3M$, $OPO_3M$, Cyanomethyl, Alkyl, Halogenalkyl, Alkenyl, Alkinyl, Alkyloxy, Halogenalkyloxy, Alkenyloxy, Alkinyloxy, Alkylthio, Alkylamino, Dialkylamino, Trialkylamino, Formyl, Alkylcarbonyl, Alkylsulfonyl, Alkylsulfoxyl, Alkylaminosulfoxyl, Dialkylaminosulfoxyl, Alkyloxycarbonyl, Alkylcarbonyloxy, Aminocarbonyl, Aminothiocarbonyl, Alkylaminocarbonyl, Alkylcarbonylamino, Dialkylaminocarbonyl, Alkylaminothiocarbonyl, Oxo (=O), Thioxo (=S), $C_1$-$C_8$-Alkylimino (=N-$C_1$-$C_8$-Alkyl) oder $C_1$-$C_8$-Alkyloximino (=N-O-$C_1$-$C_8$-Alkyl), wobei die Alkylgruppen in diesen Resten 1 bis 8 Kohlenstoffatome enthalten und die genannten Alkenyl- oder Alkinylgruppen in diesen Resten 2 bis 8 Kohlenstoffatome enthalten;

$Y^1$, $Y^2$ sind gleich oder verschieden ~($C_1$-$C_4$-Alkyl)-, ~($C_1$-$C_4$-Halogenalkyl)-, ~($C_1$-$C_4$-Alkyl)-(CO)-, ~C(O)-, ~CH=CH-C(O)-, ~C=C-C(O)-, ~$S(O)_2$-, ~$CH_2$-$S(O)_2$-, ~$NR^x$-C(O)-, ~$CH(CF_3)$-, ~$CH_2$-$NR^x$-3-Cyclobuten-1,2-dion-4-, ~$NR^x$-3-Cyclobuten-1,2-dion-4-, ~$NR^x$-3-2,5-Thiadiazol-1-oxid-4-, ~$NR^x$-3-2,5-Thiadiazol-1,1-dioxid-4-, wobei ~ die Bindung zur Gruppe A bezeichnet oder

$Y^1$, $Y^2$ bilden, unabhängig voneinander, zusammen mit dem Stickstoff, an den sie gebunden sind, und der Gruppe $R^8$ eine Gruppe ~4-1H-(1,2,3)Triazol-1-yl-, ~5-1H-(1,2,3)Triazol-1-yl-, ~$CH_2$-4-1H-(1,2,3)Triazol-1-yl- oder ~$CH_2$-5-1H-(1,2,3)Triazol-1-yl-, wobei ~ die Bindung zur Gruppe A bezeichnet;

$Y^3$ ist eine Bindung, O, S, S(O), $S(O)_2$, C(O), $CH_2$, $CHR^x_2$ oder $NR^x$;

m ist 0, 1 oder 2;

$Z^1$ ist O, S, $CH_2$ oder $NR^x$;

D ist eine Gruppe $Z^2$-$T^1$-$Y^4$ -$A^3$-$Y^5$-$T^2$-$Z^2$;

$Z^2$ ist gleich oder verschieden -O~, -S~, -$NR^x$~, -NH-C(O)~, -$CH_2$~, -$CF_2$~, -CH(OH)~, -$N(R^x)$-O-, -O-$NR^x$~, -O-N=CH~, ~4-1H-(1,2,3)Triazol-1-yl- oder ~5-1H-(1,2,3)Triazol-1-yl- wobei ~ die Bindung zur Gruppe T bezeichnet;

$T^1$, $T^2$ sind gleich oder verschieden eine geradkettige oder verzweigte Alkandiylgruppe mit 1 bis 30 C-Atomen, wobei

(i) gegebenenfalls eine oder mehrere nicht terminale $CH_2$-Gruppen durch -O-, -S-, -S(O)-, -$S(O)_2$-, -$S^+(CH_3)$-, -$P(O_2)$- und/oder -$NR^x$-ersetzt sind und/oder

(ii) gegebenenfalls ein oder mehrere H-Atome durch F, Cl, $OR^x$,-$OSO_3M$, (=O), (=S), Carboxyl, $NH_2$, $NHR^z$ und/oder $NHR^y$, ersetzt sind und/oder

(iii) gegebenenfalls eine oder mehrere nicht terminale -$CH_2CH_2$-Gruppen durch -5-1H-(1,2,3)Triazol-1-yl-, -$CR^x$=$CR^x$- und/oder - C≡C- ersetzt sind und/oder

(iv) gegebenfalls eine nicht terminale -$CH_2CH_2CH_2$-Gruppe durch -4-1H-(1,2,3)Triazol-1-yl- und/oder -O-N=CH- ersetzt ist und/oder

(v) gegebenfalls eine nicht terminale -$CH_2CH_2CH_2CH_2$-Gruppe durch Phenyl-1,4-diyl ersetzt ist;

$Y^4$, $Y^5$ sind gleich oder verschieden eine Bindung O, S, $NR^x$, S(O), $S(O)_2$, C(O), ~C(O)-$NR^x$-, ~$NR^x$-C(O)-, ~C(O)-O-, ~O-C(O)-, ~$NR^x$-CO-$NR^x$-, ~$NR^x$-$S(O)_2$-, ~$S(O)_2$-$NR^x$-, ~$CH_2$-$NR^x$-C(O)-, ~$CH_2$-C(O)-$NR^x$-, ~$CH_2$-$NR^x$-, ~$CH(CF_3)$-$NR^x$-, ~CH=N-O- oder ~O-N=CH- wobei ~ die Bindung zu Gruppe A bezeichnet;

$A^3$ ist

a) $C_1$-$C_8$-Alkandlyl, $C_2$-$C_8$-Alkendiyl, $C_2$-$C_8$-Alkindiyl, $C_4$-$C_8$-Alkadiendiyl, wobei in den genannten Gruppen

gegebenenfalls mehrere $CH_2$-Gruppen durch O, S, S(O), $S(O)_2$, $NR^x$ und/oder C(O) ersetzt sind und wobei in den genannten Gruppen gegebenenfalls ein oder mehrere H-Atome durch eine Gruppe $X^2$ ersetzt sind,

b) eine Gruppe $A^4$-$[Z^3$-$A^5]_n$,

c) 1,1'-Ferrocendiyl, 1,1'-Cobaltocendiyl, 1,1-Ruthenocen oder Dichlorplatindiaminodiyl;

$A^4$, $A^5$ sind gleich oder verschieden ein gesättigter, partiell ungesättigter oder aromatischer, mono- oder polycyclischer Kohlenwasserstoffrest mit 3 bis 14 C-Atomen oder ein drei- bis achtgliedriger, aromatischer, partiell ungesättigter oder gesättigter mono oder polycyclischer heterocyclischer Rest, wobei die genannten Gruppen jeweils gegebenenfalls durch eine oder mehrere Gruppen $X^2$ substituiert sind;

$Z^3$ ist eine Bindung, O, S, S(O), $S(O)_2$, $NR^x$ C(O) oder $CR^x_2$;

$X^2$ ist gleich oder verschieden Fluor, Chlor, Brom, Nitro, Hydroxylamino, $B(OH_2)$, $SO_3M$, $OSO_3M$, $SO_2NH_2$, $PO_3M$, $OPO_3M$, Alkyl, Halogenalkyl, Alkyloxy, Halogenalkyloxy, Alkylthio, Alkylamino, Dialkylamino,

Trialkylamino, Alkylcarbonyl, Alkylsulfonyl, Alkylsulfoxyl, Alkylaminosulfoxyl, Dialkylaminosulfoxyl, Alkyloxycarbonyl, Alkylcarbonyloxy, Aminocarbonyl, Alkylaminocarbonyl, Alkylcarbonylamino, Dialkylaminocarbonyl, Alkylaminothiocarbonyl, $C_1$-$C_4$-Alkylimino (=N-$C_1$-$C_4$-Alkyl), $C_1$-$C_4$-Alkyloximino (=N-O-$C_1$-$C_4$-Alkyl), wobei die Alkylgruppen in diesen Resten 1 bis 4 Kohlenstoffatome enthalten und die Gruppen Halogenalkyl und Halogenalkyloxy die Halogene F und/oder C1 enthalten;

n ist 0 oder 1;

$R^1$ ist gleich C(O)OM, O-$PO_3M_2$, O-$SO_3M$, C(O)-NH-$S(O)_2$-$R^x$, $SO_3M$, $PO_3M_2$ oder C(O)NOM;

$R^2$, $R^3$ sind gleich oder verschieden H oder F;

$R^4$, $R^7$ sind gleich oder verschieden H, OH, $OR^z$, $OC(O)NHR^y$ oder $NR^x$;

$R^6$ ist gleich oder verschieden H oder $R^z$;

$R^5$ ist gleich oder verschieden H, $R^x$, $R^x_2$, C(O)H, $C(O)CH_2OH$ oder C(O) Halogenalkyl;

$R^8$ ist gleich oder verschieden $R^x$;

M ist H, ein $C_1$-$C_6$-Alkyl oder ein Kation;

$R^x$ ist gleich oder verschieden H, $R^y$ oder $R^z$;

$R^y$ ist gleich oder verschieden $C_1$-$C_6$-Alkyl, Phenyl oder Benzyl und

$R^z$ ist gleich oder verschieden -C(O)-$C_1$-$C_6$-Alkyl, -C(O)-Phenyl oder - C(O)-$C_1$-$C_4$-Alkyl-Phenyl,

sowie deren pharmakologisch verträgliche Salze.

Ebenfalls Gegenstand der Erfindung ist eine pharmazeutische Zubereitung, enthaltend mindestens ein Sialinsäurederivat der Formel (I) oder ein pharmakologisch verträgliches Salz davon und einen pharmakologisch verträglichen Träger.

Weiterhin Gegenstand der Erfindung ist zudem ein Sialinsäurederivat der Formel (I) oder ein pharmakologisch verträgliches Salz davon als Arzneimittel.

Gegenstand der Erfindung ist auch ein Sialinsäurederivat der Formel (I) oder ein pharmakologisch verträgliches Salz davon zur Behandlung oder Prävention von Allergien, Autoimmunerkrankungen, chronischen Entzündungen, Querschnittslähmung, multipler Sklerose, Krebs, Viruserkrankungen, beispielsweise AIDS, bakteriellen Erkrankungen, bespielsweise Streptokokken, parasitären Erkrankungen, beispielsweise Chagas Krankheit, Krankheiten bei denen die Immunantwort im Rahmen der B-Zellenaktivierung gestört ist, wie Common Variable Immunodeficiency (CVID) und 1gA-Defizienz, bei Krankheiten der blutbildenden Organe und des Blutes sowie bei Krebs, beispielsweise Lymphome und Myelome, sowie zur Regulation des Immunsystems, beispielsweise bei Impfungen.

Ein weiterer Gegenstand der Erfindung ist ein Sialinsäurederivat der Formel (I) oder ein pharmakologisch

verträgliches Salz davon zur Verwendung bei der Herstellung eines Arzneimittels zur Regulation des Immunsystems, beispielsweise bei Impfungen sowie zur Behandlung von Allergien, Autoimmunerkrankungen, chronischen Entzündungen, Querschnittslähmung, multipler Sklerose, Krebs, Viruserkrankungen, beispielsweise AIDS, bakteriellen Erkrankungen, bespielsweise Streptokokken, parasitären Erkrankungen, beispielsweise Chagas Krankheit, Krankheiten, bei denen die Immunantwort im Rahmen der B-Zellenaktivierung gestört ist, wie Common Variable Immunodeficiency (CVID) und 1gA-Defizienz, bei Krankheiten der blutbildenden Organe und des Blutes sowie bei Krebs, beispielsweise Lymphome und Myelome.

Ebenfalls Gegenstand der Erfindung ist ein Verfahren zur Regulation des Immunsystems, beispielsweise bei Impfungen sowie zur Behandlung von Krankheiten, deren Verlauf oder Aktivität durch die Siglec-Liganden beeinflusst werden kann, insbesondere aus der Gruppe der Allergien, Autoimmunerkrankungen, chronischen Entzündungen, Querschnittslähmung, multipler Sklerose, Krebs, Viruserkrankungen, beispielsweise AIDS, Bakterielle Erkrankungen, bespielsweise Streptokokken, Parasitäre Erkrankungen, beispielsweise Chagas Krankheit, Krankheiten, bei denen die Imnunantwort im Rahmen der B-Zellenaktivierung gestört ist, wie Common Variable Immunodeficiency (CVID) und 1gA-Defizienz, bei Krankheiten der blutbildenden Organe und des Blutes sowie bei Krebs, beispielsweise Lymphome und Myelome, wobei man einer von der Krankheit betroffenen Person eine vorzugsweise therapeutisch wirksame Menge eines Sialinsäurederivats der Formel (I) oder eines pharmakologisch verträglichen Salzes davon verabreicht.

Die Sialinsäurederivate der Formel (I) können aufgrund der Divalenz an zwei Bindungstaschen bzw. zwei Proteine gleichzeitig binden. Sie weisen im Affinitätstest ein deutlich verändertes Verhalten zu Monomeren auf. Die Substitution in der 9-Position erhöht die Affinität in unerwartet starkem Maße. Sie weisen eine hohe Aktivität als Siglecliganden auf und haben, im Gegensatz zu Polymeren, jeweils eine eindeutige und definierbare Struktur. Sie enthalten zudem außer Sialinsäure keine weiteren Kohlenhydrate. Die Herstellung der Verbindungen erfolgt ohne Einsatz von Zellkulturen oder Enzymen, wodurch eine Herstellung in großem Maßstab möglich ist.

Der Begriff "Sialinsäurederivat der Formel (I)" umfasst alle stereoisomeren Formen der Verbindung der Formel (I), insbesondere E/Z oder Cis/trans-Isomere bei substituierten Doppelbindungen oder Ringen und sowie Stereoisomere, die sich durch die Chiralitätszentren der Verbindungen der Formel (I) ergeben, insbesondere Enantiomere und Diastereoisomere in reiner Form oder in Form von Gemischen jeglicher Zusammensetzung, wobei die einzelnen Chiralitätszentren jeweils in der (S)- oder (R)-Form vorliegen.

Die Herstellung der einzelnen Stereoisomeren kann beispielsweise durch Aufbesserung der Isomerengemische nach üblichen Methoden, wie Chromatographie oder Kristallisation, oder durch Verwendung von isomeren reinen Ausgangsstoffen erfolgen. Die Aufbesserung der Isomere kann auf Stufe der Edukte, Zwischenprodukte oder Endprodukte der Formel (I) erfolgen. Zu den erfindungsgemäß umfassten Isomeren zählen auch alle tautomeren Formen von Verbindungen (I) und alle mesomorphen Formen.

Weiterhin umfasst der Begriff "Sialinsäurederivate der Formel (I)" pharmakologisch verträgliche Salze der Verbindungen (I), darunter auch interne (Zwitterionen).

Im Allgemeinen kommen die Salze derjenigen Kationen oder die Säureadditionssalze derjenigen Säuren in Betracht, deren Kationen, beziehungsweise Anionen, die pharmakologische Wirkung der Verbindungen (I) nicht negativ beeinträchtigen.

Es kommen als Kationen insbesondere Ionen der Alkalimetalle, vorzugsweise Lithium, Natrium und Kalium, der Erdalkalimetalle, vorzugsweise Calcium und Magnesium, und der Übergangsmetalle, vorzugsweise Mangan, Kupfer, Zink und Eisen, sowie Ammonium, wobei hier gewünschtenfalls ein bis vier Wasserstoffatome durch $R^Y$ ersetzt sein können, vorzugsweise Ammonium, Dimethylammonium, Diisopropylammonium, Tetramethylammonium, Triethylammonium, Tetrabutylammonium, 2-(2-Hydroxyeth-1-oxy)eth-1-ylammonium, Di-(2-hydroxyeth-1-yl)-ammonium, Trimethylbenzylammonium, des weiteren Phosphoniumionen, Sulfoniumionen, vorzugsweise Tri($C_1$-$C_4$-alkyl)sulfonium und Sulfoxoniumionen, vorzugsweise Tri($C_1$-$C_4$-alkyl)-sulfoxonium, in Betracht. Bevorzugt sind Na, Li, K, Ca, Mg und Ammonium (gegebenenfalls substituiert), besonders bevorzugt sind Na, Li und K, insbesondere bevorzugt ist Na.

Anionen von pharmakologisch verträglichen Säureadditionsalzen sind beispielsweise Chlorid, Bromid, Fluorid, Hydrogensulfat, Sulfat, Dihydrogenphosphat, Hydrogenphosphat, Nitrat, Hydrogencarbonat, Carbonat, Hexafluorosilikat, Hexafluorophosphat, Benzoat, die Anionen von $C_1$-$C_4$-Alkansäuren, vorzugsweise Formiat, Acetat, Propionat und Butyrat, und anderen organischen Säuren, wie Pivalinsäure, Maleinsäure, Bernsteinsäure, Pimelinsäue, Fumarsäure, Apfelsäure, Sulfaminsäure, Phenylpropionsäure, Gluconsäure, Ascorbinsäure, Nicotinsäure, Zitronensäure und Adipinsäure.

Weiterhin umfasst der Begriff "Sialinsäurederivat der Formel (I)" Solvate, beispielsweise Hydrate oder Addukte mit Alkoholen, sowie alle Kristallmodifikationen.

Soweit nicht anders angegeben können Symbole, die mehrfach verwendet werden, unabhängig voneinander die gleiche oder verschiedene Bedeutungen haben.

Bei den in der Formel (I) angegebenen Definitionen der Symbole bedeuten:

Halogen: Fluor, Chlor, Brom und Iod;

Alkyl: gesättigte, geradkettige, verzweigte oder cyclische Kohlenwasserstoffreste mit beispielsweise 1 bis 8 Kohlenstoffatomen, wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Dimethylpropyl, 1,1-Dimethylpropyl, 1-Ethylpropyl, Hexyl, 1,1-Dimethylbutyl, 2,2-Dimethylbutyl, 3,3-Dimethylbutyl, 1,2-Dimethylbutyl, 1,3-Dimethylbutyl, 2,3-Dimethylbutyl, 1-Methylpentyl, 2-Methylpentyl, 3-Methylpentyl, 4-Methylpentyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, Heptyl, Octyl, Cyclopropyl, Cyclobutyl, 1-Methylcyclopropyl, 2-Methylcyclopropyl, Cyclopentyl, 2,2-Dimethylcylopropyl, 2,3- Dimethylcylopropyl, Cyclohexyl und Cyclooctyl;

Halogenalkyl: geradkettige, verzweigte oder cyclische Alkylgruppen mit beispielsweise 1 bis 6 Kohlenstoffatomen (wie vorstehend genannt), wobei in diesen Gruppen Wasserstoffatome teilweise oder vollständig durch Halogenatome ersetzt sind: wie Chlormethyl, Brommethyl, Dichlormethyl, Trichlormethyl, Fluormethyl, Difluormethyl, Trifluormethyl, Chlorfluonnethyl, Dichlorfluormethyl, Chlordifluormethyl, 1-Chlorethyl, 1-Bromethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Pentafluorethyl, 1-Fluor-1-methyl-ethyl, 1-Fluorcyclopropyl, Heptafluorpropyl oder Nonafluorbutyl;

Alkenyl: ungesättigte, geradkettige oder verzweigte Kohlenwasserstoffreste mit beispielsweise 2 bis 8 Kohlenstoffatomen und einer oder zwei Doppelbindungen in beliebiger Position (einschließlich aller E und Z Stereoisomeren), z.B. $C_2$-$C_6$-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1 propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl, 1-Ethyl-2-methyl-2-propenyl oder But-1,3-enyl;

Alkinyl: geradkettige oder verzweigte Kohlenwasserstoffgruppen mit 2 bis 8 Kohlenstoffatomen und einer oder zwei Dreifachbindungen in beliebiger Position, z.B. $C_2$-$C_6$-Alkinyl wie Ethinyl, 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-1-pentinyl, 3-Methyl-4-pentinyl, 4-Methyl-1-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl und 1-Ethyl-1-methyl-2-propinyl;

Alkyloxy: Alkyloxygruppen mit gesättigtem, geradkettigem, verzweigtem oder cyclischen Alkylrest, wobei dieser aus der vorstehend genannten Gruppe der Alkyle ist und 1 bis 6 Kohlenstoffatome enthält;

Halogenalkyloxy: Halogenalkyloxygruppen mit geradkettigem, verzweigtem oder cyclischem Halogenalkylrest, wobei dieser aus der vorstehend genannten Gruppe der Halogenalkyle ist und 1 bis 6 Kohlenstoffatome enthält;

Alkenyloxy: Alkenyloxygruppen mit ungesättigtem, geradkettigem oder verzweigtem Alkenylrest, wobei dieser aus der vorstehend genannten Gruppe der Alkenyle ist und 1 bis 6 Kohlenstoffatome enthält;

Alkinyloxy: Alkinyloxygruppen mit geradkettigem oder verzweigtem Alkinylrest, wobei dieser aus der vorstehend genannten Gruppe der Alkinyle ist und 1 bis 6 Kohlenstoffatome enthält;

Alkylthio: Alkylthiogruppen mit gesättigtem, geradkettigem, verzweigtem oder cyclischem Alkylrest, wobei dieser aus der vorstehend genannten Gruppe der Alkyle ist und 1 bis 4 Kohlenstoffatome enthält;

Alkylamino: Alkylaminogruppen mit gesättigtem, geradkettigem, verzweigtem oder cyclischem Alkylrest, wobei dieser aus der vorstehend genannten Gruppe der Alkyle ist und 1 bis 4 Kohlenstoffatome enthält;

Dialkylamino: Dialkylaminogruppen mit gesättigten, geradkettigen, verzweigten oder cyclischen Alkylresten, wobei diese gleich oder verschieden aus der vorstehend genannten Gruppe der Alkyle sind und 1 bis 4 Kohlenstoffatome enthalten;

Trialkylamino: Trialkylaminogruppen mit gesättigtem, geradkettigem, verzweigtem oder cyclischem Alkylresten, wobei diese gleich oder verschieden aus der vorstehend genannten Gruppe der Alkyle sind und 1 bis 4 Kohlenstoffatome enthalten;

Alkylcarbonyl: Alkylcarbonylgruppen mit gesättigtem, geradkettigem, verzweigtem oder cyclischem Alkylrest, wobei

dieser aus der vorstehend genannten Gruppe der Alkyle ist und 1 bis 5 Kohlenstoffatome enthält;
Alkylsulfonyl: Alkylsulfonylgruppen mit gesättigtem, geradkettigem, verzweigtem oder cyclischem Alkylrest, wobei dieser aus der vorstehend genannten Gruppe der Alkyle ist und 1 bis 4 Kohlenstoffatome enthält;
Alkylsulfoxyl: Alkylsulfoxylgruppen mit gesättigtem, geradkettigem, verzweigtem oder cyclischem Alkylrest, wobei dieser aus der vorstehend genannten Gruppe der Alkyle ist und 1 bis 6 Kohlenstoffatome enthält;
Alkylaminosulfoxyl: Alkylaminosulfoxylgruppen mit gesättigtem, geradkettigem, verzweigtem oder cyclischem Alkylrest, wobei dieser aus der vorstehend genannten Gruppe der Alkyle ist und 1 bis 6 Kohlenstoffatome enthält;
Dialkylaminosulfoxyl: Aialkylaminosulfoxylgruppen mit gesättigtem, geradkettigem, verzweigtem oder cyclischem Alkylrest, wobei dieser aus der vorstehend genannten Gruppe der Alkyle ist und 1 bis 6 Kohlenstoffatome enthält;
Alkyloxycarbonyl: Alkyloxycarbonylgruppen mit gesättigtem, geradkettigem, verzweigtem oder cyclischem Alkylrest, wobei dieser aus der vorstehend genannten Gruppe der Alkyle ist und 1 bis 8 Kohlenstoffatome enthält;
Alkylcarbonyloxy: Alkylcarbonyloxygruppen mit gesättigtem, geradkettigem, verzweigtem oder cyclischem Alkylrest, wobei dieser aus der vorstehend genannten Gruppe der Alkyle ist und 1 bis 8 Kohlenstoffatome enthält;
Alkylaminocarbonyl: Alkylaminocarbonylgruppen mit gesättigtem, geradkettigem, verzweigtem oder cyclischem Alkylrest, wobei dieser aus der vorstehend genannten Gruppe der Alkyle ist und 1 bis 8 Kohlenstoffatome enthält;
Dialkylaminocarbonyl: Dialkylaminocarbonylgruppen mit gesättigtem, geradkettigem, verzweigtem oder cyclischem Alkylrest, wobei dieser aus der vorstehend genannten Gruppe der Alkyle ist und 1 bis 8 Kohlenstoffatome enthält;
Alkylaminothiocarbonyl: Alkylaminothiocarbonylgruppen mit gesättigtem, geradkettigem, verzweigtem oder cyclischem Alkylrest, wobei dieser aus der vorstehend genannten Gruppe der Alkyle ist und 1 bis 8 Kohlenstoffatome enthält;
Alkylimino: Alkyliminogruppen mit gesättigtem, geradkettigem, verzweigtem oder cyclischem Alkylrest, wobei dieser aus der vorstehend genannten Gruppe der Alkyle ist und 1 bis 8 Kohlenstoffatome enthält;
Alkyloximino: Alkyloximinogruppen mit gesättigtem, geradkettigem, verzweigtem oder cyclischem Alkylrest, wobei dieser aus der vorstehend genannten Gruppe der Alkyle ist und 1 bis 8 Kohlenstoffatome enthält;
Alkylcarbonylamino: Alkylcarbonylaminogruppen mit gesättigtem, geradkettigem, verzweigtem oder cyclischem Alkylrest, wobei dieser aus der vorstehend genannten Gruppe der Alkyle ist und 1 bis 8 Kohlenstoffatome enthält;
Alkandiyl bedeutet für Rest $A^3$: gesättigte, geradkettige oder verzweigte Alkandiylgruppe mit beispielsweise 1 bis 8 Kohlenstoffatomen, wie Methan-1,1-diyl, Ethan-1,2-diyl, Propan-1,3-diyl, Butan-1,4-diyl, Pentan-1,5-diyl, Hexan-1,6-diyl, Heptan-1,7-diyl oder Octan-1,8-diyl;
Alkandiyl bedeutet für Rest $T^1$ und $T^2$: gesättigte, geradkettige oder verzweigte Alkandiylgruppe mit beispielsweise 1 bis 21 Kohlenstoffatomen, wie Methan-1,1-diyl, Ethan-1,2-diyl, Propan-1,3-diyl, Butan-1,4-diyl, Pentan-1,5-diyl, Hexan-1,6-diyl, Heptan-1,7-diyl, Octan-1,8-diyl, Nonan-1,9-diyl, Decan-1,10-diyl, Undecan-1,11-diyl, Dodecan-1,12-diyl, Tridecan-1,13-diyl, Tetradecan-1,14-diyl, Pentadecan-1,15-diyl, Hexadecan-1,16-diyl, Heptadecan-1,17-diyl, Octadecan-1,18-diyl, Nonadecan-1,19-diyl, Eicosan-1,20-diyl oder Heneicosan-1,21-diyl;
Alkendiyl (z.B. für Rest $A^3$): ungesättigte, geradkettige oder verzweigte Alkendiylgruppen (einschließlich aller E und Z Stereoisomeren) mit beispielsweise 2 bis 8 Kohlenstoffatomen, wie Ethen-1,1-diyl, Prop-2-en-1,3-diyl. But-2-en-1,4-diyl, 3,4-Dimethylbut-2-en-1,4-diyl, Pent-2-en-1,5-diyl, Hex-3-en-1,6-diyl und Oct-4-en-1,8-diyl
Alkindiyl (z.B. für Rest $A^3$): ungesättigte, geradkettige oder verzweigte Alkindiylgruppen mit beispielsweise 2 bis 8 Kohlenstoffatomen, wie Ethin-1,1-diyl, Prop-2-in-1,3-diyl, But-2-in-1,4-diyl, Pent-2-in-1,5-diyl, Hex-3-in-1,6-diyl, Oct-4-in-1,8-diyl;
Alkadiendiyl (z.B. für Rest $A^3$): ungesättigte, geradkettige oder verzweigte Alkadiendiylgruppe (einschließlich aller E und Z Stereoisomeren) mit beispielsweise 4 bis 8 Kohlenstoffatomen wie But-1,3-dien-1,4-diyl, 3,4-Dimethylbut-1,3-dien-1,4-diyl und Hex-2,4-dien-1,6-diyl.

Mono- oder polycyclischer, aromatischer, partiell ungesättiger oder gesättiger $C_3$-$C_{14}$-Kohlenwasserstoffrest bedeutet für $A^4$ und $A^5$ beispielsweise:

a) gleich oder verschieden $C_6$-$C_{14}$-Aryldiyl, insbesondere Phenylen-1,4-diyl, Phenylen-1,3-diyl, Phenylen-1,2-diyl, Naphthalin-1,2-diyl, Naphthalin-1,3-diyl, Naphthalin-1,4-diyl, Naphthalin-1,5-diyl, Naphthalin-1,6-diyl, Naphthalin-1,7-diyl, Naphthalin-1,8-diyl, Naphthalin-2,3-diyl, Naphthalin-2,6-diyl, Naphthalin-2,7-diyl, Biphenylen-1,2-diyl, Biphenylen-1,3-diyl, Biphenylen-1,4-dlyl, Biphenylen-1,5-diyl, Biphenylen-1,6-diyl, Biphenylen-1,7-diyl, Biphenylen-1,8-diyl, Biphenylen-2,3-diyl, Biphenylen-2,6-diyl, Biphenylen-2,7-diyl, Anthracen-1,2-diyl, Anthracen-1,3-diyl, Anthracen-1,4-diyl, Anthracen-1,5-diyl, Anthracen-1,6-diyl, Anthracen-1,7-diyl, Anthracen-1,8-diyl, Anthracen-1,9-diyl, Anthracen-1,10-diyl, Anthracen-2,3-diyl, Anthracen-2,6-diyl, Anthracen-2,7-diyl, Anthracen-2,9-diyl, Anthracen-2,10-diyl, Anthracen-9,10-diyl, Inden-4,7-diyl, s-Indacen-4,8-diyl, Fluoren-1,4-diyl, Phenanthren-1,4-diyl, Indendiyl, s-Indacendiyl, Fluorendiyl oder Phenanthrendiyl, 1,2,3,4-tetrahydronapht-2,6-diyl, trans-1,2,3,4-tetrahydronapht-1,4-diyl, 1,2,3,4-tetrahydronapht-5,8-diyl und 2,3-dihydroinden-,4,7-diyl;

b) $C_3$-$C_{14}$-Cycloalkendiyl oder $C_5$-$C_{14}$-Cycloalkadiendiyl, insbesondere Cycloprop-1-en-1,2-diyl, Cyclobut-1-en-1,2-diyl, Cyclopent-1-en-1,3-diyl, Cyclohex-1-en-1,4-diyl, Cyclopenta-1,3,-dien-1,3-diyl, Cyclohexa-1,3-dien-1,3-diyl und Cyclohexa-1,3-dien-1,4-diyl;

c) $C_3$-$C_8$-Cycloalkyldiyl oder beispielsweise Cyclopropan-1,2-diyl, insbesondere trans-Cyclobutan-1,2-diyl, trans-Cyclobutan-1-3-diyi, cis-Cyclopentan-1,3-diyl, cis-Cyclopentan-1,3-diyl, cis-Cyclohexan-1,4-diyl, trans-Cyclohexan-1,4-diyl, cis-Cyclohexan-1,3-diyl, trans-Cyclohexan-1,3-diyl, trans-Cycloheptan-1,3-diyl und trans-Cycloheptan-1,4-diyl.

Drei- bis achtgliedriger gesättigter, partiell ungesättigter oder aromatischer heterocyclischer Rest bedeutet für $A^4$. $A^5$ beispielsweise:

a) nicht-aromatisches, gesättigtes oder teilweise ungesättigtes 5- oder 6-gliedriges Heterocyclodiyl, enthaltend ein bis drei Stickstoffatome und/oder ein Sauerstoff-oder Schwefelatom oder ein oder zwei Sauerstoff- und/oder Schwefelatome, insbesondere trans-Tetrahydrofuran-2,5-diyl, trans-Tetrahydrofuran-2,4-diyl, cis-Tetrahydrofuran-2,5-diyl, trans-Tetrahydrothien-2,5-diyl, trans-Pyrrolidin-2,5-diyl, Isoxazolidin-2,4-diyl, Isoxazolidin-2,5-diyl, Isothiazolidin-2,4-diyl, Isothiazolidin-2,5-diyl, Pyrazolidin-1,3,diyl, trans-Oxazolidin-2,4-diyl, trans-Thiazolidin-2,5-diyl, Imidazolidin-1,3-diyl, trans-Imidazolidin-2,4-diyl, Pyrrolin-1,3-diyl, trans-Pyrrolin-2,4-diyl, trans-Pyrrolin-2,5-diyl, Piperidin-1,4-diyl, trans-Piperidin-2,5-diyl, Di-oxan-2,5-diyl, trans-Tetrahydropyran-2,5-diyl, trans-Hexahydropyridazin-3,6-diyl, Hexahydropyridazin-1,4-diyl, Trans-Hexahydropyrimidin-2,5-diyl, Hexahydropyrimidin-1,3-diyl, Hexahydropyrimidin-1,4-diyl, Piperazin-1,4-diyl, trans-Piperazin-2,5-diyl und Piperazin-1,3-diyl;

b) 5-gliedriges Heteroaryldiyl, enthaltend ein bis vier Stickstoffatome oder ein bis drei Stickstoffatome und/oder ein Schwefel- oder Sauerstoffatom, insbesondere Furan-2,4-diyl, Furan-2,5-diyl, Thiophen-2,4-diyl, Thiophen-2,5-diyl, Pyrrol-2,4-diyl, Pyrrol-2,5-diyl, Pyrazol-1,3-diyl, Oxazol-2,4-diyl, Oxazol-2,5-diyl, 1,3,4-Oxadiazol-2,5-diyl, 1,2,4-Oxadiazol-3,5-diyl, 1,2,4-Thiadiazol-3,5-diyl, 1,3,4-Thiadiazol-2,5-diyl, Thiazol-2,4-diyl, Thiazol-2,5-diyl, Imidazol-2,4-diyl, 2H-Tetrazol-2,5-diyl, 1H(1,2,4)Triazol-2,5-diyl, 1H-(1,2,3)Triazol-1,4-diyl, und 1H-(1,2,3)Triazol-1,5-diyl;

c) 6-gliedriges Heteroaryldiyl, enthaltend ein bis drei bzw. ein bis vier Stickstoffatome, insbesondere Pyridin-2,5-diyl, Pyridin-2,4-diyl, Pyridazin-2,5-diyl, Pyrimidin-2,5-diyl, Pyrimidin-2,4-diyl, Pyrazin-2,5-diyl und Tetrazin-2,5-diyl.

Polycyclischer aromatischer, partiell ungesättigter oder gesättigter heterocyclischer Rest bedeutet für $A^4$, $A^5$ beispielsweise:

l-Benzofuran-4,7-diyl, 1-Benzofuran-2,7-diyl, 2-Benzofuran-4,7-diyl, 2-Benzofuran-3,6-diyl, Chromen-5,8-diyl, Chromen-3,7-diyl, Xanthen-1,4-diyl, Xanthen-2,6-diyl, Indazol-4,7-diyl, Purin-2,8-diyl, 4H-Chinolizin-6,9-diyl, 3-Isochinolin-1,4-diyl, Phthalazin-1,4-diyl, 1,8-Naphthyridin-2,6-diyl, Chinoxalin-2,6-diyl, Chinazolin-5,8-diyl, Cinnolin-5,8-diyl, Pteridin-2,6-diyl, Indolizin-2,6-diyl, Indolizin-5,8-diyl, Indol-4,7-diyl, Indol-2,5-diyl, Indol-3,6-diyl, Isoindol-4,7-diyl, Isoindol-2,5-diyl, Carbozol-1,4-diyl, Acridin-1,4-diyl, Phenoxazin-1,4-diyl, Benzoxazol-4,7-diyl, Benzothiazol-4,7-diyl, Benzoimidazol-4,7-diyl, 1H-Benzotriazol-4,7-diyl und Benzothiophen-1,4-diyl.

Mono- oder polycyclischer, aromatischer, partiell ungesättiger oder gesättiger $C_3$-$C_{14}$-Kohlenwasserstoffrest bedeutet für $D^2$ beispielsweise:

a) gleich oder verschieden $C_6$-$C_{14}$-Aryldlyl, insbesondere Phenylen-1,4-diyl, Phenylen-1,3-diyl, Phenylen-1,2-diyl, Naphthalin-1,2-diyl, Naphthalin-1,3-diyl, Naphthalin-1,4-diyl, Naphthalin-1,5-diyl, Naphthalin-1,6-diyl, Naphthalin-1,7-diyl, Naphthalin-1,8-diyl, Naphthalin-2,3-diyl, Naphthalin-2,6-diyl, Naphthalin-2,7-diyl, Biphenylen-1,2-diyl, Biphenylen-1,3-diyl, Biphenylen-1,4-diyl, Biphenylen-1,5-diyl, Biphenylen-1,6-diyl, Biphenylen-1,7-dlyl, Biphenylen-1,8-diyl, Biphenylen-2,3-diyl, Biphenylen-2,6-diyl, Biphenylen-2,7-diyl, Anthracen-1,2-diyl, Anthracen-1,3-diyl, Anthracen-1,4-diyl, Anthracen-1,5-diyl, Anthracen-1,6-diyl, Anthracen-1,7-diyl, Anthracen-1,8-diyl, Anthracen-1,9-diyl, Anthracen-1,10-diyl, Anthracen-2,3-diyl, Anthracen-2,6-diyl, Anthracen-2,7-diyl, Anthracen-2,9-diyl, Anthracen-2,10-diyl, Anthracen-9,10-diyl, Inden-4,7-diyl, s-Indacen-4,8-diyl, Fluoren-1,4-diyl, Phenanthren-1,4-diyl und Inden-4,7-diyl;

b) $C_3$-$C_{14}$-Cycloalkenyldiyl oder $C_5$-$C_{14}$-Cycloalkadiendiyl, insbesondere Cyclopropen-1,2-diyl, Cyclobuten-1,2-diyl, Cyclopenten-1,3-diyl, Cyclohexen-1,4-diyl, Cyclohepten-1,4-diyl, Cyclopentadien-1,3-diyl und Cyclohexa-1,3-dien-1,4-diyl;

c) $C_3$-$C_8$-Cycloalkyldiyl, insbesondere trans-Cyclopropan-1,2-diyl, Cyclopropan-1,1-diyl, trans-Cyclobutan-1,3-diyl, cis-Cyclobutan-1,3-diyl, trans-Cyclopentan-1,3-diyl, cis-Cyclohexan-1,4-diyl. trans-Cyclohexan-1,4-dlyl, trans-Cycloheptan-1,4-diyl und trans-Cyclooctan-1,5-diyl.

Drei- bis achtgliedriger gesättigter, partiell ungesättigter oder aromatischer heterocyclischer Rest bedeutet für $D^2$ beispielsweise:

a) nicht-aromatisches, gesättigtes oder teilweise ungesättigtes 5- oder 6-gliedriges Heterocyclodiyl, enthaltend ein bis drei Stickstoffatome und/oder ein Sauerstoff-oder Schwefelatom oder ein oder zwei Sauerstoff- und/oder Schwefelatome, insbesondere trans-Tetrahydrofuran-2,5-diyl, trans-Tetrahydrofuran-2,4-diyl, cis-Tetrahydrofuran-2,5-diyl, trans-Tetrahydrothien-2,5-diyl, trans-Tetrahydrothien-2,4-diyl, trans-Pyrrolidin-2,5-diyl, trans-Pyrrolidin-2,4-diyl, Isoxazolidin-2,4-diyl, Isoxazolidin-2,5-diyl, Isothiazolidin-2,4-diyl, Isothiazolidin-2,5-diyl, Pyrazolidin-1,3-diyl, trans-Oxazolidin-2,4-diyl, trans-Thiazolidin-2,5-diyl, Imidazolidin-1,3-diyl, trans-Imidazolidin-2,4-diyl, Pyrrolin-1,3-diyl, trans-Pyrrolin-2,4-diyl, trans-Pyrrolin-2,5-diyl, trans-Piperidin-2,5-diyl, Piperidin-1,4-diyl, trans-Dioxan-2,5-diyl, trans-Tetrahydropyran-2,5-diyl, trans-Hexahydropyridazin-3,6-diyl, trans-Hexahydropyridazin-1,4-diyl, trans-Hexahydropyrimidin-2,5-diyl, Hexahydropyrimidin-1,3-diyl, Hexahydropyrimidin-1,4-diyl, Piperazin-1,4-diyl, trans-Piperazin-2,5-diyl und Piperazin-1,3-diyl;

b) 5-gliedriges Heteroaryldiyl, enthaltend ein bis vier Stickstoffatome oder ein bis drei Stickstoffatome und/oder ein Schwefel- oder Sauerstoffatom, insbesondere Furan-2,4-diyl, Furan-2,5-diyl, Thiophen-2,4-diyl, Thiophen-2,5-diyl, Pyrrol-2,4-diyl, Pyrrol-2,5-diyl, Pyrazol-1,3-diyl, Oxazol-2,4-diyl, Oxazol-2,5-diyl, 1,3,4-Oxadiazol-2,5-diyl, 1,2,4-Oxadiazol-3,5-diyl, 1,2,4-Thiadiazol-3,5-diyl, 1,3,4-Thiadiazol-2,5-diyl, Isooxazol-3,5-diyl, Thiazol-2,4-diyl, Thiazol-2,5-diyl, Isothiazol-3,5-diyl, Imidazol-2,4-diyl, 2H-Tetrazol-2,5-diyl, 1H(1,2,4)-Triazol-2,5-diyl, 1H(1,2,3)-Triazol-1,4-diyl und 1H(1,2,3)-Triazol-1,5-diyl;

c) 6-gliedriges Heteroaryldiyl, enthaltend ein bis drei bzw. ein bis vier Stickstoffatome, insbesondere Pyridin-2,5-diyl, Pyridin-2,4-diyl, Pyridin-2,3-diyl, Pyridazin-3,6-diyl, Pyrimidin-2,5-diyl, Pyrimidin-2,6-diyl, Pyrazin-2,5-diyl und Tetrazin-3,5-diyl.

Polycyclischer aromatischer, partiell ungesättigter oder gesättigter heterocyclischer Rest bedeutet für $D^2$ beispielsweise:

1-Benzofuran-4,7-diyi, 1-Benzofuran-2,7-diyl, 2-Benzofuran-4,7-diyl, 2-Benzofuran-3,6-diyl, Chromen-5,8-diyl, Chromen-3,7-diyl, Xanthen-1,4-diyl, Xanthen-2,6-diyl, Indazol-4,7-diyl, Purin-2,8-diyl, 4H-Chinolizin-6,9-diyl, 3-Isochinolin-1,4-diyl, Phthalazin-1,4-diyl, 1,8-Naphthyridin-2,6-diyl, Chinoxalin-2,6-diyl, Chinazolin-5,8-diyl, Cinnolin-5,8-diyl,. Pteridin-2,6-diyl, Indolizin-2,6-diyl, Indol-4,7-diyl, Indol-2,5-diyl, Indol-3,6-diyl, Isoindol-4,7-diyl, Isoindol-2,5-diyl, Carbozol-1,4-diyl, Acridin-1,4-diyl, Phenoxazin-1,4-diyl, Benzoxazol-4,7-diyl, Benzothiazol-4,7-diyl, Benzoimidazol-4,7-diyl, 1H-Benzotriazol-4,7-diyl und Benzothiophendiyl.

Mono- oder polycyclischer, aromatischer, partiell ungesättiger oder gesättiger $C_3$-$C_{14}$-Kohlenwasserstoffrest bedeutet für $D^1$ beispielsweise:

a) $C_6$-$C_{14}$-Aryl, insbesondere Phenyl, 1-Naphthyl, 2-Naphtyl, 1-Biphenylen, 2-Biphenylen, 1-Pyrenyl, 1-Anthracenyl, 2-Anthracenyl, 9-Anthracenyl, 4-Indenyl, 2-Fluorenyl, 3-Fluorenyl, 9-Fluorenyl und 3-Phenanthrenyl;

b) $C_3$-$C_{14}$-Cycloalkenyl oder $C_5$-$C_{14}$-Cycloalkadienyl, insbesondere Cyclopropenyl, Cyclobutenyl, Cyclopentenyl, Cyclohexenyl, Cycloheptenyl, Cyclooctenyl, Cyclopentadien-1-yl, Cyclohexadien-1-yl und Cyclooctadien-1-yl;

c) $C_3$-$C_8$-Cycloalkyl, insbesondere Cyclopropyl, Cyclobutyl, Cyclopentyl, Cyclohexyl, Cycloheptyl, Adamantan-1-yl, Cuban-1-yl, Bicylo[4.4.0]decan-2-yl und Cyclooctyl.

Drei- bis achtgliedriger gesättigter, partiell ungesättigter oder aromatischer heterocyclischer Rest bedeutet für $D^1$ beispielsweise:

a) nicht-aromatisches, gesättigtes oder teilweise ungesättigtes 4, 5, 6 oder 7-gliedriges Heterocyclyl, enthaltend ein bis vier Stickstoffatome und/oder ein Sauerstoff- oder Schwefelatom oder ein oder zwei Sauerstoff- und/oder Schwefelatome, insbesondere 1-Aza-2-oxocyclobut-1-yl, 2-Tetrahydrofuranyl, 3-Tetrahydrofuranyl, 2-Tetrahydrothienyl, 3-Tetrahydrothienyl, 2-Pyrrolidinyl, 3-Pyrrolidinyl, 3-Isoxazolidinyl, 4-Isoxazolidinyl, 5-Isoxazolidinyl, 3-Isothiazolidinyl, 4-Isothiazolidinyl, 5-Isothiazolidinyl, 3-Pyrazolidinyl, 4-Pyrazolidinyl, 5-Pyrazolidinyl, 2-Oxazolidinyl, 4-Oxazolidinyl, 5-Oxazolidinyl, 4,5-dihydro-1,3-oxazole-2-yl, 4,5-dihydro-1,3-oxazole-4-yl, 4,5-dihydro-1,3-oxazole-5-yl, 4,5-dihydro-1,3-thiazole-2-yl, 4,5-dihydro-1,3-thiazole-4-yl, 4,5-dihydro-1,3-thiazole-5-yl, 4,5-dihydro-4H-1,3-oxazin-2-yl, 4,5-dihydro-4H-1,3-thiazin-2-yl, 4,5,6,7-tetrahydro-1,3-oxazepin-2-yl, 4,5,6,7-tetrahydro-1,3-thiazepin-2-yl, 2-Thiazolidinyl, 4-Thiazolidinyl, 5-Thiazolidinyl, 2-Imidazolidinyl, 4-Imidazolidinyl, 2-Pyrrolin-2-yl, 2-Pyrrolin-3-yl, 3-Pyrrolin-2-yl, 3-Pyrrolin-3-yl, 1-Piperidinyl, 2-Piperidinyl, 3-Piperidinyl, 4-Piperidinyl, 4-Morpholinyl, 1,3-Dioxan-5-yl, 2-Tetrahydropyranyl, 4-Tetrahydropyranyl, 2-Tetrahydrothienyl, 3-Hexahydropyridazinyl, 4-Hexahydropyridazinyl, 2-Hexahydropyrimidinyl, 4-Hexahydropyrimidinyl, 5-Hexahydropyrimidinyl, 5H-Tetrazol-5-yl, 1H-Tetrazol-5-yl, 2H-Tetrazol-5-yl 1-Piperazinyl und 2-Piperazinyl;

b) 5-gliedriges Heteroaryl, enthaltend ein bis vier Stickstoffatome oder ein bis drei Stickstoffatome und/oder ein Schwefel- oder Sauerstoffatom: insbesondere 2-Furyl, 3-Furyl, 2-Thienyl, 3-Thienyl, 2-Pyrrolyl, 3-Pyrrolyl, 3-Pyrazolyl, 4-Pyrazolyl, 5-Pyrazolyl, 2-Oxazolyl, 4-Oxazolyl, 5-Oxazolyl, 2-Isoxazolyl, 4-Isooxazolyl, 5-Isooxazolyl, 2-Thiazolyl, 4-Thiazolyl, 5-Thiazolyl, 2-Isothiazolyl, 4-Isothiazolyl, 5-Isothiazolyl, 2-Imidazolyl, 4-Imidazolyl, 1,2,5-Thiadiazol-3-yl, 1,2,4-Thiadiazol-3-yl, 1,2,4-Thiadiazol-5-yl, 1,2,5-Oxadiazol-3-yl, 1,2,4-Oxadiazol-3-yl, 1,2,4-Oxadiazol-5-yl, 1,2,3-Thiadiazol-4-yl, 1,2,3-Thiadiazol-5-yl, 1,2,3,4-Thiatriazol-5-yl, 1H-(1,2,3)Triazol-1-yl, 1H-(1,2,3)Triazol-4-yl, 1H-(1,2,3)Triazol-5-yl, 1H-(1,3,4)Triazol-1-yl und 1H-(1,3,4)Triazol-2-yl;

c) 6-gliedriges Heteroaryl, enthaltend ein bis drei bzw. ein bis vier Stickstoffatome: insbesondere 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl und 2-Pyrazinyl;

d) 1,2-di-carba-closo-dodecaboran-1-yl, 1,7-di-carba-closo-dodecaboran-1-yl oder 1,12-di-carba-closo-dodecaboran-1-yl.

Polycyclischer aromatischer, partiell ungesättigter oder gesättigter heterocyclischer Rest bedeutet für $D^1$ beispielsweise:

1-Benzofuran-2-yl, 1-Benzofuran-3-yl, 1-Benzofuran-5-yl, 1-Benzofuran-6-yl, 1-Benzofuran-7-yl, 2-Benzofuran-1-yl, 2-Benzofuran-3-yl, 2-Benzofuran-4-yl, 2-Benzofuran-5-yl, 2-Benzofuran-6-yl, 2-Benzofuran-7-yl, 2H-Chromen-3-yl, 2H-Chromen-4-yl, 2H-Chromen-5-yl, 2H-Chromen-6-yl, 2H-Chromen-7-yl, 2H-Chromen-8-yl, Xanthen-1-yl, Xanthen-4-yl, Xanthen-9-yl, Indazol-1-yl, Indazol-3-yl, Indazol-4-yl, Indazol-5-yl, Indazol-6-yl, Indazol-7-yl, Purin-2-yl, Purin-6-yl, Purin-7-yl, Purin-8-yl, Chinolin-2-yl, Chinolin-3-yl, Chinolin-4-yl, Chinolin-6-yl, Chinolin-7-yl, Chinolin-8-yl, Isochinolin-1-yl, Isochinolin-3-yl, Isochinolin-4-yl, Isochinolin-5-yl, Isochinolin-6-yl, Isochinolin-7-yl, Isochinolin-8-yl, Phthalazin-1-yl, Phthalazin-3-yl, Phthalazin-5-yl, Phthalazin-6-yl, 1,8-Naphthyridin-2-yl, 1,8-Naphthyridin-3-yl, 1,8-Naphthyridin-4-yl, 1,8-Naphthyridin-6-yl, 1.8-Naphthyridin-7-yl, Chinoxalin-2-yl, Chinoxalin-5-yl, Chinoxalin-6-yl, Chinazolin-4-yl, Chinazolin-6-yl, Cinnolin-3-yl, Cinnolin-4-yl, Cinnolin-6-yl, Pteridin-2-yl, Pteridin-4-yl, Pteridin-6-yl, Pteridin-7-yl, Indolizin-1-yl, Indol-1-yl, Indol-2-yl, Indol-3-yl, Indol-5-yl, Indol-6-yl, Indol-7-yl, Indol-8-yl, Isoindol-1-yl, Isoindol-2-yl, Isoindol-4-yl, Isoindol-5-yl, Carbazol-9-yl, Acridin-9-yl, Phenoxazin-10-yl, 1-Benzothiophen-2-yl, 1-Benzothiophen-3-yl, 1-Benzothiophen-5-yl, 1-Benzothiophen-6-yl, 1-Benzothiophen-7-yl, 1-Benzothiophen-8-yl, Benzimidazol-1-yl, Benzimidazol-2-yl, Benzimidazol-5-yl, Benzimidazol-6-yl, Benzimidazol-7-yl, Benzimidazol-8-yl, 1H-Benzotrialzol-1-yl, 1H-Benzotrialzol-5-yl, 1H-Benzotrialzol-6-yl, 1H-Benzotrialzol-7-yl, 1H-Benzotrialzol-8-yl, 4H-3,1-Benzoxazin-2-yl, 4H-2-Benzopyran-2-yl, 2H-Isochinolin-3-yl, Benzthiazol-2-yl, Benzthiazol-5-yl, Benzthiazol-6-yl, Benzthiazol-7-yl, Benzthiazol-8-yl, Benzoxazol-2-yl, Benzoxazol-5-yl, Benzoxazol-6-yl, Benzoxazol-7-yl oder Benzoxazol-8-yl.

Die genannten linearen oder cyclischen Kohlenwasserstoffreste und Heterocyclen können unsubstituiert oder substituiert sein, wobei die Substituenten vorzugsweise aus der Gruppe $X^2$ gewählt sind. Bevorzugt sind, abhängig von der jeweiligen Ketten- oder Ringgröße, 1, 2, 3 oder 4 Substituenten; im Falle von Halogensubstituenten ist auch eine Substitution bis zur maximal möglichen Anzahl (Persubstitution) bevorzugt.

Vorteilhaft haben die Symbole in der Formel (I) folgende Bedeutungen:

$A^1$, $A^2$ sind vorteilhaft gleich oder verschieden eine Gruppe $D^1$-[$Y^3$-$D^2$-]$_m$-.

$D^1$ ist vorteilhaft gleich oder verschieden

a) $C_6$-$C_{14}$-Aryl, insbesondere Phenyl, 1-Naphthyl, 2-Naphtyl, 1-Biphenylen, 2-Biphenylen, 1-Pyrenyl, 1-Anthracenyl, 2-Fluorenyl, 3-Fluorenyl, 9-Fluorenyl oder 3-Phenanthrenyl oder,

b) $C_3$-$C_{14}$-Cycloalkenyl oder $C_5$-$C_{14}$-Cycloalkadienyl, insbesondere Cyclopropenyl, Cyclobutenyl, Cyclopentenyl, Cyclohexenyl, Cycloheptenyl oder Cyclooctenyl;

c) $C_3$-$C_8$-Cycloalkyl, insbesondere Cyclopropyl, Cyclobutyl, Cyclopentyl oder Cyclohexyl oder,

d) nicht-aromatisches, gesättigtes oder teilweise ungesättigtes 5- oder 6-gliedriges Heterocyclyl, enthaltend ein bis drei Stickstoffatome und/oder ein Sauerstoff- oder Schwefelatom oder ein oder zwei Sauerstoff- und/oder

Schwefelatome, insbesondere 2-Tetrahydrofuranyl, 3-Tetrahydrofuranyl, 2-Tetrahydrothienyl, 3-Tetrahydrothienyl, 2-Pyrrolidinyl, 3-Pyrrolidinyl, 3-Isoxazolidinyl, 4-Isoxazolidinyl, 5-Isoxazolidinyl, 3-Isothiazolidinyl, 4-Isothiazolidinyl, 5-Isothiazolidinyl, 3-Pyrazolidinyl, 4-Pyrazolidinyl, 5-Pyrazolidinyl, 2-Oxazolidinyl, 4-Oxazolidinyl, 5-Oxazolidinyl, 2-Thiazolidinyl, 4-Thiazolidinyl, 5-Thiazolidinyl, 2-Imidazolidinyl, 4-Imidazolidinyl, 2-Pyrrolin-2-yl, 2-Pyrrolin-3-yl, 3-Pyrrolin-2-yl, 3-Pyrrolin-3-yl, 2-Piperidinyl, 3-Piperidinyl, 4-Piperidinyl, 4-Morpholinyl, 1,3-Dioxan-5-yl, 2-Tetrahydropyranyl, 4-Tetrahydropyranyl, 2-Tetrahydrothienyl, 3-Hexahydropyridazinyl, 4-Hexahydropyridazinyl, 2-Hexahydropyrimidinyl, 4-Hexahydropyrimidinyl, 5-Hexahydropyrimidinyl, 1-Piperazinyl oder 2-Piperazinyl oder,

e) 5-gliedriges Heteroaryl, enthaltend ein bis vier Stickstoffatome oder ein bis drei Stickstoffatome und/oder ein Schwefel- oder Sauerstoffatom, insbesondere 2-Furyl, 3-Furyl, 2-Thienyl, 3-Thienyl, 2-Pyrrolyl, 3-Pyrrolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazo-lyl, 2-Imidazolyl, 4-Imidazolyl, 1H-Tetrazol-5-yl, 1H-(1,2,3)-Triazol-1-yl, 1H-(1,2,3)-Triazol-4-yl, 1H-(1,2,3)-Triazol-5-yl oder 1H-(1,3,4)-Triazol-2-yl oder,

f) 6-gliedriges Heteroaryl, enthaltend ein bis vier Stickstoffatome, insbesondere 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl oder 2-Pyrazinyl oder,

g) polycyclischer heterocyclischer Rest insbesondere, 1-Benzofuran-2-yl, 1-Benzofuran-3-yl, 1-Benzofuran-5-yl, 1-Benzofuran-6-yl, 1-Benzofuran-7-yl, 2-Benzofuran-5-yl, 2-Benzofuran-6-yl, 2H-Chromen-3-yl, 2H-Chromen-4-yl, 2H-Chromen-5-yl, 2H-Chromen-6-yl, 2H-Chromen-7-yl,Indazol-7-yl, Purin-8-yl, Isochinolin-3-yl, Phthalazin-1-yl, 1,8-Naphthyridin-2-yl, Chinoxalin-2-yl, Chinazolin-2-yl, Cinnolin-3-yl, Pteridin-2-yl, Indol-1-yl, Isoindol-2-yl, Carbozol-9-yl, Acridin-9-yl, 1-Benzothiophen-2-yl, Benzimidazol-2-yl, Benzthiazol-2-yl oder Benzoxazol-2-yl,

wobei die genannten Gruppen unsubstituiert oder durch eine oder mehrere Gruppen $X^1$ substituiert sind.

$D^2$ ist vorteilhaft gleich oder verschieden

a) $C_6$-$C_{14}$-Aryldiyl, insbesondere Phenylen-1,4-diyl, Phenylen-1,3-diyl, Phenylen-1,2-diyl, Naphthalin-1,4-diyl, Naphthalin-1,5-diyl, Anthracen-9,10-diyl oder Inden-4,7-diyl;

b) $C_3$-$C_8$-Cycloalkyldiyl, insbesondere Cyclopropandiyl, trans-Cyclobutan-1,3-diyl, trans-Cyclopentan-1,3-diyl und trans-Cyclohexan-1,4-diyl,

c) nicht-aromatisches, gesättigtes oder teilweise ungesättigtes 5- oder 6-gliedriges Heterocyclodiyl, enthaltend ein bis drei Stickstoffatome und/oder ein Sauerstoff-oder Schwefelatom oder ein oder zwei Sauerstoff- und/oder Schwefelatome, insbesondere Tetrahydrofuran-2,5-diyl, Tetrahydrofuran-2,4-diyl, Piperidin-1,4-diyl, Imidazolidin-1,3-diyl oder Piperazin-1,4-diyl;

d) 5-gliedriges Heteroaryldiyl, enthaltend ein bis vier Stickstoffatome oder ein bis drei Stickstoffatome und/oder ein Schwefel- oder Sauerstoffatom, insbesondere Furan-2,4-diyl, Furan-2,5-diyl, Thiophen-2,4-diyl, Thiophen-2,5-diyl, Pyrrol-2,4-diyl, Pyrrol-2,5-diyl, 1H(1,2,4)-Triazol-2,5-diyl, 1H(1,2,3)-Triazol-1,4-diyl oder 1 H(1,2,3)-Triazol-1,5-diyl

e) 6-gliedriges Heteroaryldiyl, enthaltend ein bis vier Stickstoffatome, insbesondere Pyridin-2,5-diyl, Pyridin-2,4-diyl, Pyridin-2,3-diyl, Pyridazin-3,6-diyl, Pyrimidin-2,5-diyl, Pyrimidin-2,6-diyl, Pyrazin-2,5-diyl oder Tetrazin-3,5-diyl, wobei die genannten Gruppen unsubstituiert oder durch eine oder mehrere Gruppen $X^1$ substituiert sind.

$X^1$ ist vorteilhaft gleich oder verschieden F, Cl, Br, I, Cyano, Nitro, Hydroxy, Mercapto, Amino, Carboxyl, Hydroxylamino, Azido, $SO_3M$, $OSO_3M$, $SO_2NH_2$, $OPO_3M$, Alkyl, Halogenalkyl, Alkenyl, Alkinyl, Alkyloxy, Halogenalkyloxy, Alkenyloxy, Alkinyloxy, Alkylthio, Alkylamino, Dialkylamino, Trialkylamino, Formyl, Alkylcarbonyl, Alkylsulfonyl, Alkylsulfoxyl, Aminocarbonyl, Alkylaminocarbonyl, Alkylcarbonylamino, Oxo (=O), Thioxo (=S), wobei die Alkylgruppen in diesen Resten 1 bis 8 Kohlenstoffatome enthalten und die genannten Alkenyl- oder Alkinylgruppen in diesen Resten 2 bis 8 Kohlenstoffatome enthalten.

$Y^1$, $Y^2$ sind vorteilhaft gleich oder verschieden $\sim CH_2$-, $\sim CH_2CH_2$-, $\sim CH_2CH_2CH_2$-, ~C(O)-, $\sim CH_2C$(o)-,

~$CH_2CH_2C(O)$-, ~$CH_2CH_2CH_2C(O)$-, ~CH=CH-C(O)-, ~C≡C-C(O)-, ~$S(O)_2$-, ~$CH_2S(O)_2$-, ~NH-C(O)-, ~$NR^x$-3-Cyclobuten-1,2-dion-4-, ~$CH_2$-$NR^x$-3-Cyclobuten-1,2-dion-4-, ~$NR^x$-3-2,5-thiadiazol-1,1-dioxid-4-, wobei ~ die Bindung zur Gruppe A bezeichnet oder

$Y^1$, $Y^2$ bilden, unabhängig voneinander, zusammen mit dem Stickstoff, an den sie gebunden sind, und der Gruppe $R^8$ eine Gruppe ~4-1H-(1,2,3)Triazol-1-yl-, ~5-1H-(1,2,3)Triazol-1-yl-, ~$CH_2$-4-1H-(1,2,3)Triazol-1-yl- oder ~$CH_2$-5-1H-(1,2,3)Triazol-1-yl-, wobei ~ die Bindung zur Gruppe A bezeichnet.

$Y^3$ ist vorteilhaft eine Bindung, O, S(O), $S(O_2)$, $CH_2$, C(O) oder $NR^x$.

m ist vorteilhaft 0, 1 oder 2.

$Z^1$ ist vorteilhaft O, S oder $CH_2$.

D ist vorteilhaft eine Gruppe $Z^2$-$T^1$-$Y^4$ -$A^3$-$Y^5$-$T^2$-$Z^2$.

$Z^2$ ist vorteilhaft gleich oder verschieden -O~, -S~, -$NR^x$~, -NHC(O)~, - $CH_2$~, -O-$NR^x$~ oder ~4-1H-(1,2,3)Triazol-1-yl- wobei ~ die Bindung zur Gruppe T bezeichnet.

$T^1$, $T^2$ sind vorteilhaft gleich oder verschieden eine geradkettige oder verzweigte Alkandiylgruppe mit 4 bis 30 C-Atomen, wobei

(i) gegebenenfalls eine oder mehrere nicht terminale $CH_2$-Gruppen durch -O-, -S-, -S(O)-, -$S(O)_2$-, -$P(O_2)$- und/oder -$NR^x$- ersetzt sind und/oder

(ii) gegebenenfalls ein oder mehrere H-Atome durch F, Cl, $OR^x$,-$OSO_3M$, (=O), Carboxyl, $NH_2$, $NHR^Y$ und/oder $NHR^z$ ersetzt sind und/oder

(iii) gegebenenfalls eine nicht terminale -$CH_2$-$CH_2$-Gruppe durch -5-1H-(1,2,3)Triazol-1-yl- ersetzt ist und/oder

(iv) gegebenfalls eine nicht terminale -$CH_2CH_2CH_2$-Gruppe durch -4-1H-(1,2,3)Triazol-1-yl- oder -O-N=CH- ersetzt ist.

$Y^4$, $Y^5$ sind vorteilhaft gleich oder verschieden O, $NR^x$, S(O), $S(O)_2$, C(O), ~$NR^x$-C(O)-, ~C(O)-$NR^x$-, ~$NR^x$-CO-$NR^x$-, ~$NR^x$-$S(O)_2$, ~$S(O)_2$-$NR^x$-, ~$CH_2$-$NR^x$-C(O)-, -$CH_2$-C(O)-$NR^x$- oder -$CH_2$-$NR^x$-, wobei ~ die Bindung zu Gruppe A bezeichnet.

$A^3$ ist vorteilhaft

a) $C_1$-$C_8$ Alkandiyl, $C_2$-$C_8$-Alkendiyl, $C_2$-$C_8$-Alkindiyl, $C_4$-$C_8$-Alkadiendiyl, wobei in den genannten Gruppen gegebenenfalls 1, 2, 3 oder 4 $CH_2$-Gruppen durch O, S(O), $S(O)_2$, $NR^x$ und/oder S ersetzt sind, und wobei in den genannten Gruppen gegebenenfalls ein oder mehrere H-Atome durch F oder Cl ersetzt sind,

b) eine Gruppe $A^4$-$[Z^3$-$A^5]_n$,

c) 1,1'-Ferrocendiyl, 1,1'-Cobaltocendiyl, 1,1'-Ruthenocen oder Dichlorplatindiaminodiyl.

$A^4$, $A^5$ sind vorteilhaft gleich oder verschieden

a) $C_6$-$C_{14}$-Aryldiyl, insbesondere Phenylen-1,4-diyl, Phenylen-1,3-diyl, Phenylen-1,2-diyl, Naphthalin-1,4-diyl, Naphthalin-1,5-diyl, Naphthalin-2,6-diyl, Naphthalin-2,7-diyl, Biphenylen-1,4-diyl, Biphenylen-2,6-diyl, Anthracen-1,4-diyl, Anthracen-9,10-diyl, Inden-4,7-diyl, s-Indacen-4,8-diyl, Fluoren-1,4-diyl oder Phenanthren-1,4-diyl;
b) $C_3$-$C_8$-Cycloalkyldiyl, insbesondere trans-Cyclopropan-1,2-diyl, trans-Cyclobutan-1,2-diyl, trans-Cyclobutan-1-3-diyl, trans-Cyclohexan-1,4-diyl oder trans-Cyclohexan-1,3-diyl

c) nicht-aromatisches, gesättigtes oder teilweise ungesättigtes 5-oder 6-gliedriges Heterocyclodiyl, enthaltend ein bis drei Stickstoffatome und/oder ein Sauerstoff- oder Schwefelatom oder ein oder zwei Sauerstoff- und/oder Schwefelatome, insbesondere Piperazin-1,4-diyl, Tetrahydrofuran-2,5-diyl oder Tetra-hydrofuran-2,4-diyl;
d) 5-gliedriges Heteroaryldiyl, enthaltend ein bis vier Stickstoffatome oder ein bis drei Stickstoffatome und/oder ein Schwefel- oder Sauerstoffatom, insbesondere Furan-2,4-diyl, Furan-2,5-diyl, Thiophen-2,4-diyl, Thiophen-2,5-diyl, Pyrrol-2,5-diyl, Pyrrol-2,5-diyl, Pyrazol-1,3-diyl, Oxazol-2,4-diyl, Oxazol-2,5-diyl, 1,3,4-Oxadiazol-2,5-diyl, 1,2,4-Oxadiazol-3,5-diyl, 1,2,4-Thiadiazol-3,5-diyl, 1,3,4-Thiadiazol-2,5-diyl, Thiazol-2,4-diyl, Thiazol-2,5-diyl, Imidazol-2,4-diyl, 2H-Tetrazol-2,5-diyl, 1H-(1,2,4)Triazol-2,5-diyl, 1H-(1,2,3)Triazol-1,4-diyl oder 1H-(1,2,3)Triazol-1,5-diyl;
e) 6-gliedriges Heteroaryldiyl, enthaltend ein bis drei bzw. ein bis vier Stickstoffatome, insbesondere Pyridin-2,5-diyl, Pyridin-2,4-diyl, Pyridazin-2,5-diyl, Pyrimidin-2,5-diyl, Pyrimidin-2,4-diyl, Pyrazin-2,5-diyl oder Tetrazin-2,5-diyl;
f) polycyclisches Heterocyclyl aus der Gruppe 1-Benzofuran-4,7-diyl, 2-Benzofuran-4,7-diyl oder Xanthen-1,4-diyl,

wobei die genannten Reste unsubstituiert oder durch eine oder mehrere Gruppen $X^2$ substituiert sind.

$Z^3$ ist vorteilhaft eine Bindung, O, S(O), $S(O)_2$ oder C(O).

$X^2$ ist vorteilhaft gleich oder verschieden Fluor, Chlor, Brom, Nitro, Hydroxylamino, $SO_3M$, $OSO_3M$, $SO_2NH_2$, $PO_3M$, $OPO_3M$, Alkyl, Halogenalkyl, Alkyloxy, Halogenalkyloxy, Alkylamino, Dialkylamino, Trialkylamino, Alkylcarbonyl, Alkylsulfonyl, Alkylsulfoxyl, Alkylaminosulfoxyl, Dialkylaminosulfoxyl, Alkyloxycarbonyl, Alkylcarbonyloxy, Aminocarbonyl, Alkylaminocarbonyl, Alkylcarbonylamino, Dialkylaminocarbonyl, $C_1$-$C_4$-Alkylimino (=N-$C_1$-$C_4$-Alkyl), $C_1$-$C_4$-Alkyloximino (=N-O-$C_1$-$C_4$-Alkyl), wobei die Alkylgruppen in diesen Resten 1 bis 4 Kohlenstoffatome enthalten und die Gruppen Halogenalkyl und Halogenalkyloxy die Halogene F und/oder Cl enthalten.

n ist vorteilhaft 0 oder 1.

$R^1$ ist vorteilhaft gleich ist C(O)OM, $SO_3M$, C(O)-NH-$S(O)_2$-$R^x$, $PO_3M_2$ oder C(O)NOM.

$R^2$ $R^3$ sind vorteilhaft gleich oder verschieden H oder F.

$R^4$, $R^7$ sind vorteilhaft gleich oder verschieden H, OH, $OR^z$, OC(O)$NHR^y$ oder $NR^x$.

$R^6$ ist vorteilhaft gleich oder verschieden H oder $R^z$.

$R^5$ ist vorteilhaft gleich oder verschieden H, $R^x$, C(O)$CH_2$OH, C(O)-Halogenalkyl oder C(O)H.

$R^8$ ist vorteilhaft gleich oder verschieden $R^x$.

M ist vorteilhaft H, $C_1$-$C_4$-Alkyl oder ein Kation aus der Gruppe der Alkalimetalle, Erdalkalimetalle, Mangan, Kupfer, Zink, Eisen und gegebenenfalls substituiertes Ammonium.

$R^x$ ist vorteilhaft gleich oder verschieden H, $R^y$ oder $R^z$.

$R^y$ ist vorteilhaft gleich oder verschieden $C_1$-$C_4$-Alkyl, insbesondere Methyl, Ethyl, 1-Methylethyl, Propyl, 1-Methylpropyl, 1,1-Dimethylpropyl, Butyl, Phenyl oder Benzyl.

$R^z$ ist vorteilhaft gleich oder verschieden -C(O)-$C_1$-$C_6$-Alkyl, -C(O)-Phenyl, -C(O)-$C_1$-$C_4$-Alkyl-Phenyl, insbesondere Methylcarbonyl, Ethylcarbonyl, Propylcarbonyl, 1-Methylethylcarbonyl, 1,1-Dimethylethylcarbonyl, 1-Methylpropylcarbonyl, 2-Methylpropylcarbonyl, 1,1-Dimethylpropycarbonyl, Butylcarbonyl, Pentylcarbonyl oder Benzylcarbonyl.

Vorteilhaft bedeuten die in der Formel (I) angegebenen Definitionen der Symbole:

Halogen: Fluor, Chlor und Brom;

Alkyl: gesättigte, geradkettige, verzweigte oder cyclische Kohlenwasserstoffreste mit beispielsweise 1 bis 7 Kohlenstoffatomen, wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Dimethylpropyl, 1,1-Dimethylpropyl, 1-Ethylpropyl, Hexyl, 1,1-Dimethylbutyl, 2,2-Dimethylbutyl, 3,3-Dimethylbutyl, 1,2-Dimethylbutyl, 1,3-Dimethylbutyl, 2,3-Dimethylbutyl, 1-Methylpentyl, 2-Methylpentyl, 3-Methylpentyl, 4-Methylpentyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, Heptyl, Octyl, Cyclopropyl, Cyclobutyl, 1-Methylcyclopropyl, 2-Methylcyclopropyl, Cyclopentyl, 2,2-Dimethylcylopropyl, 2,3- Dimethylcylopropyl und Cyclohexyl;
Halogenalkyl: geradkettige, verzweigte oder cyclische Alkylgruppen mit beispielsweise 1 bis 5 Kohlenstoffatomen (wie vorstehend genannt), wobei in diesen Gruppen Wasserstoffatome teilweise oder vollständig durch Halogenatome ersetzt sind: wie Chlormethyl, Brommethyl, Dichlormethyl, Trichlormethyl, Fluormethyl, Difluormethyl, Trifluormethyl, Chlorfluormethyl, Dichlorfluormethyl, Chlordifluormethyl, 1-Chlorethyl, 1-Bromethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Pentafluorethyl, 1-Fluor-1-methyl-ethyl, 1-Fluorcyclopropyl, Heptafluorpropyl oder Nonafluorbutyl;
Alkenyl: ungesättigte, geradkettige oder verzweigte Kohlenwasserstoffreste mit beispielsweise 2 bis 4 Kohlenstoffatomen und einer oder zwei Doppelbindungen in beliebiger Position (einschließlich aller E und Z Stereoisomeren), z.B. $C_2$-$C_6$-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl oder But-1,3-enyl;
Alkinyl: geradkettige oder verzweigte Kohlenwasserstoffgruppen mit 2 bis 4 Kohlenstoffatomen und einer oder zwei Dreifachbindungen in beliebiger Position, z.B. $C_2$-$C_6$-Alkinyl wie Ethinyl, 1-Propinyl, 2-Propinyl, 1-Butinyl, 2-Butinyl, 3-Butinyl oder 1-Methyl-2-propinyl;
Alkyloxy: Alkyloxygruppen mit gesättigtem, geradkettigem, verzweigtem oder cyclischen Alkylrest, wobei dieser aus der vorstehend genannten Gruppe der Alkyle ist und 1 bis 4 Kohlenstoffatome enthält;
Halogenalkyloxy: Halogenalkyloxygruppen mit geradkettigem, verzweigtem oder cyclischem Halogenalkylrest, wobei dieser aus der vorstehend genannten Gruppe der Halogenalkyle ist und 1 bis 4 Kohlenstoffatome enthält;
Alkenyloxy: Alkenyloxygruppen mit ungesättigtem, geradkettigem oder verzweigtem Alkenylrest, wobei dieser aus der vorstehend genannten Gruppe der Alkenyle ist und 1 bis 4 Kohlenstoffatome enthält;
Alkinyloxy: Alkinyloxygruppen mit geradkettigem oder verzweigtem Alkinylrest, wobei dieser aus der vorstehend genannten Gruppe der Alkinyle ist und 1 bis 4 Kohlenstoffatome enthält;
Alkylthio: Alkylthiogruppen mit gesättigtem, geradkettigem, verzweigtem oder cyclischem Alkylrest, wobei dieser aus der vorstehend genannten Gruppe der Alkyle ist und 1 bis 4 Kohlenstoffatome enthält;
Alkylamino: Alkylaminogruppen mit gesättigtem, geradkettigem, verzweigtem oder cyclischem Alkylrest, wobei dieser aus der vorstehend genannten Gruppe der Alkyle ist und 1 bis 4 Kohlenstoffatome enthält;
Dialkylamino: Dialkylaminogruppen mit gesättigten, geradkettigen, verzweigten oder cyclischen Alkylresten, wobei diese gleich oder verschieden aus der vorstehend genannten Gruppe der Alkyle sind und 1 bis 4 Kohlenstoffatome enthalten;
Trialkylamino: Trialkylaminogruppen mit gesättigtem, geradkettigem, verzweigtem oder cyclischem Alkylresten, wobei diese gleich oder verschieden aus der vorstehend genannten Gruppe der Alkyle sind und 1 bis 4 Kohlenstoffatome enthalten;
Alkylcarbonyl: Alkylcarbonylgruppen mit gesättigtem, geradkettigem, verzweigtem oder cyclischem Alkylrest, wobei dieser aus der vorstehend genannten Gruppe der Alkyle ist und 1 bis 4 Kohlenstoffatome enthält;
Alkylsulfonyl: Alkylsulfonylgruppen mit gesättigtem, geradkettigem, verzweigtem oder cyclischem Alkylrest, wobei dieser aus der vorstehend genannten Gruppe der Alkyle ist und 1 bis 4 Kohlenstoffatome enthält;
Alkylsulfoxyl: Alkylsulfoxylgruppen mit gesättigtem, geradkettigem, verzweigtem oder cyclischem Alkylrest, wobei dieser aus der vorstehend genannten Gruppe der Alkyle ist und 1 bis 4 Kohlenstoffatome enthält;
Alkylaminosulfoxyl: Alkylaminosulfoxylgruppen mit gesättigtem, geradkettigem, verzweigtem oder cyclischem Alkylrest, wobei dieser aus der vorstehend genannten Gruppe der Alkyle ist und 1 bis 4 Kohlenstoffatome enthält;
Dialkylaminosulfoxyl: Aialkylaminosulfoxylgruppen mit gesättigtem, geradkettigem, verzweigtem oder cyclischem Alkylrest, wobei dieser aus der vorstehend genannten Gruppe der Alkyle ist und 1 bis 4 Kohlenstoffatome enthält;
Alkyloxycarbonyl: Alkyloxycarbonylgruppen mit gesättigtem, geradkettigem, verzweigtem oder cyclischem Alkylrest, wobei dieser aus der vorstehend genannten Gruppe der Alkyle ist und 1 bis 4 Kohlenstoffatome enthält;
Alkylcarbonyloxy: Alkylcarbonyloxygruppen mit gesättigtem, geradkettigem, verzweigtem oder cyclischem Alkylrest, wobei dieser aus der vorstehend genannten Gruppe der Alkyle ist und 1 bis 8 Kohlenstoffatome enthält;
Alkylaminocarbonyl: Alkylaminocarbonylgruppen mit gesättigtem, geradkettigem, verzweigtem oder cyclischem Alkylrest, wobei dieser aus der vorstehend genannten Gruppe der Alkyle ist und 1 bis 4 Kohlenstoffatome enthält;
Dialkylaminocarbonyl: Dialkylaminocarbonylgruppen mit gesättigtem, geradkettigem, verzweigtem oder cyclischem Alkylrest, wobei dieser aus der vorstehend genannten Gruppe der Alkyle ist und 1 bis 4 Kohlenstoffatome enthält;
Alkylaminothiocarbonyl: Alkylaminothiocarbonylgruppen mit gesättigtem, geradkettigem, verzweigtem oder cyclischem Alkylrest, wobei dieser aus der vorstehend genannten Gruppe der Alkyle ist und 1 bis 4 Kohlenstoffatome enthält;

Alkylimino: Alkyliminogruppen mit gesättigtem, geradkettigem, verzweigtem oder cyclischem Alkylrest, wobei dieser aus der vorstehend genannten Gruppe der Alkyle ist und 1 bis 4 Kohlenstoffatome enthält;

Alkyloximino: Alkyloximinogruppen mit gesättigtem, geradkettigem, verzweigtem oder cyclischem Alkylrest, wobei dieser aus der vorstehend genannten Gruppe der Alkyle ist und 1 bis 4 Kohlenstoffatome enthält;

Alkylcarbonylamino: Alkylcarbonylaminogruppen mit gesättigtem, geradkettigem, verzweigtem oder cyclischem Alkylrest, wobei dieser aus der vorstehend genannten Gruppe der Alkyle ist und 1 bis 4 Kohlenstoffatome enthält;

Alkandiyl bedeutet für Rest $A^3$: gesättigte, geradkettige oder verzweigte Alkandiylgruppe mit beispielsweise 1 bis 6 Kohlenstoffatomen, wie Methan-1,1-diyl, Ethan-1,2-diyl, Propan-1,3-diyl, Butan-1,4-diyl, Pentan-1,5-diyl und Hexan-1,6-diyl;

Alkandiyl bedeutet für Rest $T^1$ und $T^2$: gesättigte, geradkettige oder verzweigte Alkandiylgruppe mit beispielsweise 1 bis 21 Kohlenstoffatomen, wie Methan-1,1-diyl, Ethan-1,2-diyl, Propan-1,3-diyl, Butan-1,4-diyl, Pentan-1,5-diyl, Hexan-1,6-diyl, Heptan-1,7-diyl, Octan-1,8-diyl, Nonan-1,9-diyl, Decan-1,10-diyl, Undecan-1,11-diyl, Dodecan-1,12-diyl, Tridecan-1,13-diyl, Tetradecan-1,14-diyl, Pentadecan-1,15-diyl, Hexadecan-1,16-diyl, Heptadecan-1,17-diyl, Octadecan-1,18-diyl, Nonadecan-1,19-diyl, Eicosan-1,20-diyl oder Heneicosan-1,21-diyl;

Alkendiyl (z.B. für Rest $A^3$): ungesättigte, geradkettige oder verzweigte Alkendiylgruppen (einschließlich aller E und Z Stereoisomeren) mit beispielsweise 2 bis 6 Kohlenstoffatomen, wie Ethen-1,1-diyl, Prop-2-en-1,3-diyl, But-2-en-1,4-diyl, 3,4-Dimethylbut-2-en-1,4-diyl, Pent-2-en-1,5-diyl und Hex-3-en-1,6-diyl;

Alkindiyl (z.B. für Rest $A^3$): ungesättigte, geradkettige oder verzweigte Alkindiylgruppen mit beispielsweise 2 bis 6 Kohlenstoffatomen, wie Ethin-1,1-diyl, Prop-2-in-1,3-diyl, But-2-in-1,4-diyl, Pent-2-in-1,5-diyl und Hex-3-in-1,6-diyl;

Alkadiendiyl (z.B. für Rest $A^3$): ungesättigte, geradkettige oder verzweigte Alkadiendiylgruppe (einschließlich aller E und Z Stereoisomeren) mit beispielsweise 4 bis 6 Kohlenstoffatomen wie But-1,3-dien-1,4-diyl, 3,4-Dimethylbut-1,3-dien-1,4-diyl und Hex-2,4-dien-1,6-diyl.

Vorteilhaft sind Verbindungen der Formel (I), bei denen alle Symbole, Definitionen und Indizes die vorteilhaften Bedeutungen haben.

Bevorzugt haben die Symbole und Indizes in der Formel (I) folgende Bedeutungen:

$A^1$, $A^2$ sind bevorzugt gleich oder verschieden eine Gruppe $D^1$-[$Y^3$-$D^2$-]$_m$-.

$D^1$ ist bevorzugt gleich oder verschieden

a) $C_6$-$C_{12}$-Aryl, insbesondere Phenyl, 1-Naphthyl, 2-Naphtyl, 2-Biphenylen sowie 2-Fluorenyl, 3-Fluorenyl oder 2-Phenanthrenyl;
b) $C_3$-$C_6$-Cycloalkyl, insbesondere Cyclopropyl, Cyclobutyl, Cyclopentyl oder Cyclohexyl;
c) nicht-aromatisches, gesättigtes oder teilweise ungesättigtes 5- oder 6-gliedriges Heterocyclyl, enthaltend ein bis drei Stickstoffatome und/oder ein Sauerstoff-oder Schwefelatom oder ein oder zwei Sauerstoff- und/oder Schwefelatome, insbesondere 2-Piperidinyl, 3-Piperidinyl, 4-Piperidinyl, 1,3-Dioxan-5-yl, 4-Tetrahydropyranyl, 3-Hexahydropyridazinyl, 4-Hexahydropyridazinyl, 2-Hexahydropyrimidinyl, 4-Hexahydropyrimidinyl, 5-Hexahydropyrimidinyl, 1-Piperazinyl, 2-Piperazinyl oder 4-Morpholinyl;
d) 5-gliedriges Heteroaryl, enthaltend ein bis vier Stickstoffatome oder ein bis drei Stickstoffatome und/oder ein Schwefel- oder Sauerstoffatom, insbesondere 2-Furyl, 3-Furyl, 2-Thienyl, 3-Thienyl, 2-Pyrrolyl, 3-Pyrrolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, 1,2,3-Triazol-1-yl, 1,2,3-Triazol-4-yl, 1,2,3-Triazol-5-yl oder 1,3,4-Triazol-2-yl;
e) 6-gliedriges Heteroaryldiyl, enthaltend ein bis drei Stickstoffatome, insbesondere 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl oder 2-Pyrazinyl;
f) polycyclische heterocyclische Reste, insbesondere 1-Benzofuran-2-yl, 1-Benzofuran-5-yl, 1-Benzofuran-6-yl, 2-Benzofuran-5-yl, 2-Benzofuran-6-yl, 2H-Chromen-3-yl, 2H-Chromen-6-yl, 2H-Chromen-7-yl, Indolyl, 1-Benzothiophen-2-yl oder Benzimidazolyl,

wobei die genannten Gruppen unsubstituiert oder durch eine oder mehrere Gruppen $X^1$ substituiert sind.

$D^2$ ist bevorzugt gleich oder verschieden

a) $C_6$-$C_{12}$-Aryldlyl, insbesondere Phenylen-1,4-diyl, Phenylen-1,3-diyl, Naphthalin-1,4-diyl;
b) $C_5$-$C_6$-Cycloalkyldiyl, insbesondere trans-Cyclobutan-1,3-diyl oder trans-Cyclohexan-1,4-diyl;
c) 5-gliedriges Heteroaryldiyl, enthaltend ein bis vier Stickstoffatome oder ein bis drei Stickstoffatome und/oder

ein Schwefel- oder Sauerstoffatom, insbesondere Furan-2,5-diyl, Thiophen-2,4-diyl, Thiophen-2,5-diyl, 1H(1,2,3)-Triazol-1,4-diyl oder Pyrrol-2,5-diyl;
d) 6-gliedriges Heteroaryldiyl, enthaltend ein bis vier Stickstoffatome, insbesondere Pyridin-2,5-diyl, Pyridin-2,4-diyl, Pyridazin-3,6-diyl, Pyrimidin-2,5-diyl, Pyrimidin-2,6-diyl, Pyrazin-2,5-diyl und Tetrazin-3,5-diyl;

wobei die genannten Gruppen unsubstituiert oder durch eine Gruppe $X^1$ substituiert sind.

$X^1$ ist bevorzugt gleich oder verschieden F, Cl, Br, I, Cyano, Nitro, Hydroxy, Amino, Carboxyl, Hydroxylamino, Azido, $SO_3M$, $SO_2NH_2$, Alkyl, Halogenalkyl, Alkinyl, Alkyloxy, Halogenalkyloxy, Alkinyloxy, Alkylamino, Dialkylamino, Trialkylamino, Alkylcarbonyl, Alkylsulfonyl, Alkylsulfoxyl, Aminocarbonyl, Alkylaminocarbonyl, Alkylcarbonylamino oder Oxo (=O), wobei die Alkylgruppen in diesen Resten 1 bis 6 Kohlenstoffatome enthalten und die genannten Alkinylgruppen in diesen Resten 2, 4 oder 6 Kohlenstoffatome enthalten.

$Y^1$, $Y^2$ sind bevorzugt gleich oder verschieden ~$CH_2$-, ~C(O)-, ~$CH_2$C(O)-, ~$CH_2CH_2$-C(O)-, ~CH=CH-C(O)-, ~C≡C-C(O)-, ~$CH_2$-$NR^x$-3-Cyclobuten-1,2-dion-4-, ~$S(O)_2$-, ~$CH_2S(O)_2$- oder ~NH-C(O)-, wobei ~ die Bindung zur Gruppe A bezeichnet oder

$Y^1$, $Y^2$ bilden, unabhängig voneinander, zusammen mit dem Stickstoff, an den sie gebunden sind, und der Gruppe $R^8$ eine Gruppe ~4-1H-(1,2,3)Triazol-1-yl- oder ~5-1H-(1,2,3)Triazol-1-yl-, wobei ~ die Bindung zur Gruppe A bezeichnet;

$Y^3$ ist bevorzugt eine Bindung, O, $S(O_2)$, $CH_2$ oder C(O).

m ist bevorzugt 0, 1 oder 2.

$Z^1$ ist bevorzugt O oder S.

D ist bevorzugt eine Gruppe $Z^2$-$T^1$-$Y^4$ -$A^3$-$Y^5$-$T^2$-$Z^2$.

$Z^2$ ist bevorzugt gleich O, S, ~4-1H-(1,2,3)Triazol-1-yl-, -NH-C(O)~ oder $CH_2$, wobei ~ die Bindung zur Gruppe T bezeichnet.

$T^1$, $T^2$ sind bevorzugt gleich oder verschieden eine geradkettige oder verzweigte Alkandiylgruppe mit 4 bis 30 C-Atomen, wobei

(i) gegebenenfalls eine oder mehrere nicht terminale $CH_2$-Gruppen durch -O-, -S-, -S(O)-, -$S(O)_2$- und/oder -$NR^x$- ersetzt sind und/oder
(ii) gegebenenfalls ein oder mehrere H-Atome durch Fluor, Chlor, (=O), Carboxyl, $NH_2$ oder $NHR^z$ ersetzt sind und/oder (iv) gegebenenfalls eine nicht terminale -$CH_2CH_2CH_2$-Gruppe durch -4-1H-(1,2,3)Triazol-1-yl- ersetzt ist.

$Y^4$, $Y^5$ sind bevorzugt gleich oder verschieden O, $NR^x$, ~$NR^x$-C(O)-, ~C(O)-$NR^x$-, ~$NR^x$-CO-$NR^x$-, ~$NR^x$-S(O)2-, ~$S(O)_2$-$NR^x$-, ~$CH_2$-$NR^x$-C(O)-, ~$CH_2$-C(O)-$NR^x$- oder ~$CH_2$-$NR^x$-, wobei ~ die Bindung zu Gruppe A bezeichnet.

$A^3$ ist bevorzugt

a) $C_1$-$C_6$ Alkandiyl, $C_2$-$C_4$-Alkendiyl, $C_2$-$C_4$-Alkindiyl oder $C_2$-$C_4$-Alkadiendiyl wobei in den genannten Gruppen gegebenenfalls 1, 2 oder 3 $CH_2$-Gruppen durch O und/oder S ersetzt sind und wobei in den genannten Gruppen gegebenenfalls ein oder mehrere H-Atome durch F oder Cl ersetzt sind,

b) eine Gruppe $A^4$-$[Z^3$-$A^4]_n$ oder

c) 1,1'-Ferrocendiyl.

$A^4$, $A^5$ sind bevorzugt gleich oder verschieden

a) $C_6$-$C_{10}$-Aryldiyl, insbesondere Phenylen-1,4-diyl, Phenylen-1,3-diyl, Phenylen-1,2-diyl oder Naphthalin-1,4-

diyl;
b) $C_5$-$C_6$-Cycloalkyldiyl, insbesondere trans-Cyclobutan-1,3-diyl oder trans-Cyclohexan-1,4-diyl;
c) Thiophen-2,5-diyl, Furan-2,5-diyl, Piperazin-1,4-diyl, 1H-(1,2,3)Triazol-1,4-diyl, Pyridin-2,5-diyl, Pyridin-2,4-diyl, Pyridazin-2,5-diyl, Pyrimidin-2,5-diyl, Pyrimidin-2,4-diyl, Pyrazin-2,5-diyl oder Tetrazin-2,5-diyl;

wobei die genannten Reste unsubstituiert oder durch eine oder mehrere Gruppen $X^2$ substituiert sind.

$Z^3$ ist bevorzugt eine Bindung oder O.

$X^2$ ist bevorzugt gleich oder verschieden Fluor, Chlor, Brom, Nitro, $SO_3M$, $SO_2NH_2$, $PO_3M$, Alkyl, Halogenalkyl, Alkyloxy, Halogenalkyloxy, Alkylamino, Dialkylamino, Trialkylamino, Alkylcarbonyl, Alkylsulfoxyl, Alkylaminosulfoxyl, Dialkylaminosulfoxyl, Alkyloxycarbonyl, Alkylcarbonyloxy, Aminocarbonyl, Alkylaminocarbonyl, Alkylcarbonylamino, wobei die Alkylgruppen in diesen Resten 1 bis 4 Kohlenstoffatome enthalten und die Gruppen Halogenalkyl und Halogenalkyloxy die Halogene F und/oder Cl enthalten.

n ist bevorzugt 0 oder 1.

$R^1$ ist bevorzugt gleich und ist C(O)OM, $SO_3M$, $PO_3M_2$ oder C(O)NOM.

$R^2$, $R^3$ sind bevorzugt gleich H.

$R^4$, $R^7$ sind bevorzugt gleich OH oder $OR^z$,

$R^6$ ist bevorzugt gleich H oder $R^z$.

$R^5$ ist bevorzugt gleich $R^x$, $C(O)CH_2OH$ oder C(O)-Halogenalkyl.

$R^8$ ist bevorzugt gleich oder verschieden $R^x$.

M ist bevorzugt H, Methyl, Ethyl oder ein Kation aus der Gruppe der Alkalimetalle, Erdalkalimetalle und gegebenenfalls substituiertes Ammonium.

$R^x$ ist bevorzugt gleich oder verschieden H, $R^y$ oder $R^z$.

$R^y$ ist bevorzugt gleich oder verschieden Methyl, Ethyl, 1-Methylethyl, Propyl, 1-Methylpropyl, 1,1-Dimethylpropyl oder Benzyl.

$R^z$ ist bevorzugt gleich oder verschieden Methylcarbonyl, Ethylcarbonyl, Propylcarbonyl, 1-Methylethylcarbonyl, 1,1-Dimethylethylcarbonyl oder Phenylcarbonyl.

Bevorzugt bedeuten die in der Formel (I) angegebenen Definitionen der Symbole:

Alkyl: gesättigte, geradkettige, verzweigte oder cyclische Kohlenwasserstoffreste mit beispielsweise 1 bis 5 Kohlenstoffatomen, wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Dimethylpropyl, 1,1-Dimethylpropyl, 1-Ethylpropyl, Cyclopropyl, Cyclobutyl, 1-Methylcyclopropyl, 2-Methylcyclopropyl, Cyclopentyl, 2,2-Dimethylcylopropyl und 2,3- Dimethylcylopropyl;
Halogenalkyl: geradkettige, verzweigte oder cyclische Alkylgruppen mit beispielsweise 1 bis 4 Kohlenstoffatomen (wie vorstehend genannt), wobei in diesen Gruppen Wasserstoffatome teilweise oder vollständig durch Halogenatome ersetzt sind: wie Chlormethyl, Brommethyl, Dichlormethyl, Trichlormethyl, Fluormethyl, Difluormethyl, Trifluormethyl, Chlorfluormethyl, Dichlorfluormethyl, Chlordifluormethyl, 1-Chlorethyl, 1-Bromethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Pentafluorethyl, 1-Fluor-1-methyl-ethyl, 1-Fluorcyclopropyl, Heptafluorpropyl oder Nonafluorbutyl;
Alkinyl: geradkettige oder verzweigte Kohlenwasserstoffgruppen mit 2 bis 3 Kohlenstoffatomen und einer Dreifachbindung wie Ethinyl, 1-Propinyl und 2-Propinyl;
Alkyloxy: Alkyloxygruppen mit gesättigtem, geradkettigem, verzweigtem oder cyclischen Alkylrest, wobei dieser aus der vorstehend genannten Gruppe der Alkyle ist und 1 bis 3 Kohlenstoffatome enthält;

Halogenalkyloxy: Halogenalkyloxygruppen mit geradkettigem, verzweigtem oder cyclischem Halogenalkylrest, wobei dieser aus der vorstehend genannten Gruppe der Halogenalkyle ist und 1 bis 3 Kohlenstoffatome enthält;
Alkinyloxy: Alkinyloxygruppen mit geradkettigem oder verzweigtem Alkinylrest, wobei dieser aus der vorstehend genannten Gruppe der Alkinyle ist und 1 bis 3 Kohlenstoffatome enthält;
Alkylamino: Alkylaminogruppen mit gesättigtem, geradkettigem, verzweigtem oder cyclischem Alkylrest, wobei dieser aus der vorstehend genannten Gruppe der Alkyle ist und 1 bis 3 Kohlenstoffatome enthält;
Dialkylamino: Dialkylaminogruppen mit gesättigten, geradkettigen, verzweigten oder cyclischen Alkylresten, wobei diese gleich oder verschieden aus der vorstehend genannten Gruppe der Alkyle sind und 1 bis 3 Kohlenstoffatome enthalten;
Trialkylamino: Trialkylaminogruppen mit gesättigtem, geradkettigem, verzweigtem oder cyclischem Alkylresten, wobei diese gleich oder verschieden aus der vorstehend genannten Gruppe der Alkyle sind und 1 bis 3 Kohlenstoffatome enthalten;
Alkylcarbonyl: Alkylcarbonylgruppen mit gesättigtem, geradkettigem, verzweigtem oder cyclischem Alkylrest, wobei dieser aus der vorstehend genannten Gruppe der Alkyle ist und 1 bis 3 Kohlenstoffatome enthält;
Alkylsulfonyl: Alkylsulfonylgruppen mit gesättigtem, geradkettigem, verzweigtem oder cyclischem Alkylrest, wobei dieser aus der vorstehend genannten Gruppe der Alkyle ist und 1 bis 3 Kohlenstoffatome enthält;
Alkylsulfoxyl: Alkylsulfoxylgruppen mit gesättigtem, geradkettigem, verzweigtem oder cyclischem Alkylrest, wobei dieser aus der vorstehend genannten Gruppe der Alkyle ist und 1 bis 3 Kohlenstoffatome enthält;
Alkylaminocarbonyl: Alkylaminocarbonylgruppen mit gesättigtem, geradkettigem, verzweigtem oder cyclischem Alkylrest, wobei dieser aus der vorstehend genannten Gruppe der Alkyle ist und 1 bis 4 Kohlenstoffatome enthält;
Alkylcarbonylamino: Alkylcarbonylaminogruppen mit gesättigtem, geradkettigem, verzweigtem oder cyclischem Alkylrest, wobei dieser aus der vorstehend genannten Gruppe der Alkyle ist und 1 bis 4 Kohlenstoffatome enthält;
Alkandiyl bedeutet für Rest $A^3$: gesättigte, geradkettige oder verzweigte Alkandiylgruppe mit beispielsweise 1 bis 6 Kohlenstoffatomen, wie Methan-1,1-diyl, Ethan-1,2-diyl, Propan-1,3-diyl, Butan-1,4-diyl, Pentan-1,5-diyl und Hexan-1,6-diyl;
Alkandiyl bedeutet für Rest $T^1$ und $T^2$: gesättigte, geradkettige oder verzweigte Alkandiylgruppe mit beispielsweise 2 bis 21 Kohlenstoffatomen, wie Ethan-1,2-diyl, Propan-1,3-diyl, Butan-1,4-diyl, Pentan-1,5-diyl, Hexan-1,6-diyl, Heptan-1,7-diyl, Octan-1,8-diyl, Nonan-1,9-diyl, Decan-1,10-diyl, Undecan-1,11-diyl, Dodecan-1,12-diyl, Tridecan-1,13-diyl, Tetradecan-1,14-diyl, Pentadecan-1,15-diyl, Hexadecan-1,16-diyl, Heptadecan-1,17-diyl, Octadecan-1,18-diyl, Nonadecan-1,19-diyl, Eicosan-1,20-diyl oder Heneicosan-1,21-diyl;
Alkendiyl (z.B. für Rest $A^3$): ungesättigte, geradkettige oder verzweigte Alkendiylgruppen (einschließlich aller E und Z Stereoisomeren) mit beispielsweise 2 bis 4 Kohlenstoffatomen, wie Ethen-1,1-diyl, Prop-2-en-1,3-diyl und But-2-en-1,4-diyl;
Alkindiyl (z.B. für Rest $A^3$): ungesättigte, geradkettige oder verzweigte Alkindiylgruppen mit beispielsweise 2 bis 4 Kohlenstoffatomen, wie Ethin-1,1-diyl, Prop-2-in-1,3-diyl und But-2-in-1,4-diyl;
Alkadiendiyl (z.B. für Rest $A^3$): ungesättigte, geradkettige oder verzweigte Alkadiendiylgruppe (einschließlich aller E und Z Stereoisomeren) mit beispielsweise 4 bis 6 Kohlenstoffatomen wie But-1,3-dien-1,4-diyl, 3,4-Dimethylbut-1,3-dien-1,4-diyl und Hex-2,4-dien-1,6-diyl.

Bevorzugt sind Verbindungen der Formel (I), bei denen alle Symbole, Definitionen und Indizes die bevorzugten Bedeutungen haben.

Besonders bevorzugt haben die Symbole und Indizes in der Formel (I) folgende Bedeutungen:

$A^1$, $A^2$ sind besonders bevorzugt gleich oder verschieden eine Gruppe $D^1$-[$Y^3$-$D^2$-]$_m$-.

$D^1$ ist besonders bevorzugt gleich oder verschieden Phenyl, 1-Naphthyl, 2-Naphtyl, 2-Biphenylen, 2-Fluorenyl, 3-Fluorenyl, 2-Phenanthrenyl, Cyclopropyl, Cyclobutyl, Cyclopentyl, Cyclohexyl, 2-Piperidinyl, 3-Piperidinyl, 4-Piperidinyl, 1,3-Dioxan-5-yl, 4-Tetrahydropyranyl, 3-Hexahydropyridazinyl, 4-Hexahydropyridazinyl, 2-Hexahydropyrimidinyl, 4-Hexahydropyrimidinyl, 5-Hexahydropyrimidinyl, 1-Piperazinyl, 2-Piperazinyl, 4-Morpholinyl, 2-Furyl, 3-Furyl, 2-Thienyl, 3-Thienyl, 2-Pyrrolyl, 3-Pyrrolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, 1,2,3-Triazol-1-yl, 1,2,3-Triazol-4-yl, 1,2,3-Triazol-5-yl, 1,3,4-Triazol-2-yl, 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1-Benzofuran-2-yl, 1-Benzofuran-5-yl, 1-Benzofuran-6-yl, 2-Benzofuran-5-yl, 2-Benzofuran-6-yl, 2H-Chromen-3-yl, 2H-Chromen-6-yl, 2H-Chromen-7-yl, Indol-2-yl, 1-Benzothiophen-2-yl oder Benzimidazol-2-yl,

wobei die genannten Gruppen unsubstituiert oder durch eine oder mehrere Gruppen $X^1$ substituiert sind.

$D^2$ ist besonders bevorzugt gleich oder verschieden Phenylen-1,4-diyl, Phenylen-1,3-diyl Biphenyl-4,4'-diyl, Naphthalin-1,4-diyl, trans-Cyclohexan-1,4-diyl, Furan-2,5-diyl, Thiophen-2,4-diyl, Thiophen-2,5-diyl, 1H(1,2,3)-Triazol-1,4-diyl oder Pyrrol-2,5-diyl, Pyridin-2,5-diyl, Pyridin-2,4-diyl, Pyridazin-3,6-diyl, Pyrimidin-2,5-diyl, Pyrimidin-2,6-diyl, Pyrazin-2,5-diyl oder Tetrazin-3,5-diyl,

wobei die genannten Gruppen unsubstituiert oder durch eine oder eine Gruppe $X^1$ substituiert sind.

$X^1$ ist besonders bevorzugt gleich oder verschieden F, Cl, Br, I, Cyano, Nitro, Hydroxy, Amino, Carboxyl, Azido, $SO_3M$, $SO_2NH_2$, Alkyl, Halogenalkyl, Alkyloxy, Halogenalkyloxy, Alkylamino, Dialkylamino, Trialkylamino, Alkylcarbonyl, Alkylsulfoxyl, Aminocarbonyl, Alkylaminocarbonyl, Alkylcarbonylamino, Oxo (=O), wobei die Alkylgruppen in diesen Resten 1 bis 6 Kohlenstoffatome.

$Y^1$, $Y^2$ sind besonders bevorzugt gleich oder verschieden ~$CH_2$-, ~C(O)-, ~$CH_2$-C(O)-, ~$CH_2CH_2C(O)$-, ~CH=CH-C(O)-, ~C≡C-C(O)-, ~NH-C(O)-, ~$CH_2$-$NR^x$-3-Cyclobuten-1,2-dion-4-, ~$S(O)_2$- oder ~$CH_2S(O)_2$- wobei ~ die Bindung zur Gruppe A bezeichnet oder

$Y^1$, $Y^2$ bilden, unabhängig voneinander, zusammen mit dem Stickstoff, an den sie gebunden sind, und der Gruppe $R^8$ eine Gruppe ~4-1H-(1,2,3)Triazol-1-yl- wobei ~ die Bindung zur Gruppe A bezeichnet;

$Y^3$ ist besonders bevorzugt eine Bindung, O, $S(O_2)$, $CH_2$ oder C(O).

m ist besonders bevorzugt 0, 1 oder 2.

$Z^1$ ist besonders bevorzugt O.

D ist besonders bevorzugt eine Gruppe $Z^2$-$T^1$-$Y^4$-$A^3$-$Y^5$-$T^2$-$Z^2$.

$Z^2$ ist besonders bevorzugt gleich O, S, ~4-1H-(1,2,3)Triazol-yl-, -NH-C(O)~ oder $CH_2$, wobei ~ die Bindung zur Gruppe T bezeichnet.

$T^1$, $T^2$ sind besonders bevorzugt gleich oder verschieden eine geradkettige oder verzweigte Alkandiylgruppe mit 4 bis 20 C-Atomen, wobei

(i) gegebenenfalls eine oder mehrere nicht terminale $CH_2$-Gruppen durch -S-, -S(O)-, -$S(O)_2$-, und/oder -$NR^x$- ersetzt sind und /oder
(ii) gegebenenfalls ein oder mehrere H-Atome durch Fluor, Chlor, (=O), Carboxyl, $NH_2$, oder $NHR^z$ ersetzt sind und/oder (iv) gegebenfalls eine nicht terminale -$CH_2CH_2CH_2$-Gruppe durch -4-1H-(1,2,3)Triazol-1-yl- ersetzt ist.

$Y^4$, $Y^5$ sind besonders bevorzugt gleich oder verschieden ~$NR^x$-C(O)-, ~C(O)-$NR^x$, ~$NR^x$-$S(O)_2$, ~$NR^x$-, ~$CH_2NR^x$, ~$NR_x$C(O)$NR^x$-, ~$CH_2NR^x$C(O)-, ~$CH_2$C(O)$NR^x$- oder ~$S(O)_2$-$NR^x$- wobei ~ die Bindung zu Gruppe A bezeichnet.

$A^3$ ist besonders bevorzugt

a) $C_1$-$C_6$ Alkandiyl, $C_2$-$C_4$-Alkindiyl wobei in den genannten Gruppen gegebenenfalls ein oder mehrere H-Atome durch F oder Cl ersetzt sind, oder

b) eine Gruppe $A^4$

$A^4$ ist besonders bevorzugt gleich Phenylen-1,4-diyl, Biphenyl-4,4'-diyl, Naphthalin-1,4-diyl, trans-Cyclohexan-1,4-diyl, Thiophen-2,5-diyl oder 1H-(1,2,3)Triazol-1,4-diyl, Pyridin-2,5-diyl, Pyridin-2,4-diyl, Pyridazin-2,5-diyl, Pyrimidin-2,5-diyl, Pyrimidin-2,4-diyl, Pyrazin-2,5-diyl oder Tetrazin-2,5-diyl, wobei die genannten Reste unsubstituiert oder durch eine Gruppe $X^2$ substituiert sind.

$X^2$ ist besonders bevorzugt gleich oder verschieden Fluor, Chlor, Brom, Nitro, $SO_3M$, Alkyl, Halogenalkyl, Alkyloxy, Halogenalkyloxy, Alkylamino, Dialkylamino, Trialkylamino, Alkylcarbonyl, Alkylsulfoxyl, Alkylcarbonyloxy, Aminocarbonyl, Alkylaminocarbonyl, Alkylcarbonylamino, wobei die Alkylgruppen in diesen Resten 1 bis 4 Kohlenstoffatome enthalten und die Gruppen Halogenalkyl und Halogenalkyloxy die Halogene F und/oder Cl enthalten;

$R^1$ ist besonders bevorzugt gleich und ist C(O)OM oder C(O)NOM.

$R^2$, $R^3$ sind besonders bevorzugt gleich H.

$R^4$, $R^7$ sind besonders bevorzugt gleich OH oder $OR^z$;

$R^6$ ist besonders bevorzugt gleich H oder $R^z$.

$R^5$ ist besonders bevorzugt gleich $R^x$, $C(O)CH_2OH$ oder C(O)-Halogenalkyl.

$R^8$ ist besonders bevorzugt gleich oder verschieden $R^x$.

M ist besonders bevorzugt H, Methyl, Ethyl oder ein Kation aus der Gruppe Li, Na, K, Ca, Mg und gegebenenfalls substituiertes Ammonium.

$R^x$ ist besonders bevorzugt gleich oder verschieden H, $R^y$ oder $R^z$.

$R^y$ ist besonders bevorzugt gleich oder verschieden Methyl, Ethyl, 1-Methylethyl, Propyl, 1-Methylpropyl, 1,1-Dimethylpropyl oder Benzyl.

$R^z$ ist besonders bevorzugt gleich oder verschieden Methylcarbonyl, Ethylcarbonyl, Propylcarbonyl, 1-Methylethylcarbonyl, 1,1-Dimethylethylcarbonyl oder Phenylcarbonyl.

Besonders bevorzugt bedeuten die in der Formel (I) angegebenen Definitionen der Symbole:

Alkyl: gesättigte, geradkettige, verzweigte oder cyclische Kohlenwasserstoffreste mit beispielsweise 1 bis 4 Kohlenstoffatomen, wie Methyl, Ethyl, Propyl, 1-Methylethyl, Butyl, 1-Methyl-propyl, 2-Methylpropyl, 1,1-Dimethylethyl, Cyclopropyl, Cyclobutyl, 1-Methylcyclopropyl, 2-Methylcyclopropyl;
Halogenalkyl: geradkettige, verzweigte oder cyclische Alkylgruppen mit beispielsweise 1 bis 3 Kohlenstoffatomen (wie vorstehend genannt), wobei in diesen Gruppen Wasserstoffatome teilweise oder vollständig durch Halogenatome ersetzt sind: wie Chlormethyl, Brommethyl, Dichlormethyl, Trichlormethyl, Fluormethyl, Difluormethyl, Trifluormethyl, Chlorfluormethyl, Dichlorfluormethyl, Chlordifluormethyl, 1-Chlorethyl, 1-Bromethyl, 1-Fluorethyl, 2-Fluorethyl, 2,2-Difluorethyl, 2,2,2-Trifluorethyl, 2-Chlor-2-fluorethyl, 2,2,2-Trichlorethyl, Pentafluorethyl, 1-Fluor-1-methyl-ethyl, 1-Fluorcyclopropyl und Heptafluorpropyl;
Alkinyl: geradkettige oder verzweigte Kohlenwasserstoffgruppen mit 2 bis 3 Kohlenstoffatomen und einer Dreifachbindung wie Ethinyl, 1-Propinyl und 2-Propinyl;
Alkyloxy: Alkyloxygruppen mit gesättigtem, geradkettigem, verzweigtem oder cyclischen Alkylrest, wobei dieser aus der vorstehend genannten Gruppe der Alkyle ist und 1 bis 3 Kohlenstoffatome enthält;
Halogenalkyloxy: Halogenalkyloxygruppen mit geradkettigem, verzweigtem oder cyclischem Halogenalkylrest, wobei dieser aus der vorstehend genannten Gruppe der Halogenalkyle ist und 1 bis 2 Kohlenstoffatome enthält;
Alkylamino: Alkylaminogruppen mit gesättigtem, geradkettigem, verzweigtem oder cyclischem Alkylrest, wobei dieser aus der vorstehend genannten Gruppe der Alkyle ist und 1 bis 3 Kohlenstoffatome enthält;
Dialkylamino: Dialkylaminogruppen mit gesättigten, geradkettigen, verzweigten oder cyclischen Alkylresten, wobei diese gleich oder verschieden aus der vorstehend genannten Gruppe der Alkyle sind und 1 bis 2 Kohlenstoffatome enthalten;
Trialkylamino: Trialkylaminogruppen mit gesättigtem, geradkettigem, verzweigtem oder cyclischem Alkylresten, wobei diese gleich oder verschieden aus der vorstehend genannten Gruppe der Alkyle sind und 1 bis 2 Kohlenstoffatome enthalten;
Alkylcarbonyl: Alkylcarbonylgruppen mit gesättigtem, geradkettigem, verzweigtem oder cyclischem Alkylrest, wobei dieser aus der vorstehend genannten Gruppe der Alkyle ist und 1 bis 2 Kohlenstoffatome enthält;
Alkylsulfoxyl: Alkylsulfoxylgruppen mit gesättigtem, geradkettigem, verzweigtem oder cyclischem Alkylrest, wobei dieser aus der vorstehend genannten Gruppe der Alkyle ist und 1 bis 2 Kohlenstoffatome enthält;
Alkylaminocarbonyl: Alkylaminocarbonylgruppen mit gesättigtem, geradkettigem, verzweigtem oder cyclischem Alkylrest, wobei dieser aus der vorstehend genannten Gruppe der Alkyle ist und 1 bis 3 Kohlenstoffatome enthält;
Alkylcarbonylamino: Alkylcarbonylaminogruppen mit gesättigtem, geradkettigem, verzweigtem oder cyclischem Alkylrest, wobei dieser aus der vorstehend genannten Gruppe der Alkyle ist und 1 bis 3 Kohlenstoffatome enthält;
Alkandiyl bedeutet für Rest $A^3$: gesättigte, geradkettige oder verzweigte Alkandiylgruppe mit beispielsweise 1 bis 6 Kohlenstoffatomen, wie Methan-1,1-diyl, Ethan-1,2-diyl, Propan-1,3-diyl, Butan-1,4-diyl, Pentan-1,5-diyl und Hexan-1,6-diyl;
Alkandiyl bedeutet für Rest $T^1$ und $T^2$: gesättigte, geradkettige oder verzweigte Alkandiylgruppe mit beispielsweise 2 bis 21 Kohlenstoffatomen, wie Ethan-1,2-diyl, Propan-1,3-diyl, Butan-1,4-diyl, Pentan-1,5-diyl, Hexan-1,6-diyl, Heptan-1,7-diyl, Octan-1,8-diyl, Nonan-1,9-diyl, Decan-1,10-diyl, Undecan-1,11-diyl, Dodecan-1,12-diyl, Tridecan-1,13-diyl, Tetradecan-1,14-diyl, Pentadecan-1,15-diyl. Hexadecan-1,16-diyl, Heptadecan-1,17-diyl, Octadecan-1,18-diyl, Nonadecan-1,19-diyl, Eicosan-1,20-diyl oder Heneicosan-1,21-diyl;
Alkindiyl (z.B. für Rest $A^3$): ungesättigte, geradkettige oder verzweigte Alkindiylgruppen mit beispielsweise 2 bis 3 Kohlenstoffatomen, wie Ethin-1,1-diyl, Prop-2-in-1,3-diyl;

Besonders bevorzugt sind Verbindungen der Formel (I), bei denen alle Symbole und Indizes die besonders bevorzugten Bedeutungen haben.

Ganz besonders bevorzugt haben die Symbole und Indizes in der Formel (I) folgende Bedeutungen:

$A^1$, $A^2$ sind ganz besonders bevorzugt gleich eine Gruppe $D^1$-($Y^3$-$D^2$-$]_m$-.

$D^1$ ist ganz besonders bevorzugt gleich Phenyl, 2-Naphtyl, Cyclohexyl, 2-Fluorenyl, 2-Furanyl, 2-Benzothiophenyl, 2-Thienyl, 3-Thienyl, 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl oder 2-Pyrazinyl, wobei die genannten Gruppen unsubstituiert oder durch eine Gruppe $X^1$ substituiert sind.

$D^2$ ist ganz besonders bevorzugt gleich Phenylen-1,4-diyl, Naphthalin-1,4-diyl, Thiophen-2,5-diyl, Pyridin-2,5-diyl, Pyridazin-3,6-diyl, Pyrimidin-2,5-diyl oder Pyrazin-2,5-diyl,

wobei die genannten Gruppen unsubstituiert oder durch eine Gruppe $X^1$ substituiert sind.

$x^1$ ist ganz besonders bevorzugt gleich F, Cl, Br, Nitro, Hydroxy, Carboxyl, Alkyl, Halogenalkyl, Alkyloxy, Halogenalkyloxy, Alkylamino, Dialkylamino, Trialkylamino, Alkylcarbonyl, Alkylsulfoxyl, Aminocarbonyl, Alkylaminocarbonyl, Alkylcarbonylamino, wobei die Alkylgruppen in diesen Resten 1 bis 4 Kohlenstoffatome enthalten.

$Y^1$, $Y^2$ sind ganz besonders bevorzugt gleich ~$CH_2$-, ~C(O)-, ~$CH_2$-C(O)-, ~C≡C-C(O)-, ~CH=CH-C(O)-, ~$CH_2$-$NR^x$-3-Cyclobuten-1,2-dion-4-oder ~$S(O)_2$-, wobei ~ die Bindung zur Gruppe A bezeichnet oder

$Y^1$, $Y^2$ bilden, unabhängig voneinander, zusammen mit dem Stickstoff, an den sie gebunden sind, und der Gruppe $R^8$ eine Gruppe ~4-1H-(1,2,3)Triazol-1-yl-, wobei ~ die Bindung zur Gruppe A bezeichnet.

$Y^3$ ist ganz besonders bevorzugt eine Bindung oder O.

m ist ganz besonders bevorzugt 0 oder 1.

$Z^1$ ist ganz besonders bevorzugt O.

D ist ganz besonders bevorzugt eine Gruppe $Z^2$-$T^1$-$Y^4$ -$A^3$-$Y^5$-$T^2$-$Z^2$.

$Z^2$ ist ganz besonders bevorzugt gleich O, ~4-1H-(1,2,3)Triazol-1-yl-, -NH-C(O)~ oder $CH_2$, wobei ~ die Bindung zur Gruppe T bezeichnet.

$T^1$, $T^2$ sind ganz besonders bevorzugt gleich oder verschieden eine geradkettige Alkandiylgruppe mit 4 bis 10 C-Atomen, wobei

(i) gegebenenfalls eine oder mehrere nicht terminale $CH_2$-Gruppen durch -S- und/oder -NH- ersetzt sind und/oder
(ii) gegebenenfalls ein oder mehrere H-Atome durch F, Cl, Carboxyl, $NH_2$, oder (=O) ersetzt sind.

$Y^4$, $Y^5$ sind ganz besonders bevorzugt gleich ~$NR^x$-C(O)-, ~$NR^x$-, ~$CH_2NR^x$-, ~$NR^xC(O)NR^x$-, ~$CH_2NR^xC(O)$-, ~$CH_2C(O)NR^x$- oder ~C(O)-$NR^x$-, wobei ~ die Bindung zu Gruppe A bezeichnet.

$A^3$ ist ganz besonders bevorzugt

a) $C_1$-$C_6$ Alkandiyl wobei in den genannten Gruppen gegebenenfalls ein oder mehrere H-Atome durch F oder Cl ersetzt sind, oder

b) eine Gruppe $A^4$.

$A^4$ ist ganz besonders bevorzugt gleich Phenylen-1,4-diyl, Biphenyl-4,4'-diyl, Naphthalin-1,4-diyl, Pyridin-2,5-diyl, Pyridazin-2,5-diyl, Pyrimidin-2,5-diyl, Pyrazin-2,5-diyl, Tetrazin-2,5-diyl, Ethin-1,2-diyl, Thiophen-2,5-diyl, Cyclohex-1,4-diyl oder -3-Cyclobuten-1,2-dion-4-; wobei die genannten Reste unsubstituiert oder durch eine Gruppe $X^2$ substituiert sind.

$X^2$ ist ganz besonders bevorzugt gleich Fluor, Chlor, Brom, Nitro, $SO_3M$, Alkyl, Halogenalkyl, Alkyloxy, Halogenalkyloxy, Alkylamino, Dialkylamino, Trialkylamino, Alkylsulfoxyl, Alkylcarbonyloxy, wobei die Alkylgruppen in diesen Resten 1 bis 4 Kohlenstoffatome enthalten und die Gruppen Halogenalkyl und Halogenalkyloxy die Halogene F und/oder Cl enthalten;

$R^1$ ist ganz besonders bevorzugt gleich und ist C(O)OM.

$R^2$, $R^3$ sind ganz besonders bevorzugt gleich H.

$R^4$, $R^7$ sind ganz besonders bevorzugt gleich OH oder $OR^z$;

$R^6$ ist ganz besonders bevorzugt gleich H oder $R^z$.

$R^5$ ist ganz besonders bevorzugt gleich $R^x$, $C(O)CH_2OH$ oder C(O)-Halogenalkyl

$R^8$ ist ganz besonders bevorzugt gleich $R^x$.

M ist ganz besonders bevorzugt H, Methyl, Ethyl oder ein Kation aus der Gruppe Li, Na und K.

$R^x$ ist ganz besonders bevorzugt gleich oder verschieden H, $R^y$ oder $R^z$.

$R^y$ ist ganz besonders bevorzugt gleich oder verschieden Methyl oder Ethyl.

$R^z$ ist ganz besonders bevorzugt gleich Methylcarbonyl, Ethylcarbonyl oder 1-Methylethylcarbonyl.

Ganz besonders bevorzugt bedeuten die in der Formel (I) angegebenen Definitionen der Symbole:

Alkyl: gesättigte, geradkettige, verzweigte oder cyclische Kohlenwasserstoffreste mit beispielsweise 1 bis 4 Kohlenstoffatomen, wie Methyl, Ethyl, Propyl, 1-Methylethyl, Butyl, 1-Methyl-propyl, 2-Methylpropyl, 1,1-Dimethylethyl, Cyclopropyl, Cyclobutyl, 1-Methylcyclopropyl, 2-Methylcyclopropyl;
Halogenalkyl: geradkettige, verzweigte oder cyclische Alkylgruppen mit beispielsweise 1 bis 3 Kohlenstoffatomen (wie vorstehend genannt), wobei in diesen Gruppen Wasserstoffatome teilweise oder vollständig durch Halogenatome ersetzt sind: wie Chlormethyl, Dichlormethyl, Trichlormethyl, Fluormethyl, Difluormethyl, Trifluormethyl, Dichlorfluormethyl, Chlordifluormethyl, 1-Chlorethyl, 1-Bromethyl, 1-Fluorethyl, 2-Fluorethyl, 2,2-Difluorethyl, 2,2,2-Trifluorethyl, 2,2,2-Trichlorethyl, Pentafluorethyl, 1-Fluor-1-methyl-ethyl, 1-Fluorcyclopropyl und Heptafluorpropyl;
Alkyloxy: Alkyloxygruppen mit gesättigtem, geradkettigem, verzweigtem oder cyclischen Alkylrest, wobei dieser aus der vorstehend genannten Gruppe der Alkyle ist und 1 bis 2 Kohlenstoffatome enthält;
Halogenalkyloxy: Halogenalkyloxygruppen mit geradkettigem, verzweigtem oder cyclischem Halogenalkylrest, wobei dieser aus der vorstehend genannten Gruppe der Halogenalkyle ist und 1 bis 2 Kohlenstoffatome enthält;
Alkylamino: Alkylaminogruppen mit gesättigtem, geradkettigem, verzweigtem oder cyclischem Alkylrest, wobei dieser aus der vorstehend genannten Gruppe der Alkyle ist und 1 bis 2 Kohlenstoffatome enthält;
Dialkylamino: Dialkylaminogruppen mit gesättigten, geradkettigen, verzweigten oder cyclischen Alkylresten, wobei diese gleich oder verschieden aus der vorstehend genannten Gruppe der Alkyle sind und 1 bis 2 Kohlenstoffatome enthalten;
Trialkylamino: Trialkylaminogruppen mit gesättigtem, geradkettigem, verzweigtem oder cyclischem Alkylresten, wobei diese gleich oder verschieden aus der vorstehend genannten Gruppe der Alkyle sind und 1 bis 2 Kohlenstoffatome

enthalten;
Alkylcarbonyl: Alkylcarbonylgruppen mit gesättigtem, geradkettigem, verzweigtem oder cyclischem Alkylrest, wobei dieser aus der vorstehend genannten Gruppe der Alkyle ist und 1 bis 2 Kohlenstoffatome enthält;
Alkylsulfoxyl: Alkylsulfoxylgruppen mit gesättigtem, geradkettigem, verzweigtem oder cyclischem Alkylrest, wobei dieser aus der vorstehend genannten Gruppe der Alkyle ist und 1 bis 2 Kohlenstoffatome enthält;
Alkylaminocarbonyl: Alkylaminocarbonylgruppen mit gesättigtem, geradkettigem, verzweigtem oder cyclischem Alkylrest, wobei dieser aus der vorstehend genannten Gruppe der Alkyle ist und 1 bis 2 Kohlenstoffatome enthält;
Alkylcarbonylamino: Alkylcarbonylaminogruppen mit gesättigtem, geradkettigem, verzweigtem oder cyclischem Alkylrest, wobei dieser aus der vorstehend genannten Gruppe der Alkyle ist und 1 bis 2 Kohlenstoffatome enthält;
Alkandiyl bedeutet für Rest $A^3$: gesättigte, geradkettige oder verzweigte Alkandiylgruppe mit beispielsweise 1 bis 6 Kohlenstoffatomen, wie Methan-1,1-diyl, Ethan-1,2-diyl, Propan-1,3-diyl, Butan-1,4-diyl, Pentan-1,5-diyl und Hexan-1,6-diyl;
Alkandiyl bedeutet für Rest $T^1$ und $T^2$: gesättigte, geradkettige oder verzweigte Alkandiylgruppe mit beispielsweise 2 bis 21 Kohlenstoffatomen, wie Ethan-1,2-diyl, Propan-1,3-diyl, Butan-1,4-dlyl, Pentan-1,5-diyl, Hexan-1,6-diyl, Heptan-1,7-diyl, Octan-1,8-diyl, Nonan-1,9-diyl, Decan-1,10-diyl, Undecan-1,11-diyl, Dodecan-1,12-diyl, Tridecan-1,13-diyl, Tetradecan-1,14-diyl, Pentadecan-1,15-diyl, Hexadecan-1,16-diyl, Heptadecan-1,17-diyl, Octadecan-1,18-diyl, Nonadecan-1,19-diyl, Eicosan-1,20-diyl oder Heneicosan-1,21-diyl.

Ganz besonders bevorzugt sind Verbindungen der Formel (I), bei denen alle Symbole, Definitionen und Indizes die ganz besonders bevorzugten Bedeutungen haben.
Stark bevorzugt haben die Symbole und Indizes in der Formel (I) folgende Bedeutungen:

$A^1$, $A^2$ sind stark bevorzugt gleich eine Gruppe $D^1$-[$Y^3$-$D^2$-]$_m$-.

$D^1$ ist stark bevorzugt gleich Phenyl, Cyclohexyl, Napht-2-yl, Fluoren-2-yl, Furan-2-yl, Benzothiophen-2-yl, Thien-2-yl, Thien-3-yl, Pyridin-2-yl, Pyridin-3-yl, Pyridin-4-yl, Pyridazin-3-yl, Pyridazin-4-yl, Pyrimidin-2-yl, Pyrimidin-4-yl, Pyrimidin-5-yl oder Pyrazin-2-yl, wobei die genannten Gruppen unsubstituiert oder durch eine Gruppe $X^1$ substituiert sind.

$D^2$ ist stark bevorzugt gleich Phenylen-1,4-diyl, Naphthalin-1,4-diyl, Thiophen-2,5-diyl, Pyridin-2,5-diyl, Pyridazin-3,6-diyl, Pyrimidin-2,5-diyl oder Pyrazin-2,5-diyl,

wobei die genannten Gruppen unsubstituiert oder durch eine Gruppe $X^1$ substituiert sind.

$X^1$ ist stark bevorzugt gleich F, Cl, Nitro, Hydroxy, Carboxyl, Alkyl, Halogenalkyl, Alkyloxy, Halogenalkyloxy, Alkylamino, Dialkylamino, Trialkylamino, Alkylsulfoxyl, Aminocarbonyl, Alkylaminocarbonyl, Alkylcarbonylamino, wobei die Alkylgruppen in diesen Resten 1 bis 3 Kohlenstoffatome enthalten.

$Y^1$, $Y^2$ sind stark bevorzugt gleich ~$CH_2$-, ~C(O)-, ~$CH_2$-C(O)-, ~C≡C-C(O)-, ~CH=CH-C(O)-, ~$CH_2$-$NR^x$-3-Cyclobuten-1,2-dion-4- oder ~$S(O)_2$-, wobei ~ die Bindung zur Gruppe A bezeichnet oder

$Y^1$, $Y^2$ bilden, unabhängig voneinander, zusammen mit dem Stickstoff, an den sie gebunden sind, und der Gruppe $R^8$ eine Gruppe ~4-1H-(1,2,3)Triazol-1-yl-, wobei ~ die Bindung zur Gruppe A bezeichnet.

$Y^3$ ist stark bevorzugt eine Bindung.

m ist stark bevorzugt 0 oder 1.

$Z^1$ ist stark bevorzugt O.

D ist stark bevorzugt eine Gruppe $Z^2$ -$T^1$-$Y^4$ -$A^3$-$Y^5$-$T^2$-$Z^2$.

$Z^2$ ist stark bevorzugt gleich O, $CH_2$ oder ~4-1H-(1,2,3)Triazol-1-yl-, wobei ~ die Bindung zur Gruppe T bezeichnet.

$T^1$, $T^2$ sind stark bevorzugt gleich oder verschieden eine geradkettige Alkandiylgruppe mit 4 bis 6 C-Atomen, wobei

(i) gegebenenfalls eine oder mehrere nicht terminale $CH_2$-Gruppen durch -S-, und/oder NH ersetzt sind und/oder

(ii) gegebenenfalls ein oder mehrere H-Atome durch F, Cl, Carboxyl, $NH_2$, oder (=O) ersetzt sind.

$Y^4$, $Y^3$ sind stark bevorzugt gleich oder verschieden ~C(O)-$NR^x$-, ~$NR^X$-, ~$CH_2NR^x$-, ~NHC(O)NH-, ~$CH_2$NHC(O)-, ~$CH_2$C(O)NH- oder ~$NR^x$-C(O)-, wobei ~ die Bindung zu Gruppe A bezeichnet.

$A^3$ ist stark bevorzugt a) $C_1$-$C_6$ Alkandiyl, wobei in den genannten Gruppen gegebenenfalls ein oder mehrere H-Atome durch F oder Cl ersetzt sind, oder
b) eine Gruppe $A^4$.

$A^4$ ist stark bevorzugt gleich Phenylen-1,4-diyl, Biphenyl-4,4'-diyl, Naphthalin-1,4-diyl, Pyridin-2,5-diyl, Pyridazin-2,5-diyl, Pyrimidin-2,5-diyl, Pyrazin-2,5-diyl, Tetrazin-2,5-diyl, Ethin-1,2-diyl, Thiophen-2,5-diyl, Cyclohex-1,4-diyl oder -3-Cyclobuten-1,2-dion-4-; wobei die genannten Reste unsubstituiert oder durch eine Gruppe $X^2$ substituiert sind.

$X^2$ ist stark bevorzugt gleich Fluor, Chlor, Brom, Nitro, $SO_3M$, Alkyl, Halogenalkyl, Alkyloxy, Halogenalkyloxy, Alkylamino, Dialkylamino, Trialkylamino, Alkylsulfoxyl, Alkylcarbonyloxy, wobei die Alkylgruppen in diesen Resten 1 bis 3 Kohlenstoffatome enthalten und die Gruppen Halogenalkyl und Halogenalkyloxy die Halogene F und/oder Cl enthalten;

$R^1$ ist stark bevorzugt C(O)OM.

$R^2$, $R^3$ sind stark bevorzugt gleich H.

$R^4$, $R^7$ sind stark bevorzugt gleich OH oder $OR^z$;

$R^6$ ist stark bevorzugt gleich H oder $R^z$.

$R^5$ ist stark bevorzugt gleich $R^x$, C(O)$CH_2$OH oder C(O)-Halogenalkyl.

$R^8$ ist stark bevorzugt gleich H.

M ist stark bevorzugt H, Methyl oder ein Kation aus der Gruppe Li, Na und K.

$R^x$ ist stark bevorzugt gleich oder verschieden H, $R^y$ oder $R^z$.

$R^y$ ist stark bevorzugt gleich oder verschieden Methyl oder Ethyl.

$R^z$ ist stark bevorzugt gleich Methylcarbonyl, Ethylcarbonyl oder 1-Methylethylcarbonyl.

Stark bevorzugt bedeuten die in der Formel (I) angegebenen Definitionen der Symbole:

Alkyl: gesättigte, geradkettige, verzweigte oder cyclische Kohlenwasserstoffreste mit beispielsweise 1 bis 3 Kohlenstoffatomen, wie Methyl, Ethyl, Propyl, 1-Methylethyl oder Cyclopropyl;
Halogenalkyl: geradkettige, verzweigte oder cyclische Alkylgruppen mit beispielsweise 1 bis 2 Kohlenstoffatomen (wie vorstehend genannt), wobei in diesen Gruppen Wasserstoffatome teilweise oder vollständig durch Halogenatome ersetzt sind: wie Chlormethyl, Dichlormethyl, Trichlormethyl, Fluormethyl, Difluormethyl, Trifluormethyl, Dichlorfluormethyl, Chlordifluormethyl, 1-Chlorethyl, 1-Bromethyl, 1-Fluorethyl, 2-Fluorethyl, 2,2-Difluorethyl, 2,2,2-Trifluorethyl, 2,2,2-Trichlorethyl oder Pentafluorethyl;
Alkyloxy: Alkyloxygruppen mit gesättigtem, geradkettigem, verzweigtem oder cyclischen Alkylrest, wobei dieser aus der vorstehend genannten Gruppe der Alkyle ist und 1 bis 2 Kohlenstoffatome enthält;
Halogenalkyloxy: Halogenalkyloxygruppen mit geradkettigem, verzweigtem oder cyclischem Halogenalkylrest, wobei dieser aus der vorstehend genannten Gruppe der Halogenalkyle ist und 1 bis 2 Kohlenstoffatome enthält;
Alkylamino: Alkylaminogruppen mit gesättigtem, geradkettigem, verzweigtem oder cyclischem Alkylrest, wobei dieser aus der vorstehend genannten Gruppe der Alkyle ist und 1 bis 2 Kohlenstoffatome enthält;
Dialkylamino: Dialkylaminogruppen mit gesättigten, geradkettigen, verzweigten oder cyclischen Alkylresten, wobei diese gleich oder verschieden aus der vorstehend genannten Gruppe der Alkyle sind und 1 bis 2 Kohlenstoffatome enthalten;
Trialkylamino: Trialkylaminogruppen mit gesättigtem, geradkettigem, verzweigtem oder cyclischem Alkylresten, wobei diese gleich oder verschieden aus der vorstehend genannten Gruppe der Alkyle sind und 1 bis 2 Kohlenstoffatome enthalten;
Alkylsulfoxyl: Alkylsulfoxylgruppen mit gesättigtem, geradkettigem, verzweigtem oder cyclischem Alkylrest, wobei dieser aus der vorstehend genannten Gruppe der Alkyle ist und 1 bis 2 Kohlenstoffatome enthält;
Alkylaminocarbonyl: Alkylaminocarbonylgruppen mit gesättigtem, geradkettigem, verzweigtem oder cyclischem Alkylrest, wobei dieser aus der vorstehend genannten Gruppe der Alkyle ist und 1 bis 2 Kohlenstoffatome enthält;
Alkylcarbonylamino: Alkylcarbonylaminogruppen mit gesättigtem, geradkettigem, verzweigtem oder cyclischem Alkylrest, wobei dieser aus der vorstehend genannten Gruppe der Alkyle ist und 1 bis 2 Kohlenstoffatome enthält;
Alkandiyl bedeutet für Rest $A^3$: gesättigte, geradkettige oder verzweigte Alkandiylgruppe mit beispielsweise 1 bis 6 Kohlenstoffatomen, wie Methan-1,1-diyl, Ethan-1,2-diyl, Propan-1,3-diyl, Butan-1,4-diyl, Pentan-1,5-diyl und Hexan-1,6-diyl;
Alkandiyl bedeutet für Rest $T^1$ und $T^2$: gesättigte, geradkettige oder verzweigte Alkandiylgruppe mit beispielsweise 4 bis 21 Kohlenstoffatomen wie Butan-1,4-diyl, Pentan-1,5-diyl, Hexan-1,6-diyl, Hexadecan-1,16-diyl oder Heneicosan-1,21-diyl.

Stark bevorzugt sind Verbindungen der Formel (I), bei denen alle Symbole, Definitionen und Indizes die stark bevorzugten Bedeutungen haben.
Insbesondere bevorzugt haben die Symbole und Indizes in der Formel (I) folgende Bedeutungen:

$A^1$ $A^2$ sind insbesondere bevorzugt gleich eine Gruppe $D^1$-[$Y^3$-$D^2$-]$_m$.

$D^1$ ist insbesondere bevorzugt gleich Phenyl, Pyridin-2-yl, Fluoren-2-yl, Cyclohexyl, Napht-2-yl, Benzothiophen-2-yl, Furan-2-yl, Thien-2-yl oder Thien-3-yl, wobei die genannten Gruppen unsubstituiert oder durch eine Gruppe $X^1$ substituiert sind.

$D^2$ ist insbesondere bevorzugt gleich Phenylen-1,4-diyl oder Thiophen-2,5-diyl, wobei die genannten Gruppen unsubstituiert oder durch eine Gruppe $X^1$ substituiert sind.

$X^1$ ist insbesondere bevorzugt gleich F, Chlor, Nitro, Hydroxy, Carboxyl, Methyl, Trifluormethyl, Methyloxy.

$Y^1$, $Y^2$ sind insbesondere bevorzugt gleich ~C(O)-, ~$CH_2$-, ~C≡C-C(O)-, ~CH=CH-C(O)-, ~$S(O)_2$-, ~$CH_2$-NH-3-Cyclobuten-1,2-dion-4- oder ~$CH_2$-C(O)-, wobei ~ die Bindung zur Gruppe A bezeichnet oder

$Y^1$, $Y^2$ bilden, unabhängig voneinander, zusammen mit dem Stickstoff, an den sie gebunden sind, und der Gruppe $R^8$ eine Gruppe ~4-1H-(1,2,3)Triazol-1-yl-, wobei ~ die Bindung zur Gruppe A bezeichnet.

$Y^3$ ist insbesondere bevorzugt eine Bindung.

m ist insbesondere bevorzugt 0 oder 1.

$Z^1$ ist insbesondere bevorzugt O.

D ist insbesondere bevorzugt eine Gruppe $Z^2$ -$T^1$-$Y^4$ -$A^3$-$Y^3$-$T^2$-$Z^2$.

$Z^2$ ist insbesondere bevorzugt gleich O, $CH_2$, ~CONH- oder ~4-1H-(1,2,3)Triazol-1-yl-, wobei ~ die Bindung zur Gruppe T bezeichnet.

$T^1$, $T^2$ sind insbesondere bevorzugt gleich oder verschieden ~$CH_2CH_2CH_2SCH_2CH_2$-, ~$CH_2CH_2CH_2S(O)CH_2CH_2$-, ~$CH_2C(O)NHCH_2CH_2CH_2$-, ~$CH_2$-4-(1H-(1,2,3)Triazol)-1-$CH_2CH_2$-, Butan-1,4-diyl, Pentan-1,5-diyl oder Hexan-1,6-diyl, wobei ~ die Bindung zur Gruppe $Z^2$ bezeichnet.

$Y^4$, $Y^5$ sind insbesondere bevorzugt gleich ~C(O)-NH-, ~NH-, ~$CH_2$NH-, ~NHC(O)NH-, ~$CH_2$NHC(O)-, ~$CH_2$C(O)NH- oder ~NH-C(O)-, wobei ~ die Bindung zu Gruppe A bezeichnet.

$A^3$ ist insbesondere bevorzugt Methandiyl, Ethan-1,2-diyl, Butan-1,4-diyl, Hexan-1,6-diyl, -3-Cyclobuten-1,2dion-4-, Thiophen-2,5-diyl, Cyclohexan-1,4-diyl, Phenylen-1,4-diyl, Naphtalin-1,4-diyl, Ethin-1,2-diyl, Biphenyl-wobei die genannten Reste unsubstituiert oder durch eine oder mehrere Gruppen $X^2$ substituiert sind.

$X^2$ ist insbesondere bevorzug gleich Brom, Nitro, Methyl oder Pentyloxy.

$R^1$ ist insbesondere bevorzugt C(O)ONa.

$R^2$, $R^3$ sind insbesondere bevorzugt gleich H.

$R^4$, $R^7$ sind insbesondere bevorzugt gleich OH.

$R^6$ ist insbesondere bevorzugt gleich H.

$R^5$ ist insbesondere bevorzugt gleich $C(O)CH_3$, $C(O)CH_2F$ oder $C(O)CH_2OH$.

$R^8$ ist insbesondere bevorzugt gleich H.

Insbesondere bevorzugt sind Verbindungen der Formel (I), bei denen alle Symbole und Indizes die insbesondere bevorzugten Bedeutungen haben.

Weiterhin bevorzugte Sialinsäurederivate der Formel (I) sind solche der Formeln (Ia) - (Ik), wobei die Symbole die in der Formel (I) angegebenen Bedeutungen und Bevorzugungen haben.

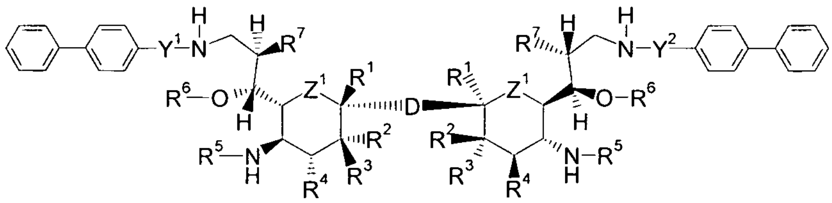

Ia

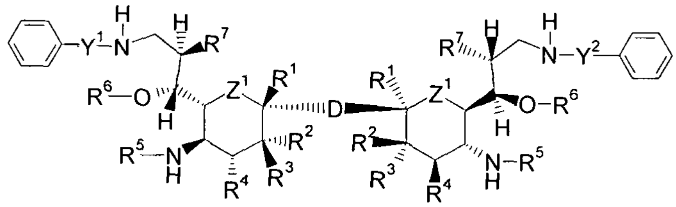

Ib

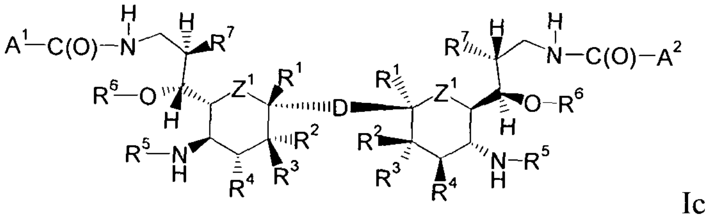

Ic

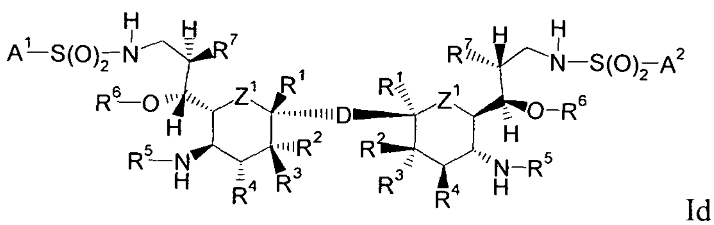

Id

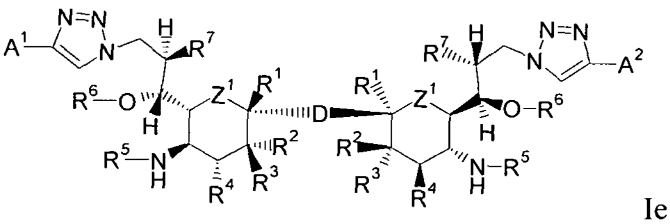

Ie

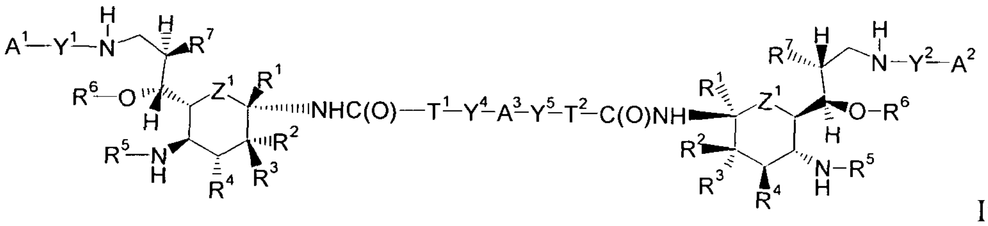

If

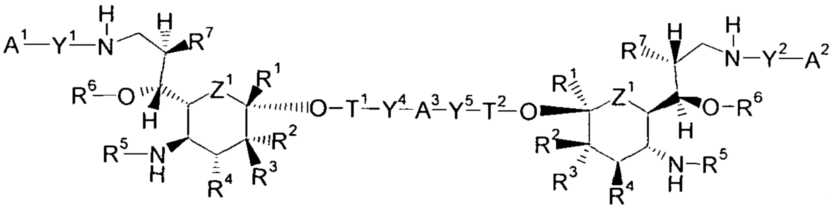

Ig

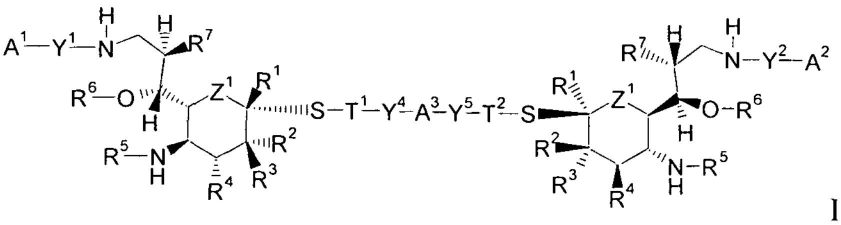

Ih

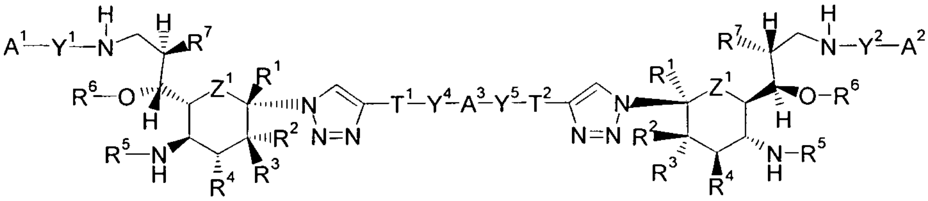

Ii

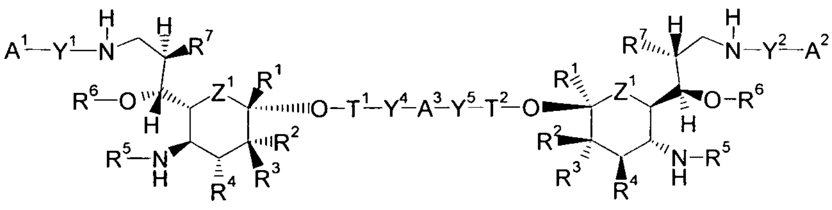

Ij

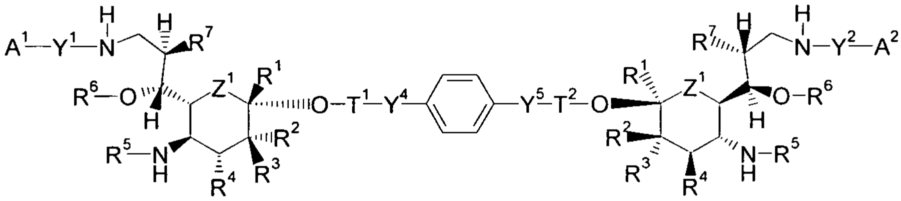

Ik

Besonders bevorzugt sind weiterhin Sialinsäurederivate der Formeln (Iaa)-(Iac), wobei die Symbole die in der Formel (I) angegebenen Bedeutungen haben:

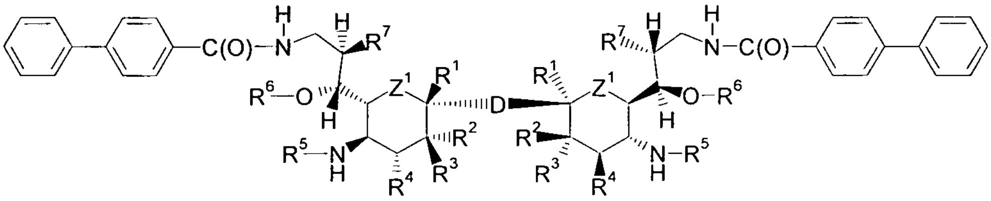

Iaa

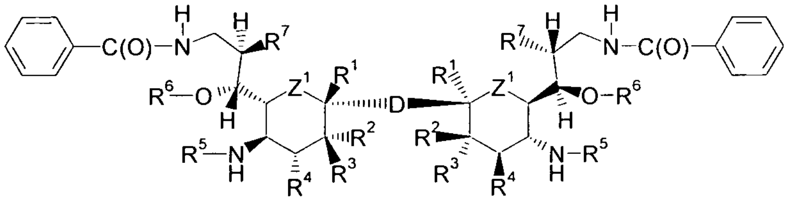

Iab

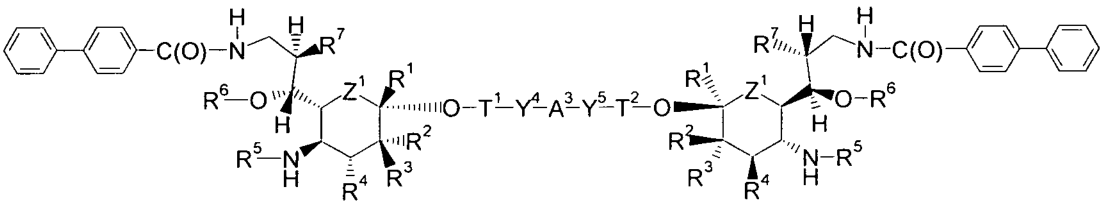

Iac

Ebenso besonders bevorzugt sind Sialinsäurederivate der Formeln (Iad)-(Iag), wobei die Symbole die in der Formel (I) angegebenen Bedeutungen haben:

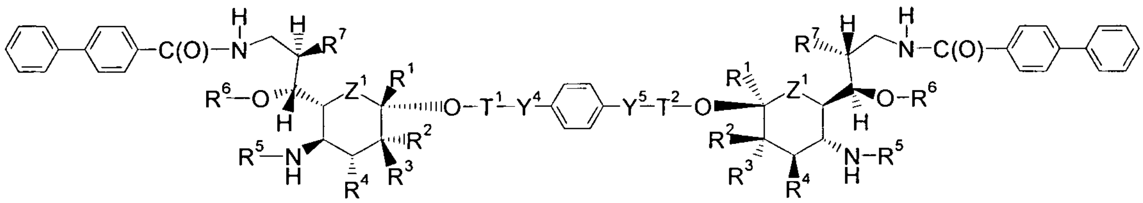

Iad

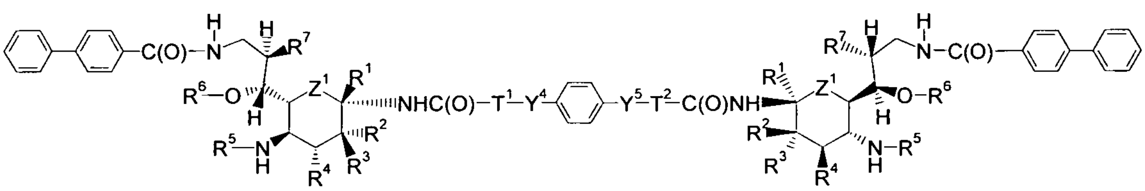

Iac

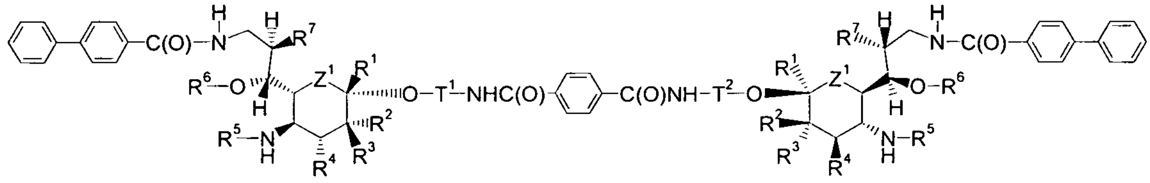

Iaf

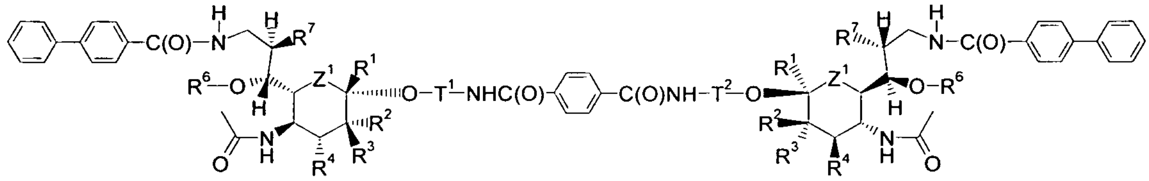

Iag

Die erfindungsgemäßen Sialinsäurederivate (I) sind unter Anwendung im Prinzip bekannter Syntheseverfahren erhältlich. Bevorzugt werden die Verbindungen nach den nachfolgend näher erläuterten erfindungsgemäßen Herstellungsverfahren, insbesondere den Syntheseschemata I - XX, erhalten:

Die Herstellung monomerer Ausgangsverbindungen kann beispielsweise gemäß den Syntheseschemata I bis XIV erfolgen.

## Schema I

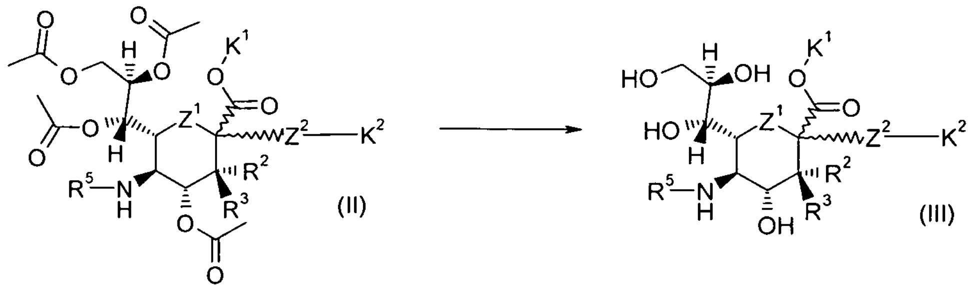

(II) (III)

$K^1$ ist Alkyl, insbesondere $C_1$-$C_6$-Alkyl
$K^2$ ist z.B.: Alkenyl, Alkinyl, geschütztes Aminoalkyl

Verbindungen der Formel (III) können durch Abspaltung der O-Acetylgruppen von Verbindungen der Formel (II) hergestellt werden (Schema I). Abspaltungen von O-Acetylgruppen sind beispielsweise beschrieben in "Protecting Groups" Philip J. Kocienski, 3rd Edition, Thieme 2005 und "Carbohydrates: Best synthetic methods" H.M.I. Osborn, Academic Press 2003.
Beispielhaft wurde Verbindung *100* hergestellt.

## Schema II

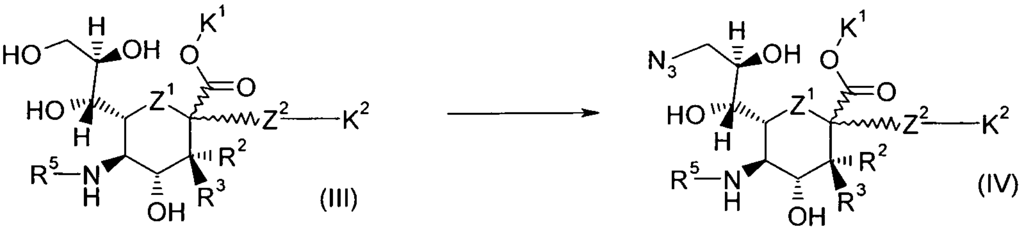

(III) (IV)

$K^1$ ist Alkyl, insbesondere $C_1$-$C_6$-Alkyl
$K^2$ ist ein beliebiger Rest, z.B.: Alkyl, Alkenyl, Alkinyl, Aryl, geschütztes Aminoalkyl

Verbindungen der Formel (IV) können durch Einführung einer Azidogruppe aus den Verbindungen der Formel (III) hergestellt werden (Schema II). Die Einführung von Azidogruppen ist beispielsweise beschrieben in Angewandte Chemie 2005, 117, 5320-5374 und Chem Rev 1988, 88 (2), 297-368
Beispielhaft wurden Verbindungen *4*, *5,96*, *105*, *112* und *118* hergestellt.

## Schema III

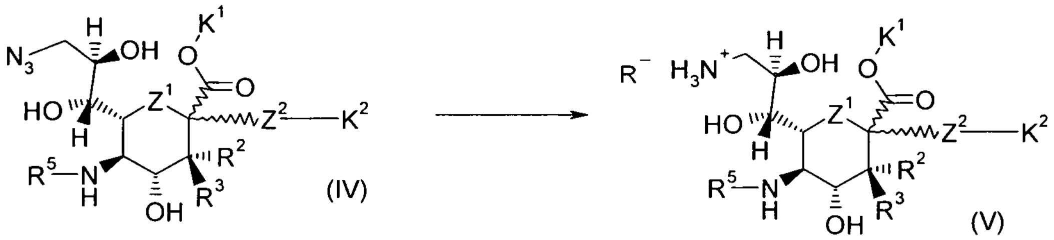

(IV) (V)

$K^1$ ist Alkyl, insbesondere $C_1$-$C_6$-Alkyl
$K^2$ ist z.B.: Alkyl, Alkenyl, Alkinyl, Aryl, geschütztes Aminoalkyl

Verbindungen der Formel V können durch Reduzierung der Azidogruppe aus den Verbindungen der Formel (IV) hergestellt werden (Schema III). Die Reduktion von Azidogruppen ist beispielsweise beschrieben in Angewandte Chemie 2005, 117, 5320-5374 und Chem Rev 1988, 88 (2), 297-368
Beispielhaft wurden Verbindungen 6, *7*, *97*, *107*, *113* und *119* hergestellt.

## Schema IV

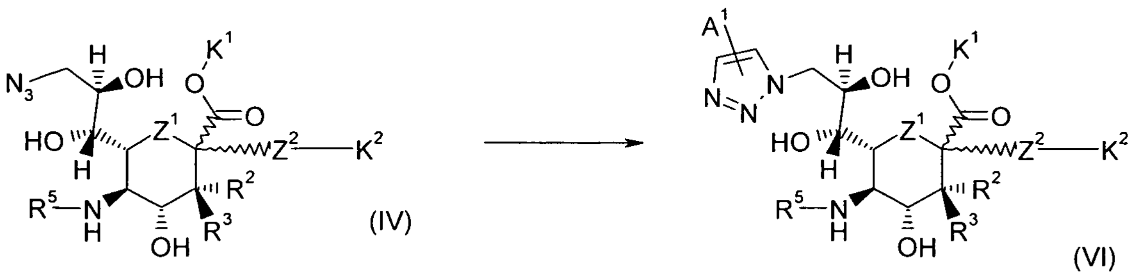

(IV) (VI)

$K^1$ ist Alkyl, insbesondere $C_1$-$C_6$-Alkyl
$K^2$ ist z.B.: Alkyl, Alkenyl, Alkinyl, Aryl, geschütztes Aminoalkyl

Verbindungen der Formel (VI) können durch Bildung eines Triazol-Ringes aus Verbindungen der Formel (IV) hergestellt werden (Schema IV). Reaktionen von Aziden zu Triazolen sind beispielsweise beschrieben in Angewandte Chemie 2005, 117, 5320-5374 und Medicinal Research Reviews 2008, 28, 278-308 Beispielhaft wurde Verbindung *93* hergestellt.

## Schema V

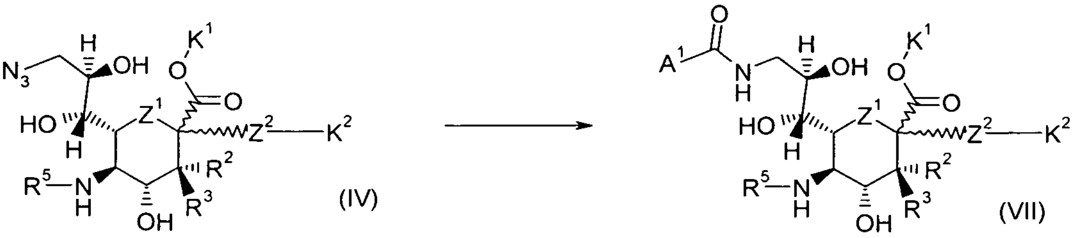

(IV) (VII)

$K^1$ ist Alkyl, insbesondere $C_1$-$C_6$-Alkyl
$K^2$ ist z.B.: Alkyl, Alkenyl, Alkinyl, Aryl, geschütztes Aminoalkyl

Verbindungen der Formel (VII) können durch Bildung einer Amid-Bindung aus Verbindungen der Formel (IV) hergestellt werden (Schema V). Reaktionen von Aziden zu Amiden sind beispielsweise beschrieben in Angewandte Chemie 2005, 117, 5320-5374; Chem Rev 1988, 88 (2), 297-368; J. Am. Chem. Soc. 2003, 125, 7754-7755 und Carbohydrate Res. 2008, 343, 1636-1643
Beispielhaft wurde Verbindung *9* hergestellt.

## Schema VI

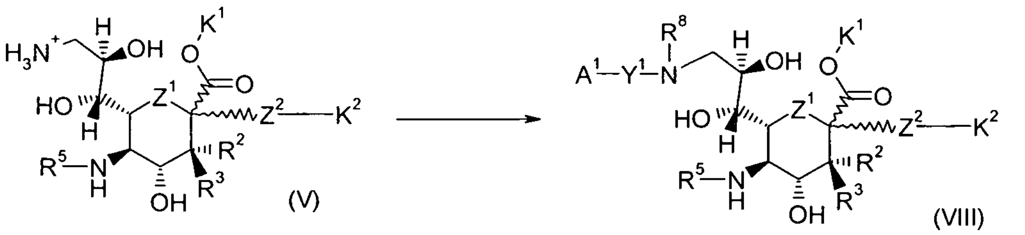

(V) (VIII)

$K^1$ ist ein Alkyl, insbesondere $C_1$-$C_6$-Alkyl
$K^2$ ist z.B.: Alkyl, Alkenyl, Alkinyl, Aryl, geschütztes Aminoalkyl

Verbindungen der Formel (VIII) können auch durch Bildung einer Amid-Bindung aus Verbindungen der Formel (V) hergestellt werden (Schema VI). Reaktionen von Aminen zu Amiden sind beispielsweise beschrieben in Tetrahedron 2005, 61, 10827-10852. Beispielhaft wurden Verbindungen *11, 42, 60, 99, 309, 419, 1049* und 1070 hergestellt. Alternativ kann auch eine Schutzgruppe für die Aminogruppe in Verbindung der Formel (V) eingeführt werden. Entsprechende Schutzgruppen sind beispielhaft in Chem. Rev. 2009, 109, 2455-2504 und "Protecting Groups" Philip J. Kocienski, 3rd Edition, Thieme 2005 beschrieben.
Beispielhaft wurde Verbindung *59* hergestellt.

## Schema VII

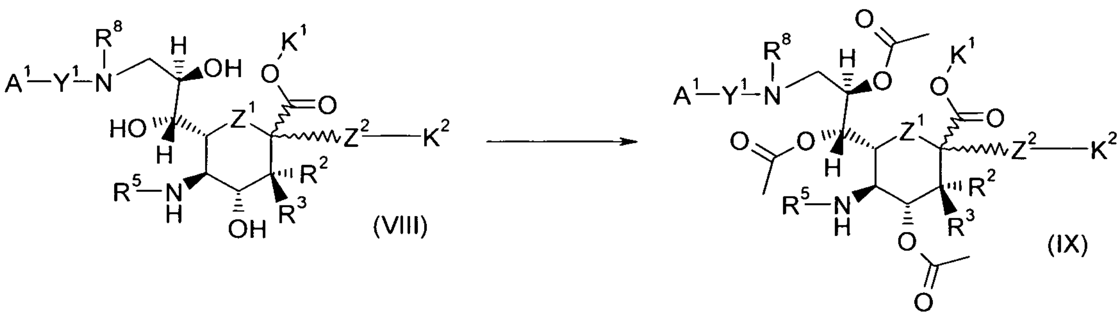

(VIII) (IX)

$K^1$ ist Alkyl, insbesondere $C_1$-$C_6$-Alkyl
$K^2$ ist ein beliebiger Rest, z.B.: Alkyl, Alkenyl, Alkinyl, Aryl, geschütztes Aminoalkyl

Verbindungen der Formel (IX) können durch Acetylierung der Hydroxygruppen von Verbindungen der Formel (VIII) hergestellt werden (Schema VII). Acetylierungen von Hydroxgruppen sind beispielsweise beschrieben in "Protecting Groups" Philip J. Kocienski, 3rd Edition, Thieme 2005 und "Carbohydrates: Best synthetic methods" H.M.I. Osborn, Academic Press 2003
Beispielhaft wurden Verbindungen 86 und *99* hergestellt

## Schema VIII

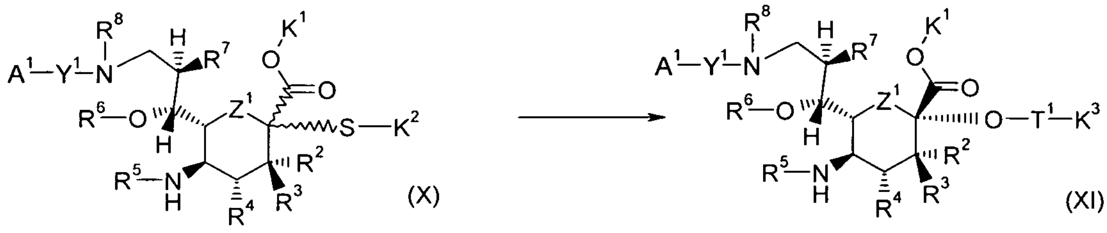

(X) (XI)

$K^1$ ist Alkyl, insbesondere $C_1$-$C_6$-Alkyl
$K^2$ ist z.B.: Alkyl, Aryl
$K^3$ ist z.B. geschütztes Amin, Carbonsäure, Alken, Alkin

Verbindungen der Formel (XI) können durch Reaktion der Verbindungen der Formel (X) mit Alkoholen hergestellt werden (Schema VIII). Solche Reaktionen sind beispielsweise beschrieben in "Carbohydrate: Best synthetic methods" H.M.I. Osborn, Academic Press 2003 .
Beispielhaft wurde Verbindung *100* hergestellt.

## Schema IX

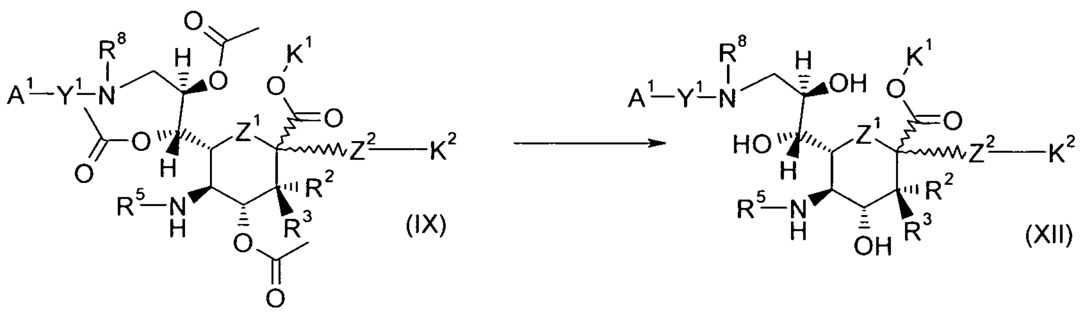

(IX) (XII)

$K^1$ ist Alkyl
$K^2$ ist z.B.: Alkenyl, Alkinyl, geschütztes Aminoalkyl

Verbindungen der Formel (XII) können durch Abspaltung der O-Acetylgruppen aus Verbindungen der Formel (IX) hergestellt werden (Schema IX). Die Abspaltung von O-Acetylgruppen ist beispielsweise beschrieben in "Protecting Groups" Philip J. Kocienski, 3rd Edition, Thieme 2005 und "Carbohydrates: Best synthetic methods" H.M.I. Osborn, Academic Press 2003 .
Beispielhaft wurde die Verbindung *100* hergestellt.

## Schema X

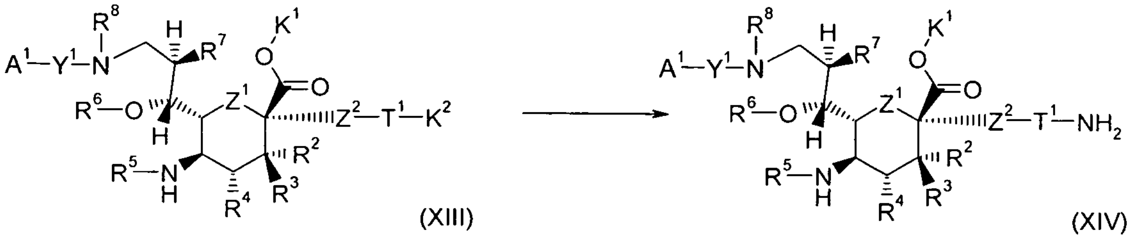

(XIII) (XIV)

$K^1$ ist Alkyl, insbesondere $C_1$-$C_6$-Alkyl
$K^2$ ist ein geschütztes Amin

Verbindungen der Formel (XIV) können durch Entschützen der Aminogruppe aus der Verbindung (XIII) hergestellt werden (Schema X). Schutzgruppen und Reaktionen zum Entschützen von Aminogruppen sind beispielsweise beschrieben in "Protecting Groups" Philip J. Kocienski, 3rd Edition, Thieme 2005 und Chem. Rev. 2009, 109, 2455-2504. Beispielhaft wurden Verbindungen *101, 109* und 115 hergestellt.

## Schema XI

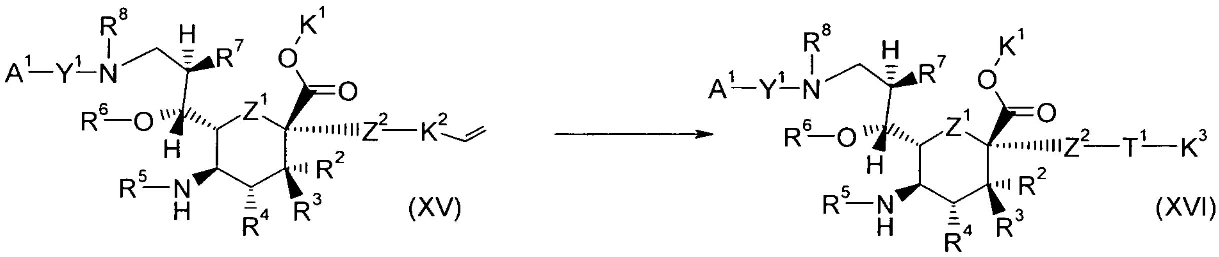

(XV) (XVI)

$K^1$ ist ein Alkyl oder ein Kation, insbesondere $C_1$-$C_6$-Alkyl, Li, Na oder K
$K^2$ ist Alkandiyl, Aryldiyl
$K^3$ ist z.B.: Amino, Carboxy, Hydroxy

Verbindungen der Formel (XVI) können aus Verbindungen (XV) durch Reaktion der terminalen Doppelbindung mit einer reaktiven Gruppe hergestellt werden (Schema XI). Reaktionen von Doppelbindung sind beispielsweise beschrieben in J. Org. Chem. 2000, 65, 958-963.
Beispielhaft wurden Verbindungen *10, 11, 18, 50, 56, 60, 94. 121, 138* und *144* hergestellt.

## Schema XII

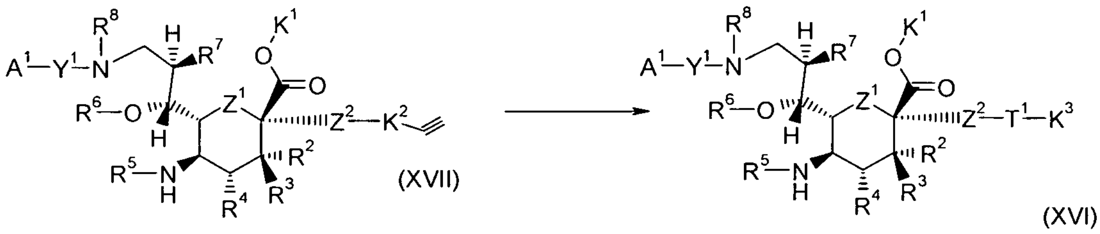

(XVII) (XVI)

$K^1$ ist Alkyl, insbesondere $C_1$-$C_6$-Alkyl
$K^2$ ist ein Alkandiyl, Aryldiyl
$K^3$ ist z.B.: geschütztes Amin, Carboxy, Hydroxy

Verbindungen der Formel (XVI) können durch Reaktion einer terminalen Dreifachbindung in Verbindungen der Formel (XVII) mit einem Azid hergestellt werden (Schema XII). Die Reaktion von Alkinen mit Aziden ist beispielsweise

beschrieben in Angewandte Chemie 2005, 117, 5320-5374 und Medicinal Research Reviews 2008, 28, 278-308. Beispielhaft wurde Verbindung 124 hergestellt.

## Schema XIII

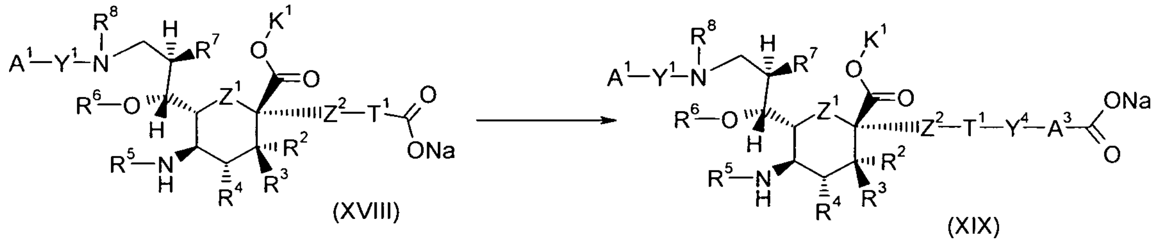

(XVIII) (XIX)

$K^1$ ist Alkyl, insbesondere $C_1$-$C_6$-Alkyl

Verbindungen der Formel (XIX) können durch Reaktion der Carboxylgruppe in Verbindungen der Formel (XVIII) mit einem Amin hergestellt werden (Schema XIII). Reaktionen von Carbonsäuren mit Aminen sind beispielsweise beschrieben in Tetrahedron 2005, 61, 10827-10852 und Chem. Eur. J. 2009, 15, 9404-9416.
Beispielhaft wurde Verbindung *53* hergestellt.

## Schema XIV

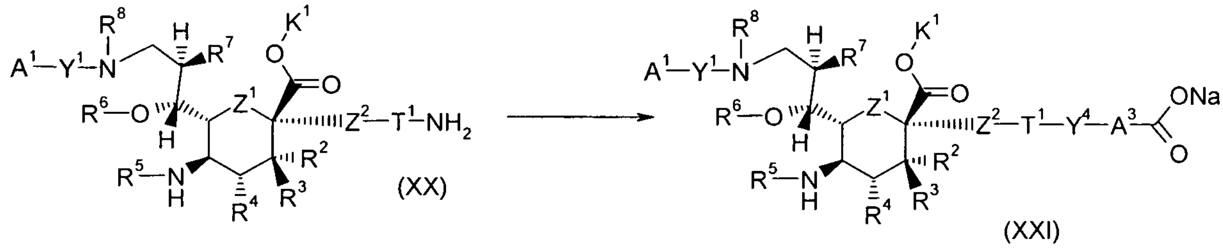

(XX) (XXI)

$K^1$ ist Alkyl, insbesondere $C_1$-$C_6$-Alkyl

Verbindungen der Formel (XXI) können durch Reaktion des Amines in Verbindungen der Formel (XX) mit einer reaktiven Gruppe hergestellt werden (Schema XIV). Reaktionen reaktiver Gruppen mit Aminen sind beispielsweise beschrieben in Tetrahedron 2005, 61, 10827-10852 und Chem. Eur. J. 2009, 15, 9404-9416.
Beispielhaft wurde Verbindung 91 hergestellt.

Die Herstellung von dimeren Ausgangsverbindungen ist beispielhaft in den Schemata XV und XVI angegeben.

## Schema XV

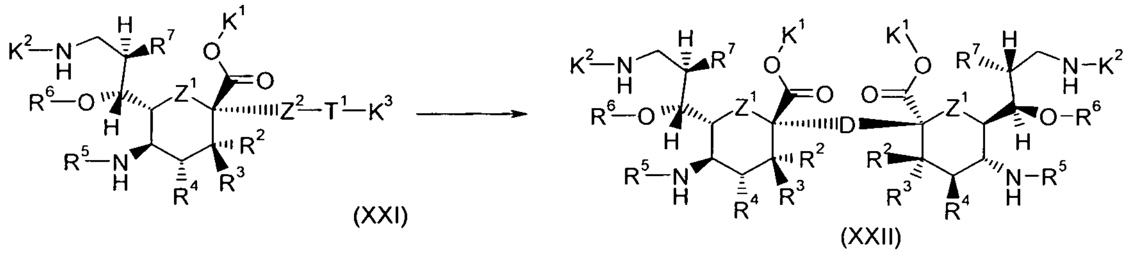

(XXI) (XXII)

$K^1$ ist Alkyl, insbesondere $C_1$-$C_6$-Alkyl
$K^2$ ist eine Schutzgruppe, insbesondere Fmoc
$K^3$ ist Amino oder Carboxyl

Verbindungen der Formel (XXII) können durch Reaktion eines Diamines/Dicarbonsäure mit den Carboxyl/Amino-Gruppen von Verbindungen der Formel (XXI) unter Bildung zweier Amidbindungen hergestellt werden (Schema XV). Reaktionen zur Bildung von Amidbindungen sind beispielsweise beschrieben in Tetrahedron 2005, 61, 10827-10852 und Chem. Eur. J. 2009, 15, 9404-9416.
Beispielhaft wurden Verbindungen *61* und *126* hergestellt.

## Schema XVI

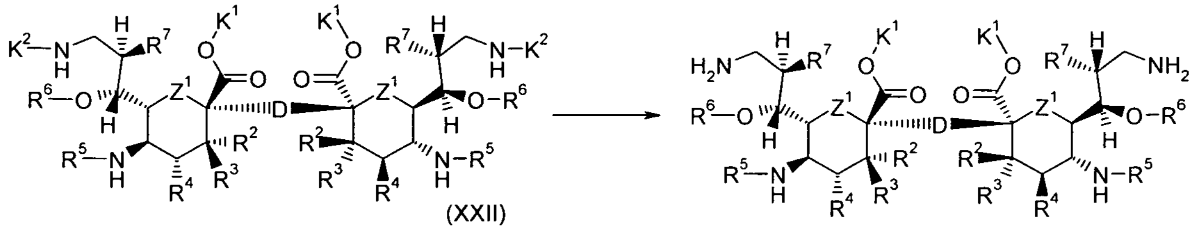

(XXII)

(XXIII)

$K^1$ ist Alkyl, insbesondere $C_1$-$C_6$-Alkyl
$K^2$ ist eine Schutzgruppe
$K^3$ ist Alky oder ein Kation, insbesondere $C_1$-$C_6$-Alkyl, Li oder Na

Verbindungen der Formel (XXIII) können durch teilweise oder vollständige Abspaltung der Schutzgruppen aus Verbindungen der Formel (XXII) hergestellt werden (Schema XVI). Schutzgruppen und deren Abspaltung ist beispielsweise beschrieben in "Protecting Groups" Philip J. Kocienski, 3rd Edition, Thieme 2005 und Chem. Rev. 2009, 109, 2455-2504.
Beispielhaft wurde Verbindung *130* hergestellt.

Die Herstellung von Sialinsäurederivaten der Formel (I) ist beispielhaft in den Schemata XVII bis XX beschrieben.

## Schema XVII

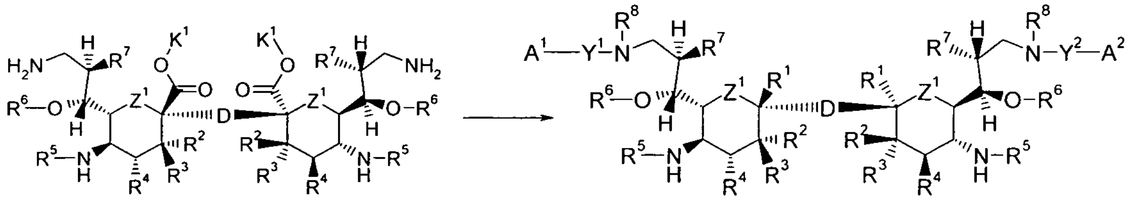

(XXIII)

(I)

$K^1$ ist Alkyl oder Kation, insbesondere $C_1$-$C_6$-Alkyl, Na oder Li

Verbindungen der Formel (I) können durch Reaktion der Aminogruppen von Verbindungen der Formel (XXIII) mit reaktiven Verbindungen hergestellt werden (Schema XVII). Reaktionen von Aminen mit reaktiven Gruppen sind beispielsweise in Tetrahedron 2005, 61, 10827-10852 und Chem. Eur. J. 2009, 15, 9404-9416. beschrieben. Alternativ können die Verbindungen auch durch den Einsatz von Kupplungsreagenzien hergestellt werden. Geeignete Kupplungsreagenzien und deren Reaktionen sind beispielsweise beschrieben in Tetrahedron 2005, 61, 10827-10852 und Chem. Eur. J. 2009, 15, 9404-9416.. Die anschließend eventuell notwendige Abspaltung von Schutzgruppen ist beispielsweise beschrieben in "Protecting Groups" Philip J. Kocienski, 3rd Edition, Thieme 2005 und Chem. Rev. 2009, 109, 2455-2504. Beispielhaft wurden Verbindungen *62 bis 73, 84, 85, 110, 116, 127, 128, 129, 131, 134, 139* und *143* hergestellt.

## Schema XVIII

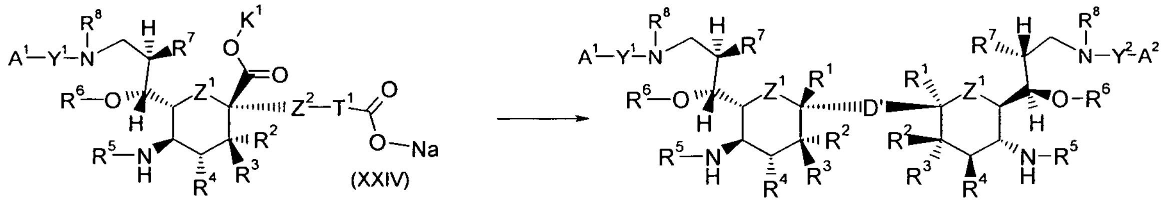

(XXIV)

(I')

$K^1$ ist Alkyl, insbesondere $C_1$-$C_6$-Alkyl

D' = $Z^2$-$T^1$-C(O)$NR^x$-$A^3$-$NR^x$-C(O)-$T^2$-$Z^2$

Verbindungen der Formel (I') können durch Reaktion der Carboxylgruppe in Verbindungen der Formel (XXIV) mit Diaminen hergestellt werden (Schema XVIII). Die Reaktion kann durch den Einsatz von Kupplungsreagenzien oder Aktivierung der Carboxylgruppe ausgeführt werden. Kupplungsreagenzien und die Aktivierung von Carbonsäuren sind beispielsweise beschrieben in Tetrahedron 2005, 61, 10827-10852 und Chem. Eur. J. 2009, 15, 9404-9416.. Die anschließend eventuell notwendige Abspaltung von Schutzgruppen ist beispielsweise beschrieben in "Protecting Groups" Philip J. Kocienski, 3rd Edition, Thieme 2005 und Chem. Rev. 2009, 109, 2455- 2504.
Beispielhaft wurden Verbindungen *39 bis 45* und *90* hergestellt.

## Schema XIX

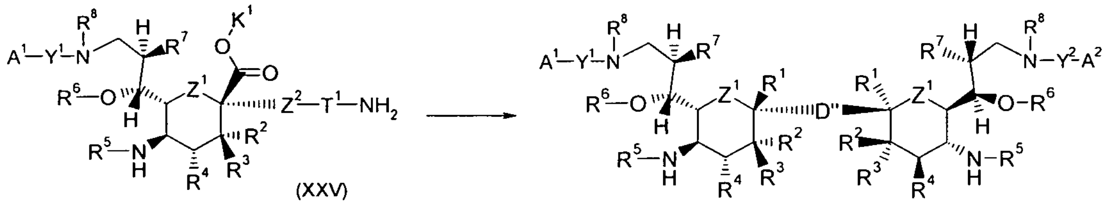

(XXV)

(I")

$K^1$ ist Alkyl, insbesondere Methyl

D" = $Z^2$-$T^1$-$Y^4$-$A^3$-$Y^5$-$T^2$-$Z^2$ und
$Y^6$ = $NR^x$, -$NR^x$-C(O)~, -$NR^X$-C(O)-$NR^x$~ oder $NR^x$-$S(O)_2$~

Verbindungen der Formel (I") können durch Reaktion der Aminogruppe in Verbindungen der Formel (XXV) mit divalenten, reaktiven Verbindungen hergestellt werden (Schema XIX). Reaktionen von Aminen mit reaktiven Verbindungen sind beispielsweise beschrieben in Tetrahedron 2005, 61, 10827-10852 und Chem. Eur. J. 2009, 15, 9404-9416. Die Reaktion kann auch durch den Einsatz von Kupplungsreagenzien oder durch in situ Aktivierungen einer divalenten Verbindung durchgeführt werden. Kupplungsreagenzien und deren Reaktionen sind beschrieben in Tetrahedron 2005, 61, 10827-10852 und Chem. Eur. J. 2009, 15, 9404-9416. Die anschließend eventuell notwendige Abspaltung von Schutzgruppen ist beispielsweise beschrieben in "Protecting Groups" Philip J. Kocienski, 3rd Edition, Thieme 2005 und Chem. Rev. 2009, 109, 2455- 2504.

Beispielhaft wurden Verbindungen *19 bis 32, 49, 51, 52, 57, 58, 95, 102, 122, 125, 140* und *149* hergestellt.

## Schema XX

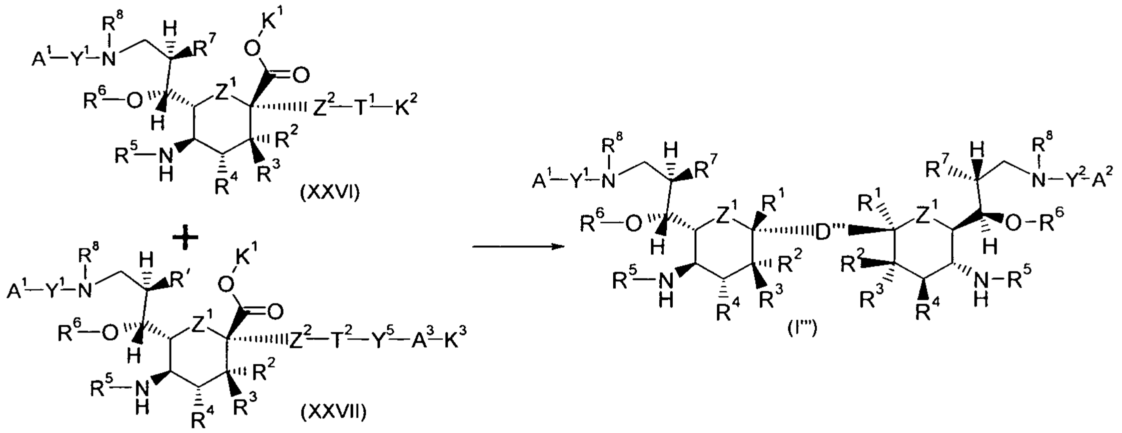
(XXVI)
(XXVII)
(I''')

$K^1$ ist Alkyl, insbesondere $C_1$-$C_6$-Alkyl
$K^2$ ist eine Aminogruppe/Carboxylgruppe
$K^3$ ist eine Carboxylgruppe/Aminogruppe

$D''' = Z^2\text{-}T^1\text{-}Y^7\text{-}A^3\text{-}Y^5\text{-}T^2\text{-}Z^2$ und
$Y^7$ ist $-NR^x-C(O)\sim$ oder $-C(O)-NR^x\sim$

Gegebenenfalls unsymmetrische Verbindungen der Formel (I''') können durch Reaktion der Amino/Carboxylgruppe in Verbindungen der Formel (XXVI) mit der Amino/Carboxylgruppe von Verbindungen der Formel (XXVII) hergestellt werden (Schema XX). Die Reaktion kann durch den Einsatz von Kupplungsreagenzien oder durch Aktivierung der Carbonsäure erfolgen. Amidbildungen, Kupplungsreagenzien und Aktivierungen sind beispielsweise beschrieben in Tetrahedron 2005, 61, 10827-10852 und Chem. Eur. J. 2009, 15, 9404-9416.. Eine anschließende eventuelle Abspaltung von Schutzgruppen ist beispielsweise beschrieben in "Protecting Groups" Philip J. Kocienski, 3rd Edition, Thieme 2005 und Chem. Rev. 2009, 109, 2455- 2504.
Beispielhaft wurden Verbindungen *22*, *54* und *103* hergestellt.

Die Sialinsäurederivate der Formel (I) eignen sich als pharmakologisch wirksame Verbindungen und Wirkstoffe für Arzneimittelzubereitungen. Sie wirken als Siglec-Liganden insbesondere von Siglec-2 (CD22), Siglec-4 (MAG), Siglec-7, zur Regulation des Immunsystems, insbesondere als Hilfsstoff bei Impfungen sowie zur Behandlung von Krankheiten, deren Verlauf oder Aktivität durch die Siglec-Liganden beeinflusst werden kann insbesondere Allergien, Autoimmunerkrankungen, chronischen Entzündungen, Querschnittslähmung, multipler Sklerose, Krebs, Viruserkrankungen, beispielsweise AIDS, sowie bei bakterielle Erkrankungen, bespielsweise Streptokokken, parasitäre Erkrankungen, beispielsweise Chagas Krankheit, Krankheiten, bei denen die Immunantwort im Rahmen der B-Zellenaktivierung gestört ist, wie Common Variable Immunodeficiency (CVID) und 1gA-Defizienz, bei Krankheiten der blutbildenden Organe und des Blutes sowie bei Krebs, beispielsweise Lymphome und Myelome. Bevorzugte Indikationen sind Allergien, Autoimmunerkrankungen und CVID.

Behandlung bedeutet im Sinne der Erfindung eine therapeutische Behandlung, sowohl zur Heilung als auch zur Linderung von Symptomen, wie auch eine vorbeugende Behandlung.

Die Sialinsäurederivate können in Kombination mit anderen pharmakologisch wirksamen Substanzen, insbesondere solchen, welche die Wirksamkeit der erfindungsgemäßen Verbindungen verstärken, eingesetzt werden.

Gegenstand der Erfindung ist auch ein Verfahren zur Behandlung einer Siglec vermittelten Krankheit, insbesondere aus der Gruppe der Allergien, Autoimmunerkrankungen, chronischen Entzündungen, Querschnittslähmung, multipler Sklerose, Krebs, Viruserkrankungen, beispielsweise AIDS, Krankheiten, bei denen die Immunantwort im Rahmen der B-Zellenaktivierung gestört ist, wie Common Variable Immunodeficiency (CVID) und 1gA-Defizienz, wobei man einer von der Krankheit betroffenen Person eine vorzugsweise therapeutisch wirksame Menge eines Sialinsäurederivats der Formel (I) verabreicht.

Weiterhin Gegenstand der Erfindung ist ein Sialinsäurederivat der Formel (I) oder ein pharmakologisch verträgliches Salz davon als Arzneimittel, insbesondere zur Behandlung von Siglec-vermittelten Krankheiten, wie den oben beschriebenen.

Gegenstand der Erfindung ist zudem ein Sialinsäurederivat der Formel (I) oder ein pharmakologisch verträgliches Salz davon zur Verwendung in einem Verfahren zur Behandlung von Siglec-vermittelten Erkrankungen, insbesondere den oben beschriebenen.

Ein weiterer Gegenstand der Erfindung ist ein Sialinsäurederivat der Formel (I) zur Verwendung zur Herstellung eines Arzneimittels, zur Behandlung von Siglec vermittelten Krankheiten, insbesondere den oben beschriebenen.

Ebenfalls Gegenstand der Erfindung ist eine pharmazeutische Zubereitung (=Arzneimittel), enthaltend mindestens ein Sialinsäurederivat der Formel (I) oder ein pharmakologisch verträgliches Salz davon und einen pharmakologisch verträglichen Träger.

Die zur Erzielung einer entsprechenden Wirkung bei der Behandlung oder Prophylaxe erforderliche Dosierung hängt üblicherweise von der zu verabreichenden Verbindung, vom Patienten, von der Art und Schwere der Krankheit oder des Zustandes und der Art und Häufigkeit der Verabreichung ab und liegt im Ermessen des zu behandelnden Arztes. Zweckmäßigerweise kann die Dosierung bei intravenöser Gabe im Bereich von 0,1 bis 1000 mg, vorzugsweise 0,5 bis 500 mg , und bei oraler Gabe im Bereich von 1 bis 1000 mg vorzugsweise 10 bis 500 mg , jeweils ein- oder mehrmals täglich, liegen. Hierzu lassen sich die erfindungsgemäßen Verbindungen der Formel I, gegebenenfalls in Kombination mit anderen Wirksubstanzen, zusammen mit einem oder mehreren inerten üblichen Trägerstoffen und/oder Verdünnungsmitteln, z.B. mit Maisstärke, Michzucker, Rohrzucker, mikrokristalliner Zellulose, Magnesiumstearat, Polyvinylpyrrolidon, Zitronensäure, Weinsäure, Wasser, Wasser/Ethanol, Wasser/Glycerin, Wasser/Sorbit, Wasser/Polyethylenglykol, Propylenglykol. Cetylstearylalkohol, Carboxymethylcellulose oder fetthaltigen Substanzen wie Hartfett oder deren geeigneten Gemischen, in übliche galenische Zubereitungen wie Tabletten, Dragees, Kapseln, Pulver, Lösungen, Suspensionen oder Zäpfchen bearbeiten.

Das Verabreichen der erfindungsgemäßen Sialinsäurederivate (I) kann durch jede konventionelle Methode erfolgen, einschließlich oraler und parenteraler, z.B. durch subcutane oder intramuskuläre Injektionen.

Die Sialinsäurederivate (I) können auch für andere als die genannten Zwecke eingesetzt werden, beispielsweise als Diagnostika, z.B. in Verfahren zur Bestimmung der Aktivität von Siglec-Liganden, als biochemische Sonden oder als Zwischenprodukte zur Herstellung weiterer, insbesondere pharmakologisch aktiven Verbindungen.

Die Erfindung wird durch die Beispiele näher erläutert ohne sie dadurch zu beschränken.

Beispiele

A. Synthesebeispiele werden in Schemata 1 bis 18 dargestellt.

## Schema 1

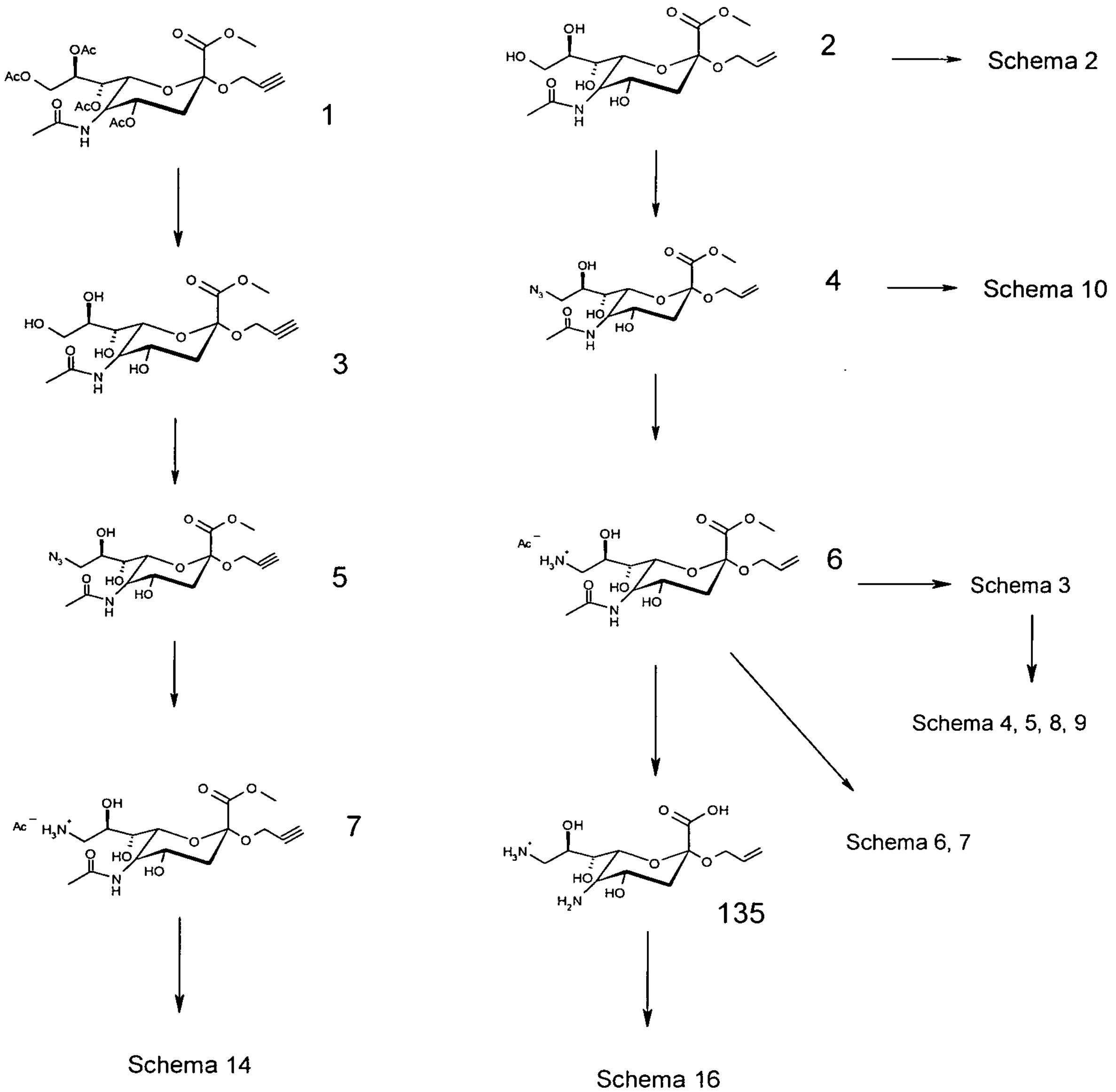

1

2

Schema 2

3

4

Schema 10

5

6

Schema 3

Schema 4, 5, 8, 9

7

Schema 6, 7

135

Schema 14

Schema 16

## Schema 2

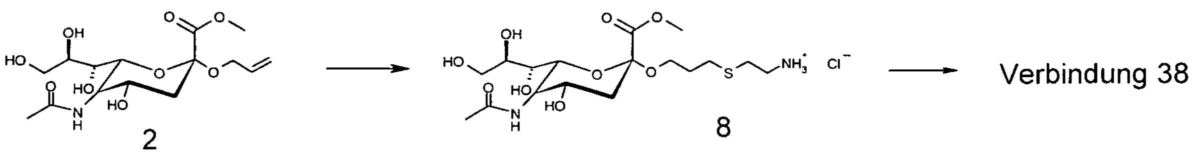

2

8

Verbindung 38

## Schema 3

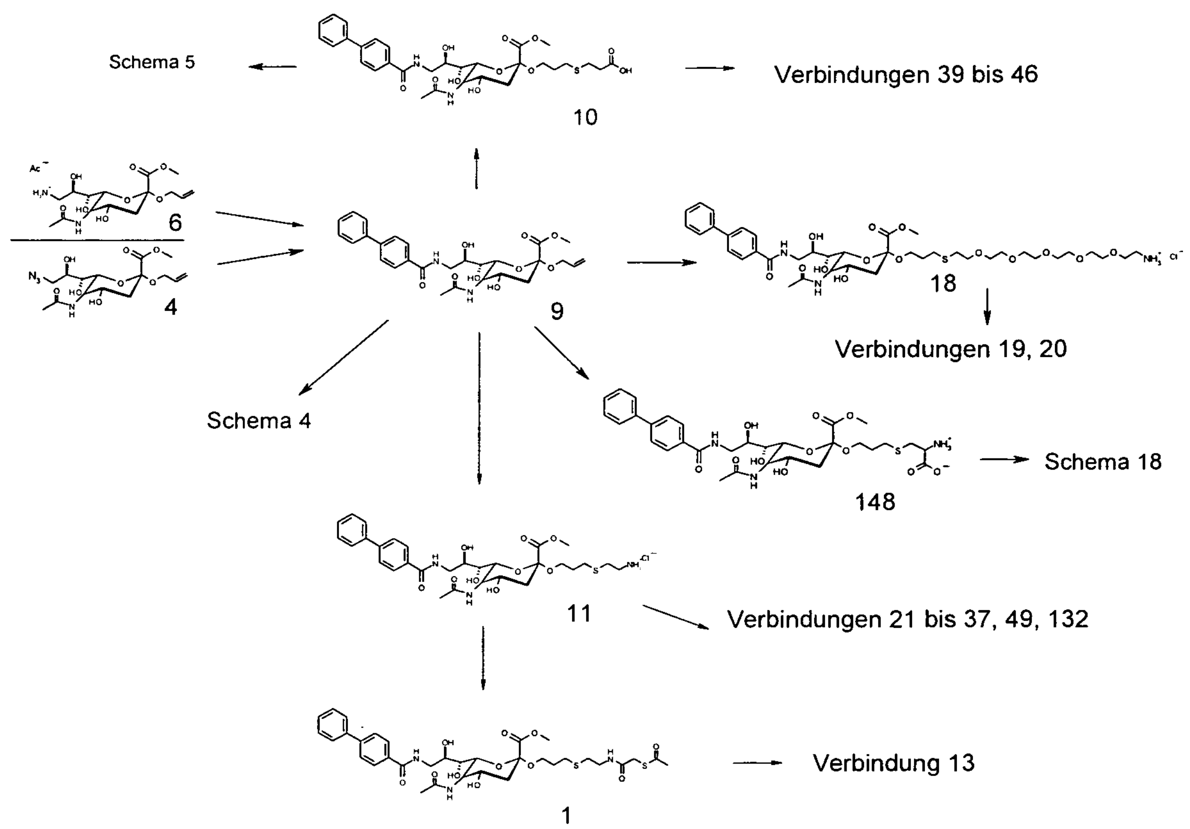


## Schema 4

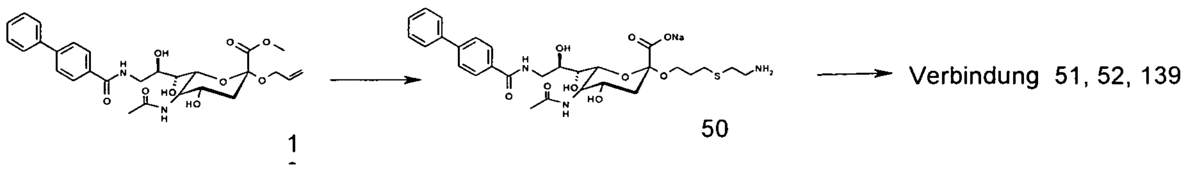


## Schema 5

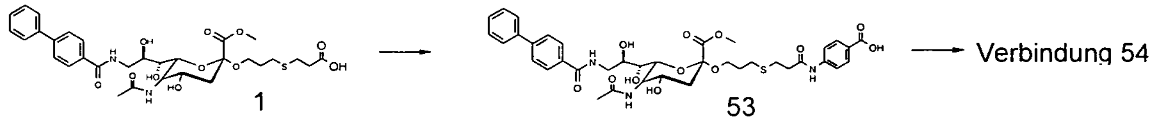

Schema 6

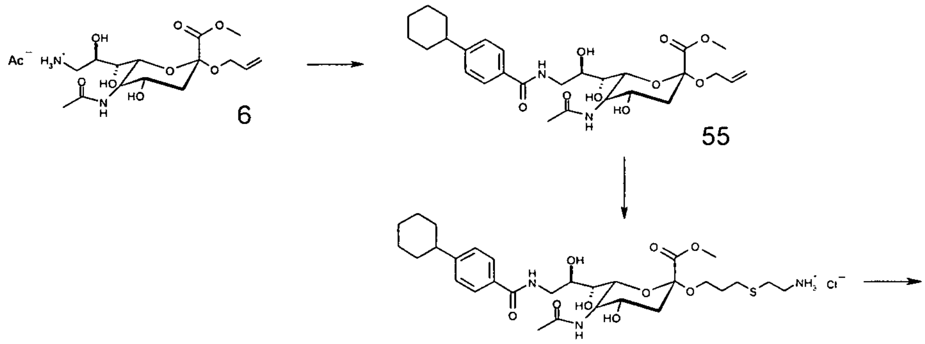

6

55

Verbindungen 57, 58

Schema 7

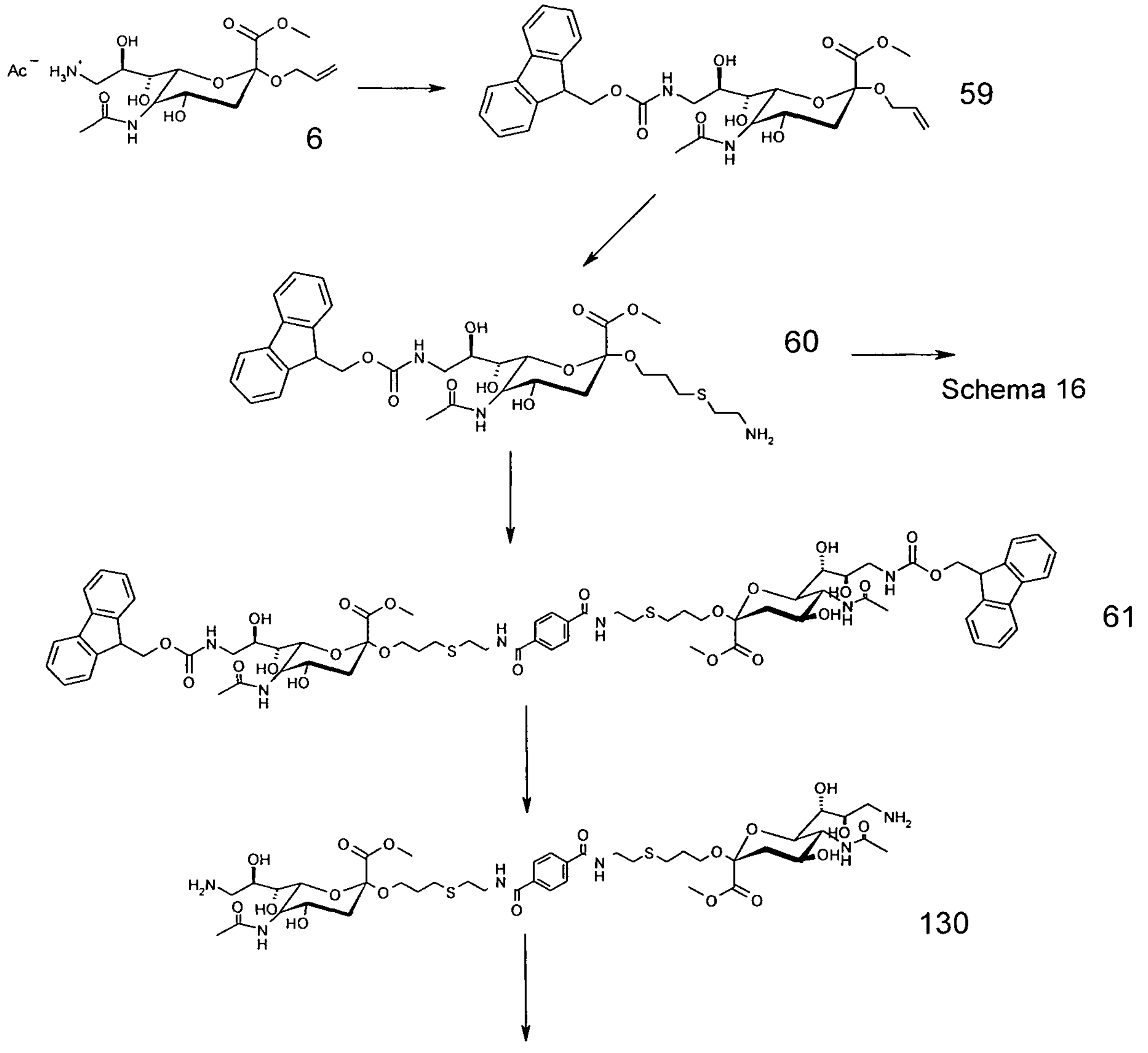

6

59

60

Schema 16

61

130

Verbindungen 62 bis 85 und 134

# Schema 8

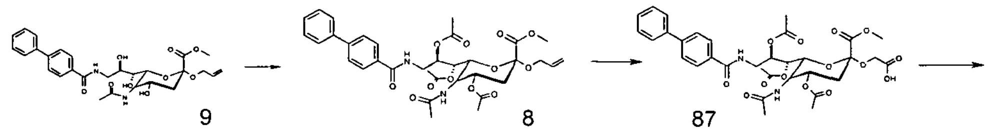

9 8 87

Verbindung 90

# Schema 9

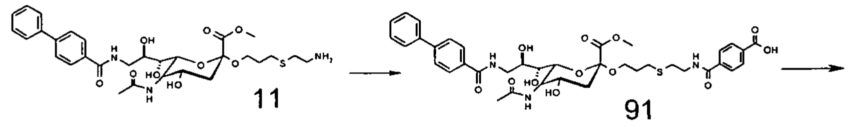

11 91

Verbindungen 92, 103

# Schema 10

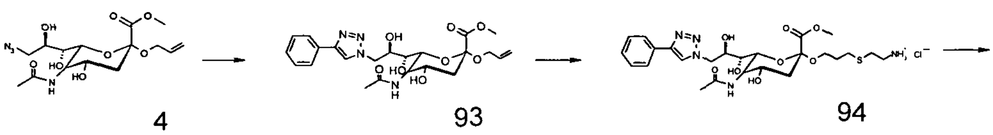

4 93 94

Verbindung 95

# Schema 11

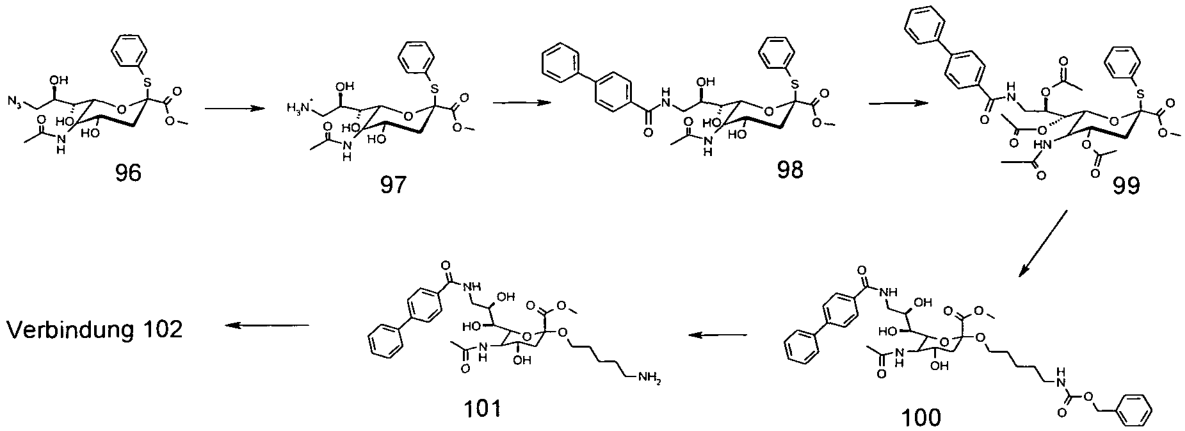

96 97 98 99

Verbindung 102

101 100

# Schema 12

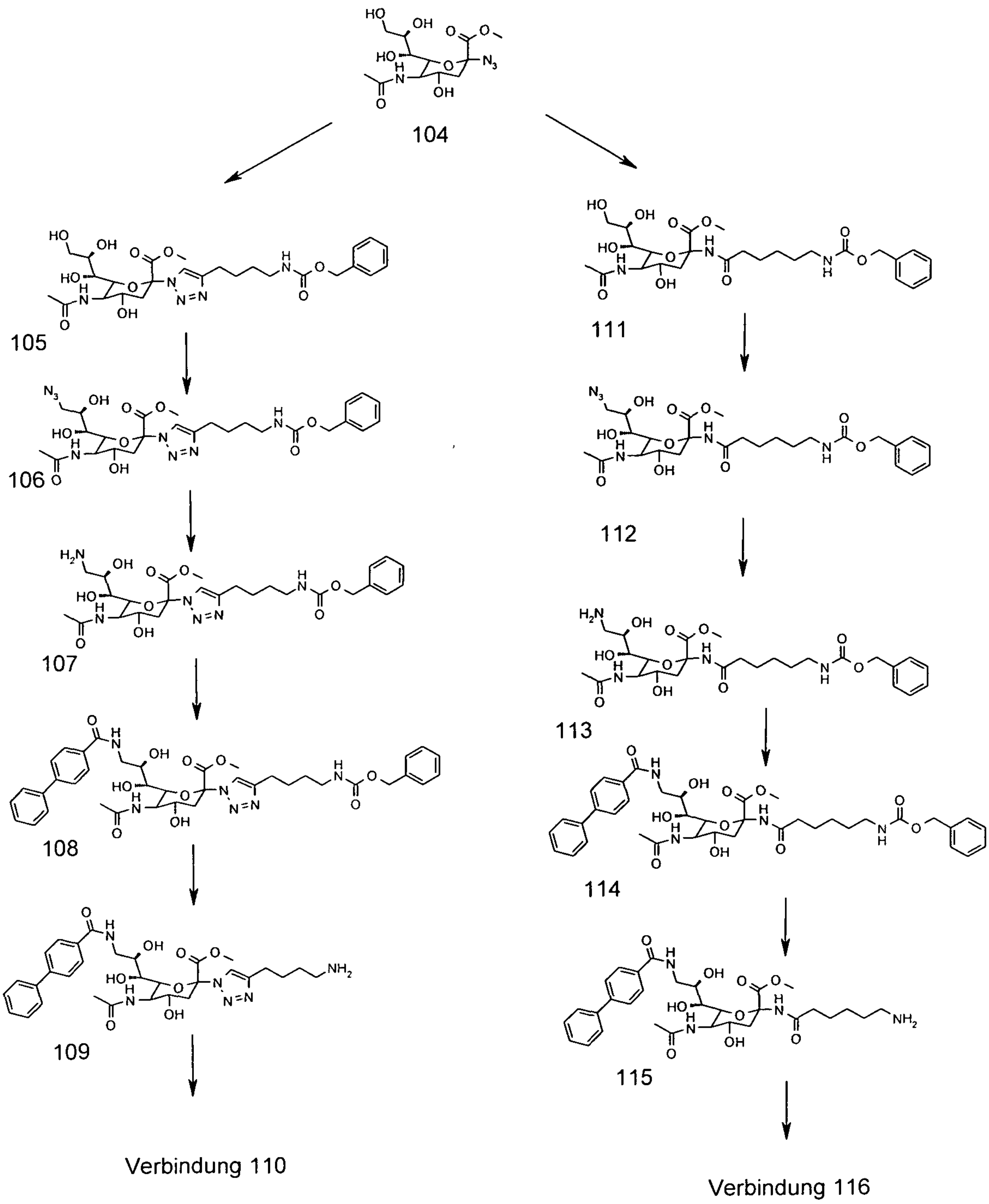

104

105

111

106

112

107

113

108

114

109

115

Verbindung 110

Verbindung 116

## Schema 13

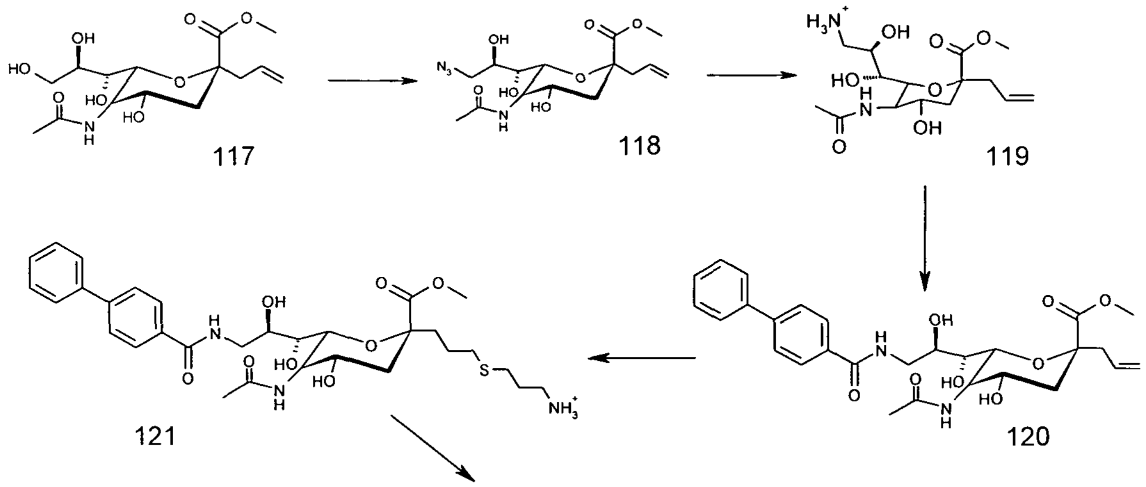

117 118 119 120 121

Verbindung 122

## Schema 14

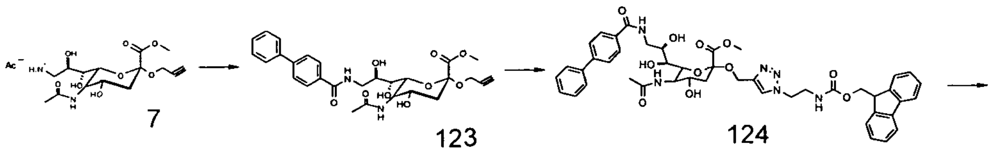

7 123 124

Verbindung 125

## Schema 15

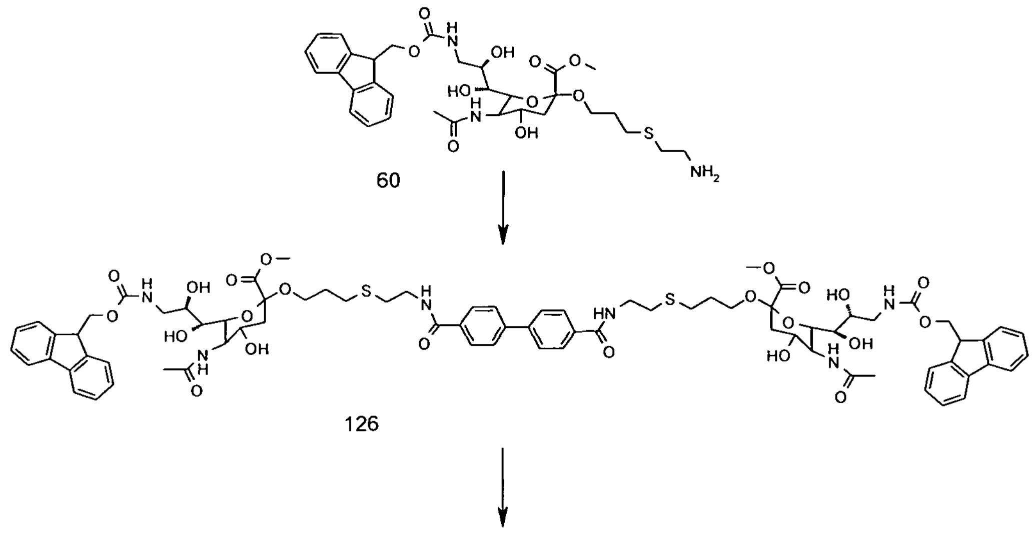

60 126

Verbindungen 127, 128, 129, 131

# Schema 16

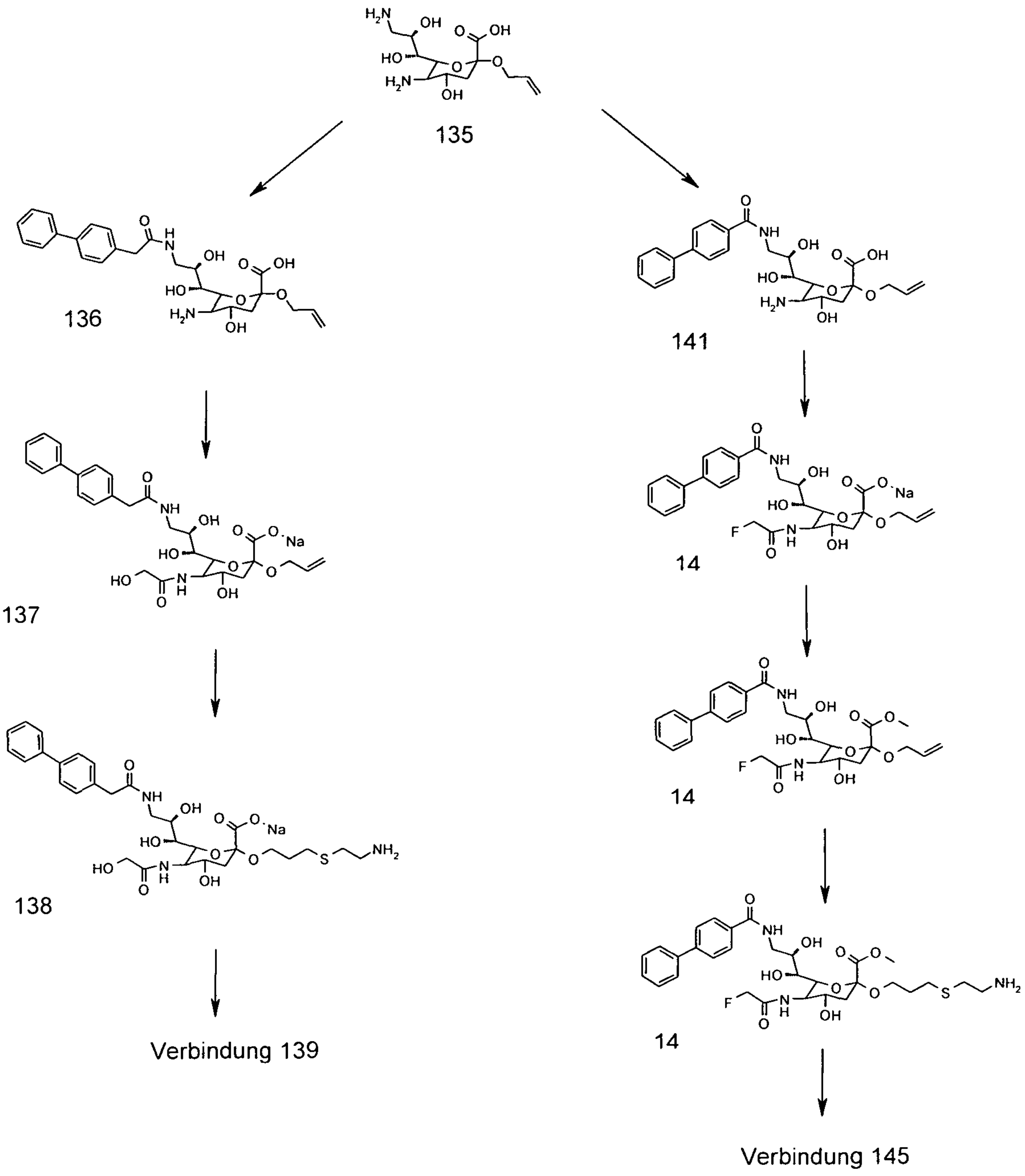

135

136

137

138

Verbindung 139

141

14

14

14

Verbindung 145

# Schema 17

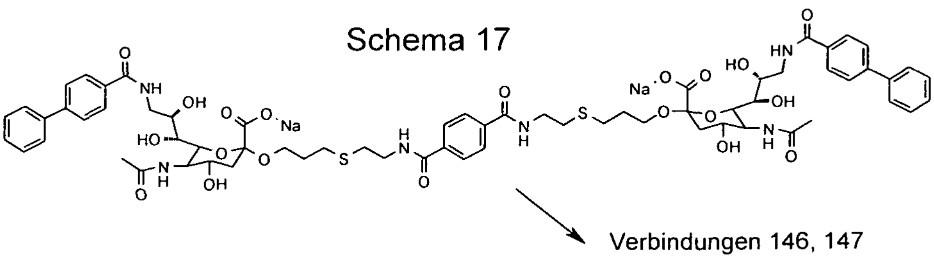

Verbindungen 146, 147

## Schema 18

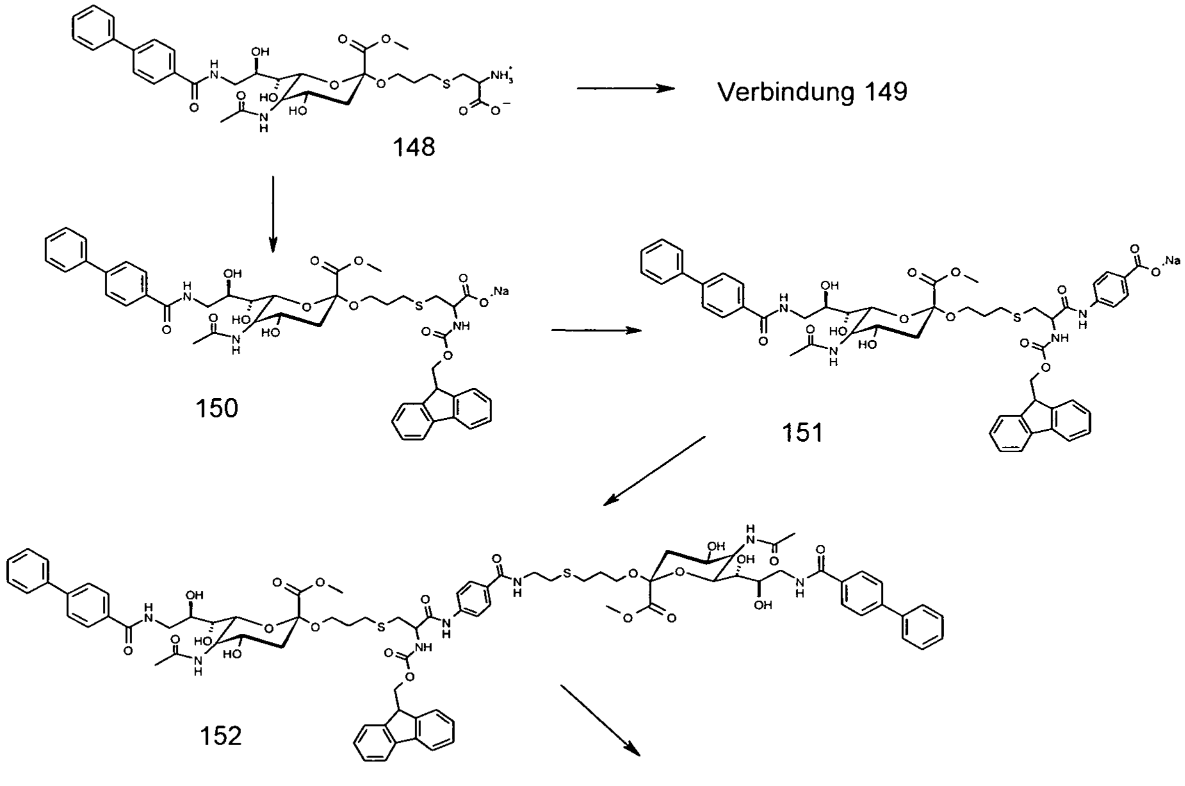

148

Verbindung 149

150

151

152

Verbindung 153

Die angegebenen Verbindungen wurden wie im Folgenden beschrieben erhalten:

Alle verwendeten aber nicht beschriebenen Verbindungen sind käuflich erhältlich oder wurden nach bekannten Literaturvorschriften hergestellt.

Für Reinigungen mit Kieselgel wurde Kieselgel Si60 43-60μm verwendet. Pro Gramm Substanz wurden ca. 100g Kieselgel benutzt.

Für Reinigungen über RP-18-Kieselgel (YMC CO LTD., YMC ODS-AQ) wurde dieses in Methanol suspendiert, in eine Säule gefüllt, mit Wasser vorgewaschen und die Substanz als Lösung oder Suspension mit Wasser aufgetragen. Das Säulenvolumen betrug ca. 5cm in der Höhe und ca. 1 cm im Durchmesser. Das Lösungsmittel wurde mit geringem Druck, erzeugt durch einen handbetriebenen Druckball, durch die Säule gedrückt. Laufmittel sind in Klammern angegeben. Gradienten sind mit dem Zeichen >> ausgedrückt z.B.: "(H2O >> MeOH)" bedeutet, dass ein Gradient von Wasser zu Methanol benutzt wurde).

Lösungsmittel wurden mittels eines Vakuumrotationsverdampfers bei reduziertem Druck und einer Badtemperatur von 40°C vollständig entfernt. In den folgenden Synthesen ist dieser Arbeitschritt als "entfernen des Lösungmittels" oder "Konzentration" benannt.

Lyophilisiert wurde, wenn nicht anders erwähnt, aus Wasser oder einer Wasser-Dioxan Mischung.

Photochemische Reaktionen wurden mittels des Photochemical Reactor, Model #RPR-100, The Souther New England Ultraviolet Company, durchgeführt.

Die Reaktionen und Substanzen wurden mit Dünnschichtchromatographie kontrolliert. Hierfür wurden kieselgelbeschichtete Aluminiumplatten mit Fluorescenceindikator verwendet (Merck TLC Silica gel 60 $F_{254}$). Die Substanzdetektion erfolgte unter UV-Licht bei 366 und 254nm. Die Chromatogramme wurden anschließend mit verdünnter Schwefelsäure besprüht und erhitzt, um die Kohlenhydrate nachzuweisen. Amine wurden zum Nachweis mit Ninhydrin-Lösung besprüht und erhitzt, Thiole mit Nitroprussid-Lösung. Einzelheiten und weitere Nachweisverfahren sind in "Anfärbereagenzien für Dünnschicht- und Papierchromatographie" Merck, 1970 erläutert.

Alle Substanzen wurden mit Massenspektrometrie geprüft.

Maldi-TOF: Matrix: 2,5 Dihydroxybenzoesäure Methode: dried droplet

| | |
|---|---|
| | Gerät: Bruker BIFLEX III |
| ESI: | Bruker ApexQe hybrid 9.4 T FT-ICR (ESI) |
| NMR: | Varian 500MHz or 300MHz system |

Abkürzungen

| | |
|---|---|
| abs. | absolut (wasserfrei) |
| AIBN | Azaisobutyronitril |
| CH3CN | Acetonitril |
| DMAP | Dimethylaminopyridin |
| DMF | N,N-Dimethylformamid |
| DIAD | Düsopropylazodicarboxylat |
| DIPEA | N,N-Diisopropylethylamin |
| EE | Ethylacetat |
| EtOH | Ethanol |
| Fmoc | Fluorenylmethyloxycarbonyl |
| HAc | Essigsäure |
| HATU | O-(7-Azabenzotriazol-1-yl)-N,N,N',N'-tetramethyluronium |
| Fmoc | (9H-Fluoren-9-ylmethoxy)carbonyl |
| MeOH | Methanol |
| RT | Raumtemperatur |
| RF | Rückfluss |
| TEA | Triethylamin |
| Z | Benzyloxycarbonyl |
| >> | Gradient |
| eq | Equivalente |

**Verbindung 1**

Hergestellt nach: Synthesis 2001, 7, 1049-1052

**Verbindung 2**

Hergestellt nach: J. Carbohydrate Chemistry, 1987, 6 (1), 161-165

**Verbindung 3**

Hergestellt analog zu Verbindung 2

**Verbindung 4**

In einer Lösung von 4g der Verbindung 2 in 40ml abs. DMF wurden unter Rühren 2,16g (4eq) trockenes Lithiumazid und 8,02g (2,2eq) Tetrabrommethan gelöst. Die gelbe Lösung wurde bei 0°C mit 3,18g (1,1eq) Triphenylphosphin versetzt, langsam auf RT erwärmt und 20h gerührt. Nach Zugabe von 20ml MeOH wurde 1h bei Raumtemperatur gerührt, wobei es zu geringer Gasentwicklung kam. Das Lösungsmittel wurde entfernt, der Rückstand mit wenig $H_2O$ versetzt und 3x mit Toluol gewaschen, anschließend wurde das Produkt mit EE extrahiert, mit $MgSO_4$ getrocknet, filtriert und konzentriert. Ausbeute: 2,75g einer leicht gelblichen Substanz.

Alternativ wurde Lithiumazid durch Natriumazid ersetzt, welches sich nicht vollständig löst. Nach Zugabe des Methanols wurde zwei Tage bei RT gerührt.

**Verbindung 5**

Hergestellt analog zu Verbindung 4

**Verbindung 6**

2,2g der Verbindung 4 wurden in 100ml MeOH gelöst, 4,45g (3eq) Triphenylphosphin und 6ml $H_2O$ zugegeben und die Suspension 18h bei Raumtemperatur gerührt. Unter Rühren wurden 20ml 20%ige Essigsäure und 90ml $H_2O$ zugegeben, eine halbe Stunde gerührt, die Suspension auf 100ml konzentriert, und dreimal mit 100ml Dichlormethan ausgeschüttelt. Die wässrige Phase wurde lyophilisiert. Ausbeute: 2,67g Feststoff
Alternativ wurde 1 eq HCl statt 20ml 20%iger HAc zugegeben.

**Verbindung 7**

Hergestellt analog zu Verbindung 6

**Verbindung 8**

Eine Lösung aus 620mg Verbindung 2 und 388mg (2eq) Cysteaminhydrochlorid in 2ml MeOH und 6ml $H_2O$ wurde 20min mit Stickstoff gespült. Nach Zugabe einer katalytischen Menge (2-3mg) AIBN wurde 24h mit UV-Licht bestrahlt, das Lösungsmittel entfernt, und der Rückstand über eine Kieselgelsäule gereinigt (MeOH:EE:HAc(20%) 1:4:1). Die Produktfraktionen wurden vereinigt und lyophilisiert. Ausbeute: 700mg eines weißen Feststoffes.

**Verbindung 9**

Eine Lösung von 1,0g Verbindung 6 in 6ml abs. DMF wurde nach Zugabe von 607□L (1,5eq) Diisopropylethylamin und 832mg (1,1eq) 4-Biphenylcarbonsäure-Nitrophenylester 17h bei RT gerührt. Das Lösungsmittel wurde entfernt, über eine Kieselgelsäule (EtOH:EE:HAc(20%) 1:8:1) gereinigt. Ausbeute: 1,06g eines weißer Feststoff.

**Verbindung 10**

Eine Lösung von 460mg Verbindung 9 in 3ml MeOH wurde mit 10eq 3-Mercaptonatriumpropinat in 3ml $H_2O$ versetzt und 20min mit Stickstoff gespült. Nach Zugabe einer katalytischen Menge (2-3mg) AIBN wurde 24h mit UV-Licht bestrahlt. Das Lösungsmittel wurde entfernt, der Rückstand mit $H_2O$ gelöst und über eine RP-18 Säule gereinigt ($H_2O$»MeOH). Die Produktfraktionen wurden vereinigt, das MeOH entfernt und lyophilisiert. Ausbeute: 564mg weißer Feststoff.

**Verbindung 11**

Eine Lösung von 460mg Verbindung 9 in 3ml MeOH wurde mit 193mg (2eq) Cysteaminhydrochlorid in 3ml $H_2O$ versetzt und 20min mit Stickstoff gespült. Nach Zugabe einer katalytischen Menge (2-3mg) AIBN wurde 24h mit UV-Licht bestrahlt, das Lösungsmittel entfernt, der Rückstand mit $H_2O$ gelöst und über eine RP-18 Säule gereinigt (HCl pH4 >> MeOH / HCl pH4). Die Produktfraktionen wurden mit verd. NaOH neutralisiert, das MeOH entfernt und lyophilisiert. Ausbeute: 525mg weißer Feststoff.

**Verbindung 12**

Es wurden 45mg Verbindung 11 in 2ml abs. DMF gelöst, 19,5□l (2eq) TEA und 31mg (1,5eq) Pentafluorophenyl-S-acetylthioglycolsäure zugegeben und über Nacht gerührt. Das Lösungsmittel wurde entfernt, der Rückstand mit wenig MeOH gelöst, $H_2O$ zugegeben bis eine kolloidale Trübung auftrat, über eine RP-18 Säule gereinigt ($H_2O$ >> EtOH) und nach Entfernen des Ethanols lyophilisiert. Ausbeute: 50mg weißer Feststoff.

**Verbindung 13**

Eine Lösung von 40mg Verbindung 12 in 5ml $H_2O$ wurde mit 400□l 2M NaOH versetzt. Nach vollständiger Verseifung (DC-Kontrolle) wurde mit verd. HCl auf pH 9 eingestellt, 24h Luft durch die Lösung geblubbert, und über eine RP18-Säule ($H_2O$>>$CH_3CN$) gereinigt. Ausbeute: 12mg weißer Feststoff.
$^1$H-NMR (300MHz, $CD_3OD$): δ ppm 1.60 (t, *J* = 11.27, 11.27 Hz, 2H, H3-a H3-a'), 1.73-1.83 (m, 4H, CH2CH2CH2 CH2CH2CH2), 2.00 (s, 6H, NHAc NHAc'), 2.55-2.66 (m, 8H, CH2SCH2 CH2SCH2'), 2.79-2.87 (m, 2H, H3-e H3-e'), 3.36 (dt, *J* = 7.06, 7.01, 2.33 Hz, 4H, CH2NH CH2NH'), 3.42-3.93 (m, 20H, H4 H4' H5 H5' H6 H6' H7 H7' H9a H9a' H9b H9b' OCH2a OCH2a' OCH2b OCH2b' COCH2S COCH2S), 4.09 (dt, *J* = 8.28, 8.21, 2.62 Hz, 2H, H8), 7.36 (t, *J* = 7.28, 7.28 Hz, 2H, Ar), 7.45 (t, *J* = 7.37, 7.37 Hz, 4H, Ar), 7.64 (d, *J* = 7.12 Hz, 4H, Ar), 7.69 (d, *J* = 8.41 Hz, 4H, Ar), 7.91 (d,

*J* = 8.35 Hz, 4H, Ar) $^{13}$C NMR (75MHz, CD3OD): δ ppm 22.7 29.4 31.4 32.1 40.7 42.8 43.0 44.6 54.2 63.8 69.6 71.6 72.5 74.3 101.9 128.0 128.2 129.0 129.1 130.1 134.5 141.3 145.5 170.0 171.3 174.47 175.48
HRMS (ESI-neg) [(M-2Na)/2] berechnet: 677,2082 gefunden: 677,2097

**N-(19-Phenyl-3,6,9,12,15,18-Hexaoxa-Nonadecyl) Trifluoroacetamide (Verbindung 14)**

Zu einer Lösung von 800mg 19-Phenyl-3,6,9,12,15,18-Hexaoxa-Nonadecylamonium Acetat in 5ml MeOH wurden 75 μl TEA und 609 μl Trifluoressigsäureethylester zugegeben und 17h bei RT gerührt. Lösungsmittel und überschüssige Reagenzien wurden entfernt und der Rückstand über eine Kieselgelsäule filtriert (EE). Ausbeute: 672mg farbloses Öl.

**N-(17-hydroxy-3,6,9,12,15-pentaoxa-heptadecyl) trifluoroacetamide (Verbindung 15)**

672mg Verbindung 14 wurden in 5ml MeOH gelöst, mit HCl auf pH 3 eingestellt und nach Zugabe von 76mg Pd auf Kohle (10%) 17h unter Normaldruck hydriert. Die Suspension wurde über Celite filtriert, konzentriert und mit Toluol zweifach nachgedampft. Lösungsmittelreste wurden im Ölpumpenvakuum über Nacht entfernt. Ausbeute: 480mg farbloses Öl.

**N-(17-acetylthio-3,6,9,12,15-pentaoxa-heptadecyl) trifluoroacetylamide (Verbindung 16)**

Zu einer Lösung von 1,632g (2,1eq) Triphenylphosphin in 10ml abs. DMF wurden bei 0°C 999μl (1,6eq) DIAD zugetropft und 20min gerührt. Anschließend wurde eine Mischung aus 672mg Verbindung 15 und 933 μl (4eq) Thioessigsäure zugetropft, 4h bei 0°C, dann über Nacht bei RT gerührt. Das Lösungsmittel wurde entfernt und der Rückstand über eine Kieselgelsäule ($CHCl_{3>>}CHCl_3$:MeOH 50:1) und über eine weitere Kieselgelsäule (Diethylether) gereinigt. Man erhält ein farbloses Öl.

**17-Amino-3,6,9,12,15-Pentaoxa-Heptadecanethiol hydrochlorid (Verbindung 17)**

Eine Lösung von Verbindung 16 in 1ml abs. MeOH wurde auf-40°C gekühlt, und mit 1,3ml frisch hergestellter NaOMe-Lösung (auf -40°C gekühlt, 1,3mM) versetzt. Die Lösung wurde innerhalb 30min auf RT erwärmt, mit 1ml $H_2O$ versetzt, nach 20min mit 1M HCl auf pH 6 eingestellt und das Lösungsmittel entfernt. Der Rückstand wurde ohne weitere Reinigung eingesetzt.

**Verbindung 18**

40mg Verbindung 17 und 80mg Verbindung 9 wurden in 2ml $H_2O$-MeOH-Mischung 1:1 gelöst, 20min mit Stickstoff gespült und nach Zugabe einer katalytischen Menge (2-3mg) AIBN 24h mit UV-Licht bestrahlt. Das Lösungsmittel wurde entfernt, der Rückstand mit $H_2O$ gelöst und über eine RP-18 Säule gereinigt (HCl pH4 >> MeOH / HCl pH4). Die Produktfraktionen wurden mit verd. NaOH neutralisiert, das MeOH entfernt und lyophilisiert. Ausbeute: 63mg weißer Feststoff.

**Verbindung 19**

2.4mg (1eq) Adipinsäure, 40mg (2,8eq) Verbindung 18 und 9mg (1,44eq) HATU wurden in 1ml abs. DMF gelöst. Nach Zugabe von 10,3 μl (6,5eq) DIPEA wurde 30min bei RT gerührt, das Lösungsmittel entfernt, der Rückstand in wenig MeOH gelöst, $H_2O$ zugegeben bis eine kolloidale Trübung auftrat und über eine RP-18 Säule ($H_2O$/HAc pH3 >> Dioxan) gereinigt Die Produktfraktionen wurden konzentriert, mit wenig MeOH gelöst, mit $H_2O$ bis zum Auftreten einer Trübung verdünnt und mit 2M NaOH auf pH 12-13 gebracht. Nach Beenden der Reaktion wurde mit 20%HAc neutralisiert und über eine RP18 Säule ($H_2O$ >> Dioxan) gereingt, das MeOH entfernt und lyophilisiert. Ausbeute: 21 mg weißer Feststoff.
$^1$H-NMR (300MHz, $CD_3OD$): δ ppm 1.53-1.65 (m, 6H, CH2CH2CH2CH2, H3-a H3-a'), 1.74-1.84 (m, 4H, CH2CH2CH2 CH2CH2CH2'), 2.00 (s, 6H, NHAc NHAc'), 2.16-2.25 (m, 4H, CH2CH2CH2CH2), 2.64-2.57 (m, 4H, CH2SCH2 CH2SCH2'), 2.66 (t, *J* = 6.78, 6.78 Hz, 4H, CH2SCH2 CH2SCH2'), 2.83 (dd, *J* = 12.14, 4.09 Hz, 2H, H3-e H3-e'), 3.35 (t, *J* = 5.42, 5.42 Hz, 4H, CH2NHCO CH2NHCO'), 3.44 (dd, *J* = 8.95, 1.69 Hz, 2H, H7 H7'), 3.47-3.91 (m, 66H, H4 H4' H5 H5' H6 H6' H9a H9a' H9b H9b' OCH2a OCH2a' OCH2b OCH2b' NHCH2 NHCH2' 3xCH2OCH2CH2OCH2 3xCH2OCH2CH2OCH2'), 4.07 (ddd, *J* = 8.73, 7.63, 3.16 Hz, 2H, H8 H8'), 7.38 (d, *J* = 7.30 Hz, 2H, Ar), 7.46 (t, *J* = 7.35, 7.35 Hz, 4H, Ar), 7.69 (dd, *J* = 15.46, 7.82 Hz, 8H, Ar), 7.93 (d, *J* = 8.63 Hz, 4H, Ar) $^{13}$C NMR (75MHz, CD30D): δ ppm 22.7 26.5 29.9 31.6 32.2 36.8 40.3 42.8 44.6 54.3 64.0 64.5 69.6 71.0 71.1 71.2 71.30 71.35 71.4 71.6 71.8 72. 6 74.3

102.0 128.1 128.2 129.0 129.1 130.1 134.5 141.3 145.6 169.9 174.4 175.5 176.0

**Verbindung 20**

Hergestellt analog zu Verbindung 19
$^1$H-NMR (300MHz, $CD_3OD$): δ1.58 (t, *J* = 11.68, 11.68 Hz, 2H, H3-a H3-a'), 1.73-1.83 (m, 4H, CH2CH2CH2 CH2CH2CH2'), 2.00 (s, 6H, NHAc NHAc'), 2.53-2.70 (m, 8H, CH2SCH2 CH2SCH2'), 2.83 (dd, *J* = 12.21, 3.90 Hz, 2H, H3-e H3-e'), 3.41-3.92 (m, 68H, H4 H4' H5 H5' H6 H6' H7 H7' H9a H9a' H9b H9b' OCH2a OCH2a' OCH2b OCH2b' CH2NH CH2NH' 3x CH2OCH2CH2OCH2 3x CH2OCH2CH2OCH2'), 4.07 (dt, *J* = 8.20, 8.15, 3.19 Hz, 2H, H8 H8'), 7.33-7.49 (m, 6H, Ar), 7.67 (dd, *J* = 14.28, 7.92 Hz, 8H, Ar), 7.89-7.94 (m, 8H, Ar) $^{13}$C NMR (75MHz, CD30D): δ ppm 22.7 29.9 31.6 32.2 41.0 42.8 44.6 54.3 64.0 69.7 70.9 71.1 71.3 71.3 71.5 71.8 72.7 74.3 102.0 128.0 128.2 128.6 129.0 130.1 134.5 138.4 141.3 145.5 169.4 169.9 174.4 175.5
HRMS (ESI-neg) [(M-2Na)/2] berechnet: 889,3672 gefunden: 889,3667

**Verbindung 21**

4,0mg Terephthalsäure (1eq), 38mg (2,4eq) Verbindung 11 und 22mg (2,4eq) HATU wurden in 1ml abs. DMF gelöst. Nach Zugabe von 25μl (6eq) DIPEA wurde 5min bei RT gerührt, das Lösungsmittel entfernt, der Rückstand mit wenig MeOH gelöst, $H_2O$ zugegeben bis eine Trübung auftrat und über eine RP-18 Säule ($H_2O$>>MeOH) gereinigt. Die Produktfraktionen wurden konzentriert, mit wenig MeOH gelöst, mit $H_2O$ bis zum Auftreten einer Trübung verdünnt und mit 2M NaOH auf pH 12-13 gebracht. Nach Beenden der Reaktion wurde mit 20%HAc neutralisiert, über eine RP18 Säule ($H_2O$>>$CH_3CN$) gereinigt, das $CH_3CN$ entfernt und lyophilisiert. Ausbeute: 30mg weißer Fesstoff.
$^1$H-NMR (500MHz, $CD_3OD$): δ ppm 1.57 (t, *J* = 11.85, 11.85 Hz, 2H, H3-a, H3-a'), 1.79-1.86 (m, 4H, CH2CH2CH2 CH2CH2CH2'), 2.01 (s, 6H, NHCOCH3 NHCOCH3'), 2.66 (dt, *J* = 7.12, 7.08, 3.77 Hz, 4H, SCH2CH2CH2 SCH2CH2CH2'), 2.74 (dt, *J* = 6.78, 6.68, 4.69 Hz, 4H, CH2S CH2S'), 2.83 (dd, *J* = 12.25, 4.25 Hz, 2H, H3-e H3-e'), 3.44 (dd, *J* = 8.99, 1.81 Hz, 2H, H7 H7'), 3.47-3.58 (m, 6H, H9a H9a' CH2NHCO CH2NHCO'), 3.59 (td, *J* = 9.55, 6.12, 6.12 Hz, 2H, OCH2a OCH2a'), 3.64 *(dd, J* = 10.45, 1.96 Hz, 2H, H6 H6'), 3.68-3.73 (m, 4H, H4 H4' H5 H5'), 3.85 (dd, *J* = 13.43, 3.25 Hz, 2H, H9b H9b'), 3.90 (td, *J* = 9.50, 6.02, 6.02 Hz, 2H, OCH2b OCH2b'), 4.11 (dt, *J* = 8.70, 8.39, 3.39 Hz, 2H, H8 H8'), 7.35 (t, *J* = 7.36, 7.36 Hz, 2H, Ar), 7.43 (t, *J* = 7.56, 7.56 Hz, 4H, Ar), 7.62 (dd, *J* = 8.29, 1.19 Hz, 4H, Ar), 7.67 (d, *J* = 8.53 Hz, 4H, Ar), 7.86 (s, 4H, Ar), 7.89 (d, *J* = 8.34 Hz, 4H, Ar)$^{13}$C NMR (125MHz, CD30D): δ ppm 22.7 29.4 31.3 32.0 41.0 42.5 44.7 54.1 63.8 69.4 71.5 72.5 74.5 101.4 128.0 128.1 128.6 129.0 129.1 130.0 134.5 138.4 141.3 145.5 169.3 170.0 173.7 175.5
HRMS (ESI-neg) [(M-2Na)/2] berechnet: 669,2362 gefunden: 669,2353

**Verbindungen 22, 23, 24, 25, 26, 27, 28, 29, 30, 31, 32, 33, 34, 35, 36, 37**

Hergestellt analog zu Verbindung 21

**Verbindung 22:**

$^1$H-NMR (300MHz, $CD_3OD$): δ ppm 1.53-1.65 (m, 6H, H3-a H3-a' CH2CH2CH2CH2), 1.72-1.84 (m, 4H, CH2CH2CH2 CH2CH2CH2'), 2.00 (s, 6H, NHCOCH3 NHCOCH3'), 2.14-2.22 (m, 4H, CH2CH2CH2CH2), 2.55-2.64 (m, 8H, CH2SCH2 CH2SCH2'), 2.84 (dd, *J* = 11.63, 3.31 Hz, 2H, H3-e H3-e'), 3.26-3.35 (m, 4H, CH2NH CH2NH'), 3.41-3.93 (m, 16H, H4 H4' H5 H5' H6 H6' H7 H7' H9a H9a' H9b H9b'OCH2a OCH2a' OCH2b OCH2b'), 4.09 (dt, *J* = 8.18, 8.03, 3.24 Hz, 2H, H8 H8'), 7.36 (t, *J* = 7.28, 7.28 Hz, 2H, Ar), 7.45 (t, *J* = 7.51, 7.51 Hz, 4H, Ar), 7.65 (d, *J* = 7.67 Hz, 4H, Ar), 7.70 *(d, J* = 8.34 Hz, 4H, Ar), 7.91 (d, *J* = 8.37 Hz, 4H, Ar) $^{13}$C NMR (75MHz, $CD_3OD$): δ ppm 22.7 26.5 29.3 31.4 32.2 36.8 40.3 42.8 44.6 54.2 63.8 69.6 71.5 72.6 74.3 102.0 128.0 128.2 129.0 129.1 130.1 134.5 134.6 141.3 145.6 170.01 174.4 175.5 175.9
HRMS (ESI-neg) [(M-2Na)/2] berechnet: 659,2518 gefunden: 659,2519

**Verbindung 23:**

$^1$H-NMR (300MHz, $CD_3OD$): δ ppm 1.66-1.52 (m, 2H, H3-a H3-a'), 1.73-1.85 (m, 4H, CH2CH2CH2 CH2CH2CH2'), 2.00 (s, 6H, NHAc NHAc'), 2.56-2.68 (m, 8H, CH2SCH2 CH2SCH2'), 2.79-2.88 (m, 2H, H3-e H3-e'), 3.33-3.94 (m, 20H, H4 H4' H5 H5' H6 H6' H7 H7' H9a H9a' H9b H9b' OCH2a OCH2a' OCH2b OCH2b' CH2NH CH2NH'), 4.09 (dt, *J* = 8.32, 8.14, 2.80 Hz, 2H, H8 H8'), 6.30 (dd, *J* = 11.43, 2.77 Hz, 2H, CHCHCHCH), 7.17 (dd, *J* = 11.26, 2.90 Hz, 2H, CHCHCHCH), 7.32-7.40 (m, 2H, Ar), 7.44 (t, *J* = 7.35, 7.35 Hz, 4H, Ar), 7.66 (dd, *J* = 14.84, 7.78 Hz, 8H, Ar), 7.90 (d, *J* = 8.34 Hz, 4H, Ar) $^{13}$C NMR (75MHz, CD30D): δ ppm 22.7 29.3 31.4 32.1 40.5 42.8 44.6 54.2 63.8 69.7 71.6

72.6 74.3 101.9 128.0 128.2 129.0 129.1 130.1 134.3 141.3 145.6 131.3 138.8 167,8 170.1 175.5 175.6
HRMS (ESI-neg) [(M-2Na)/2] berechnet: 657,2362 gefunden: 657,2396

**Verbindung 24:**

$^1$H-NMR (300MHz, $CD_3OD$): δ ppm 1.60 (dt, *J* = 11.72, 11.60, 7.26 Hz, 2H, H3-a H3-a'), 1.78-1.89 (m, 4H, CH2CH2CH2 CH2CH2CH2'), 2.00 (s, 6H, NHCOCH3 NHCOCH3'), 2.68 (dt, *J* = 7.03, 6.84, 4.36 Hz, 4H, CH2SCH2 CH2SCH2'), 2.76 (t, *J* = 7.06, 7.06 Hz, 4H, CH2SCH2 CH2SCH2'), 2.85 (dd, *J* = 12.16, 3.88 Hz, 2H, H3-e H3-e'), 3.42-3.97 (m, 20H, H4 H4' H5 H5' H6 H6' H7 H7' H9a H9a' H9b H9b' OCH2a OCH2a' OCH2b OCH2b' CH2NH CH2NH'), 4.12 (dt, *J* = 7.96, 7.92, 3.27 Hz, 2H, H8 H8'), 7.30-7.37 (m, 2H, Ar), 7.41 (t, *J* = 7.29, 7.29 Hz, 4H, Ar), 7.58-7.73 (m, 12H, Ar), 7.89 *(d, J* = 8.25 Hz, 8H, Ar) $^{13}$C NMR (75MHz, CD30D): δ ppm 22.7 29.4 31.5 32.1 41.0 42.8 44.6 54.2 63.9 69.7 71.5 72.7 74.3 102.0 128.0 128.2 128.3 129.0 129.1 130.0 141.2 144.3 145.5 169.7 170.0 174.5 175.5
HRMS (ESI-neg) [(M-2Na)/2] berechnet: 707,2518 gefunden: 707,2504

**Verbindung 25**:

$^1$H-NMR (300MHz, $CD_3OD$): δ ppm 1.59 (t, *J* = 11.72, 11.72 Hz, 2H, H3-e H3-e'), 1.73-1.84 (m, 4H, CH2CH2CH2, CH2CH2CH2'), 2.00 (s, 6H, NHCOCH3 NHCOCH3'), 2.51-2.68 (m, 8H, CH2SCH2 CH2SCH2'), 2.83 (dd, *J* = 12.24, 3.93 Hz, 2H, H3-e H3-e'), 3.24 (t, *J* = 6.81, 6.81 Hz, 4H, CH2NH CH2NH'), 3.35-3.94 (m, 16H, H4 H4' H5 H5' H6 H6' H7 H7' H9a H9a' H9b H9b' OCH2a OCH2a' OCH2b OCH2b'), 4.04-4.12 (m, 2H, H8 H8'), 7.36 (t, *J* = 7.25, 7.25 Hz, 2H, Ar), 7.45 (t, *J* = 7.42, 7.42 Hz, 4H, Ar), 7.64 (d, *J* = 7.42 Hz, 4H, Ar), 7.69 (d, *J* = 8.39 Hz, 4H, Ar), 7.91 (d, *J* = 8.37 Hz, 4H, Ar) $^{13}$C NMR (75MHz, CD30D): δ ppm 22.7 29.3 31.4 32.1 40.7 42.8 44.5 54.2 63.8 69.7 71.5 72.6 74.3 102.0 128.0 128.2 129.0 129.1 130.1 134.5 141.3 145.6 153.6 170.0 174.5 175.5
HRMS (ESI-neg) [(M-2Na)/2] berechnet: 643,2205 gefunden: 643,2181

**Verbindung 26:**

$^1$H-NMR (300MHz, $CD_3OD$): δ ppm 1.59 (t, *J* = 11.73, 11.73 Hz, 2H, H3-a H3-a'), 1.76-1.86 (m, 4H, CH2CH2CH2 CH2CH2CH2'), 2.00 (s, 6H, NHAc NHAc'), 2.64 (dt, *J* = 6.87, 6.76, 1.44 Hz, 4H, CH2SCH2 CH2SCH2'), 2.72 (t, *J* = 7.13, 7.13 Hz, 4H, CH2SCH2 CH2SCH2'), 2.84 (dd, *J* = 12.07, 3.48 Hz, 2H, H3-e H3-e'), 3.41-3.94 (m, 20H, H4 H4' H5 H5' H6 H6' H7 H7' H9a H9a' H9b H9b' OCH2a OCH2a' OCH2b OCH2b' CH2NH CH2NH'), 4.10 (dt, *J* = 8.40, 8.25, 3.15 Hz, 2H, H8 H8'), 7.31-7.54 (m, 7H, Ar), 7.59-7.69 (m, 8H, Ar), 7.86-7.95 (m, 6H, Ar), 8.28-8.30 (m, 1H, Ar) $^{13}$C NMR (75MHz, CD30D): δ ppm 22.7 29.3 31.4 32.0 41.0 42.8 44.6 54.2 63.8 69.7 71.5 72.6 74.3 102.0 127.3 128.0 128.1 129.0 129.1 129.9 130.0 131.4 134.5 136.1 141.3 145.5 169.3 170.0 174.5 175.5
HRMS (ESI-neg) [(M-2Na)/2] berechnet: 669,2362 gefunden: 669,2392

**Verbindung 27:**

$^1$H-NMR (300MHz, $CD_3OD$): δ ppm 1.54-1.72 (m, 2H, H3-a H3-a'), 1.76-1.88 (m, 4H, CH2CH2CH2 CH2CH2CH2'), 1.98-2.03 (m, 6H, NHAc NHAc'), 2.57-2.89 (m, 10H, H3-e H3-e' CH2SCH2 CH2SCH2'), 3.40-3.97 (m, 20H, H4 H4' H5 H5' H6 H6' H7 H7' H9a H9a' H9b H9b' OCH2 OCH2' CH2NH CH2NH'), 4.06-4.20 (m, 2H, H8 H8'), 7.32-7.38 (m, 2H, Ar), 7.40-7.47 (m, 4H, Ar), 7.56-7.72 (m, 8H, Ar), 7.85-7.94 (m, 4H, Ar), 8.06 *(d, J* = 8.04 Hz, 1H, Ar), 8.21-8.27 (m, 2H, Ar) $^{13}$C NMR (75MHz, CD30D): δ ppm 22.7 29.4 29.6 31.5 40.4 41.3 42.7 44.6 44.6 54.1 54.2 63.8 63.9 69.6 71.4 71.5 72.5 72.7 74.3 101.91 101.9 122.7 128.0 128.1 129.0 129.1 130.0 133.7 134.50 134.53 137.8 141.3 145.52 145.53 148.8 152.9 165.9 167.5 167.0 174.40 174.43 175.5 175.6
HRMS (ESI-neg) [(M-2Na)/2] berechnet: 669,7338 gefunden: 669,7335

**Verbindung 28**:

$^1$H-NMR (300MHz, $CD_3OD$): 1.68-1.82 (m, 6H, H3-a H3-a' CH2CH2CH2 CH2CH2CH2'), 1.99 (s, 6H, NHAc NHAc'), 2.50-2.62 (m, 8H, CH2SCH2 CH2SCH2'), 2.71 (dd, *J* = 12.73, 4.24 Hz, 2H, H3-e H3-e'), 3.24-3.32 (m, 4H, CH2NH CH2NH'), 3.40-3.93 (m, 20H, H4 H4' H5 H5' H6 H6' H7 H7' H9a H9a' H9b H9b' OCH2a OCH2a' OCH2b OCH2b' CH2ArCH2), 4.08 (dt, *J* = 8.14, 7.97, 3.17 Hz, 2H, H8 H8'), 7.21 (s, 4H, Ar), 7.37 (t, *J* = 7.24, 7.24 Hz, 2H, Ar), 7.45 (t, *J* = 7.55, 7.55 Hz, 4H, Ar), 7.64 (d, *J* = 7.84 Hz, 4H, Ar), 7.69 (d, *J* = 8.19 Hz, 4H, Ar), 7.90 (d, *J* = 8.19 Hz, 4H, Ar) $^{13}$C NMR (75MHz, CD30D): δ ppm 22.7 29.2 31.0 32.1 40.5 42.0 43.6 44.9 54.0 63.7 68.9 71.4 72.3 74.8 100.2 128.1 128.2 129.0 129.1 130.1 130.5 134.4 135.5 141.3 145.6 170.3 172.2 174.1 175.3
HRMS (ESI-neg) [(M-2Na)/2] berechnet: 683,2518 gefunden: 683,2503

**Verbindung 29:**

$^{1}$H-NMR (300MHz, $CD_3OD$): δ ppm 1.68-1.86 (m, 6H, H3-a H3-a' CH2CH2CH2 CH2CH2CH2'), 1.99 (s, 6H, NHAc NHAc'), 2.59-2.75 (m, 10H, H3-e H3-e' CH2SCH2 CH2SCH2'), 3.42-3.95 (m, 20H, H4 H4' H5 H5' H6 H6' H7 H7' H9a H9a' H9b H9b' OCH2a OCH2a' OCH2b OCH2b'CH2NH CH2NH'), 4.09 (dt, *J* = 7.95, 7.94, 3.17 Hz, 2H, H8 H8'), 7.35 (t, *J* = 7.22, 7.22 Hz, 2H, Ar), 7.57 (s, 2H, Ar), 7.61 (d, *J* = 7.45 Hz, 4H, Ar), 7.67 (d, *J* = 8.34 Hz, 4H, Ar), 7.89 (d, *J* = 8.27 Hz, 4H, Ar) $^{13}$C NMR (75MHz, CD30D): δ ppm 22.7 29.3 31.0 31.9 40.9 41.8 44.9 53.9 63.7 68.9 71.4 72.3 74.8 100.2 128.0 128.1 129.1 129.1 129.7 130.0 134.4 141.2 144.0 145.6 163.7 170.3 172.1 175.3
HRMS (ESI-neg) [(M-2Na)/2] berechnet: 672,2144 gefunden: 672,2146

**Verbindung 30**:

$^{1}$H-NMR (300MHz, $CD_3OD$): δ ppm 0.91 (t, *J* = 6.29, 6.29 Hz, 3H, CH3), 1.30-1.44 (m, 4H, CH2CH2CH3), 1.68-1.87 (m, 8H, ArOCH2CH2 H3-a H3-a' CH2CH2CH2 CH2CH2CH2'), 1.99 (s, 6H, NHAc NHAc'), 2.58-2.76 (m, 10H, CH2SCH2 CH2SCH2' H3-e H3-e'), 3.44-4.15 (m, 24H, H4 H4' H5 H5'H6 H6' H7 H7' H8 H8' H9a H9a' H9b H9b' OCH2 OCH2' CH2NHCO CH2NHCO' ArOCH2), 7.29-7.49 (m, 8H, Ar), 7.55-7.68 (m, 8H, Ar), 7.82-7.96 (m, 5H, Ar) $^{13}$C NMR (75MHz, CD30D): δ ppm 14.6 22.7 22.8 23.5 29.0 29.2 29.5 29.9 31.0 31.9 32.2 40.3 41.0 41.8 44.9 53.9 63.7 68.8 70.6 71.4 72.4 74.8 100.1 112.9 120.3 125.6 128.0 128.1 129.0 129.1 130.0, 132.34, 134.32 139.64 141.14 145.49 158.3 167.3 169.0 170.1 170.2 172.1 175.2
HRMS (ESI-neg) [(M-2Na)/2] berechnet: 712.2727 gefunden: 6712.2708

**Verbindung 31:**

$^{1}$H-NMR (300MHz, $CD_3OD$): δ ppm 1.50-1.95 (m, 16H, H3-a H3-a' CH2CH2CH2 CH2CH2CH2', Cyclohexyl), 2.00 (s, 6H, NHCOCH3 NHCOCH3'), 2.53-2.65 (m, 8H, CH2SCH2 CH2SCH2'), 2.83 (dd, *J* = 12.10, 3.57 Hz, 2H, H3-e H3-e'), 3.26-3.31 (m, 4H, CH2NHCO CH2NHCO'), 3.41-3.93 (m, 16H, H4 H4' H5 H5' H6 H6' H9a H9a' H9b H9b' OCH2 OCH2'), 4.09 (dt, *J* = 8.15, 8.06, 3.07 Hz, 2H, H8 H8'), 7.36 (*t, J* = 7.25, 7.25 Hz, 2H, Ar), 7.45 (t, *J* = 7.50, 7.50 Hz, 4H, Ar), 7.64 *(d, J* = 7.75 Hz, 4H, Ar), 7.69 (d. *J* = 8.21 Hz, 4H, Ar), 7.91 (d, *J* = 8.28 Hz, 4H, Ar) $^{13}$C NMR (75MHz, CD30D): δ ppm 22.7 27,6 29.4 31.4 32.2 40.2 42.8 42,9 44.5 54.2 63.9 69.6 71.6 72.5 74.3 102.0 128.0 128.1 129.0 129.1 130.1 134.5 141.3 145.52 170.0 174.5 175.5, 178.1 HRMS (ESI-neg) [(M-2Na)/2] berechnet: 672,2596 gefunden: 672,2631

**Verbindung 32:** $^{1}$H-NMR (300MHz, $CD_3OD$): δ ppm 1.50-1.62 (m, 2H, H3-a H3-a'), 1.75-1.87 (m, 4H. CH2CH2CH2 CH2CH2CH2'), 1.99 (s, 3H, NHCOCH3), 2.00 (s, 3H, NHCOCH3'), 2.58-2.80 (m, 8H, CH2SCH2 CH2SCH2'), 2.82 (dd, *J* = 12.03, 2.46 Hz, 2H, H3-e H3-e'), 3.39-3.95 (m, 20H, H4 H4' H5 H5' H6 H6' H7 H7' H9a H9a' H9b H9b' OCH2a OCH2a' OCH2b OCH2b' CH2NHArNHCH2), 4.10 (dt, *J* = 8.41, 8.26, 3.24 Hz, 2H, H8 H8'), 7.35 (t, *J* = 7.32, 7.32 Hz, 2H, Ar), 7.39-7.48 (m, 5H, Ar), 7.62 (d, *J* = 7.57 Hz, 4H, Ar), 7.67 (dd, *J* = 8.35, 1.62 Hz, 4H, Ar), 7.81 (dd, *J* = 7.94, 1.57 Hz, 1H, Ar), 7.89 (dd, *J* = 8.36, 2.78 Hz, 4H, Ar), 8.02 (d, *J* = 1.53 Hz, 1H, Ar) $^{13}$C NMR (75MHz, CD30D): δ ppm 22.7 29.4 29.5 31.5 31.7 31.9 32.0 40.9 41.1 42.8 44.6 54.2 63.8 69.6 71.5 72.6 74.3 102.0 120.7 128.0 128.2 129.0 129.1 130.0 133.1 134.5 138.3 141.3 142.4 145.6 167.8 170.0 170.2 174.5 175.5
HRMS (ESI-neg) [(M-2Na)/2] berechnet: 708,1914 gefunden: 708,1918

**Verbindung 33**:

$^{1}$H-NMR (300MHz, $CD_3OD$): δ ppm 1.51-1.64 (m, 2H, H3-a H3-a'), 1.76-1.88 (m, 4H, CH2CH2CH2 CH2CH2CH2'), 2.00 (s, 6H, NHCOCH3 NHCOCH3'), 2.61-2.86 (m, 10H, H3-e H3-e' CH2SCH2 CH2SCH2'), 3.41-3.95 (m, 20H, H4 H4' H5 H5' H6 H6' H7 H7' H9a H9a' H9b H9b' OCH2a OCH2a' OCH2b OCH2b' CH2NH CH2NH'), 4.10 (ddd, *J* = 8.97, 8.23, 3.14 Hz, 2H, H8 H8'), 7.35 (t, *J* = 7.22, 7.22 Hz, 2H, Ar), 7.43 (t, *J* = 7.49, 7.49 Hz, 4H, Ar), 7.60-7.69 (m, 9H, Ar), 7.89 (dd, *J* = 8.28, 3.29 Hz, 4H, Ar), 8.16 (dd, *J* = 7.89, 1.55 Hz, 1H, Ar), 8.46 (d, *J* = 1.58 Hz, 1H, Ar) $^{13}$C NMR (75MHz, $CD_3OD$): δ ppm 22.7 29.3 29.4 31.4 31.8 31.9 40.9 41.2 42.8 44.5 54.2 63.8 69.6 71.5 72.6 74.3 101.9 124.6 128.0 128.1 129.0 129.1 130.0 130.7 133.5 134.5 136.2 138.1 141.3 145.5 148.0 167.0 168.7 170.0 174.5 175.5
HRMS (ESI-neg) [(M-2Na)/2] berechnet: 691,7287 gefunden: 691,7339

**Verbindung 34**:

$^{1}$H-NMR (300MHz, $CD_3OD$): δ ppm 1.43 (t, *J* = 11.61, 11.61 Hz, 2H, H3-a H3-a'), 1.78-1.88 (m, 4H, CH2CH2CH2 CH2CH2CH2'), 2.00 (s, 6H, NHCOCH3 NHCOCH3'), 2.62-2.89 (m, 10H, H3-e H3-e' CH2SCH2 CH2SCH2'), 3.39-3.74 (m, 16H, H4 H4' H5 H5' H6 H6' H7 H7' H9a H9a' OCH2a OCH2a' CH2NH CH2NH'), 3.84 (dd, *J* = 13.57, 3.14 Hz, 2H, H9b H9b'), 3.86-3.94 (m, 2H, OCH2b OCH2b'), 4.13 (ddd, *J* = 8.94, 7.94, 3.21 Hz, 2H, H8 H8'), 7.36 (t, *J* = 7.24, 7.24

Hz, 2H, Ar), 7.44 (t, *J* = 7.31, 7.31 Hz, 4H, Ar), 7.53 (dd, *J* = 6.53, 3.32 Hz, 1H, Ar), 7.59-7.69 (m, 10H, Ar), 7.88 (d, *J* = 8.61 Hz, 4H, Ar), 8.22 (dd, *J* = 6.58, 3.28 Hz, 2H, Ar) $^{13}$C NMR (75MHz, CD3OD): δ ppm 22.7 29.6 31.4 32.2 41.0 42.7 44.5 54.2 63.8 69.5 71.5 72.6 74.3 101.9 125.5 126.8 128.0 128.1 128.3 129.0 129.1 130.0 131.6 134.5 137.9 141.3 145.5 170.0 172.0 174.5 175.5
HRMS (ESI-neg) [(M-2Na)/2] berechnet: 694,2440 gefunden: 694,2447

**Verbindung 35:**

$^{1}$H-NMR (300MHz, $CD_3OD$): δ ppm 7.91 (d, *J* = 8.55 Hz, 4H, Ar), 7.70 (d, *J* = 8.44 Hz, 4H, Ar), 7.65 (d, *J* = 7.15 Hz, 4H, Ar), 7.45 (t, *J* = 7.41, 7.41 Hz, 4H, Ar), 7.36 (t, *J* = 7.27, 7.27 Hz, 2H, Ar), 4.08 (ddd, *J* = 8.65, 7.99, 3.20 Hz, 2H, H8 H8'), 3.88 (td, *J* = 9.21, 6.36, 6.36 Hz, 2H, OCH2a OCH2a'), 3.83 (dd, *J* = 13.27, 2.74 Hz, 2H, H9a H9a'), 3.74-3.52 (m, 8H, H4 H4' H5 H5' H6 H6' OCH2b OCH2b'), 3.52 (dd, *J* = 13.55, 7.70 Hz, 2H, H9b H9b'), 3.44 (dd, J= 8.92, 1.17 Hz, 2H, H7 H7'), 3.31-3.26 (m, 4H, CH2NH CH2NH'), 2.83 (dd, *J* = 11.95, 3.62 Hz, 2H, H3-e H3-e'), 2.63-2.55 (m, 8H, CH2SCH2 CH2SCH2'), 2.45 (s, 4H, COCH2CH2CO), 2.00 (s, 6H, CH3 CH3'), 1.84-1.72 (m, 4H, CH2CH2CH2 CH2CH2CH2'), 1.59 (t, *J* = 11.77, 11.77 Hz, 2H, H3-a H3-a') $^{13}$C NMR (125MHz, CD30D): δ ppm 175.5 174.6 174.5 170.0 145.5 141.3 134.5 130.0 129.1 129.0 128.2 128.0 102.0 74.3 72.6 71.5 69.7 63.8 54.2 44.6 42.8 40.4 32.6 32.1 31.4 29.3 22.7
HRMS (ESI-neg) [(M-2Na)/2] berechnet: 645,2362 gefunden: 645,2343

**Verbindung 36:**

$^{1}$H-NMR (500MHz, $CD_3OD$): δ ppm 7.89 (d, *J* = 8.44 Hz, 4H, Ar), 7.67 (d, *J* = 8.01 Hz, 4H, Ar), 7.63 *(d, J* = 8.23 Hz, 4H, Ar), 7.49 (dd, *J* = 7.45, 1.43 Hz, 2H, Ar), 7.44 (t, *J* = 7.71, 7.71 Hz, 4H, Ar), 7.42-7.38 (m, 2H, Ar), 7.38-7.34 (m, 4H, Ar), 7.08 (d, *J* = 7.37 Hz, 2H, Ar), 4.09 (ddd, *J* = 8.59, 8.18, 3.30 Hz, 2H, H8 H8'), 3.84 (dd, *J* = 13.64, 2.99 Hz, 2H, H9a H9a'), 3.84 (td, *J* = 9.03, 6.05, 6.05 Hz, 2H, OCH2a OCH2a'), 3.73-3.68 (m, 4H, H4 H4' H5 H5'), 3.64 (dd, *J* = 10.08, 1.61 Hz, 2H, H6 H6'), 3.53 (td, *J* = 10.25, 6.38, 6.38 Hz, 2H, OCH2b OCH2b'), 3.51 (dd, *J* = 14.02, 7.48 Hz, 2H, H9b H9b'), 3.44 *(dd, J* = 8.97, 1.62 Hz, 2H, H7 H7'), 3.29-3.11 (m, 4H, CH2NH CH2NH'), 2.83 (dd, *J* = 12.16, 4.00 Hz, 2H, H3-e H3-e'), 2.53-2.42 (m, 4H, SCH2 SCH2'), 2.30-2.14 (m, 4H, CH2S CH2S'), 2.00 (s, 6H, CH3 CH3'), 1.75-1.68 (m, 4H, CH2CH2CH2 CH2CH2CH2'), 1.59 (t, *J* = 11.73, 11.73 Hz, 2H, H3-a H3-a') $^{13}$C NMR (500 MHz, CD30D): δ ppm 175.5 174.5 172.4 170.0 145.5 141.3 140.3 137.4 134.5 130.8 130.7 130.1 129.1 129.0 128.4 128.2 128.0 102.0 74.3 72.6 71.6 69.7 63.9 54.2 44.6 42.8 40.4 31.5 31.3 29.3 22.7
HRMS (ESI-neg) [(M-2Na)/2] berechnet: 707,2518 gefunden: 707,2496

**Verbindung 37**:

$^{1}$H-NMR (500MHz, $CD_3OD$): δ ppm 7.91 (d, *J* = 8.25 Hz, 2H, Ar), 7.70 (d, *J* = 8.24 Hz, 2H, Ar), 7.65 (dd, *J* = 8.18, 1.38 Hz, 2H, Ar), 7.45 (t, *J* = 7.80, 7.80 Hz, 2H, Ar), 7.36 (t, *J* = 7.37, 7.37 Hz, 1H, Ar), 4.08 (ddd, *J* = 8.68, 7.99, 3.27 Hz, 2H, H8 H8'), 3.87 (td, *J* = 9.96, 6.27, 6.27 Hz, 2H, OCH2a OCH2a'), 3.84 (dd, *J* = 13.58, 3.23 Hz, 2H, H9a H9a'), 3.73-3.68 (m, 4H, H4 H4' H5 H5'), 3.64 (dd, *J* = 10.27, 1.73 Hz, 2H, H6 H6'), 3.57 (td, *J* = 9.69, 6.22, 6.22 Hz, 2H, OCH2b OCH2b'), 3.51 (dd, *J* = 13.70, 7.76 Hz, 2H, H9b H9b'), 3.44 (dd, *J* = 8.90, 1.81 Hz, 2H, H7 H7'), 3.37-3.32 (m, 4H, CH2NH CH2NH'), 3.19 (dd, *J* = 19.27, 7.94 Hz, 2H, COCH2CO), 2.83 (dd, *J* = 12.21, 3.83 Hz, 2H, H3-e H3-e'), 2.65-2.55 (m, 8H, CH2SCH2 CH2SCH2'), 2.00 (s, 6H, CH3 CH3'), 1.81-1.75 (m, 4H, CH2CH2CH2 CH2CH2CH2'), 1.59 (t, *J* = 11.74, 11.74 Hz, 2H, H3-a H3-a) $^{13}$C NMR (125MHz, CD30D): δ ppm 175.5 174.5 170.0 169.6 145.6 141.3 134.6 130.1 129.1 129.0 128.2 128.1 102.0 74.3 72.7 71.5 69.7 63.8 54.2 44.6 43.8 42.8 40.4 32.0 31.4 29.3 22.7
HRMS (ESI-neg) [(M-2Na)/2] berechnet: 639,2362 gefunden: 639,2320

**Verbindung 38**

Hergestellt analog zu Verbindung 21 mit Verbindung 8 anstelle von Verbindung 11 HRMS (ESI-neg) [(M-2Na)/2] berechnet: 490,1627 gefunden: 490, 1617

**Verbindung 39**

Eine Lösung von 36mg (2,4eq) Verbindung 10 und 20mg (2,4eq) HATU in 1ml abs. DMF wurde mit 22,5μl (6eq) DIPEA versetzt, 5min gerührt und nach Zugabe von 4mg (1eq) p-Phenylendiamindihydrochlorid weitere 5min gerührt. Das Lösungsmittel wurde entfernt, der Rückstand mit wenig MeOH gelöst, $H_2O$ zugegeben bis eine Trübung auftrat und über eine RP-18 Säule ($H_2O$>>MeOH) gereinigt. Die Produktfraktionen wurden konzentriert, mit wenig MeOH gelöst, mit $H_2O$ bis zum Auftreten einer Trübung verdünnt und mit 2M NaOH auf pH 12-13 gebracht. Nach Beenden der

Reaktion wurde mit 20%HAc neutralisiert, über eine RP18 Säule ($H_2O$>>$CH_3CN$) gereinigt, das $CH_3CN$ entfernt und lyophilisiert. Ausbeute: 23mg weißer Feststoff.

$^1H$ NMR (300MHz, CD3OD): δ ppm 1.68-1.77 (m, 2H, H3-a H3-a'), 1.75-1.86 (m, 4H, CH2CH2CH2 CH2CH2CH2'), 1.99 (s, 6H, NHAc NHAc'), 2.55-2.66 (m, 8H, CH2SCH2 CH2SCH2'), 2.67-2.75 (m, 2H, H3-e H3-e'), 2.75-2.83 (m, 4H CH2CO CH2CO'), 3.45-3.94 (m, 16H, H4 H4' H5 H5' H6 H6' H7 H7' H9a H9a' H9b H9b' OCH2a OCH2a' OCH2b OCH2b'), 4.04-4.15 (m, 2H, H8 H8'), 7.14-7.22 (m, 2H, Ar), 7.26-7.48 (m, 8H, Ar), 7.58-7.69 (m, 8H, Ar), 7.86-7.92 (m, 4H, Ar) $^{13}C$ NMR (75MHz, CD3OD): δ ppm 22.8 28.6 29.4 31.0 38.5 41.9 44.6 53.9 63.8 68.9 71.4 72.4 74.8 100.3 113.2 117.1 128.1 128.2 129.0 129.1 130.0 134.4 140.2 141.3 145.6 170.3 172.2 172.6 175.3

HRMS (ESI-neg) [(M-2Na)/2] berechnet: 669,2362 gefunden: 669,2397

**Verbindungen 40, 41, 42, 43, 44, 45, 46**

Hergestellt analog zu Verbindung 39

**Verbindung 40:** $^1H$-NMR (300MHz, $CD_3OD$): δ ppm 1.53-1.64 (m, 2H, H3-a H3-a'), 1.73-1.83 (m, 4H, CH2CH2CH2 CH2CH2CH2'), 2.00 (s, 6H, NHAc NHAc'), 2.45 (t, *J* = 7.17, 7.17 Hz, 4H, CH2SCH2 CH2SCH2'), 2.59 (dt, *J* = 7.07, 7.00, 2.63 Hz, 4H, CH2SCH2 CH2SCH2'), 2.73 (t, *J* = 7.11, 7.11 Hz, 4H, CH2CO CH2CO'), 2.83 (dd, *J* = 12.14, 4.24 Hz, 2H, H3-e H3-e'), 3.30-3.35 (m, 4H, CH2CH2NHCO CH2CH2NHCO'), 3.45 (dd, *J* = 8.87, 1.72 Hz, 2H, H7 H7'), 3.47-3.91 (m, 30H, H4 H4' H5 H5' H6 H6' H9a H9a' H9b H9b' OCH2a OCH2a' OCH2b OCH2b' CH2OCH2CH2OCH2 CH2OCH2CH2OCH2'), 4.09 (dt, *J* = 8.20, 8.13, 3.37 Hz, 2H, H8 H8'), 7.36 (t, *J* = 7.28, 7.28 Hz, 2H, Ar), 7.45 (t, *J* = 7.45, 7.45 Hz, 4H, Ar), 7.68 (dd, *J* = 15.02, 7.76 Hz, 8H, Ar), 7.92 (d, *J* = 8.36 Hz, 4H, Ar) $^{13}C$ NMR (75MHz, CD30D): δ ppm 22.7 28.8 29.4 31.4 37.5 40.5 42.8 44.6 45.0 54.3 64.0 69.7 71.4 70.7 71.4 71.5 71.7 72.7 74.3 102.0 128.0 128.2 129.0 129.1 130.1 134.6 141.3 145.6 170.0 174.5 174.6 175,5

HRMS (ESI-neg) [(M-2Na)/2] berechnet: 802,3101 gefunden: 802,3104

**Verbindung 41:**

$^1H$-NMR (300MHz, $CD_3OD$): δ ppm 1.67-1.78 (m, 2H, H3-a H3-a'), 1.75-1.86 (m, 4H, CH2CH2CH2 CH2CH2CH2'), 1.98 (s, 6H, NHAc NHAc'), 2.56-2.70 (m, 8H, CH2SCH2 CH2SCH2'), 2.73 (dd, *J* = 12.66, 4.29 Hz, 2H, H3-e H3-e'), 2.78-2.87 (m, 4H, CH2CO CH2CO'), 3.42-3.62 (m, 6H, H7 H7' H9a H9a' OCH2a OCH2a'), 3.63-3.95 (m, 10H, H4 H4' H5 H5' H6 H6' H9b H9b' OCH2b OCH2b'), 4.09 (dt, *J* = 8.33, 8.30, 3.24 Hz, 2H, H8 H8'), 7.16 *(dd, J* = 6.01, 3.52 Hz, 2H, Ar), 7.31-7.52 (m, 8H, Ar), 7.59-7.70 (m, 8H, Ar), 7.89 *(d, J* = 8.52 Hz, 4H, Ar) $^{13}C$ NMR (75MHz, CD30D): δ ppm 22.7 28.7 29.5 31.0 38.0 41.9 44.8 54.0 63.8 69.0 71.4 72.3 74.7 100.4 126.5 127.1 128.0 128.2 129.0 129.1 130.1 132.0 134.3 141.3 145.6 170.3 172.6 173.2 175.3

HRMS (ESI-neg) [(M-2Na)/2] berechnet: 669,2362 gefunden: 669,2368

**Verbindung 42:**

$^1H$-NMR (300MHz, $CD_3OD$): δ ppm 1.70 (dd, *J* = 12.14, 11.63 Hz, 2H, H3-a H3-a'), 1.77-1.86 (m, 4H, CH2CH2CH2 CH2CH2CH2'), 1.99 (s, 6H, NHCOCH3 NHCOCH3'), 2.57-2.66 (m, 8H, CH2SCH2 CH2SCH2'), 2.73 *(dd, J* = 12.63, 4.27 Hz, 2H, H3-e H3-e'), 2.81 (t, *J* = 7.14, 7.14 Hz, 4H, CH2CO CH2CO'), 3.47 (dd, *J* = 8.72, 1.52 Hz, 2H, H7 H7'), 3.50-3.60 (m, 4H, OCH2a OCH2a' H9a H9a'), 3.64-3.94 (m, 10H, H4 H4' H5 H5' H6 H6' H9b H9b' OCH2b OCH2b'), 4.09 (ddd, *J* = 8.42, 7.66, 3.29 Hz, 2H, H8 H8'), 7.31-7.50 (m, 10H, Ar), 7.65 (dd, *J* = 16.48, 7.78 Hz, 8H, Ar), 7.90 (d, *J* = 8.59 Hz, 4H, Ar) $^{13}C$ NMR (75MHz, CD30D): δ ppm 22.7 28.7 29.5 31.0 38.4 41.1 44.8 54.0 63.8 69.0 71.4 72.4 74.7 100.5 121.8 128.1 128.3 127.0 129.1 130.1 134.4 136.0 141.3 145.7 170.3 172.5 172.7 175.3

HRMS (ESI-neg) [(M-2Na)/2] berechnet: 669,2362 gefunden: 669,2370

**Verbindung 43:**

$^1H$-NMR (300MHz, $CD_3OD$): δ ppm 1.52-1.22 (m, 8H, CH2CH2CH2CH2CH2CH2), 1.67-1.83 (m, 6H, CH2CH2CH2 CH2CH2CH2' H3-a H3-a'), 1.99 (s, 6H, NHCOCH3 NHCOCH3'), 2.41 (t, *J* = 7.10, 7.10 Hz, 4H, CH2CO CH2CO'), 2.57 (t, *J* = 7.14, 7.14 Hz, 4H, CH2SCH2 CH2SCH2'), 2.66-2.78 (m, 6H, CH2SCH2 CH2SCH2' H3-e H3-e'), 3.12 (t, *J* = 6.85, 6.85 Hz, 4H, NHCH2 NHCH2'), 3.41-3.93 (m, 16H, H4 H4' H5 H5' H6 H6' H7 H7' H9a H9a' H9b H9b' OCH2a OCH2a' OCH2b OCH2b'), 4.08 (dt, *J* = 8.47, 8.21, 2.79 Hz, 2H, H8 H8'), 7.32-7.48 (m, 6H, Ar), 7.66 (dd, *J* = 16.40, 7.46 Hz, 8H, Ar), 7.91 (d, *J* = 7.87 Hz, 4H, Ar) $^{13}C$ NMR (75MHz, CD30D): δ ppm 22.8 27.5 28.8 29.3 30.3 30.9 37.5 40.4 41.9 44.8 54.0 63.8 68.9 71.4 72.3 74.8 100.3 128.0 128.1 129.0 129.1 130.0 134.3 141.2 145.6 170.2 172.4 174.2 175.3

HRMS (ESI-neg) [(M-2Na)/2] berechnet: 687.2831 gefunden: 687.2809

**Verbindung 44:**

$^1H$-NMR (300MHz, $CD_3OD$): 1.66-1.84 (m, 6H, H3-a H3-a' CH2CH2CH2 CH2CH2CH2'), 1.99 (s, 6H, NHAc NHAc'), 2.47 (t, *J* = 7.11, 7.11 Hz, 4H, CH2SCH2 CH2SCH2'), 2.58 (t, *J* = 7.11, 7.11 Hz, 4H, CH2SCH2 CH2SCH2'), 2.68-2.78 (m, 6H, H3-e H3-e' CH2CO CH2CO'), 3.44-3.91 (m, 16H, H4 H4' H5 H5' H6 H6' H7 H7' H9a H9a' H9b H9b' OCH2a OCH2a' OCH2b OCH2b'), 4.07 (dt, *J* = 7.86, 7.85, 3.37 Hz, 2H, H8 H8'), 4.31 (s, 4H, ArCH2), 7.22 (s, 4H, Ar), 7.37 (t, *J* = 7.23, 7.23 Hz, 2H, Ar), 7.45 (t, *J* = 7.37, 7.37 Hz, 4H, Ar), 7.64 *(d, J* = 7.17 Hz, 2H, Ar), 7.69 (d, *J* = 8.49 Hz, 2H, Ar), 7.89 (d, *J* = 8.39 Hz, 4H, Ar) $^{13}C$ NMR (75MHz, CD30D): δ ppm 22.7 28.8 29.4 31.0 37.5 41.9 43.9 44.8 54.0 63.8 68.9 71.4 72.4 74.8 100.3 128.1 128.2 128.8 129.0 129.1 130.1 134.4 138.8 141.3 145.6 170.3 172.3 174.3 175.3
HRMS (ESI-neg) [(M-2Na)/2] berechnet: 683.2518 gefunden: 683.2507

**Verbindung 45:**

$^1H$ NMR (300MHz, CD30D): δ ppm 1.71 (dd, *J* = 12.14, 11.65 Hz, 2H, H3-a H3-a'), 1.79-1.90 (m, 4H, CH2CH2CH2 CH2CH2CH2'), 1.99 (s, 6H, NHCOCH3 NHCOCH3'), 2.10 (s, 12H, 4xArCH3), 2.69 (dd, *J* = 15.09, 7.37 Hz, 8H, CH2SCH2 CH2SCH2'), 2.75 (dd, *J* = 12.69, 4.33 Hz, 2H, H3-e H3-e'), 2.86 (t, *J* = 6.80, 6.80 Hz, 4H, CH2CO CH2CO'), 3.43-3.97 (m, 16H, H4 H4' H5 H5' H6 H6' H7 H7' H9a H9a' H9b H9b' OCH2a OCH2a' OCH2b OCH2b'), 4.08 (dt, *J* = 8.00, 7.93, 3.30 Hz, 2H, H8 H8'), 7.36 (t, *J* = 7.27, 7.27 Hz, 2H, Ar), 7.45 (t, *J* = 7.32, 7.32 Hz, 4H, Ar), 7.64 (d, *J* = 7.06 Hz, 4H, Ar), 7.68 (d, *J* = 8.41 Hz, 4H, Ar), 7.89 (d, *J* = 8.37 Hz, 4H, Ar) $^{13}C$ NMR (75MHz, CD30D): δ ppm 15.7 22.7 28.8 29.5 31.2 37.2 42.1 44.8 54.0 63.9 69.1 71.3 72.5 74.7 100.6 128.1 128.2 129.0 129.1 130.1 134.4 134.6 141.3 145.6 170.2 172.7 175.3
HRMS (ESI-neg) [(M-2Na)/2] berechnet: 713,2624 gefunden: 713,2637

**Verbindung 46:**

$^1H$-NMR (500MHz, $CD_3OD$): δ ppm 7.91 (d, *J* = 8.46 Hz, 4H, Ar), 7.70 (d, *J* = 8.51 Hz, 4H, Ar), 7.65 (dd, *J* = 8.25, 1.07 Hz, 4H, Ar), 7.45 (t, *J* = 7.65, 7.65 Hz, 4H, Ar), 7.36 (t, *J* = 7.38, 7.38 Hz, 2H, Ar), 4.08 (ddd, *J* = 8.95, 7.87, 3.25 Hz, 2H, H8 H8'), 3.86 (td, *J* = 9.65, 6.17, 6.17 Hz, 2H, H9a H9a'), 3.84 (dd, *J* = 13.60, 3.28 Hz, 2H, OCH2a OCH2a'), 3.73-3.68 (m, 4H, H4 H4' H5 H5'), 3.64 (dd, *J* = 10.42, 1.97 Hz, 2H, H6 H6'), 3.58 (td, *J* = 9.72, 6.21, 6.21 Hz, 2H, OCH2b OCH2b'), 3.52 (dd, *J* = 13.67, 7.70 Hz, 2H, H9b H9b'), 3.44 (dd, *J* = 8.96, 1.85 Hz, 2H, H7 H7'), 2.84 (dd, *J* = 12.21, 4.20 Hz, 2H, H3-e H3-e'), 2.71 (dt, *J* = 7.26, 6.99, 3.72 Hz, 4H, CH2S CH2S'), 2.62-2.54 (m, 4H, SCH2 SCH2'), 2.44 (t, *J* = 7.38, 7.38 Hz, 4H, NHCH2CH2NH), 2.00 (s, 6H, COCH3 COCH3'), 1.81-1.74 (m, 4H, CH2CH2CH2 CH2CH2CH2'), 1.59 (t, *J* = 11.85, 11.85 Hz, 2H, H3-a H3-a') $^{13}C$ NMR (125MHz, CD30D): δ ppm 175.5 174.7 174.5 170.0 145.6 141.3 134.5 130.0 129.1 129.0 128.2 128.0 102.0 74.3 72.6 71.5 69.7 63.9 54.244.542.8 40.037.631.329.4 28.7 22.7
HRMS (ESI-neg) [(M-2Na)/2] berechnet: 645,2362 gefunden: 645,2340

**3,4-Di[2-[2-[2-(tert-butyloxycarbonylamino)ethyloxy]ethyloxy]ethylamine]-3-cyclobutene-1,2-dione (Verbindung 47)**

Eine Lösung von 50mg (1eq) 3,4-Diethoxy-3-cyclobutene-1,2-dione und 201mg (2,75eq) Verbindung 247 in 2m1 MeOH wurde nach Zugabe von 0,40m1 (10eq) TEA 2h bei RT gerührt. Anschließend wurden 50μm 2M NaOH zugegeben, 10min bei RT gerührt, das Lösungsmittel entfernt und der Rückstand über eine Kieselgelsäule (EE >> EE:EtOH 5:1) gereinigt. Ausbeute: 140mg farbloses Öl.

**3,4-Di[2-[2-[2-(Amino)ethyloxy]ethyloxy]ethylamine]-3-cyclobutene-1,2-dione dihydrochlorid (Verbindung 48)**

Eine Lösung von 30mg Verbindung 47 in 0,5ml EtOH und 1ml $H_2O$ wurde mit 0,2ml 2M HCl versetzt, über Nacht gerührt, das Lösungmittel entfernt und dreimal mit $H_2O$ gelöst und wieder konzentriert. Ausbeute: 23mg weißer Feststoff.

**Verbindung 49**

Eine Lösung von 5 mg 1,4-Phenylendiisocyanat und 43mg (2,1eq) Verbindung 11 in 1ml DMF wurde mit 16μl DIPEA versetzt, 30min bei RT und 15min auf 50°C gerührt. Das Lösungsmittel wurde entfernt, der Rückstand mit wenig MeOH gelöst, $H_2O$ zugegeben bis eine kolloidale Trübung auftrat und über eine RP-18 Säule ($H_2O$ >> MeOH) gereinigt. Die Produktfraktionen wurden konzentriert, mit wenig MeOH gelöst, mit $H_2O$ bis zum Auftreten einer Trübung verdünnt und mit 2M NaOH auf pH 12-13 gebracht. Nach Beenden der Reaktion wurde mit 20%HAc neutralisiert und über eine RP18 Säule ($H_2O$ >> MeOH) gereingt, das MeOH entfernt und lyophilisiert. Ausbeute: 38mg weißer Feststoff.

$^{1}H$ NMR (300MHz, CD30D): δ ppm 1.53-1.66 (m, 2H, H3-a H3-a'), 1.74-1.86 (m, 4H, CH2CH2CH2 CH2CH2CH2'), 2.00 (s, 6H, NHAc NHAc'), 2.57-2.67 (m, 8H, CH2SCH2 CH2SCH2'), 2.85 (dd, *J* = 12.09, 3.71 Hz, 2H, H3-e H3-e'), 3.31-3.37 (m, 4H, CH2NH CH2NH'), 3.42-3.93 (m, 16H, H4 H4' H5 H5' H6 H6' H7 H7' H9a H9a' H9b H9b'OCH2a OCH2a' OCH2b OCH2b'), 4.09 (dt, *J* = 8.20, 8.09, 3.02 Hz, 2H, H8 H8'), 7.24 (s, 4H, Ar), 7.28-7.47 (m, 6H, Ar), 7.65 (dd, *J* = 15.38, 7.85 Hz, 8H, Ar), 7.90 (d, *J* = 8.20 Hz, 4H, Ar) $^{13}C$ NMR (75MHz, CD30D): δ ppm 22.7 29.2 31.3 33.2 40.3 42.8 44.5 54.2 63.7 69.6 71.6 72.6 74.3 102.0 121.4 128.0 128.2 129.0 129.1 130.0 134.5 135.8 141.3 146.0 158.4 170.0 174.6 175.5 HRMS (ESI-neg) [(M-2Na)/2] berechnet: 684,2471 gefunden: 684,2517

**Verbindung 50**

Eine Lösung von 30 mg Verbindung 11 in 2ml $H_2O$ wurde mit 100μL 2M NaOH versetzt, 2h bei RT gerührt, mit 2M HCl neutralisiert und über eine RP-18-Säule ($H_2O$>>EtOH) von gereinigt.

**Verbindung 51**

Eine Lösung von 15mg Verbindung 11 und 7,0mg (1,8eq) 3,4-Diethoxy-3-cyclobutene-1,2-dione in 1ml MeOH wurde mit 65μl (20eq) TEA versetzt, 30min gerührt, eingeengt, über eine RP-18 Säule ($H_2O$>>EtOH) gereinigt und konzentriert. Der Rückstand wurde in 1ml $H_2O$ gelöst, mit 15mg Verbindung 13 und 100μL 2M NaOH versetzt, 2h gerührt, mit 20%iger HAc neutralisiert, über RP-18 Säule ($H_2O$>>EtOH) gereinigt, das EtOH entfernt und lyophilisiert. Ausbeute: 19mg weißer Feststoff
$^{1}H$-NMR (300MHz, $CD_3OD$): δ ppm 1.59 (t, *J* = 11.73, 11.73 Hz, 2H, H3-a H3-a'), 1.81 (p, *J* = 7.08, 7.08, 6.90, 6.90 Hz, 4H, CH2CH2CH2 CH2CH2CH2'), 1.99 (s, 6H, NHCOCH3 NHCOCH3'), 2.63 (dt, *J* = 7.03, 6.78, 3.06 Hz, 4H, CH2S CH2S'), 2.71 (t, *J* = 6.84, 6.84 Hz, 4H, SCH2 SCH2'), 2.83 (dd, *J* = 12.23, 4.08 Hz, 2H, H3-e H3-e'), 3.45 (dd, *J* = 8.94, 1.36 Hz, 2H, H7 H7'), 3.51-3.76 (m, 14H, H4 H4' H5 H5' H6 H6' H9a H9a' OCH2a OCH2a' CH2NH CH2NH'), 3.81 (dd, *J* = 13.78, 3.31 Hz, 2H, H9b H9b'), 3.88 (td, *J* = 8.50, 5.83, 5.83 Hz, 2H, OCH2b OCH2b'), 4.08 (ddd, *J* = 8.94, 7.61, 3.19 Hz, 2H, H8 H8'), 7.36 (t, *J* = 7.29, 7.29 Hz, 2H, Ar), 7.45 (t, *J* = 7.34, 7.34 Hz, 4H, Ar), 7.65 (dd, *J* = 7.19, 1.51 Hz, 4H, Ar), 7.70 (d, *J* = 8.37 Hz, 4H, Ar), 7.92 (d, *J* = 8.64 Hz, 4H, Ar) $^{13}C$ NMR (75MHz, CD30D): δ ppm 22.7 29.3 31.4 34.1 42.7 44.3 44.5 54.1 63.9 69.7 71.7 72.5 74.2 102.0 128.0 128.1 129.0 130.0 134.5 141.3 145.7 170.0 174.5 175.4 182.3 189.9

**Verbindung 52**

Eine Lösung von 35,0 mg (2,3eq) Verbindung 11 und 2,8 mg (1eq) Terephthaldialdehyd in abs. MeOH wurde mit 5,8μl (2eq) TEA, nach einer Stunde mit 4,6 mg (3eq) Natriumcyanoborhydrid und nach 24h mit 100μl Essigsäure(20%ig) versetzt. Das Lösungsmittel wurde entfernt, der Rückstand mit $H_2O$ gelöst, über eine RP-18 Säule ($H_2O$ >> MeOH) gereinigt, die Produktfraktionen konzentriert und mit 2M NaOH auf pH 12-13 gebracht. Nach Beenden der Reaktion wurde mit 20%HAc neutralisiert, über eine RP18 Säule ($H_2O$>>$CH_3CN$) gereinigt, das $CH_3CN$ entfernt und lyophilisiert. Ausbeute: 7mg weißer Feststoff.
$^{1}H$-NMR (300MHz, $CD_3OD$): δ ppm 1.56 (t, *J* = 11.57, 11.57 Hz, 2H, H3-a H-3a'), 1.70-1.80 (m, 4H, CH2CH2CH2 CH2CH2CH2'), 2.01 (s, 6H, NHCOCH3 NHCOCH3'), 2.59 (t, *J* = 6.90, 6.90 Hz, 4H, CH2S CH2S'), 2.78-2.88 (m, 6H, H3-e H3-e' SCH2 SCH2'), 3.19 (t, *J* = 7.27, 7.27 Hz, 4H, CH2NH CH2NH'), 3.33-3.93 (m, 18H, H4 H4' H5 H5' H6 H6' H7 H7' H9a H9a' H9b H9b' OCH2a OCH2a' OCH2b OCH2b' CH2ArCH2-a), 4.08 (dt, *J* = 8.35, 8.34, 3.13 Hz, 2H, H8 H8'), 4.25 (s, 2H, CH2ArCH2-b), 7.36 (t, *J* = 7.28, 7.28 Hz, 2H, Ar), 7.45 (t, *J* = 7.32, 7.32 Hz, 4H, Ar), 7.58 (s, 4H, Ar), 7.64 (d, *J* = 7.11 Hz, 4H, Ar), 7.69 (d, *J* = 8.42 Hz, 4H, Ar), 7.90 (d, *J* = 8.51 Hz, 4H, Ar)
HRMS (ESI-neg) [(M-2Na)/2] berechnet: 655,2569 gefunden: 655,2548

**Verbindung 53**

Zu einer Lösung von 25mg Verbindung 10 und 16,1 mg (1,1eq) HATU werden 19,9μl (3eq) DIPEA zzugegeben, 5min bei RT gerührt und 7,9mg (1,5eq) 4-Aminobenzoesäure zugegeben. Nach weiteren 5min wird eingeengt und über eine RP-18 ($H_2O$>>EtOH) gereinigt, das EtOH entfernt und lyophilisert. Ausbeute: 15mg weißer Feststoff

**Verbindung 54**

Eine Lösung von 20mg (1eq) Verbindung 53 und 10mg (1eq) HATU in 1ml abs. DMF wurde mit 13,4μl (3eq) DIPEA versetzt, 5min bei RT gerührt, mit 19mg (1,2eq) Verbindung 11 versetzt und 5min gerührt. Das Lösungsmittel wurde entfernt, der Rückstand mit MeOH gelöst, $H_2O$ zugegeben bis eine Trübung auftrat, und über eine RP-18 Säule ($H_2O$ >> MeOH) gereinigt. Das Produkt wurde mit wenig MeOH gelöst, mit $H_2O$ bis zum Auftreten einer Trübung verdünnt

und mit 2M NaOH auf pH 12-13 gebracht. Nach Beenden der Reaktion wurde mit 20%HAc neutralisiert, über eine RP 18 Säule ($H_2O$>>$CH_3CN$) gereinigt, das $CH_3CN$ entfernt und lyophilisiert. Ausbeute: 26mg weißer Feststoff.
$^1$H NMR (300MHz, CD30D): δ ppm, 1.68-1.87 (m, 6H, H3-a H3-a' CH2CH2CH2 CH2CH2CH2'), 1.99 (s, 6H, NHCOCH3 NHCOCH3'), 2.53-2.75 (m, 10H, CH2SCH2 CH2SCH2' H3-e H3-e'), 2.81 (t, *J* = 7.02, 7.02 Hz, 2H, CH2CONH), 3.44-3.94 (m, 18H, H4 H4' H5 H5' H6 H6' H7 H7' H9a H9a' H9b H9b' OCH2 OCH2' CH2NHCO), 4.04-4.13 (m, 2H, H8 H8'), 7.32-7.47 (m, 6H, Ar), 7.58-7.77 (m, 12H, Ar), 7.86-7.94 (m, 4H, Ar) $^{13}$C NMR (75MHz, CD30D): δ ppm 22.7 22.8 28.5 29.0 29.4 30.9 31.0 32.0 38.5 40.1 41.9 44.9 53.9 63.8 68.9 71.4 72.4 74.8 100.2 120.4 128.0 128.1 129.0 129.129.2 130.0 130.6 134.4 141.3 143.0 145.6 170.3 169.6 172.2 172.9 175.3
HRMS (ESI-neg) [(M-2Na)/2] berechnet: 669,2362 gefunden: 6692392

**Verbindung 55**

Hergestellt analog zu Verbindung 9

**Verbindung 56**

Hergestellt analog zu Verbindung 11

**Verbindung 57 und 58**

Hergestellt analog zu Verbindung 21

**Verbindung 57:**

$^1$H-NMR (300MHz, $CD_3OD$): δ ppm 1.21-1.50 (m, 10H, Cyclohexyl Cyclohexyl'), 1.57 (t, *J* = 11.82, 11.82 Hz, 2H, H3-a H3a'), 1.70-1.87 (m, 14H, Cyclohexyl Cyclohexyl' CH2CH2CH2 CH2CH2CH2'), 1.99 (s, 6H, NHAc NHAc'), 2.47-2.59 (m, 2H, Cyclohexyl Cyclohexyl'), 2.65 (dt, *J* = 6.88, 6.71, 1.42 Hz, 4H, CH2SCH2 CH2SCH2'), 2.74 (dt, *J* = 7.11, 7.07, 0.92 Hz, 4H, CH2SCH2 CH2SCH2'), 2.82 (dd, *J* = 12.24, 4.00 Hz, 2H, H3-e H3-e'), 3.40-3.93 (m, 20H, H4 H4' H5 H5' H6 H6' H7 H7' H9a H9a' H9b H9b' OCH2a OCH2a' OCH2b OCH2b' CH2NH CH2NH'), 4.07 (dt, *J* = 7.84, 7.81, 3.23 Hz, 2H, H8 H8'), 7.26 (d, *J* = 8.35 Hz, 4H, Ar), 7.73 (d, *J* = 8.32 Hz, 4H, Ar), 7.90 (s, 4H, Ar) $^{13}$C NMR (75MHz, CD30D): δ ppm 22.7 27.2 27.9 35.4 29.4 31.4 31.9 41.0 42.7 44.4 45.9 54.2 63.8 69.6 71.6 72.5 74.3 102.0 128.0 128.4 128.6 133.3 138.4 153.2 169.3 170.3 174.5 175.5
HRMS (ESI-neg) [(M-2Na)/2] berechnet: 677,2082 gefunden: 677,2097

**Verbindung 58:**

$^1$H-NMR (300MHz, $CD_3OD$): δ ppm 1.28-1.51 1.81-1.89 (m, 24H, Cyclohexyl Cyclohexyl'), 1.56-1.66 (m, 6H, CH2CH2CH2CH2 H3-a H3-a'), 1.72-1.82 (m, 4H, CH2CH2CH2 CH2CH2CH2'),, 1.98 (s, 6H, NHAc NHAc'), 2.15-2.25 (m, 4H, CH2CH2CH2CH2), 2.50-2.67 (m, 10H, CH2SCH2 CH2SCH2' (CH)Cyclohexyl (CH)Cyclohexyl'), 2.83 (dd, *J* = 12.29, 4.15 Hz, 2H, H3-e H3-e'), 3.27-3.36 (m, 4H, CH2NH CH2NH'), 3.38-3.93 (m, 16H, H4 H4' H5 H5' H6 H6' H7 H7' H9a H9a' H9b H9b' OCH2a OCH2a' OCH2b OCH2b'), 4.05 (dt, *J* = 7.77, 7.68, 3.20 Hz, 2H, H8 H8'), 7.29 (d, *J* = 8.28 Hz, 4H, Ar), 7.75 (d, *J* = 8.29 Hz, 4H, Ar) $^{13}$C NMR (75MHz, CD30D): δ ppm 22.7 26.6 27.2 27,9 29.3 31.4 32.2 35.5 36.7 40.3 42.8 44.4 45.9 54.2 63.8 69.6 71.5 72.5 74.3 102.0 128.0 128.5 133.3 153.2 170.3 174.5 175.5 175.9
HRMS (ESI-neg) [(M-2Na)/2] berechnet: 655,2988 gefunden: 655,3010

**Verbindung 59**

Zu einer Lösung von 1,06g (1eq) Verbindung 6 und 660ml TEA (2eq) in 10ml abs. DMF wurden 937mg (1,2eq) Fmoc-NHS zugegeben. Nach 2h rühren wurde das Lösungsmittel entfernt, der Rückstand über eine Kieselgelsäule (EE:EtOH 10:1 >> 5:1) gereinigt, konzentriert und aus $H_2O$-Dioxan lyophilisiert. Ausbeute: 1145mg weißer Feststoff.

**Verbindung 60**

Eine Lösung von 1145mg Verbindung 59 in 5ml MeOH wurde mit 2228mg (10eq) Cysteaminhydrochlorid in versetzt und 20min mit Stickstoff gespült. Nach Zugabe einer katalytischen Menge (2-3mg) AIBN wurde 24h mit UV-Licht bestrahlt, das Lösungsmittel entfernt, der Rückstand mit $H_2O$ gelöst und über eine RP-18 Säule gereinigt (HCl pH4 >> MeOH / HCl pH4). Die Produktfraktionen wurden mit verd. NaOH neutralisiert, das MeOH entfernt und lyophilisiert. Ausbeute: 1030mg weißer Feststoff.

**Verbindung 61**

Eine Lösung von 40mg Terephthalsäure, 370mg (2,2eq) Verbindung 60 und 220mg (2,4eq) HATU in 5ml abs. DMF wurde mit 165ml (4eq) DIPEA versetzt, 5min bei RT gerührt. Das Lösungsmittel wurde entfernt, der Rückstand mit wenig MeOH gelöst, $H_2O$ zugegeben bis eine kolloidale Trübung auftrat und über eine RP-18 Säule ($H_2O$>>MeOH) gereinigt. Die Produktfraktionen wurden konzentriert, mit Dioxan-H2O-Gemisch gelöst und lyophilisiert. Ausbeute: 396mg weißer Feststoff.

**Verbindung 62**

Eine Lösung von 35mg (1eq) Verbindung 61 in 800μl absolutem DMF wurde mit 200μl Piperidin versetzt und 20min bei RT gerührt. Das Lösungsmittel wurde entfernt, der Rückstand mit 1ml abs. DMF gelöst und erneut konzentriert. Nach Lösen des Rückstandes in 1ml abs. DMF wurden 12mg (2,4eq) 2,2'-Bithiothiophene-5-carbonsäure und 22mg (2,4eq) HATU zugegeben, anschließend unter Rühren 25μl (6eq) Dipea zugegeben und 5min bei RT gerührt. Das Lösungsmittel wurde entfernt, der Rückstand mit wenig MeOH gelöst, H2O zugegeben bis eine kolloidale Trübung auftrat, und über eine RP-18 Säule ($H_2O$ >> MeOH) gereinigt. Die Produktfraktionen wurden konzentriert, über Dünnschichtplatten (EtOH:EE:HAc(20%) 1:5:1) gereinigt, mit wenig MeOH gelöst, mit $H_2O$ bis zum Auftreten einer Trübung verdünnt und mit 2M NaOH auf pH 12-13 gebracht. Nach Beenden der Reaktion wurde mit 20%HAc neutralisiert, über eine RP18 Säule ($H_2O$>>$CH_3CN$) gereinigt, das $CH_3CN$ entfernt und lyophilisiert. Ausbeute: 12 mg weißer Feststoff.
$^1$H-NMR (300MHz, $CD_3OD$): δ ppm 1.57 (t, *J* = 11.75, 11.75 Hz, 2H, H3-a H3-a'), 1.87-1.77 (m, 4H, CH2CH2CH2 CH2CH2CH2'), 2.00 (s, 6H, NHCOCH3 NHCOCH3'), 2.66 (t, *J* = 7.10, 7.10 Hz, 4H, CH2SCH2 CH2SCH2'), 2.75 (dt, *J* = 7.12, 7.10, 0.92 Hz, 4H, CH2SCH2 CH2SCH2'), 2.83 (dd, *J* = 12.26, 3.62 Hz, 2H, H3-e H3-e'), 3.42 (dd, *J* = 8.98, 1.57 Hz, 2H, H7 H7'), 3.40-3.74 (m, 10H, H4 H4' H5 H5' H6 H6' H9a H9a' OCH2a OCH2a'), 3.79 (dd, *J* = 13.64, 3.07 Hz, 2H, H9b H9b'), 3.89 (td, *J* = 9.47, 6.18, 6.18 Hz, 2H, OCH2b OCH2b'), 4.06 (ddd, *J* = 8.94, 8.07, 3.05 Hz, 2H, H8 H8'), 7.04 (dd, *J* = 5.12, 3.65 Hz, 2H, Ar), 7.16 (d, *J* = 3.92 Hz, 2H, Ar), 7.28 (dd, *J* = 3.63, 1.11 Hz, 2H, Ar), 7.39 (dd, *J* = 5.12, 1.12 Hz, 2H, Ar), 7.59 (d, *J* = 3.95 Hz, 2H, Ar), 7.88 (s, 4H, Ar) $^{13}$C NMR (75MHz, CD3OD): δ ppm 22.7 29.4 31.5 32.0 41.0 42.8 44.4 54.2 63.8 69.6 71.5 72.5 74.3 102.0 125.1 126.1 127.0 128.6 129.2 130.5 134.5 137.6 138.4 138.5 143.3 164.2 169.4 175.5
HRMS (ESI-neg) [(M-2Na)/2] berechnet: 681,1490 gefunden: 681,1490

**Verbindung 63, 64, 65, 66, 67, 68, 69, 70, 71, 72, 73, 74, 75, 76, 77, 78, 79, 80, 81, 82, 83**

Hergestellt analog zu Verbindung 62. Es wurde Verbindng 130 eingesetzt.

**Verbindung 63:**

$^1$H-NMR (300MHz, $CD_3OD$): δ ppm 1.51-1.62 (m, 2H, H3-a H3-a'), 1.78-1.84 (m, 4H, CH2CH2CH2 CH2CH2CH2'), 1.99 (s, 6H, NHAc NHAc'), 2.65 (t, *J* = 6.86, 6.86 Hz, 4H, CH2SCH2 CH2SCH2'), 2.74 (dt, *J* = 7.20, 7.07, 1.46 Hz, 4H, CH2SCH2 CH2SCH2'), 2.82 (dd, *J* = 12.13, 3.14 Hz, 2H, H3-e H3-e'), 3.37-3.94 (m, 20H, H4 H4' H5 H5' H6 H6' H7 H7' H9a H9a' H9b H9b' OCH2a OCH2a' OCH2b OCH2b' CH2NH CH2NH'), 4.07 (dt, *J* = 8.02, 7.97, 3.05 Hz, 2H, H8 H8'), 6.96 (d, *J* = 8.83 Hz, 4H, Ar), 7.02 (dd, *J* = 8.63, 1.06 Hz, 4H, Ar), 7.17 (t, *J* = 7.42, 7.42 Hz, 2H, Ar), 7.38 (dd, *J* = 8.53, 7.42 Hz, 4H, Ar), 7.82 (d, *J* = 8.79 Hz, 4H, Ar), 7.89 (s, 4H, Ar) $^{13}$C NMR (75MHz, CD30D): δ ppm 22.7 29.4 31.5 32.0 41.1 42.8 44.5 54.2 63.8 69.6 71.5 72.6 74.3 102.0 118.6 120.9 125.5 128.6 130.2 130.4 131.2 138.4 157.5 162.0 169.4 169.6 173.7 175.5
HRMS (ESI-neg) [(M-2Na)/2] berechnet: 685,2311 gefunden: 685,2362

**Verbindung 64:**

$^1$H-NMR (300MHz, $CD_3OD$): δ ppm 1.57 (t, *J* = 11.77, 11.77 Hz, 2H, H3-a H3-a'), 1.74-1.87 (m, 4H, CH2CH2CH2 CH2CH2CH2'), 2.00 (s, 6H, NHCOCH3 NHCOCH3'), 2.62-2.70 (m, 4H, CH2SCH2 CH2SCH2'), 2.74 (dt, *J* = 7.13, 7.02, 1.38 Hz, 4H, CH2SCH2 CH2SCH2'), 2.83 (dd, *J* = 12.23, 4.03 Hz, 2H, H3-e H3-e'), 3.41-3.74 (m, 16H, H4 H4' H5 H5' H6 H6' H7 H7' H9a H9a' OCH2a OCH2a' CH2NHCO CH2NHCO'), 3.84 (dd, *J* = 13.60, 3.29 Hz, 2H, H9b H9b'), 3.85-3.94 (m, 2H, OCH2b OCH2b'), 4.10 (ddd, *J* = 8.91, 8.00, 3.28 Hz, 2H, H8 H8'), 7.05 (t, *J* = 8.74, 8.74 Hz, 4H, Ar), 7.50 (t, *J* = 8.50, 8.50 Hz, 2H, Ar), 7.57 (dd, *J* = 8.35, 1.49 Hz, 4H, Ar), 7.87 (s, 4H, Ar), 7.90 (d, *J* = 8.45 Hz, 4H, Ar) $^{13}$C NMR (75MHz, CD3OD): δ ppm 22.7 29.4 31.5 32.0 41.0 42.8 44.6 54.2 63.8 69.6 71.4 72.7 74.3 102.0 105.3 (dd, *J* = 26.80, 25.89 Hz) 112.9 (dd, *J* = 21.42, 3.91 Hz) 128.6 128.7 130.0 130.1 133.01 (dd, *J* = 9.69, 4.65 Hz) 135.1 138.4 139.4 164.34 (dd, *J* = 223.41, 12.15 Hz) 169.3 169.8 174.5 175.5 HRMS (ESI-neg) [(M-2Na)/2] berechnet: 705,2173 gefunden: 705,2179

**Verbindung 65:**

$^{1}$H-NMR (300MHz, $CD_3OD$): $\delta$ ppm 1.59 (t, $J$ = 11.67, 11.67 Hz, 2H, H3-a H3-a'), 1.76-1.87 (m, 4H, CH2CH2CH2 CH2CH2CH2'), 2.00 (s, 6H, NHCOCH3 NHCOCH3'), 2.65 (t, $J$ = 7.09, 7.09 Hz, 4H, SCH2CH2CH2 SCH2CH2CH2'), 2.74 (t, $J$ = 6.99, 6.99 Hz, 4H, CH2S CH2S'), 2.82 (dd, $J$ = 12.18, 3.64 Hz, 2H, H3-e H3-e'). 3.39-3.75 (m, 16H, H4 H4' H5 H5' H6 H6' H7 H7' H9a' H9b' CH2NH CH2NH' OCH2a OCH2a'), 3.81 (dd, $J$ = 13.49, 3.14 Hz, 2H, H9b H9b'), 3.89 (td, $J$ = 9.60, 6.16, 6.16 Hz, 2H, OCHb OCH2b'), 4.08 (ddd, $J$ = 8.80, 8.19, 3.05 Hz, 2H, H8 H8'), 7.26-7.45 (m, 8H, Ar), 7.59-7.70 (m, 6H, Ar), 7.87 (s, 4H, Ar) $^{13}$C NMR (75MHz, CD30D): $\delta$ ppm 22.7 29.4 31.4 32.0 41.0 42.7 44.4 54.2 63.8 69.6 71.6 72.5 74.3 101.8 124.8 124.9 127.0 127.1 128.6 129.6 129.7 130.2 130.7 134.9 138.4 139.0 150.2 164.4 169.3 174.3 175.5

**Verbindung 66:**

$^{1}$H-NMR (300MHz, $CD_3OD$): $\delta$ ppm 1.54 (t, $J$ = 11.66, 11.66 Hz, 2H, H3-a H3-a'), 1.70-1.81 (m, 4H, CH2CH2CH2 CH2CH2CH2'), 1.93 (s, 6H, NHCOCH3 NHCOCH3'), 2.61 (t, $J$ = 7.18, 7.18 Hz, 4H, SCH2CH2CH2 SCH2CH2CH2'), 2.71 (dt, $J$ = 6.85, 6.85, 3.64 Hz, 4H, SCH2 SCH2'), 2.81 (dd, $J$ = 12.22, 4.11 Hz, 2H, H3-e H3-e'), 3.21 (dd, $J$ = 13.65, 8.01 Hz, 2H, H9a H9a'), 3.30-3.36 (m, 2H, H7 H7'), 3.46-3.75 (m, 18H, H4 H4' H5 H5' H6 H6' H9b H9b' CH2NH CH2NH' CH2Ar CH2Ar' OCH2a OCH2a'), 3.82 (td, $J$ = 9.52, 6.05, 6.05 Hz, 2H, OCH2b OCH2b'), 3.95 (dt, $J$ = 8.31, 8.23, 2.87 Hz, 2H, H8 H8'), 7.37-7.46 (m, 6H, Ar), 7.74-7.81 (m, 6H, Ar), 7.89 (s, 4H, Ar) $^{13}$C NMR (75MHz, CD30D): $\delta$ ppm 22.6 29.4 31.5 32.0 41.1 42.8 44.0 44.1 54.2 63.7 69.6 71.4 72.3 74.2 101.9 126.7 127.2 128.4 128.6 128.7 128.8 128.9 129.2 133.9 134.7 135.0 138.4 169.4 174.1 174.5 175.5
HRMS (ESI-neg) [(M-2Na)/2] berechnet: 657,2362 gefunden: 657,2367

**Verbindung 67:**

$^{1}$H-NMR (300MHz, $CD_3OD$): $\delta$ ppm 1.58 (t, $J$ = 11.73, 11.73 Hz, 2H, H3-a H3-a'), 1.77-1.87 (m, 4H, CH2CH2CH2 CH2CH2CH2'), 2.00 (s, 6H, NHCOCH3 NHCOCH3'), 2.65 (t, $J$ = 6.98, 6.98 Hz, 4H, CH2S CH2S'), 2.73 (t, $J$ = 7.12, 7.12 Hz, 4H, SCH2 SCH2'), 2.83 (dd, $J$ = 11.89, 3.30 Hz, 2H, H3-e H3-e'), 3.41-3.75 (m, 16H, H4 H4' H5 H5' H6 H6' H7 H7' H9a H9a' OCH2a OCH2a' CH2NH CH2NH'), 3.80-3.94 (m, 4H, H9b H9b' OCH2b OCH2b'), 4.10 (dt, $J$ = 8.20, 8.10, 3.17 Hz, 2H, H8 H8'), 6.85 (d, $J$ = 8.61 Hz, 4H, Ar), 7.47 (d, $J$ = 8.48 Hz, 4H, Ar), 7.59 (d, $J$ = 8.26 Hz, 4H, Ar), 7.84 (d, $J$ = 8.57 Hz, 4H, Ar), 7.85 (s, 4H, Ar) $^{13}$C NMR (75MHz, CD30D): $\delta$ ppm 22.7 29.3 31.5 31.9 41.0 42.8 44.5 54.2 63.8 69.6 71.5 72.6 74.3 102.0 116.8 127.3 128.6 128.9 129.3 132.5 133.5 138.4 145.5 158.9 169.3 170.1 174.5 175.5
HRMS (ESI-neg) [(M-2Na)/2] berechnet: 685,2311 gefunden: 685,2311

**Verbindung 68:**

$^{1}$H-NMR (300MHz, $CD_3OD$): $\delta$ ppm 1.62 (t, $J$ = 11.41, 11.41 Hz, 2H, H3-a H3-a'), 1.77-1.87 (m, 4H, CH2CH2CH2), 2.00 (s, 6H, NHCOCH3 NHCOCH3'), 2.61-2.83 (m, 10H, CH2SCH2 CH2SCH2' H3-e H3-e'), 3.42-3.96 (m, 20H, H4 H4' H5 H5' H6 H6' H7 H7' H9a H9a' H9b H9b' OCH2a OCH2a' OCH2b OCH2b' CH2NH CH2NH'), 4.12 (dt, $J$ = 8.20, 8.08, 3.10 Hz, 8H, H8 H8'), 7.73 (d, $J$ = 8.51 Hz, 8H, Ar), 7.84 (s, 4H, Ar), 7.92 (d, $J$ = 8.25 Hz, 4H, Ar), 8.08 (d, $J$ = 8.28 Hz, 4H, Ar)
HRMS (ESI-neg) [(M-2Na)/2] berechnet: 713,2260 gefunden: 713,2239

**Verbindung 69:**

$^{1}$H-NMR (300MHz, $CD_3OD$): $\delta$ ppm 1.57 (t, $J$ = 11.69, 11.69 Hz, 2H, H3-a H3-a'), 1.77-1.87 (m, 4H, CH2CH2CH2 CH2CH2CH2'), 2.02 (s, 6H, NHCOCH3 NHCOCH3'), 2.66 (t, $J$ = 7.02, 7.02 Hz, 4H, CH2S CH2S'), 2.71-2.78 (m, 4H, SCH2 SCH2'), 2.83 (dd, $J$ = 12.51, 4.04 Hz, 2H, H3-e H3-e'), 3.41-3.97 (m, 20H, H4 H4' H5 H5' H6 H6' H7 H7' H9a H9a' H9b H9b' OCH2a OCH2a' OCH2b OCH2b' CH2NH CH2NH'), 4.13 (dt, $J$ = 8.28, 8.22, 2.88 Hz, 2H, H8 H8'), 7.53 (d, $J$ = 7.99 Hz, 2H, Ar), 7.71 (d, $J$ = 8.56 Hz, 2H, Ar), 7.86 (s, 4H, Ar), 8.26 (dd, $J$ = 7.95, 1.79 Hz, 2H, Ar), 8.61 (dd, $J$ = 8.47, 2.35 Hz, 2H, Ar), 8.75 (d, $J$ = 1.43 Hz, 2H, Ar), 9.03 (d, $J$ = 2.23 Hz, 2H, Ar) $^{13}$C NMR (75MHz, CD3OD): $\delta$ ppm 22.7 29.4 31.5 32.0 41.0 42.8 44.9 54.2 63.8 69.6 71.4 72.7 74.3 102.0 121.1 125.0 128.6 129.0 132.4 133.6 133.9 136.4 138.0 138.4 141.0 148.1 148.5 149.4 167.3 169.3 174.5 175.6
HRMS (ESI-neg) [(M-2Na)/2] berechnet: 804,1914 gefunden: 804,1964

**Verbindung 70:**

$^{1}$H-NMR (300MHz, $CD_3OD$): $\delta$ ppm 1.69 (t, $J$ = 11.97, 11.97 Hz, 2H, H3a H3a'), 1.77-1.87 (m, 4H, CH2CH2CH2

CH2CH2CH2'), 1.99 (s, 6H, NHCOCH3 NHCOCH3'), 2.61-2.77 (m, 10H, H3-e H3-e' CH2SCH2 CH2SCH2'), 3.43-3.95 (m, 20H, H4 H4' H5 H5' H6 H6' H7 H7' H9a H9a' H9b H9b' OCH2a OCHa' OCH2b OCH2b' CH2NH CH2NH'), 4.09 (dt, *J* = 7.93, 7.92, 3.21 Hz, 2H, H8 H8'), 7.36-7.42 (m, 4H, Ar), 7.82-7.89 (m, 8H, Ar), 7.93 (s, 2H, Ar)
HRMS (ESI-neg) [(M-2Na)/2] berechnet: 649,1769 gefunden: 649,1724

**Verbindung 71:**

$^{1}$H-NMR (300MHz, $CD_3OD$): $\delta$ ppm 1.57 (t, *J* = 11.68, 11.68 Hz, 2H, H3-a H3-a'), 1.77-1.87 (m, 4H, CH2CH2CH2 CH2CH2CH2'), 2.00 (s, 6H, NHCOCH3 NHCOCH3'), 2.66 (t, *J* = 7.08, 7.08 Hz, 4H, CH2S CH2S'), 2.75 (dt, *J* = 7.10, 7.02, 2.21 Hz, 4H, SCH2 SCH2'), 2.83 (dd, *J* = 12.07, 3.85 Hz, 2H, H3-e H3-e'), 3.39-3.74 (m, 16H, H4 H4' H5 H5' H6 H6' H7 H7' H9a H9a' OCH2a OCH2a' CH2NH CH2NH'), 3.79-3.92 (m, 4H, H9b H9b' OCH2b OCH2b'), 4.09 (dt, *J* = 8.31, 8.20, 3.17 Hz, 2H, H8 H8'), 7.74 (d, *J* = 8.36 Hz, 4H, Ar), 7.90 (s, 4H, Ar), 7.99 (d, *J* = 8.15 Hz, 4H, Ar) $^{13}$C NMR (75MHz, CD30D): $\delta$ ppm 22.7 29.3 31.3 32.0 41.1 42.8 44.7 54.2 63.8 69.6 71.3 72.7 74.3 102.0 126.5 128.6 129.2 138.4 139.7 169.0 169.4 174.5 175.6 HRMS (ESI-neg) [(M-2Na)/2] berechnet: 661,1922 gefunden: 661,1973

**Verbindung 72:**

$^{1}$H-NMR (500MHz, $CD_3OD$): $\delta$ ppm 1.56 (t, *J* = 11.71, 11.71 Hz, 2H, H3-a H3-a'), 1.78-1.85 (m, 4H, CH2CH2CH2 CH2CH2CH2'), 2.00 (s, 6H, NHCOCH3 NHCOCH3'), 2.66 (dt, *J* = 7.02, 6.86, 2.63 Hz, 4H, CH2S CH2S'), 2.75 (dd, *J* = 12.31, 6.75 Hz, 4H, SCH2 SCH2'), 2.82 (dd, *J* = 12.25, 3.85 Hz, 2H, H3-e H3-e'), 3.42 (dd, *J* = 8.97, 1.88 Hz, 2H, H7 H7'), 3.47 (dd, *J* = 13.68, 7.86 Hz, 2H, H9a H9a'), 3.51-3.73 (m, 12H, H4 H4' H5 H5' H6 H6' OCH2a OCH2a' CH2NH CH2NH'), 3.80 (dd, *J* = 13.57, 3.19 Hz, 2H, H9b H9b'), 3.88 (td, *J* = 9.71, 6.19, 6.19 Hz, 2H, OCH2b OCH2b'), 4.07 (dt, *J* = 8.71, 8.39, 3.24 Hz, 2H, H8 H8'), 7.43 (d, *J* = 8.84 Hz, 4H, Ar), 7.80 (d, *J* = 8.84 Hz, 4H, Ar), 7.90 (s, 4H, Ar) $^{13}$C NMR (125MHz, CD30D): $\delta$ ppm 22.7 29.4 31.5 32.0 41.1 42.8 44.6 54.2 63.8 69.6 71.4 72.6 74.3 102.0 128.6 129.7 130.1 134.6 138.4 138.6 169.2 169.4 174.5 175.5
HRMS (ESI-neg) [(M-2Na)/2] berechnet: 627,1659 gefunden: 627,1640

**Verbindung 73:**

$^{1}$H-NMR (300MHz, $CD_3OD$): $\delta$ ppm 1.57 (t, *J* = 11.80, 11.80 Hz, 2H, H3-a H3-a'), 1.76-1.87 (m, 4H, CH2CH2CH2 CH2CH2CH2'), 1.99 (s, 6H, NHCOCH3 NHCOCH3'), 2.13 (s, 6H, NHCOCH3 NHCOCH3'), 2.61-2.69 (m, 4H, CH2S CH2S'), 2.70-2.77 (m, 4H, SCH2 SCH2'), 2.82 (dd, *J* = 12.22, 4.05 Hz, 2H, H3-e H3-e'), 3.39-3.73 (m, 16H, H4 H4' H5 H5' H6 H6' H7 H7' H9a H9a' OCH2a OCH2a' CH2NH CH2NH'), 3.80 (dd, *J* = 13.64, 3.10 Hz, 2H, H9b H9b'), 3.88 (td, *J* = 9.66, 6.03, 6.03 Hz, 2H, OCH2b OCH2b'), 4.07 (dt, *J* = 8.18, 7.96, 2.98 Hz, 2H, H8 H8'), 7.63 (d, *J* = 8.74 Hz, 4H, Ar), 7.78 (d, *J* = 8.74 Hz, 4H, Ar), 7.89 (s, 4H, Ar) $^{13}$C NMR (75MHz, CD30D): $\delta$ ppm 22.7 24.1 29.4 31.5 32.0 41.0 42.8 44.4 54.2 63.8 69.6 71.5 72.5 74.3 102.0 120.3 128.6129.2 130.8 138.4 143.1 169.4 169.7 171.9 174.5 175.5
HRMS (ESI-neg) [(M-2Na)/2] berechnet: 650,2263 gefunden: 650,2258

**Verbindung 74:**

$^{1}$H-NMR (300MHz, $CD_3OD$): $\delta$ ppm 1.58 (t, *J* = 11.80, 11.80 Hz, 2H, H3-a H3-a'), 1.77-1.87 (m, 4H, CH2CH2CH2 CH2CH2CH2'), 2.01 (s, 6H, NHCOCH3 NHCOCH3'), 2.66 (dt, *J* = 7.34, 7.20, 0.97 Hz, 4H, CH2S CH2S'), 2.74 (dt, *J* = 7.25, 7.14, 1.46 Hz, 4H, SCH2 SCH2'), 2.83 (dd, *J* = 12.14, 4.08 Hz, 2H, H3-e H3-e'), 3.45 (dd, *J* = 8.95, 1.70 Hz, 2H, H7 H7'), 3.47-3.79 (m, 14H, H4 H4' H5 H5' H6 H6' H9a H9a' OCH2a OCH2a' CH2NH CH2NH'), 3.77 (s, 6H, OCH3 OCH3'), 3.84 (dd, *J* = 13.57, 3.24 Hz, 2H, H9b H9b'), 3.90 (td, *J* = 9.68, 6.13, 6.13 Hz, 2H, OCH2b OCH2b'), 4.11 (ddd, *J* = 8.74, 7.80, 3.30 Hz, 2H, H8 H8'), 7.00 (dt, *J* = 7.45, 7.45, 1.08 Hz, 2H, Ar), 7.06 (dd, *J* = 8.32, 0.89 Hz, 2H), 7.27 (dd, *J* = 7.59, 1.66 Hz, 2H, Ar), 7.32 (dd, *J* = 7.33, 1.74 Hz, 2H, Ar), 7.34 (dd, *J* = 7.37, 1.75 Hz, 4H, Ar), 7.54 (d, *J* = 8.63 Hz, 4H, Ar), 7.83 (d, *J* = 8.62 Hz, 4H, Ar), 7.86 (s, 4H, Ar) $^{13}$C NMR (75MHz, CD3OD): $\delta$ ppm 22.7 29.4 31.5 31.9 41.0 42.8 44.6 54.2 56.1 63.8 69.6 71.4 72.7 74.3 102.0 112.7 122.0 127.9 128.6 130.5 130.7 130.9 131.6 134.0 138.4 143.5 158.0 169.3 170.2 174.5 175.5
HRMS (ESI-neg) [(M-2Na)/2] berechnet: 699,2467 gefunden: 699,2469

**Verbindung 75:**

$^{1}$H-NMR (500MHz, $CD_3OD$): $\delta$ ppm 1.57 (t, *J* = 11.87, 11.87 Hz, 2H, H3-a H3-a'), 1.79-1.85 (m, 4H, CH2CH2CH2 CH2CH2CH2'), 2.00 (s, 6H, NHCOCH3 NHCOCH3'), 2.66 (dt, *J* = 7.09, 7.05, 3.84 Hz, 4H, CH2S CH2S'), 2.74 (dt, *J* = 6.86, 6.75, 4.55 Hz, 4H, SCH2 SCH2'), 2.83 (dd, *J* = 12.21, 4.22 Hz, 2H, H3-e H3-e'), 3.45 (dd, *J* = 8.89, 1.93 Hz, 2H, H7 H7'), 3.49-3.74 (m, 14H, H4 H4' H5 H5' H6 H6' H9a H9a' OCH2a OCH2a' CH2NH CH2NH'), 3.84 (dd, *J* = 13.62,

3.31 Hz, 2H, H9b H9b'), 3.90 (td, *J* = 9.62, 6.22, 6.22 Hz, 2H, OCH2b OCH2b'), 4.11 (ddd, *J* = 8.84, 7.76, 3.30 Hz, 2H, H8 H8'), 7.33-7.37 (m, 6H, Ar), 7.46-7.50 (m, 6H, Ar), 7.86-7.91 (m, 8H, Ar) $^{13}$C NMR (125MHz, CD30D): δ ppm 22.7 29.4 31.5 31.9 41.0 42.8 44.6 54.2 63.8 69.7 71.5 72.6 74.3 102.0 112.3 128.2 128.4 128.6 130.4 130.7 131.1 132.4 133.3 135.1 138.4 141.0 144.0 169.3 170.0 174.5 175.5
HRMS (ESI-neg) [(M-2Na)/2] berechnet: 703,1972 gefunden: 703,1992

**Verbindung 76:**

$^1$H-NMR (300MHz, $CD_3OD$): δ ppm 1.57 (t, *J* = 11.76, 11.76 Hz, 2H, H3-a H3-a'), 1.75-1.88 (m, 4H, CH2CH2CH2 CH2CH2CH2'), 2.00 (s, 6H, NHCOCH3 NHCOCH3'), 2.36 (s, 6H, ArCH3 ArCH3'), 2.66 (dt, *J* = 7.19, 7.11, 1.11 Hz, 4H, CH2S CH2S'), 2.73 (dt, *J* = 7.04, 6.95, 1.14 Hz, 4H, SCH2 SCH2'), 2.83 (dd, *J* = 12.19, 4.00 Hz, 2H, H3-e H3-e'), 3.45 (dd, *J* = 8.96, 1.42 Hz, 2H, H7 H7'), 3.48-3.74 (m, 14H, H4 H4' H5 H5' H6 H6' H9a H9a' OCH2a OCH2a' CH2NH CH2NH'), 3.84 (dd, *J* = 13.61, 3.18 Hz, 2H, H9b H9b'), 3.90 (td, *J* = 10.39, 6.52, 6.52 Hz, 2H, OCH2b OCH2b'), 4.10 (ddd, *J* = 8.80, 7.84, 3.23 Hz, 2H, H8 H8'), 7.24 (d, *J* = 8.06 Hz, 2H, Ar), 7.51 (d, *J* = 8.13 Hz, 4H, Ar), 7.62-7.69 (m, 6H, Ar), 7.84-7.92 (m, 8H, Ar) $^{13}$C NMR (125MHz, CD30D): δ ppm 22.2 22.7 29.4 31.5 31.9 41.0 42.8 44.6 54.2 63.8 69.6 71.5 72.7 74.3 102.0 127.7 128.0 128.6 128.9 130.0 130.7 134.2 138.4 139.1 141.3 145.5 169.3 170.0 174.5 175.5
HRMS (ESI-neg) [(M-2Na)/2] berechnet: 683,2518 gefunden: 683,2521

**Verbindung 77:**

$^1$H-NMR (300MHz, $CD_3OD$): δ ppm 1.58 (t, *J* = 11.81, 11.81 Hz, 2H, H3-a H3-a'), 1.77-1.87 (m, 4H, CH2CH2CH2 CH2CH2CH2'), 2.01 (s, 6H, NHCOCH3 NHCOCH3'), 2.66 (dt, *J* = 6.95, 6.93, 1.17 Hz, 4H, CH2S CH2S'), 2.74 (dt, *J* = 7.33, 7.14, 1.23 Hz, 4H, SCH2 SCH2'), 2.83 (dd, *J* = 12.19, 3.94 Hz, 2H, H3-e H3-e'), 3.45 (dd, *J* = 9.02, 1.68 Hz, 2H, H7), 3.47-3.76 (m, 14H, H4 H4' H5 H5' H6 H6' H9a H9a' OCH2a OCH2a' CH2NH CH2NH'), 3.85 (dd, *J* = 13.76, 3.42 Hz, 2H, H9b H9b'), 3.90 (td, *J* = 9.37, 5.93, 5.93 Hz, 2H, OCH2b OCH2b'), 4.11 (ddt, *J* = 8.84, 7.98, 3.15 Hz, 2H, H8 H8'), 7.38 (ddd, *J* = 6.28, 4.92, 2.32 Hz, 2H, Ar), 7.86 (s, 4H, Ar), 7.86-7.90 (m, 4H, Ar), 7.94 (d, *J* = 8.67 Hz, 4H, Ar), 8.01 (d, *J* = 8.66 Hz, 4H, Ar), 8.60-8.63 (m, 2H, Ar) $^{13}$C NMR (75MHz, CD30D): δ ppm 22.7 29.3 31.5 31.9 41.0 42.8 44.6 54.2 63.8 69.6 71.5 72.6 74.3 102.0 122.8 124.3 128.2 128.6 128.9 129.1 136.3 138.4 139.0 143.2 150.6 157.7 169.3 169.8 174.5 175.5
HRMS (ESI-neg) [(M-2Na)/2] berechnet: 670,2314 gefunden: 670,2324

**Verbindung 78:**

$^1$H-NMR (500MHz, $CD_3OD$): δ ppm 1.59 (t, *J* = 11.82, 11.82 Hz, 2H, H3-a H3-a'), 1.79-1.86 (m, 4H, CH2CH2CH2 CH2CH2CH2'), 2.02 (s, 6H, NHCOCH3 NHCOCH3'), 2.66 (dt, *J* = 6.97, 6.90, 3.90 Hz, 4H, CH2S CH2S'), 2.73 (dt, *J* = 6.97, 6.94, 2.36 Hz, 4H, SCH2 SCH2'), 2.84 (dd, *J* = 12.14, 4.17 Hz, 2H, H3-e H3-e'), 3.43-3.75 (m, 16H, H4 H4' H5 H5' H6 H6' H7 H7' H9a H9a' OCH2a OCH2a' CH2NH CH2NH'), 3.85 (dd, *J* = 13.68, 3.10 Hz, 2H, H9b H9b'), 3.92 (td, *J* = 9.39, 6.14, 6.14 Hz, 2H, OCH2b OCH2b'), 4.12 (ddd, *J* = 8.86, 8.30, 3.13 Hz, 2H, H8 H8'), 7.33 (dt, *J* = 7.47, 7.45, 0.94 Hz, 2H, Ar), 7.51 (dd, *J* = 7.47, 1.11 Hz, 2H, Ar), 7.54 (d, *J* = 7.26 Hz, 2H, Ar), 7.58 (dd, *J* = 7.53, 0.65 Hz, 4H, Ar), 7.80 (s, 4H, Ar), 7.93-7.97 (m, 4H, Ar) $^{13}$C NMR (75MHz, CD30D): δ ppm 22.7 29.3 31.4 32.0 41.0 42.8 44.6 54.2 63.9 69.6 71.6 72.6 74.3 102.0 121.7 122.5 123.8 125.2 128.5 131.1 135.2 135.4 135.6 136.5 136.7 138.2 144.8 148.3 168.8 169.2 174.5 175.6, 194.3
HRMS (ESI-neg) [(M-2Na)/2] berechnet: 695,2154 gefunden: 695,2165

**Verbindung 79:**

$^1$H-NMR (500MHz, $CD_3OD$) δ ppm 1.55 (t, *J* = 11.79, 11.79 Hz, 2H, H3-a, H3-a'), 1.83-1.73 (m, 4H, CH2CH2CH2 CH2CH2CH2'), 1.97 (s, 6H, NHCOCH3 NHCOCH3'), 2.63 (t, *J* = 7.17, 7.17 Hz, 4H, SCH2CH2CH2 SCH2CH2CH2'), 2.72 (dt, *J* = 6.95, 6.94, 2.62 Hz, 4H, CH2S CH2S'), 2.81 (dd, *J* = 12.26, 4.02 Hz, 2H, H3-e H3-e'), 3.21 (dd, *J* = 13.66, 7.93 Hz, 2H, H9a H9a'), 3.35 (dd, *J* = 9.13, 1.71 Hz, 2H, H7 H7'), 3.49-3.75 (m, H, CH2NHCO CH2NHCO' OCH2a OCH2a' H4 H4' H5 H5' H6 H6' H9b H9b' CH2Ar CH2Ar'), 3.84 (td, *J* = 9.57, 6.08, 6.08 Hz, 2H, OCH2b OCH2b'), 3.95 (dt, *J* = 8.33, 8.16, 2.87 Hz, 2H, H8 H8'), 7.29 *(t, J* = 7.31, 7.31 Hz, 2H, Ar), 7.36 (d, *J* = 8.11 Hz, 4H, Ar), 7.41 (dd, *J* = 8.38, 1.24 Hz, 4H, Ar), 7.53 (d, *J* = 8.37 Hz, 4H, Ar), 7.57 (dd, *J* = 8.33, 1.27 Hz, 4H, Ar), 7.90 (s, 4H, Ar) $^{13}$C NMR (125MHz, CD30D): δ ppm 22.7 29.4 31.5 32.0 41.1 42.8 43.5 44.1 54.2 63.7 69.6 71.3 72.3 74.2 101.9 127.9 128.2 128.3 128.6 129.9 130.7 136.2 138.4 141.0 142.1 169.4 174.1 174.5 175.5
HRMS (ESI-neg) [(M-2Na)/2] berechnet: 683,2518 gefunden: 683,2566

**Verbindung 80:**

$^{1}$H-NMR (500MHz, $CD_3OD$): δ 7.86 (s, 4H, Ar), 7.83 (d, *J* = 8.71 Hz, 4H, Ar), 7.68 (d, *J* = 8.70 Hz, 4H, Ar), 7.45 (dd, *J* = 3.64, 1.14 Hz, 2H, Ar), 7.42 (dd, *J* = 5.09, 1.12 Hz, 2H, Ar), 7.10 (dd, *J* = 5.10, 3.64 Hz, 2H, Ar), 4.10 (ddd, *J* = 8.93, 7.87, 3.33 Hz, 2H, H8 H8'), 3.90 (td, *J* = 9.70, 6.19, 6.19 Hz, 2H, OCH2a OCH2a'), 3.83 (dd, *J* = 13.60, 3.28 Hz, 2H, H9a H9a'), 3.72-3.69 (m, 4H, H4 H4' H5 H5'), 3.64 (dd, *J* = 10.48, 1.97 Hz, 2H, H6 H6'), 3.59 (td, *J* = 9.61, 6.11, 6.11 Hz, 2H, OCH2b OCH2b'), 3.54 (ddd, *J* = 14.63, 6.83, 0.51 Hz, 4H, CH2NH CH2NH'), 3.49 (dd, *J* = 13.68, 7.82 Hz, 2H, H9b H9b'), 3.44 (dd, *J* = 8.98, 1.84 Hz, 2H, H7 H7'), 2.83 (dd, *J* = 12.26, 4.24 Hz, 2H, H3-e H3-e'), 2.74 (ddd, *J* = 7.45, 6.79, 4.16 Hz, 4H, CH2S CH2S'), 2.66 (dt, *J* = 7.08, 7.06, 4.19 Hz, 4H, SCH2 SCH2'), 2.00 (s, 6H, CH3 CH3'), 1.85-1.79 (m, 4H, CH2CH2CH2 CH2CH2CH2'), 1.57 (dd, *J* = 12.16, 11.64 Hz, 2H, H3-a H3-a') $^{13}$C NMR (125MHz, CD3OD): δ ppm 175.5 174.5 169.7 169.3 144.2 138.8 138.4 134.4 129.4 129.2 128.6 127.1 126.5 125.5 102.0 74.3 72.7 71.5 69.6 63.8 54.2 44.5 42.8 41.0 31.9 31.5 29.3 22.7
HRMS (ESI-neg) [(M-2Na)/2] berechnet: 675,1926 gefunden: 675,1924

**Verbindung 81:**

$^{1}$µH-NMR (500MHz, $CD_3OD$): δ ppm 7.86 (s, 4H, Ar), 7.85 (d, *J* = 8.39 Hz, 4H, Ar), 7.73-7.69 (m, 6H, Ar), 7.50-7.46 (m, 4H, Ar), 4.10 (ddd, *J* = 9.37, 8.29, 3.22 Hz, 2H, H8 H8'), 3.90 (td, *J* = 9.71, 6.23, 6.23 Hz, 2H, OCH2a OCH2a'), 3.84 (dd, *J* = 13.63, 3.20 Hz, 2H, H9a H9a'), 3.73-3.62 (m, 6H, H4 H4' H5 H5' H6 H6'), 3.59 (td, *J* = 9.85, 6.09, 6.09 Hz, 2H, OCH2b OCH2b'), 3.54 (dd, *J* = 14.82, 7.28 Hz, 4H, CH2NH CH2NH'), 3.49 (dd, *J* = 13.30, 7.02 Hz, 2H, H9b H9b'), 3.44 (d, *J* = 8.93 Hz, 2H, H7 H7'), 2.83 (dd, *J* = 12.22, 4.14 Hz, 2H, H3-e H3-e'), 2.74 (dt, *J* = 6.89, 6.82, 4.18 Hz, 4H, CH2S CH2S'), 2.66 (dt, *J* = 6.98, 6.91, 4.66 Hz, 4H, SCH2 SCH2'), 2.00 (s, 6H, COCH3 COCH3'), 1.82 ( p, *J* = 6.70, 6.70, 6.68, 6.68 Hz, 4H, CH2CH2CH2 CH2CH2CH2'), 1.57 (t, *J* = 11.85, 11.85 Hz, 2H, H3-a H3-a') $^{13}$C NMR (125 MHz, CD30D): δ ppm 175.5 174.5 169.9 169.3 142.4 140.2 138.4 134.1 129.0 128.6 127.7 127.2 127.1 122.7 102.0 74.3 72.7 71.5 69.6 63.8 54.2 44.6 42.8 41.0 32.0 31.5 29.4 22.7
HRMS (ESI-neg) [(M-2Na)/2] berechnet: 675,1926 gefunden: 675,1929

**Verbindung 82:**

$^{1}$H-NMR (500MHz, $CD_3OD$): δ ppm 7.87 (s, 4H, Ar), 7.84 (d, *J* = 8.65 Hz, 4H, Ar), 7.73 (d, *J* = 8.53 Hz, 4H, Ar), 7.58 (dd, *J* = 1.73, 0.57 Hz, 2H, Ar), 6.86 (dd, *J* = 3.39, 0.53 Hz, 2H, Ar), 6.53 (dd, *J* = 3.41, 1.81 Hz, 2H, Ar), 4.09 (ddd, *J* = 8.68, 7.90, 3.28 Hz, 2H, H8 H8'), 3.89 (td, *J* = 9.58, 6.15, 6.15 Hz, 2H, OCH2a OCH2a'), 3.82 (dd, *J* = 13.65, 3.27 Hz, 2H, H9a H9a'), 3.74-3.62 (m, 6H, H4 H4' H5 H5' H6 H6'), 3.59 (td, *J* = 9.63, 5.99, 5.99 Hz, 2H, OCH2b OCH2b'), 3.54 (dd, *J* = 14.87, 7.73 Hz, 4H, CH2NH CH2NH'), 3.49 (dd, *J* = 13.90, 8.00 Hz, 2H, H9b H9b'), 3.44 (dd, *J* = 8.93, 1.84 Hz, 2H, H7 H7'), 2.83 (dd, *J* = 12.24, 4.16 Hz, 2H, H3-e H3-e'), 2.74 (dt, *J* = 6.88, 6.83, 4.20 Hz, 4H, CH2S CH2S'), 2.66 (dt, *J* = 7.06, 7.00, 4.10 Hz, 4H, SCH2 SCH2'), 2.00 (s, 6H, COCH3 COCH3'), 1.85-1.79 (m, 4H, CH2CH2CH2 CH2CH2CH2'), 1.57 (t, *J* = 11.85, 11.85 Hz, 2H, H3-a H3-a') $^{13}$C NMR (125MHz, CD30D): δ ppm 175.5 174.5 169.8 169.3 154.3 144.4 138.4 135.0 134.0 129.0 128.6 124.5 113.1 107.9 102.0 74.3 72.6 71.5 69.6 63.8 54.2 44.5 42.8 41.0 32.0 31.5 29.4 22.7
HRMS (ESI-neg) [(M-2Na)/2] berechnet: 660,2233 gefunden: 660,2210

**Verbindung 83:**

$^{1}$H-NMR (500MHz, $CD_3OD$): δ ppm 7.89 (s, 4H, Ar), 7.55-7.48 (m, 6H, Ar ArCH=), 7.38-7.31 (m, 6H, Ar), 6.70 (d, *J* = 15.79 Hz, 2H, COCH=), 4.01 (ddd, *J* = 9.30, 8.40, 2.75 Hz, 2H, H8 H8'), 3.88 (td, *J* = 9.50, 6.18, 6.18 Hz, 2H, OCH2a OCH2a'), 3.79 (dd, *J* = 13.72, 2.20 Hz, 2H, H9a H9a'), 3.73-3.66 (m, 4H, H4 H4' H5 H5'), 3.65-3.51 (m, 8H, H6 H6' OCH2b OCH2b' CH2NH'), 3.40 (dd, *J* = 8.91, 1.85 Hz, 2H, H7 H7'), 3.34 (dd, *J* = 13.75, 7.93 Hz, 2H, H9b H9b'), 2.83 (dd, *J* = 12.21, 3.78 Hz, 2H, H3-e H3-e'), 2.76 (dt, *J* = 6.86, 6.80, 4.04 Hz, 4H, CH2S CH2S'), 2.66 (t, *J* = 7.04, 7.04 Hz, 4H, SCH2 SCH2'), 2.01 (s, 6H, COCH3 COCH3'), 1.85-1.79 (m, 4H, CH2CH2CH2 CH2CH2CH2), 1.57 (t, *J* = 11.86, 11.86 Hz, 2H, H3-a H3-a') $^{13}$C NMR (500 MHz, CD30D): δ ppm 175.5 174.5 169.4 168.8 141.6 138.4 136.5 130.7 129.9 128.9 128.6 122.2 102.0 74.3 72.4 71.4 69.7 63.8 54.3 44.1 42.8 41.1 32.0 31.5 29.4 22.7
HRMS (ESI-neg) [(M-2Na)/2] berechnet: 620,2283gefunden: 620,2241

**Verbindung 84**

Eine Lösung von 35mg (1eq) Verbindung 61 in 800µl absolutem DMF wurde mit 200µl Piperidin versetzt und 20min bei RT gerührt. Das Lösungsmittel wurde entfernt, der Rückstand mit 1ml abs. DMF gelöst und erneut konzentriert. Nach Lösen des Rückstandes in 0,5ml abs. DMF und 0,5ml $H_2O$ wurden 12mg (6eq) $NaHCO_3$ und 18,3mg (3eq)

Biphenyl-4-sulfonylchlorid zugegeben. Es wurde 15min bei RT gerührt, das Lösungsmittel entfernt, der Rückstand mit MeOH gelöst, $H_2O$ zugegeben bis eine kolloidale Trübung auftrat, und über eine RP-18 Säule ($H_2O$>>MeOH) gereinigt. Die Produktfraktionen wurden konzentriert, mit wenig MeOH gelöst, mit $H_2O$ bis zum Auftreten einer Trübung verdünnt und mit 2M NaOH auf pH 12-13 gebracht. Nach Beenden der Reaktion wurde mit 20%HAc neutralisiert, über eine RP18 Säule ($H_2O$>>$CH_3CN$) gereinigt, das $CH_3CN$ entfernt und lyophilisiert. Ausbeute: 16mg weißer Feststoff.
$^1$H-NMR (300MHz, $CD_3OD$): $\delta$ ppm 1.53 (t, *J* = 11.66, 11.66 Hz, 2H, H3-a H3-a'), 1.66-1.77 (m, 4H, CH2CH2CH2 CH2CH2CH2'), 2.00 (s, 6H, NHCOCH3 NHCOCH3'), 2.59 (dt, *J* = 7.24, 7.19, 1.27 Hz, 4H, SCH2CH2CH2 SCH2CH2CH2'), 2.73 (dt, *J* = 7.32, 7.10, 2.10 Hz, 4H, SCH2 SCH2'), 2.79 (dd, *J* = 12.62, 4.66 Hz, 2H, H3-e H3-e'), 2.91 (dd, *J* = 12.81, 7.79 Hz, 2H, H9a H9a'), 3.32-3.38 (m, 4H, H7 H7' H9 H9b'), 3.46-3.4 9(m, 14H, H4 H4' H5 H5' H6 H6' OCH2a OCH2a' CH2NH CH2NH'), 3.74 (td, *J* = 9.92, 6.05, 6.05 Hz, 2H, OCH2b OCH2b'), 3.87 (ddd, *J* = 9.08, 8.22, 2.59 Hz, 2H, H8 H8'), 7.39 (t, *J* = 7.27, 7.27 Hz, 2H, Ar), 7.46 (t, *J* = 7.46, 7.46 Hz, 4H, Ar), 7.67 (d, *J* = 7.69 Hz, 4H, Ar), 7.78 (d, *J* = 8.39 Hz, 4H, Ar), 7.89-7.94 (m, 8H, Ar) $^{13}$C NMR (75MHz, CD30D): $\delta$ ppm 22.7 29.2 31.6 31.9 41.0 42.8 47.5 54.3 63.6 69.4 71.1 72.0 74.2 101.9 128.4 128.6 128.7 129.5 130.2 138.4 140.6 140.7 146.5 169.3 174.4 175.7

**Verbindung 85**

Eine Lösung von 50mg Verbindung 61 in 1 ml MeOH und 1 ml $H_2O$ wurde mit 60$\mu$l 2M NaOH versetzt. Nach 2h rühren wurde mit Essigsäure neutralisiert, das Lösungsmittel entfernt, in $H_2O$ gelöst und mit EE ausgeschüttelt. Die wässrige Phase wurde konzentriert, in 2ml MeOH gelöst und nach Zugabe von 9,2$\mu$l (0,07eq) TEA und 11,3mg (1,8eq) Biphenyl-4-aldehyd eine Stunde gerührt. Anschließend wurden 4,8mg (2,2eq) Natriumcyanoborhydrid zugegeben, weitere 30min gerührt und 0,1ml 20%iger Essigsäure zugegeben. Das Lösungsmittel wurde entfernt, der Rückstand mit wenig MeOH gelöst, $H_2O$ zugegeben bis eine kolloidale Trübung auftrat, und über eine RP-18 Säule ($H_2O$>>MeOH) gereinigt. Die Produktfraktionen wurden konzentriert und mit Dioxan-$H_2O$ lyophilisiert. Ausbeute: 12mg weißer Feststoff.
$^1$H-NMR (300MHz, $CD_3OD$): $\delta$ ppm 1.58 (t, *J* = 11.84, 11.84 Hz, 2H, H3-a H3-a'), 1.76-1.86 (m, 4H, CH2CH2CH2 CH2CH2CH2'), 2.02 (s, 6H, NHCOCH3 NHCOCH3'), 2.65 (t, *J* = 7.18, 7.18 Hz, 2H, CH2SCH2 CH2SCH2'), 2.74 (t, *J* = 7.00, 7.00 Hz, 2H, CH2SCH2 CH2SCH2'), 2.83 (dd, *J* = 12.06, 4.84 Hz, 2H, H3-e H3-e'), 3.01 (dd, *J* = 12.61, 9.63 Hz, 2H, H9a H9a'), 3.35-3.78 (m, 16H, H4 H4' H5 H5' H6 H6' H7 H7' H9b H9b' OCH2a OCH2a' CH2NHCO CH2NHCO'), 3.85 (td, *J* = 9.27, 6.22, 6.22 Hz, 2H, OCH2b OCH2b'), 4.19 (dt, *J* = 9.33, 9.33, 3.05 Hz, 2H, H8 N8'), 4.27 (s, 4H, NHCH2Ar NHCH2Ar'), 7.35 (t, *J* = 7.25, 7.25 Hz, 2H, Ar), 7.43 (t, *J* = 7.35, 7.35 Hz, 4H, Ar), 7.54 (d, *J* = 8.34 Hz, 4H, Ar), 7.60 (dd, *J* = 7.13, 1.41 Hz, 4H, Ar), 7.66 (d, *J* = 8.29 Hz, 4H, Ar), 7.85 (s, 4H, Ar) $^{13}$C NMR (75MHz, CD30D): $\delta$ ppm 22.7 29.4 31.3 32.1 40.9 42.6 51.4 52.0 54.2 63.8 68.4 69.3 72.8 74.1 102.1 128.1 128.6 128.7 129.0 130.1 131.4 131.7 138.4 141.3 143.7 169.4 174.4 175.9
HRMS (ESI-neg) [(M-2Na)/2] berechnet: 655,2569 gefunden: 655,2568

**Verbindung 86**

Eine Lösung von 150mg Verbindung 9 in 3ml Pyridin wurde auf 0°C gekühlt und langsam 1ml Essigsäureanhydrid zugetropft. Nach Zugabe von einer Spatelspitze DMAP wurde 3h gerührt, konzentriert und mit Toluol nachgedampft. Der Rückstand wurde in $CH_2Cl_2$ gelöst, mit HCl-saurem $H_2O$ ausgeschüttelt. Die wässrige Phase wurde mit wenig $CH_2Cl_2$ gewaschen, die org. Phasen vereinigt, getrocknet ($MgSO_4$), filtriert und konzentriert. Ausbeute: 180mg weißer Feststoff.

**Verbindung 87**

Eine Lösung von 225mg Natriumacetat (12,25eq) in 25ml $H_2O$ wurde mit 302mg (5,85eq) $KIO_4$ versetzt. Nach Zugabe von 150mg Verbindung 86 in 25ml Dioxan wurden 90mg (2,55eq) Kaliumpermanganat zugegeben, 17h gerührt, über Celite filtriert, auf ca. 3-5ml konzentriert, mit $H_2O$ verdünnt, mit verdünnter HCl angesäuert und 3x mit Dichlormethan ausgeschüttelt. Die org. Phase wurde mit $MgSO_4$ getrocknet, filtriert und eingeengt. Ausbeute: 149mg weißer Feststoff.

**1,4-Bis-[benzyloxycarbonylamino-3-propylcarbonylamino] benzene (Verbindung 88)**

Zu einer Lösung von 30mg 1,4-Phenylendiamindihydrochlorid in 1ml abs. DMF wurden 130mg (2,4eq) Z-Aminobuttersäure-NPE und 56$\mu$l (2,4eq) TEA zugegeben und über 17h gerührt, das Lösungmittel entfernt und über eine RP-18-Säule ($H_2O$>>EtOH) gereinigt. Das Produkt blieb ungelöst und wurde mit DMF von der Säule gewaschen. Ausbeute: 90mg weißer Feststoff.

**1,4-Bis-13-aminopropylcarbonylaminol benzene dihydrochlorid (Verbindung 89)**

Zu einer Suspension von 70mg Verbindung 88 in 15ml MeOH und 3ml $H_2O$ wurden 25mg Palladium auf Kohle und 0,5ml 2N HCl zugegeben und über 17h bei Normaldruck unter Rühren hydriert, über Celite filtriert und das Lösungsmittel entfernt. Ausbeute: 48mg Feststoff. DC-RF: 0,37 in 1:1:1 EtOH:EE:HAc(20%)

**Verbindung 90**

Zu einer Lösung von 6mg Verbindung 89 und 25,8mg (2,2eq) Verbindung 87 in 1ml abs. DMF wurden 14,3mg (2,2eq) HATU und 9μl (3eq) DIPEA zugegeben, nach 5min eingeengt und über RP-18 gereinigt ($H_2O$>>EtOH). Die Sunbstanz wurde in wenig EtOH gelöst, mit H2O versetzt bis eine Trübung auftrat, mit 2M NaOH auf pH 12-13 gebracht, nach der Reaktion mit Essigsäure neutralisiert, dass EtOH entfernt und über RP-18 ($H_2O$>>EtOH) gereinigt. Nach Entfernen des Ethanols wurde lyophilisiert. Ausbeute: 14mg weiße Substanz.
$^1$H-NMR (300MHz, $CD_3OD$): δ ppm 1.71 (t, *J* = 11.70, 11.70 Hz, 2H, H3-a H3-a'), 1.84-1.96 (m, 4H, CH2<u>CH2</u>CH2 CH2<u>CH2</u>CH2'), 2.00 (s, 6H, NHCO<u>CH3</u> NHCO<u>CH3</u>'), 2.32-2.46 (m, 4H, CH2CO CH2CO'), 2.90 (dd, *J* = 12.13, 3.89 Hz, 2H, H3-e H3-e'), 4.39-3.30 (m, 22H, H4 H4' H5 H5' H6 H6' H7 H7' H8 H8' H9a H9a' H9b H9b' OCH2a OCH2a' OCH2b OCH2b' CH2NH CH2NH'), 7.36 (t, *J* = 7.27, 7.27 Hz, 2H, Ar), 7.40-7.51 (m, 8H, Ar), 7.57-7.74 (m, 8H, Ar), 7.87-7.95 (m, 4H, Ar) $^{13}$C NMR (75MHz, CD30D): δ ppm 22.7 26.8 35.2 39.5 42.1 44.5 54.0 64.7 69.5 71.5 72.4 74.6 101.9 121.7 128.0 128.1 129.0 129.1 130.0 134.5 136.1 141.3 145.6 170.0 173.1 173.8 175.0 175.5
HRMS (ESI-neg) [(M-2Na)/2] berechnet: 666,2543 gefunden: 666,2532

**Verbindung 91**

Eine Lösung von 155mg (17eq) Terephtalsäure, 40mg (1eq) Verbindung 9 und 25mg (1,2eq) HATU in 1ml abs. DMF wurde mit 235 μl (25eq) DIPEA versetzt, 5min gerührt, konzentriert, mit $NaHCO_3$-Lösung pH8 gelöst und über eine RP18-Säule ($H_2O$>>MeOH) gereinigt. Die Produktfraktionen wurden vereinigt, das MeOH entfernt und lyophilisiert. Ausbeute: 38mg weiße Substanz

**Verbindung 92**

Eine Lösung von 10,0mg (1eq) Verbindung 91, 8,88mg (1,1eq) Verbindung 173 und 5,78mg (1,2eq) HATU wurde mit 6,5μl (3eq) DIPEA versetzt und 5min gerührt. Nach Entfernen des Lösungsmittels wurde der Rückstand mit wenig MeOH gelöst, $H_2O$ zugegeben bis eine Trübung auftrat und über eine RP-18 Säule ($H_2O$>>MeOH) gereinigt. Die Produktfraktionen wurden konzentriert, mit wenig MeOH gelöst, mit $H_2O$ bis zum Auftreten einer Trübung verdünnt und mit 2M NaOH auf pH 12-13 gebracht. Nach Beenden der Reaktion wurde mit 20%HAc neutralisiert, über eine RP18 Säule ($H_2O$>>$CH_3CN$) gereinigt, das $CH_3CN$ entfernt und lyophilisiert. Ausbeute: 20mg weißer Fesstoff.
$^1$H-NMR (50OMHz, $CD_3OD$): δ ppm 1.35-1.46 (m, 4H, <u>CH2CH2</u> <u>CH2CH2'</u>), 1.51-1.64 (m, 6H, H3-a H3-a' <u>CH2</u>CH2CH2<u>CH2</u> <u>CH2</u>CH2CH2<u>CH2'</u>), 1.76-1.87 (m, 2H, SCH2<u>CH2</u>CH2), 2.00 (s, 6H, NHCO<u>CH3</u> NHCO<u>CH3'</u>), 2.66 (dt, *J* = 6.87, 6.57, 2.19 Hz, 2H, SCH2), 2.74 (t, *J* = 6.93, 6.93 Hz, 2H, CH2S), 2.80-2.87 (m, 2H, H3-e H3-e'), 3.32-3.95 (m, 20H, H4 H4' H5 H5' H6 H6' H7 H7' H9a H9a' H9b H9b' OCH2a OCH2a' OCH2b OCH2b' CH2NH CH2NH'), 4.02-4.15 (m, 2H, H8 H8'), 7.35 (t, *J* = 7.23, 7.23 Hz, 2H, Ar), 7.43 (t, *J* = 7.48, 7.48 Hz, 4H, Ar), 7.61 (d, *J* = 7.56 Hz, 4H, Ar), 7.67 (d, *J* = 7.80 Hz, 4H, Ar), 7.85 (s, 4H, Ar), 7.89 (dd, *J* = 8.26, 1.49 Hz, 4H, Ar) $^{13}$C NMR (125MHz, CD30D): δ ppm 22.7 26.9 27.9 29.4 30.4 30.9 31.5 32.0 41.0 41.2 42.8 44.6 54.2 63.8 65.2 69.6 71.5 72.6 74.3 102.0 102.1 128.0 128.1 128.5 128.9 129.1 130.0 134.5 138.2 138.6 141.3 145.5 169.3 170.0 174.5 174.5 175.5
HRMS (ESI-neg) [(M-2Na)/2] berechnet: 660,2580 gefunden: 660,2597

**Verbindung 93**

Zu einer Lösung von 50mg Verbindung 4 in 1ml $H_2O$ und 2ml tert.-Butanol wurden 3mal im Abstand von 17h je 42mg (2eq) Phenylacetylen, 5,4mg (0,15eq) Ascorbinsäure und 4,8mg (0,15eq) $CuSO_4$ zugegeben. Anschließend wurde eingeengt, über eine RP-18 Säule ($H_2O$>>EtOH) gereinigt, das EtOH entfernt und lyophilisiert. Ausbeute: 27mg weißer Feststoff

**Verbindung 94**

Hergestellt analog zu Verbindung 11

**Verbindung 95**

Hergestellt analog zu Verbindung 21
$^1$H-NMR (300MHz, $CD_3OD$): δ ppm 1.56 (t, *J* = 11.89, 11.89 Hz, 2H, H3-a H3-a'), 1.75-1.84 (m, 4H, CH2CH2CH2 CH2CH2CH2'), 1.99 (s, 6H, NHCOCH3 NHCOCH3'), 2.64 (dt, *J* = 6.95, 6.86, 4.05 Hz, 4H, CH2SCH2 CH2SCH2'), 2.72 (dt, *J* = 7.15, 7.12, 1.96 Hz, 4H, CH2SCH2 CH2SCH2'), 2.81 (dd, *J* = 12.26, 4.15 Hz, 2H, H3-e H3-e'), 3.38 (dd, *J* = 9.07, 1.91 Hz, 2H, H7 H7'), 3.48-3.74 (m, 12H, H4 H4' H5 H5' H6 H6' OCH2a OCH2a' CH2NH CH2NH'), 3.84 (td, *J* = 9.81, 6.15, 6.15 Hz, 2H, OCH2b OCH2b'), 4.27 (ddd, *J* = 8.96, 7.78, 2.34 Hz, 2H, H8 H8'), 4.50 (dd, *J* = 14.00, 7.72 Hz, 2H, H9a H9a'), 4.82 (dd, *J* = 14.13, 2.39 Hz, 2H, H9b H9b'), 7.31 (t, *J* = 7.35, 7.35 Hz, 2H, Ar), 7.40 (t, *J* = 7.40, 7.40 Hz, 4H, Ar), 7.79 (dd, *J* = 8.35, 1.27 Hz, 4H, Ar), 7.90 (s, 4H, Ar), 8.31 (s, 2H, Ar) $^{13}$C NMR (75MHz, CD3OD): δ ppm 22.6 29.3 31.4 31.9 41.0 42.8 54.2 54.6 63.8 69.5 71.6 74.1 102.0 126.7 128.6 129.3 130.0 131.9 138.4 148.5 169.4 174.5 175.6
HRMS (ESI-neg) [(M-2Na)/2] berechnet: 617,2161 gefunden: 617,2165

**Verbindung 96**

Hergestellt analog zu Verbindung 4

**Verbindung 97**

Hergestellt analog zu Verbindung 6

**Verbindung 98**

Hergestellt analog zu Verbindung 9

**Verbindung 99**

Hergestellt anaolg zu Verbindung 86

**Verbindung 100**

276mg Verbindung 99 und 481mg (5eq) 6-Benzyloxycarbonylaminohexanol wurden in einer Mischung aus 2ml $CH_3CN$ und 1ml $CH_2Cl_2$ gelöst und nach Zugabe von 150mg frisch ausgeglühtem, gemahlenem Molsieb A4 1h unter Argon gerührt. Bei -78°C wurden 172mg (2eq) NIS und 10μl (0,3eq) Trifluormethansulfonsäure zugegeben und 3h bei -40°C gerührt. Nach Zugabe von 30ml $CH_2Cl_2$ wurde mit ges. $Na_2CO_3$-Lösung (20ml), anschließend mit 1M $Na_2SO_3$ ausgeschüttelt, getrocknet ($MgSO_4$), filtriert, eingeengt, und über eine Kieselgelsäule (EE) gereinigt. Man erhält 130mg eines Isomerengemisches α:β ~ 3:2). Das Gemisch wurde in 1ml abs. MeOH gelöst, mit 0,1ml 0,1M NaOMe-Lösung versetzt, nach 1h RT mit 2ml 20%iger HAc neutralisiert, konzentriert, und über eine Kieselgelsäule (EtOH:EE:HAc(20%) 1:12:1) gereinigt und lyophilisiert. Ausbeute: 40mg weiße Substanz

**Verbindung 101**

40mg Verbindung 100 wurde in einer Mischung aus 2ml MeOH und 1ml $H_2O$ gelöst, mit HCl auf pH 3 eingestellt und nach Zugabe von 10mg Pd auf Kohle (10%) 4h bei Normaldruck hydriert. Nach Filtration wurde mit verd. NaOH neutralisiert, das MeOH entfernt und lyophilisiert. Ausbeute: 39mg weißer Feststoff

**Verbindung 102**

Hergestellt analog zu Verbindung 21 ausgehend von Verbindung 101
$^1$H-NMR (300MHz, $CD_3OD$): δ ppm 1.34-1.67 (m, 7H, CH2CH2CH2CH2CH2CH2 CH2CH2CH2CH2CH2CH2' H3-a H3-a'), 2.00 (s, 6H, NHCOCH3 NHCOCH3'), 2.84 (dd, *J* = 12.36, 4.01 Hz, 2H, H3-e H3-e'), 3.29-3.92 (m, 20H, H4 H4' H5 H5' H6 H6' H7 H7' H9a H9a' H9b H9b' OCH2a OCH2a' OCH2b OCH2b' CH2NHCO CH2NHCO'), 4.06 (dt, *J* = 7.93, 7.89, 3.30 Hz, 2H, H8 H8'), 7.35 (t, *J* = 7.22, 7.22 Hz, 2H, Ar), 7.43 (t, *J* = 7.35, 7.35 Hz, 4H, Ar), 7.62 (d, *J* = 7.89 Hz, 4H, Ar), 7.67 (d, *J* = 8.05 Hz, 4H, Ar), 7.84 (s, 4H, Ar), 7.90 (d, *J* = 8.29 Hz, 4H, Ar) $^{13}$C NMR (75MHz, CD30D): δ ppm 22.7 26.9 27.9 30.4 30.929.4 41.2 42.9 44.6 54.2 65.1 69.7 71.5 72.7 74.3 102.1 128.0 128.1 128.5 129.0 129.1 130.0 134.5 138.5 141.3 145.5 169.3 170.0 174.6 175.5
HRMS (ESI-neg) [(M-2Na)/2] berechnet: 651,2797 gefunden: 651,2797

**Verbindung 103**

Hergestellt analog zu Verbindung 92
Anstelle von Verbindung 101 wurde Verbindung 56 eingesetzt
$^1$H-NMR (500MHz, $CD_3OD$): δ ppm 1.21-1.46 (m, 6H, Cylcohexyl), 1.57 (t, *J* = 11.92, 11.92 Hz, 1H, H3-a), 1.58 (t, *J* = 11.68, 11.68 Hz, 1H, H3-a'), 1.71-1.86 (m, 8H, Cyclohexyl CH2CH2CH2 CH2CH2CH2'), 1.99 (s, 3H, NHCOCH3), 2.01 (s, 3H, NHCOCH3'), 2.49-2.57 (m, 1H, Cyclohexyl), 2.62-2.69 (m, 4H, CH2S CH2S'), 2.71-2.77 (m, 4H, SCH2 SCH2'), 2.80-2.85 (m, 2H, H3-e H3-e'), 3.41-3.74 (m, 16H, H4 H4' H5 H5' H6 H6' H7 H7' H9a H9a' OCH2a OCH2a' CH2NH CH2NH'), 3.80 (dd, *J* = 13.66, 3.32 Hz, 1H, H9b), 3.84 (dd, *J* = 13.54, 3.19 Hz, 1H, H9b'), 3.86-3.92 (m, 2H, OCH2b OCH2b'), 4.05-4.13 (m, 2H, H8 H8'), 7.25 (d, *J* = 8.13 Hz, 2H, Ar), 7.36 (t, *J* = 7.37, 7.37 Hz, 1H, Ar), 7.44 (t, *J* = 7.90, 7.90 Hz, 2H, Ar), 7.63 (d, *J* = 7.20 Hz, 2H, Ar), 7.68 (d, *J* = 8.13 Hz, 2H, Ar), 7.72 (d, *J* = 8.42 Hz, 2H, Ar), 7.92-7.87 (m, 6H, Ar) $^{13}$C NMR (75MHz, CD30D): δ ppm 22.7 27.2 27.9 35.4 29.4 31.5 31.9 41.0 42.8 44.5 44.6 45.9 54.2 63.8 69.6 71.5 72.6 74.3 102.0 128.0 128.1 128.4 128.6 129.0 129.1 130.0 133.3 134.5 138.4 141.3 145.5 153.2 169.3 170.0 170.3 174.5 175.5
HRMS (ESI-neg) [(M-2Na)/2] berechnet: 672,2596 gefunden: 672,2591

**Verbindung 104**

Hergestellt nach: Eur. J. Org.Chem. 2009, 16, 2611-2620

**Verbindung 105**

200mg (0,575mmol) Verbindung 104 und 159mg (0,690mmol) 4-(Benzyloxycarbonyl-amino)hex-1-in wurden in 2ml tert-Butanol gelöst. 50mg (0,29mmol) Ascorbinsäure und 46mg (0,29mmol) $CuSO_4$ wurden in 2ml $H_2O$ gelöst und zu der Lösung gegeben, nach 2 Tagen wurde das tert-Butanol entfernt, 1 ml 20%HAc zugegeben und über RP18 ($H_2O$>>EtOH) gereinigt und lyophilisiert. Ausbeute: 110mg Feststoff

**Verbindung 106**

Hergestellt analog Verbindung 4

**Verbindung 107**

Hergestellt analog Verbindung 6

**Verbindung 108**

Hergestellt analog Verbindung 9

**Verbindung 109**

Hergestellt analog Verbindung 101
Statt HCl wurde HAc verwendet, die Substanz anschliessend mit $H_2O$ auf eine RP-18 Säule aufgetragen, mit verd. HCl gespült, mit $H_2O$ gespült und anschliessend mit $H_2O$>>EtOH von der Säule gespült. Das so erhalten Hydrochlorid-Salz der Verbindung wurde lyophilisiert.

**Verbindung 110**

Hergestellt analog Verbindung 21.
$^1$H-NMR (500MHz, $CD_3OD$): δ ppm 7.94 (s, 2H, Triazol), 7.90 (d, *J* = 8.12 Hz, 4H, Ar), 7.84 (s, 4H, Ar), 7.68 (dd, *J* = 7.84, 0.78 Hz, 4H, Ar), 7.63 (d, *J* = 7.79 Hz, 4H, Ar), 7.44 (t, *J* = 7.64 Hz, 4H, Ar), 7.36 (t, *J* = 7.37 Hz, 2H, Ar), 4.08 (ddd, *J* = 8.94, 8.37, 3.18 Hz, 2H, H8 H8'), 3.98 (ddd, *J* = 11.14, 9.77, 6.13 Hz, 2H, H4 H4'), 3.89-3.86 (m, 4H, H5 H5' H6 H6'), 3.83 (dd, *J* = 13.57, 3.04 Hz, 2H, H9a H9a'), 3.56-3.49 (m, 4H, H7 H7' H9b H9b'), 3.43 (dd, *J* = 12.55, 4.69 Hz, 2H, H3-e H3-e'), 3.39 (t, *J* = 6.93 Hz, 4H, CH2NH CH2NH'), 2.73 (t, *J* = 7.28 Hz, 4H, CH2Triazol CH2Triazol'), 2.30 (t, *J* = 11.84 Hz, 2H, H3-a H3-a'), 2.02 (s, 6H, COCH3 COCH3'), 1.77-1.69 (m, 4H, CH2CH2CH2CH2 CH2CH2CH2CH2'), 1.69-1.61 (m, 4H, CH2CH2CH2CH2 CH2CH2CH2CH2') $^{13}$C NMR (125MHz, CD30D): δ ppm 175.4 171.5 170.1 169.3 148.3 145.6 141.3 138.5 134.5 130.0 129.1 129.0 128.5 128.1 121.6 92.2 76.0 72.2 71.3 69.5 53.8 44.6 41.2 40.8 29.8 27.9 25.9 22.7

HRMS (ESI-neg) [(M-2Na)/2] berechnet: 674,2706 gefunden: 674,2723

**Verbindung 111**

190mg Verbindung 104 wurden in 1ml abs. MeOH gelöst, mit 10mg Pd auf Kohle (10%) versetzt, 1h bei Normaldruck hydriert, über Celite filtriert und abgedampft. Der Rückstand (177mg; 0,55mmol) wurde mit 728mg (2,75mmol) 6-(Benzyloxycarbonyl-amino)hexansäure in 6ml abs. DMF gelöst, 10min nach Zugabe von 835mg (2,2mmol) HATU und 0,939ml (5,5mmol) DIPEA eingeengt und über RP-18 gereinigt. Die Produkte wurden gefriergetrocknet, in 5ml abs. MeOH gelöst und mit 0,5ml frischer ca. 0,5M NaOMe-Lösung versetzt, nach Ende der Reaktion mit 20%iger Hac neutralisiert, eingeengt und erneut über RP-18 gereinigt. Ausbeute: 125mg

**Verbindung 112**

Hergestellt analog Verbindung 4

**Verbindung 113**

Hergestellt analog Verbindung 6

**Verbindung 114**

Hergestellt analog Verbindung 9

**Verbindung 115**

Hergestellt analog Verbindung 109

**Verbindung 116**

Hergestellt analog Verbindung 21
$^1$H-NMR (500MHz, $CD_3OD$): δ 7.90 (d, *J* = 8.36 Hz, 4H, Ar), 7.82 (s, 4H, Ar), 7.68 (d, *J* = 8.34 Hz, 4H, Ar), 7.61 (d, *J* = 7.46 Hz, 4H, Ar), 7.45 (t, *J* = 7.56 Hz, 4H, Ar), 7.37 (t, *J* = 7.14 Hz, 2H, Ar), 4.11 (dd, *J* = 10.05, 1.36 Hz, 2H, H6 H6'), 3.99 (dt, *J* = 8.41, 3.04 Hz, 2H, H8 H8'), 3.88-3.77 (m, 4H, H4 H4' H9a H9a'), 3.80 (t, *J* = 10.43 Hz, 2H, H5 H5'), 3.48 (dd, *J* = 13.49, 8.22 Hz, 1H, H9b H9b'), 3.44 (dd, *J* = 8.82, 1.41 Hz, 2H, H7 H7'), 3.36 (t, *J* = 7.04 Hz, 4H, CH2NH CH2NH'), 2.77 (dd, *J* = 12.47, 3.98 Hz, 2H, H3-e H3-e'), 2.22 (t, *J* = 7.22 Hz, 4H, CH2CO CH2CO), 2.02 (s, 6H, CH3 CH3'), 1.91 (t, *J* = 11.49 Hz, 2H, H3-a H3-a'), 1.69-1.56 (m, 8H, <u>CH2</u>CH2<u>CH2</u> <u>CH2</u>CH2<u>CH2</u>'), 1.44-1.37 (m, 4H, CH2<u>CH2</u>CH2 CH2<u>CH2</u>CH2') $^{13}$C NMR (125MHz, CD30D): δ ppm 175.8 175.6 175.3 170.4 169.5 145.5 141.0 138.3 134.1 130.1 130.0 129.2 129.2 19.0 128.5 128.1 128.086.674.672.271.469.053.944.641.641.037.1 30.027.426.1 22.8
HRMS (ESI-neg) [(M-2Na)/2] berechnet: 664,2750 gefunden: 664,2748

**Verbindung 117**

Hergestellt nach "Liebigs Ann. Chem. 1991, 487-495

**Verbindung 118**

Hergestellt analog zu Verbindung 4

**Verbindung 119**

Hergestellt analog zu Verbindung 6

**Verbindung 120**

Hergestellt analog zu Verbindung 9

**Verbindung 121**

Hergestellt analog zu Verbindung 11

**Verbindung 122**

Hergestellt analog zu Verbindung 21
$^1$H-NMR (500MHz, $CD_3OD$): δ ppm 1.60-1.74 (m, 6H, H3-a H3-a' CH2CH2NH CH2CH2NH'), 1.80-1.88 (m, 8H, CH2CH2CH2S CH2CH2CH2S'), 2.00 (s, 6H, NHCOCH3 NHCOCH3'), 2.48-2.59 (m, 8H, CH2SCH2 CH2SCH2'), 2.70 (dd, *J* = 12.58, 4.58 Hz, 2H, H3-e H3-e'), 3.38-3.46 (m, 6H, H7 H7' CH2NH CH2NH'), 3.48 (ddd, *J* = 13.43, 7.85, 1.42 Hz, 2H, H9a H9a'), 3.62-3.72 (m, 4H, H5 H5' H6 H6'), 3.84 (dd, *J* = 13.52, 3.22 Hz, 2H, H9b H9b'), 4.04 (ddd, *J* = 8.52, 8.05, 3.40 Hz, 2H, H8 H8'), 7.35 (t, *J* = 7.41, 7.41 Hz, 2H, Ar), 7.43 (t, *J* = 7.61, 7.61 Hz, 4H, Ar), 7.62 (dd, *J* = 8.20, 1.02 Hz, 4H, Ar), 7.67 (d, *J* = 8.55 Hz, 4H, Ar), 7.83 (s, 4H, Ar), 7.89 (d, *J* = 8.19 Hz, 4H, Ar)
HRMS (ESI-neg) [(M-2Na)/2] berechnet: 667,2569 gefunden: 667,2593

**Verbindung 123**

Eine Lösung von 300mg Verbindung 7 und 198μL (2eq) Diisopropylethylamin in 10ml abs. DMF wurde mit 250mg (1,1eq) 4-Biphenylcarbonsäure-Nitrophenylester versetzt und 3h gerührt. Das Lösungsmittel wurde entfernt, der Rückstand über eine Kieselgelsäule ($CHCl_3$:MeOH 10:1) gereinigt, konzentriert und aus Dioxan lyophilisiert. Ausbeute: 300mg weißer Feststoff.

**Verbindung 124**

Zu einer Lösung von 50mg Verbindung 123 und 40mg (1,4eq) 1-Azido-2-Fmoc-amino-ethan in 2ml tert.Butanol und 1ml DMF wurden 8,1mg (0,5eq) Ascorbinsäure und 7,4mg (0,5eq) $CuSO_4$ in 0,3ml $H_2O$ zugegeben, 2h bei RT gerührt, eingeengt, über RP-18 ($H_2O$>>EtOH) gereinigt, das EtOH entfernt und lyophilisiert. Ausbeute: 65mg weißer Feststoff

**Verbindung 125**

Eine Lösung von 40,5mg (2,4eq) Verbindung 124 in 1,6ml DMF und 0,4ml Piperidin wurde 20min gerührt, konzentriert, mit DMF nachgedampft und mit 3,3mg (1eq) Terephthalsäure in 1ml DMF gelöst versetzt. 5min nach Zugabe von 18,1mg (2,4eq) HATU und 22μl (6eq) DIPEA wurde konzentriert und über eine RP-18 Säule ($H_2O$>>EtOH) gereinigt. Die Produktfraktionen wurden konzentriert, mit wenig EtOH gelöst, mit $H_2O$ bis zum Auftreten einer Trübung verdünnt und mit 2M NaOH auf pH 12-13 gebracht. Nach Beenden der Reaktion wurde mit 20%HAc neutralisiert und über eine RP18 Säule ($H_2O$ >> EtOH) gereingt, das EtOH entfernt und lyophilisiert. Ausbeute: 10mg weißer Feststoff.
$^1$H-NMR (500MHz, $CD_3OD$): δ ppm 1.59 (t, *J* = 11.68, 11.68 Hz, 2H, H3-a H3-a'), 2.00 (s, 6H, NHCOCH3 NHCOCH3'), 2.87 (dd, *J* = 11.87, 3.69 Hz, 2H, H3-e H3-e'), 3.44 (dd, *J* = 8.90, 1.50 Hz, 2H, H7 H7'), 3.49 (dd, *J* = 13.71, 7.91 Hz, 2H, H9a H9a'), 3.65-3.85 (m, 12H, H4 H4' H5 H5' H6 H6' H9b H9b' CH2NH CH2NH'), 4.02 (dt, *J* = 8.31, 8.26, 3.16 Hz, 2H, H8 H8'), 4.60 (t, *J* = 5.86, 5.86 Hz, 4H, CH2N CH2N2), 4.70 (d, *J* = 12.31 Hz, 2H, OCH2a OCH2a'), 4.96 (d, *J* = 12.54 Hz, 2H, OCH2b OCH2b'), 7.36 (t, *J* = 7.39, 7.39 Hz, 2H, Ar), 7.44 (t, *J* = 7.75, 7.75 Hz, 4H, Ar), 7.63 (dd, *J* = 8.27, 1.28 Hz, 4H, Ar), 7.67 (d, *J* = 8.36 Hz, 4H, Ar), 7.79 (s, 4H, Ar), 7.89 (d, *J* = 8.39 Hz, 4H, Ar), 7.99 (s, 2H, Triazol)
HRMS (ESI-neg) [(M-2Na)/2] berechnet: 676,2498 gefunden: 676,2499

**Verbindung 126**

Hergestellt analog zu Verbindung 61

**Verbindungen 127, 128, 129**

Hergestellt analog zu Verbindung 62
**Verbindung 127:** $^1$H-NMR (500MHz, $CD_3OD$): δ 7.93 (d, *J* = 8.43 Hz, 4H, Ar), 7.81-7.74 (m, 12H, Ar), 7.45-7.39 (m, 6H, Ar), 3.98 (ddd, *J* = 9.00, 8.25, 3.10 Hz, 2H, H8 H8'), 3.85 (td, *J* = 9.59, 6.17 Hz, 2H, OCH2a OCH2a'), 3.75-3.64 (m, 10H, OCH2b OCH2b' ArCH2 ArCH2' H5 H5' H6 H6'), 3.62-3.50 (m, 8H, H4 H4' H7 H7' CH2NH CH2NH'), 3.35-3.31 (m, 2H, H9a H9a'), 3.22 (dd, *J* = 13.61, 8.06 Hz, 2H, H9b H9b'), 2.83 (dd, *J* = 12.26, 3.97 Hz, 2H, H3-e H3-e'), 2.77-2.71 (m, 4H, CH2SCH2 CH2SCH2'), 2.63 (t, *J* = 7.30 Hz, 4H, CH2SCH2 CH2SCH2'), 1.94 (s, 6H, CH3 CH3'), 1.82-1.75 (m, 4H, CH2CH2CH2, CH2CH2CH2'), 1.57 (t, *J* = 11.94 Hz, 2H, H3-a H3-a')

HRMS (ESI-neg) [(M-2Na)/2] berechnet: 695,2518 gefunden: 695,2551

**Verbindung 128:**

$^{1}H$-NMR (500MHz, $CD_3OD$): $\delta$ ppm 7.93 (d, *J* = 8.45 Hz, 4H, Ar), 7.76 (d, *J* = 8.43 Hz, 4H, Ar), 7.57 (dd, *J* = 8.23, 1.30 Hz, 4H, Ar), 7.54 (d, *J* = 8.23 Hz, 4H, Ar), 7.42-7.35 (m, 8H, Ar), 7.30 (t, *J* = 7.38 Hz, 2H, Ar), 3.98 (ddd, *J* = 9.07, 8.18, 3.08 Hz, 2H, H8 H8'), 3.87 (td, *J* = 9.78, 6.23 Hz, 2H, OCH2a OCH2a'), 3.73-3.65 (m, 6H, OCH2b OCH2b' H5 H5' H6 H6'), 3.63-3.51 (m, 12H, CH2Ar CH2Ar' H4 H4' H9a H9a' CH2NH CH2NH'), 3.35 (dd, *J* = 9.04, 1.94 Hz, 2H, H7 H7'), 3.22 (dd, *J* = 13.66, 7.95 Hz, 2H, H9b H9b'), 2.83 (dd, *J* = 12.10, 3.96 Hz, 2H, H3-e H3-e'), 2.75 (dt, *J* = 6.84, 2.89 Hz, 4H, OCH2S OCH2S'), 2.66 (dt, *J* = 6.96, 2.85 Hz, 4H, SCH2 SCH2'), 1.98 (s, 6H, CH3 CH3'), 1.81 (t, *J* = 6.43 Hz, 4H, CH2CH2CH2 CH2CH2CH2'), 1.58 (t, *J* = 11.84 Hz, 2H, H3-a H3-a')
HRMS (ESI-neg) [(M-2Na)/2] berechnet: 721,2675 gefunden: 721,2703

**Verbindung 129:**

$^{1}H$-NMR (500MHz, $CD_3OD$): $\delta$ ppm 7.96-7.88 (m, 4H, Ar), 7.81-7.70 (m, 4H, Ar), 7.57-7.50 (m, 4H, Ar), 7.46-7.42 (m, 2H, Ar), 7.40-7.36 (m, 4H, Ar), 4.03 (ddd, *J* = 8.96, 8.09, 2.93 Hz, 2H, H8 H8'), 3.90 (td, *J* = 9.90, 6.16 Hz, 2H, OCH2a OCH2a'), 3.75 (dd, *J* = 13.84, 2.91 Hz, 2H, H9a H9a'), 3.73-3.49 (m, 14H, H4 H4' H5 H5' H6 H6' H9b H9b' OCH2b OCH2b' CH2NH CH2NH'), 3.39 (dd, *J* = 8.96, 1.94 Hz, 2H, H7 H7'), 2.84 (dd, *J* = 12.23, 4.08 Hz, 2H, H3-e H3-e'), 2.82-2.63 (m, 8H, CH2SCH2 CH2SCH2'), 2.03 (s, 6H, Ch3 CH3'), 1.88-1.80 (m, 4H CH2CH2CH2 CH2CH2CH2'), 1.60 (t, *J* = 11.79 Hz, 2H, H3-a H3-a') $^{13}C$-NMR (125MHz, $CD_3OD$): $\delta$ ppm 175.7 174.5 169.9 156.0 144.4 134.9 133.5 131.1 129.8 129.0 128.3 121.6 86.2 83.8 74.2 72.3 71.1 69.6 64.0 54.1 44.2 42.7 41.0 32.1 31.4 29.4 22.7
HRMS (ESI-neg) [(M-2Na)/2] berechnet: 665,2205 gefunden: 665,2208

**Verbindung 130:**

Eine Lösung von 35mg (1eq) Verbindung 61 in 800µl absolutem DMF wurde mit 200µl Piperidin versetzt und 20min bei RT gerührt. Das Lösungsmittel wurde entfernt, der Rückstand mit 1ml abs. DMF gelöst und erneut konzentriert. Der Rückstand wurde über RP-18 ($H_2O$ pH4 (HCl) >> EtOH) gereinigt und lyophilisiert.

**Verbindung 131:**

Hergestellt analog zu Verbindung Verbindung 21. Es wurde kein HATU zugegeben und Fluoresceincarbonsäure-NHS eingesetzt.
HRMS (ESI-neg) [(M-2Na)/2] berechnet: 885,2420 gefunden: 885,2459

**Verbindung 132**

4,0mg Terephthalsäure (1eq), 38mg (2,4eq) Verbindung 11 und 22mg (2,4eq) HATU wurden in 1ml abs. DMF gelöst. Nach Zugabe von 25µl (6eq) DIPEA wurde 5min bei RT gerührt, das Lösungsmittel entfernt, der Rückstand mit wenig MeOH gelöst, $H_2O$ zugegeben bis eine Trübung auftrat und über eine RP-18 Säule ($H_2O$>>MeOH) gereinigt. Das Produkt wurde in einer Mischung aus 2ml tert.-Butanol und 0,5ml $H_2O$ gelöst, 1ml 30%iges $H_2O_2$ zugegeben und 9 Tage bei RT stehengelassen. Nach vorsichtiger Zugabe von Braunstein wurde filtriert, konzentriert, mit wenig MeOH gelöst, mit $H_2O$ bis zum Auftreten einer Trübung verdünnt und mit 2M NaOH auf pH 12-13 gebracht. Nach Beenden der Reaktion wurde mit 20%HAc neutralisiert, über eine RP18 Säule ($H_2O$>>$CH_3CN$) gereinigt, das $CH_3CN$ entfernt und lyophilisiert. Ausbeute: 30mg weißer Fesstoff.
$^{1}H$-NMR (500MHz, $CD_3OD$): $\delta$ ppm 1.49-1.66 (m, 2H, H3-a H3-a'), 1.95-2.06 (m, 10H, NHCOCH3 NHCOCH3' CH2CH2CH2 CH2CH2CH2'), 2.76-3.28 (m, 10H, H3-e H3-e' CH2SOCH2 CH2SOCH2'), 3.39-4.01 (m, 20H, H4 H4' H5 H5' H6 H6' H7 H7' H9a H9a' H9b H9b' OCH2a OCH2a' OCH2b OCH2b' CH2NH CH2NH'), 4.02-4.11 (m, 2H, H8 H8'), 7.36 (t, *J* = 7.07, 7.07 Hz, 2H, Ar), 7.44 (t, *J* = 7.47, 7.47 Hz, 4H, Ar), 7.59-7.79 (m, 8H, Ar), 7.87-7.93 (m, 8H, Ar) $^{13}C$ NMR (125MHz, CD3OD): $\delta$ ppm 22.7 24.5 35.3 35.5 42.8 44.6 50.5 50.6 52.5 52.7 54.2 63.5 69.6 71.5 72.6 74.3 102.0 128.0 128.1 128.7 129.0 130.0 134.5 138.2 141.3 145.5 169.5 170.0 174.4 174.5 175.5
HRMS (ESI-neg) [(M-2Na)/2] berechnet: 685,2311 gefunden: 685,2295

**2,2',4'-Trinitro-biphenyl-4-carboxylic acid (Verbindung 133)**

Zu einer Lösung von 10ml konzentrierter Salpetersäure und 14ml konzentrierter Schwefelsäure wurde bei 0°C 1g Biphenyl-4-carbonsäure in kleinen Portionen zugegeben. Nachdem die Reaktion beendet war, wurde 30min bei 95°C

gerührt, auf RT abgekühlt, auf Eis gegeben, abgesaugt und aus Nitromethan rekristallisiert. Ausbeute: 500mg gelbliche Nadeln

**Verbindung 134**

Eine Lösung von 35mg (1eq) Verbindung 61 in 800µl absolutem DMF wurde mit 200µl Piperidin versetzt, 20min bei RT gerührt und mit HAc neutralisiert. Das Lösungsmittel wurde entfernt und über eine RP-18 Säule gereinigt ($H_2O$ >> EtOH). Nach Lösen der Zwischenverbindung in 0,5ml EtOH wurden 30mg (3eq) 3-Benzylamino-4-Ethoxy-cyclobutene-1,2-dione, 1ml $H_2O$ und 0,1 ml 2M NaOH zugegeben, über 17h gerührt, weitere 2eq 3-Benzylamino-4-Ethoxy-cyclobutene-1,2-dione zugegeben und weitere 17h gerührt. Das Lösungsmittel wurde entfernt, der Rückstand mit wenig MeOH gelöst, $H_2O$ zugegeben bis eine kolloidale Trübung auftrat, und über eine RP-18 Säule ($H_2O$ >> EtOH) gereinigt, das EtOH entfernt und lyophilisiert. Ausbeute: 24 mg weißer Feststoff.
$^1$H-NMR (300MHz, $CD_3OD$): δ ppm 1.55 (t, *J* = 11.78, 11.78 Hz, 2H, H3-a H3-a'), 1.72-1.83 (m, 4H, CH2<u>CH2</u>CH2 CH2<u>CH2</u>CH2'), 1.99 (s, 6H, NHCO<u>CH3</u> NHCO<u>CH3</u>'), 2.61 (dt, *J* = 6.81, 6.79, 4.62 Hz, 4H, CH2S CH2S'), 2.73 (dt, *J* = 6.90, 6.81, 1.00 Hz, 4H, SCH2 SCH2'), 2.80 (dd, *J* = 12.14, 3.47 Hz, 2H, H3-e H3-e), 3.44 (dd, *J* = 8.57, 1.46 Hz, 2H, H7 H7'), 3.47-3.77 (m, 16H, H4 H4' H5 H5' H6 H6' H9a H9a' H9b H9b' OCH2a OCH2a' CH2NH CH2NH'), 3.82 (td, *J* = 9.49, 6.15, 6.15 Hz, 2H, OCH2b OCH2b'), 3.98 (ddd, *J* = 8.42, 7.20, 2.58 Hz, 2H, H8 H8'), 4.78 (s, 4H, ArCH2 ArCH2'), 7.25-7.35 (m, 10H, Ar), 7.90 (s, 4H, Ar) $^{13}$C NMR (75MHz, CD3OD): δ ppm 22.8 29.4 31.3 32.0 41.0 42.6 47.9 48.8 54.3 63.7 69.4 71.6 72.2 74.3 101.9 128.7 128.8 129.9 138.4 139.8 169.4 174.5 175.6, 183.7
HRMS (ESI-neg) [(M-2Na)/2] berechnet: 674,2263 gefunden: 674,2267

**Verbindung 135**

Hergestellt in Analogie zu: Bioorganic & Med. Chem. Letters 1995, 5 (23), 2809-2814

**Verbindung 136**

380mg (1,24mmol) Verbindung 135 wurden in 30ml DMF und 5ml $H_2O$ gelöst, 355mg (1,67mmol) 4-Biphenylessigsäure-NHS und 0,5ml ges. $NaHCO_3$-Lösung zugegeben. Nach 72h wurde eingeengt, über eine Kieselgelsäule (EtOH:EE:Hac20% 1:8:1) gereinigt und das Produkt über RP-18 gereinigt und lyophilisiert. Ausbeute: 310mg

**Verbindung 137**

270mg (054mmol) Verbindung 136 und 174mg (0,73mmol) Acetoxyessigsäure-NPE wurden in 5ml abs. DMF gelöst, 0,154ml (1,08mmol) TEA zugegeben und 17h gerührt. Nach Zugabe von 2ml 2M NaOH wurde 17h gerührt, mit 20%iger HAc neutralisiert, eingeengt, über eine Kieselgelsäule (EtOH:EE:HAc20% 1:8:1) gereinigt. Ausbeute: 270mg

**Verbindung 138**

Hergestellt analog zu Verbindung 11

**Verbindung 139**

6,0mg Terephthalsäure (1eq) und 22mg (2,4eq) HATU wurden in 1 ml abs. DMF gelöst. 2min nach Zugabe von 25µl (6eq) DIPEA wurden 55mg (2,4eq) Verbindung 138 zugegeben und 5min bei RT gerührt, das Lösungsmittel entfernt, der Rückstand mit wenig MeOH gelöst, $H_2O$ zugegeben bis eine Trübung auftrat und über eine RP-18 Säule ($H_2O$>>MeOH) gereinigt. Ausbeute: 30mg weißer Fesstoff.
$^1$H-NMR (500MHz, $CD_3OD$): δ ppm 7.90 (s, 4H, Ar), 7.57 (dd, *J* = 8.26, 1.23 Hz, 4H, Ar), 7.53 (dd, *J* = 8.20, 1.39 Hz, 4H, Ar), 7.39 (t, *J* = 7.35, 7.35 Hz, 4H, Ar), 7.35 (d, *J* = 8.15 Hz, 4H, Ar), 7.29 (t, *J* = 7.38, 7.38 Hz, 2H, Ar), 4.04 (d, *J* = 1.94 Hz, 4H, CH2OH CH2OH'), 3.96 (ddd, *J* = 9.06, 8.54, 2.91 Hz, 2H, H8 H8'), 3.85 (td, *J* = 9.61, 6.11, 6.11 Hz, 2H, OCH2a OCH2a'), 3.82-3.77 (m, 2H, H5 H5'), 3.75 (dd, *J* = 10.04, 1.72 Hz, 2H, H6 H6'), 3.71 (dd, *J* = 13.90, 2.83 Hz, 2H, H9a H9a'), 3.69-3.66 (m, 2H, H4 H4'), 3.61-3.47 (m, 10H, Ar<u>CH2</u> Ar<u>CH2</u>' OCH2b OCH2b' CH2NH CH2NH'), 3.36 (dd, *J* = 8.84, 1.97 Hz, 2H, H7 H7'), 3.18 (dd, *J* = 13.18, 7.64 Hz, 2H, H9b H9b'), 2.83 (dd, *J* = 12.29, 4.66 Hz, 2H, H3-e H3-e'), 2.72 (dd, *J* = 12.84, 6.52 Hz, 4H, SCH2 SCH2'), 2.63 (dt, *J* = 6.58, 6.51, 1.19 Hz, 4H, CH2S CH2S), 1.81-1.75 (m, 4H, SCH2<u>CH2</u> SCH2<u>CH2</u>'), 1.56 (t, *J* = 11.88, 11.88 Hz, 2H, H3-a H3-a') $^{13}$C NMR (125Hz, CD30D): δ ppm 29.5 31.5 32.0 41.1 42.8 43.5 44.1 53.8 62.7 63.8 69.4 71.5 72.3 74.0 102.0 127.9 128.2 128.3 128.6 129.9 130.7 136.2 138.4 141.0 142.1 169.4 174.1 174.6 177.3
HRMS (ESI-neg) [(M-2Na)/2] berechnet: 699,2467 gefunden: 699,2520

**Verbindung 140**

Hergestellt analog Verbindung 139. Anstelle Verbindung 138 wurde Verbindung 86 eingesetzt. Die Substanz wurde nicht mit NaOH versetzt.
HRMS (ESI-neg) [(M-2Na)/2] berechnet: 723,2194 gefunden: 723,2176

**Verbindung 141**

Hergestellt analog Verbindung 136

**Verbindung 142**

Hergestellt analog Verbindung 137

**Verbindung 143**

54mg (0,095mmol) Verbindung 142 wurden in wenig abs. MeOH gelöst, über Dowex W 650C H+ in abs. MeOH laufen gelassen und direkt mit Diazomethan in Ether vesetzt bis die Lösung gelblich blieb. Nach Zugabe von wenig verdünnter HAc wurde eingeengt. Ausbeute: 48mg

**Verbindung 144**

Hergestellt analog Verbindung 11

**Verbindung 145**

Hergestellt analog Verbindung 21
$^1$H-NMR (500MHz, $CD_3OD$): δ ppm 7.90 (d, *J* = 8.50 Hz, 4H, Ar), 7.87 (s, 4H, Ar), 7.68 (d, *J* = 8.44 Hz, 4H, Ar), 7.63 (dd, *J* = 7.62, 1.74 Hz, 4H, Ar), 7.44 (t, *J* = 7.61 Hz, 4H, Ar), 7.36 (t, *J* = 7.38 Hz, 2H, Ar), 4.85 (d, *J* = 46.96 Hz, 4H, FCH2 FCH2'), 4.11 (ddd, *J* = 8.85, 7.95, 3.20 Hz, 2H, H8 H8'), 3.91 (td, *J* = 9.79, 6.52 Hz, 2H, OCH2a OCH2a'), 3.88 (dd, *J* = 10.02, 9.44 Hz, 2H, H5 H5'), 3.86 (dd, *J* = 13.67, 3.23 Hz, 2H, H9a H9a'), 3.81 (dd, *J* = 10.58, 1.61 Hz, 2H, H6 H6'), 3.83-3.77 (m, 2H, H4 H4'), 3.60 (td, *J* = 9.31, 6.05 Hz, 2H, OCH2b OCH2b'), 3.54 (dd, *J* = 13.64, 6.56 Hz, 4H, CH2NH CH2NH'), 3.51 (dd, *J* = 13.60, 7.74 Hz, 2H, H9b H9b'), 3.48 (dd, *J* = 8.96, 1.77 Hz, 2H, H7 H7'), 2.85 (dd, *J* = 12.22, 4.67 Hz, 2H, H3-e H3-e'), 2.74 (dt, *J* = 6.94, 2.42 Hz, 4H, SCH2 SCH2'), 2.70-2.63 (m, 4H, CH2S CH2S), 1.86-1.79 (m, 4H, CH2<u>CH2</u>CH2 CH2<u>CH2</u>CH2'), 1.60 (t, *J* = 11.99 Hz, 2H, H3-a H3-a') $^{13}$C NMR (125 MHz, $CD_3O$): δ ppm 174.6 170.1 169.3 172.19 (d, *J* = 18.61 Hz) 145.6 141.3 138.4 134.5 130.0 129.1 129.0 128.6 128.2 128.0 102.0 81.00 (d, *J* = 182.75 Hz) 73.7 72.4 71.8 69.6 63.9 53.7 44.542.8 41.8 32.0 31.5 29.4
HRMS (ESI-neg) [(M-2Na)/2] berechnet: 687,2267 gefunden: 687,2244

**Verbindung 146 und 147**

25mg (1eq) Verbindung 21 wurden mit 20μl (20eq) TEA und 41mg (10eq) Benzoesäureanhydrid drei Tage bei 38°C gerührt, das TEA entfernt, mit Wasser verdünnt und über RP18 ($H_2O$>>EtOH) gereinigt. Die beiden Produkte wurden jeweils mit EE auf eine Kieselgelsäule aufgetragen, mit EE gewaschen, mit MeOH von der Säule gespült und aus $H_2O$/Dioxan lyophilisiert. Ausbeute: 10mg Verbindung 146 und 10mg Verbindung 147

**Verbindung 146:**

$^1$H-NMR (500MHz, $CD_3OD$): δ ppm 8.00 (dd, *J* = 8.14, 1.08 Hz, 4H, Ar), 7.91 (d, *J* = 8.28 Hz, 4H, Ar), 7.85 (s, 4H, Ar), 7.77-7.71 (m, 4H, Ar), 7.68 (d, *J* = 8.40 Hz, 4H, Ar), 7.64-7.57 (m, 6H, Ar), 7.49-7.42 (m, 4H, Ar), 7.36 (t, *J* = 7.34 Hz, 2H, Ar), 5.24 (ddd, *J* = 11.41, 10.61, 4.78 Hz, 2H, H4 H4'), 4.36 (t, *J* = 10.35 Hz, 2H, H5 H5'), 4.12 (ddd, *J* = 8.50, 7.72, 3.24 Hz, 2H, H8 H8'), 4.02 (dd, *J* = 10.68, 1.47 Hz, 2H, H6 H6'), 3.95 (td, *J* = 9.19, 6.08 Hz, 2H, OCH2a OCH2a'), 3.88 (dd, *J* = 13.76, 3.10 Hz, 2H, H9a H9a'), 3.63-3.49 (m, 10H, H7 H7' H9b H9b' OCH2b OCH2b' CH2NH CH2NH'), 2.90 (dd, *J* = 12.74, 5.06 Hz, 2H, H3-e H3-e'), 2.73 (t, *J* = 7.06 Hz, 4H, CH2S CH2S'), 2.66 (q, *J* = 6.87 Hz, 4H, SCH2 SCH2'), 1.96 (t, *J* = 12.14 Hz, 2H, H3-a H3-a'), 1.84 (s, 6H, CH3 CH3'), 1.89-1.80 (m, 4H, CH2<u>CH2</u>CH2 CH2<u>CH2</u>CH2')
HRMS (ESI-neg) [(M-2Na)/2] berechnet: 773,2624 gefunden: 773,2621

**Verbindung 147:**

$^1$H-NMR (500MHz, $CD_3OD$): δ ppm 8.00 (d, *J* = 7.27 Hz, 2H, Bz), 7.90 (t, *J* = 8.31 Hz, 4H, BIP), 7.85 (s, 4H, Ar), 7.75 (t, *J* = 7.48 Hz, 2H, Bz), 7.70-7.65 (m, 4H, BIP), 7.64-7.58 (m, 5H, 1xBz 4xBIP), 7.45-7.41 (m, 4H, BIP), 7.38-7.33 (m, 2H, BIP), 5.24 (ddd, *J* = 11.60, 10.46, 4.99 Hz, 1H, H4), 4.36 (t, *J* = 10.52 Hz, 1H, H5), 4.15-4.07 (m, 2H, H8 H8'), 4.02 (dd, *J* = 10.61, 1.28 Hz, 1H, H6), 3.96 (td, *J* = 9.54, 6.29 Hz, 1H, OCH2a), 3.92-3.82 (m, 2H, H4' OCH2a'), 3.86 (dd, *J* = 13.82, 3.11 Hz, 1H, H9a), 3.80 (t, *J* = 10.04 Hz, 1H, H5'), 3.75-3.69 (m, 1H, OCH2b'), 3.68 (dd, *J* = 10.46, 1.41 Hz, 1H, H6'), 3.62-3.43 (m, 10H, H7 H7' H9a' H9b H9b' OCH2b CH2NH CH2NH'), 2.90 (dd, *J* = 12.32, 4.84 Hz, 1H, H3-e), 2.76-2.61 (m, 9H, H3-e' CH2SCH2 CH2SCH2'), 2.00 (s, 3H, CH3'), 1.96 (t, *J* = 12.22 Hz, 1H, H3-a), 1.88-1.79 (m, 5H, CH3 CH2CH2CH2), 1.76-1.67 (m, 2H, CH2CH2CH2'), 1.63-1.57 (m, 1H, H3-a')
HRMS (ESI-neg) [(M-2Na)/2] berechnet: 721,2493 gefunden: 721,2482

**Verbindung 148**

Hergestellt analog zu Verbindung 11. Statt Cysteamin wurde Cystein eingesetzt.

**Verbindung 149**

Eine Lösung von 3mg (1eq) Terephthalsäure und 21,5mg (2,3eq) HATU in 1ml abs. DMF wurde mit 14μl DIPEA (6eq) versetzt. Nach Zugabe von 27,6mg (2,3eq) Verbindung 148 wurde 5min gerührt, eingeengt und über RP18 gereinigt. Das Produkt wurde analog zu Verbindung 25 verseift. Ausbeute 13mg.
$^1$H-NMR (500MHz, $CD_3OD$): δ ppm 1.58 (t, *J* = 11.68, 11.68 Hz, 2H, H3-a H3-a'), 1.77-1.84 (m, 4H, CH2CH2CH2 CH2CH2CH2'), 1.99 (s, 6H, NHCOCH3 NHCOCH3'), 2.67 (dd, *J* = 14.57, 7.13 Hz, 4H, SCH2CH2CH2 SCH2CH2CH2'), 2.81 (dd, *J* = 12.15, 4.01 Hz, 2H, H3-e H3-e'), 3.02 (dd, *J* = 13.84, 8.18 Hz, 2H, CH2CHa), 3.17 (dd. *J* = 13.79, 4.65 Hz, 2H, CH2CHb), 3.44 (dd, *J* = 8.92, 1.73 Hz, 2H, H7 H7'), 3.52-3.75 (m, 10H, H4 H4' H5 H5' H6 H6' H9a H9a' OCH2a OCH2a'), 3.80 (dd, *J* = 13.62, 3.07 Hz, 2H, H9b H9b'), 3.88 (td, *J* = 9.40, 6.20, 6.20 Hz, 2H, OCH2b OCH2b'), 4.10 (ddd, *J* = 8.78, 7.95, 3.16 Hz, 2H, H8 H8'), 4.70 (dd, *J* = 8.11, 4.65 Hz, 2H, CH2CH CH2CH'), 7.35 (t, *J* = 7.36, 7.36 Hz, 2H, Ar), 7.44 (t, *J* = 7.63, 7.63 Hz, 4H, Ar), 7.62 (d, *J* = 7.21 Hz, 4H, Ar), 7.67 (d, *J* = 8.44 Hz, 4H, Ar), 7.87-7.94 (m, 8H, Ar)
$^{13}$C NMR (125MHz, CD30D): δ ppm 22.7 30.1 31.3 34.9 42.6 44.5 54.1 55.4 63.8 69.6 71.5 72.5 74.3 101.8 128.0 128.2 128.7 129.0 129.1 130.0 134.5 138.3 141.3 145.5 169.1 170.1 174.3 175.3 175.4
HRMS (ESI-neg) [(M-2Na)/2] berechnet: 713,2260 gefunden: 713,2331

**Verbindung 150**

Zu einer Lösung von 190mg Verbindung 148 in abs. DMF wurden 115mg (1,2eq) Fmoc-NHS und 85μl (2,2eq) TEA zugegeben. Nach 30min wurde eingeengt, über eine RP-18 Säule ($H_2O$>>EtOH) gereinigt und gefriergetrocknet. Ausbeute: 218mg weißer Feststoff

**Verbindung 151**

Zu einer Lösung von 87mg Verbindung 150 in 1ml abs. DMF wurden 51μl (3eq) DIPEA und 45mg (1,2eq) HATU zugegeben. Anschließend wurden 403mg (30eq) 4-Aminobenzoesäure und 530μl (30eq) DIPEA in 2ml abs. DMF zugegeben, nach 5min das Lösungmittel entfernt und über eine Kieselgelsäule (EtOH:EE:HAc20% 1:12:1) gereinigt. Das Produkt wurde weiter über eine RP-18 Säule ($H_2O$>>EtOH) gereinigt und nach Entfernen des Ethanols lyophilisert. Ausbeute: 41mg weißer Feststoff

**Verbindung 152**

Zu einer Lösung von 33mg Verbindung 151, 26mg (1,2eq) Verbindung 11 und 12,7mg (1,2eq) HATU in 3ml abs. DMF wurden 17,4μl DIPEA gegeben, 5min bei RT gerührt, eingeengt, über eine RP-18 Säule ($H_2O$>>EtOH) gereinigt, das Ethanol entfernt und lyophilisiert. Ausbeute: 43mg weißer Feststoff.

**Verbindung 153**

Eine Lösung von 11mg Verbindung 152 in wenig Ethanol wurde mit Wasser versetzt bis eine Trübung auftrat, mit 2M NaOH auf pH12-13 eingestellt, nach 17h mit Wasser verdünnt, mit HAc20% auf pH7-8 neutralisiert, über eine RP-18 Säule ($H_2O$>>EtOH) gereinigt, das Ethanol entfernt und lyophilisiert. Ausbeute: 8mg weißer Festoff
$^1$H-NMR (300MHz, $CD_3OD$): δ ppm 1.50-1.68 (m, 2H, H3-a H3-a'), 1.75-1.87 (m, 4H, CH2CH2CH2 CH2CH2CH2'), 2.00

(s, 6H, NHCOCH3 NHCOCH3'), 2.60-2.76 (m, 8H, H3-e H3-e' CH2SCH2 CH2SCH2), 2.79-2.89 (m, 1H, SCH2a), 2.91-3.03 (m, 1H, SCH2b), 3.33-3.95 (m, 19H, H4 H4' H5 H5' H6 H6' H7 H7' H9a H9a' H9b H9b' OCH2a OCH2a' OCH2b OCH2b'CH2NH2 CHNH), 4.05-4.15 (m, 2H, H8 H8'), 7.35 (t, *J* = 7.28, 7.28 Hz, 2H, Ar), 7.44 (t, *J* = 7.64, 7.64 Hz, 4H, Ar), 7.62 (dd, *J* = 8.29, 1.28 Hz, 4H, Ar), 7.65-7.72 (m, 6H, Ar), 7.77 (d, *J* = 8.47 Hz, 2H, Ar), 7.90 (d, *J* = 8.30 Hz, 4H, Ar)

**2-(18-biotinoylamino-1,4,7,10,13,16-hexaoxo-octadecyl)-Terephthalsäure (Verbindung 154)**

Eine Lösung von 240mg (1eq) Terephtalsäuredimethylester und 386mg (1,1eq) 17-azido-1-hydroxy-3,6,9,12,15-heptaoxo-heptadecan in 5ml abs. DMF wurde bei 0°C mit 449mg (1,5eq) Triphenylphosphin und 330μl (1,5eq) DIAD versetzt und über nacht gerührt. 2h Nach Zugabe von 2ml MeOH wurde eingeengt, in $CH_2Cl_2$ aufgenommen, mit $H_2O$ gewaschen, mit $MgSO_4$ getrocknet, filtriert, über Kieselgel gereinigt (Hexan>>EE) und konzentriert. Das erhaltene Öl wurde in 5ml MeOH und 1ml $H_2O$ gelöst, mit Pd auf Kohle (10%) und 80μl HAc(20%) versetzt, 2h hydriert, eingeengt und in $H_2O$ gelöst. Die Lösung wurde mit HCl auf pH 3-4 eingestellt und über RP18 gereingt ($H_2O$>>EtOH). Das Produkt wurde in DMF gelöst, mit Biotin-NHS (1eq) und DIPEA (3eq) versetzt, eingeengt und über RP-18 gereingt. Die erhaltene Substanz wurde mit EtOH-$H_2O$ Gemisch gelöst, der pH mit 2M NaOH auf 12-13 eingestellt, nach 3h mit HCl auf pH 3 eingestellt und erneut über RP18 gereinigt.

**Substanz 155**

Hergestellt analog zu Verbindung 21 mit Verbindung 154.
$^1$H NMR (500MHz, $CD_3OD$): δ ppm 7.92-7.87 (m, 4H), 7.94 (d, *J* = 8.08 Hz, 1H), 7.69-7.65 (m, 4H), 7.63-7.60 (m, 4H), 7.52 (d, *J* = 1.37 Hz, 1H), 7.46 (dd, *J* = 8.19, 1.55 Hz, 1H), 7.46-7.40 (m, 4H), 7.38-7.33 (m, 2H), 4.46 (ddd, *J* = 7.86, 5.05, 0.66 Hz, 1H), 4.30-4.25 (m, 3H), 4.13-4.06 (m, 2H), 3.94-3.83 (m, 6H), 3.75-3.42 (m, 34H), 3.33-3.31 (m, 2H), 3.15 (ddd, *J* = 8.76, 5.64, 4.59 Hz, 1H), 2.89 (dd, *J* = 12.75, 5.00 Hz, 1H), 2.86-2.81 (m, 2H), 2.77-2.72 (m, 4H), 2.71-2.61 (m, 5H), 2.19 (t, *J* = 7.37 Hz, 2H), 2.01 (s, 6H), 1.87-1.79 (m, 4H), 1.75-1.51 (m, 6H), 1.44-1.32 (2H, m) $^{13}$C-NMR (125MHz, $CD_3OD$): δ ppm 175.5 173.7 170.0 169.3 145.5 141.3 138.4 134.5 130.0 129.1 129.0 128.6 128.1 128.0 101.4 74.5 72.5 71.5 69.4 63.8 54.1 44.7 42.5 41.0 32.0 31.3 29.4 22.7
HRMS (Esi-neg) [M-2Na)/2] berechnt: 1844.7254 gefunden 1844.7362

**Biologische Tests**

Die Affinität einiger Dimere wurde mit einem bekannten Test gemessen (Bock et al. Methods Mol. Biol. 2006, 347, 359-375). Es wurde eine stark erhöhte Affinität gefunden. Exakte Werte konnten allerdings aufgrund von schwankenden Werten nicht bestimmt werden. Um verläßlich die Affinität der neuen Derivate bestimmen zu können wurde ein neuer Test entworfen. Für diesen Test wurde die Verbindung 155 hergestellt.

| | |
|---|---|
| 155 | 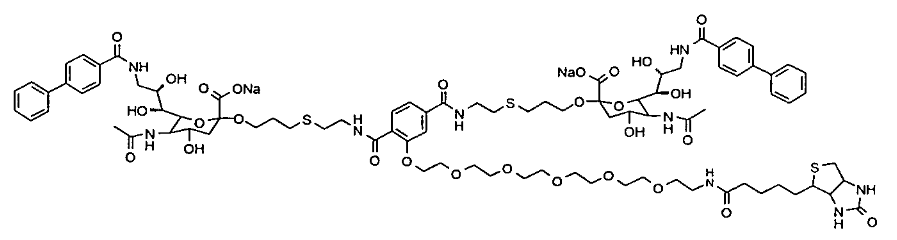 |

Mit der Substanz wurde der folgende Test durchgeführt.

Zur Bestimmung der Affinität der Verbindung der Formel (I) wurden ca. 5x $10^6$ bis 2x$10^8$ Zellen der humanen B-Zell-Lymphom-Linie "Daudi" mit Sialidase (A. ureafaciens) 1h bei 37°C inkubiert (Endkonzentration 0,1 Unit/ml). Anschliessend wurde das Enzym mit 1,5mM 2,3-DehydrNeu5Ac blockiert und die Zellen 2mal mit FACS-Puffer (PBS, 0,1% BSA, 0,01%$NaN_3$) gewaschen. Je 1-2x$10^6$ Zellen wurden in Probengefässe überführt und auf Eis 15min mit seriellen Konzentrationen (10fache Verdünnungen, 1mM bis 1x$10^{-7}$ mM) der zu messenden Substanz (Inhibitor) inkubiert. Das Reaktionsvolumen betrug 50μl. Anschliessend wurden 50microliter Verbindung 155 (4micromolar) zugegeben und 20min inkubiert. Nach Waschen mit FACS-Puffer (PBS, 0,1 BSA, 0,01% Natriumazid) wurde 15min mit Streptavidin-PE im Dunkeln inkubiert, gewaschen und die gebundene Menge an Substanz 390 über das Streptavidin-PE im FACS gemessen. Zur Bestimmung von 0% Verdrängung der Verbindung 155 wurden Zellen ohne Inhibitor gemessen. Zur Bestimmung von 100% Verdrängung wurden Zellen ohne Verbindung 155 und ohne Inhibitor gemessen. Pro Test wurden 4 Substanzen und eine Referenzsubstanz gemessen. Die bestimmten IC50-Werte jeden Testes wurden anschliessend

in rIP-Werte (relative Inhibitorische Potenz) umgerechnet. Zur Bestimmung der rIP-Werte lief die Substanz 21 als Referenz in den Testen mit.

Es wurden stark erhöhte rIP-Werte für die Dimeren gefunden. Der Vergleich des Monomeren "BPCNeu5Ac" (J. Exp. Med. 2002, 195, 1207-1213) mit den Dimeren (Verbindungen der Formel (I)) ist auch mit dem neuen Test nicht exakt möglich, da sich die IC50-Werte der beiden Substanzklassen unabhängig voneinander verhalten und von Test zu Test schwankten.

Die IC50 Werte innerhalb der Gruppe der Dimere schwankten auch. Überraschenderweise schwankten sie aber synchron. Dadurch war es möglich stabile rIP-Werte zu einer dimeren Referenzverbindung zu bilden. Tabelle I zeigt die bekannten monomeren Verbindung BPCNeu5Ac sowie die dimeren Verbindungen 38 und 21 mit rIP-Werten aus verschiedenen Testen.

Tabelle I

| Nr. | Struktur | rIP (Literatur -Assay) | rIP (Neuer Assay) |
|---|---|---|---|
| BPC Neu 5Ac | 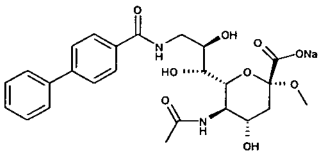 | 1 | |
| 38 | 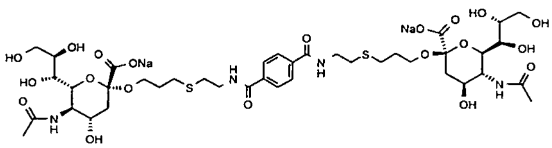 | ~1-3 | 1 |
| 21 | 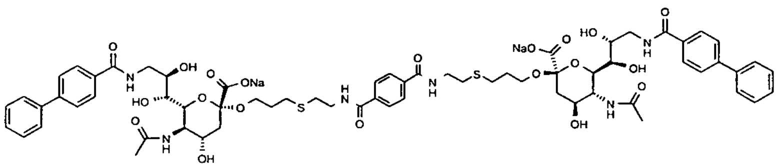 | > 2000 | 2979 |

Durch einfache Dimerisierung der Sialinsäure wurde bereits eine stark erhöhte Affinität erzielt. Durch Einführen eines substituierten Stickstoffes in die 9-Position des Dimeren wurde die Affinität überraschenderweise weiter besonders stark erhöht.

Alle Verbindungen der Formel (I) (in den Syntheseschemata ausschließlich mit einer Nummer gekennzeichnet) werden in Tabelle II aufgeführt. Die rIP-Werte in Tabelle II sind in Bezug auf Verbindung 38 gesetzt.

Tabelle II

| Nr. | Struktur | rIP |
|---|---|---|
| 38 | 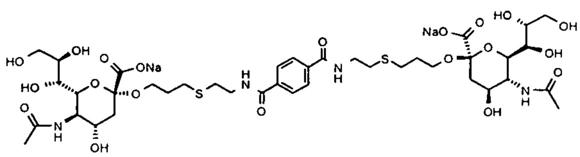 | 1 |
| 13 | 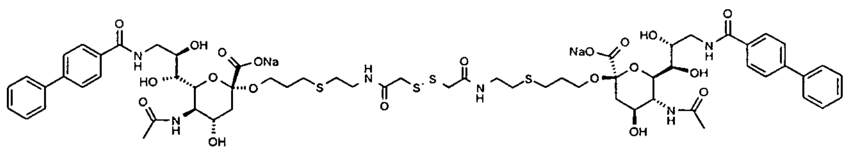 | 251 |

(fortgesetzt)

| Nr. | Struktur | rIP |
|---|---|---|
| 19 | 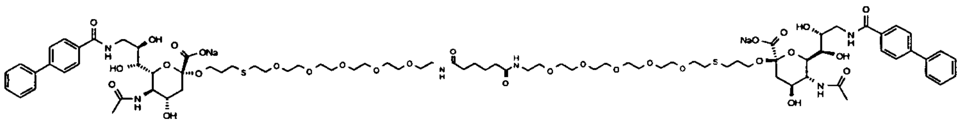 | 368 |
| 20 | 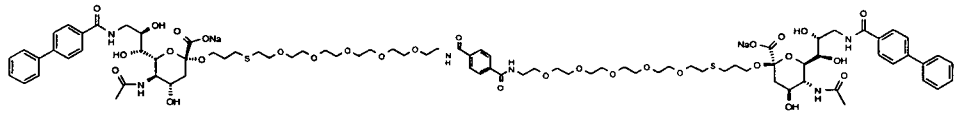 | 292 |
| 21 | 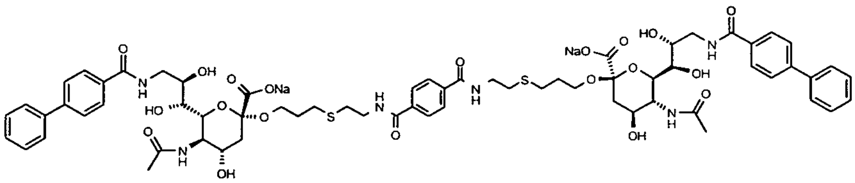 | 2979 |
| 22 | 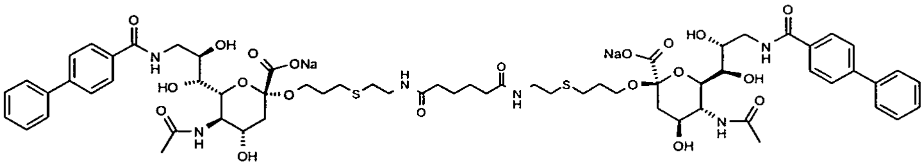 | 192 |
| 23 | 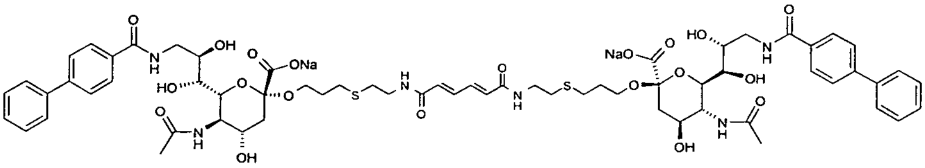 | 309 |
| 24 | 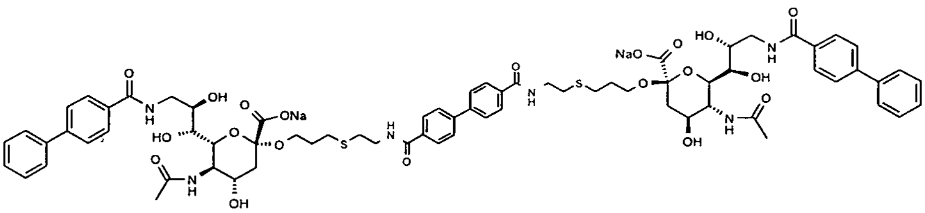 | 75 |
| 25 | 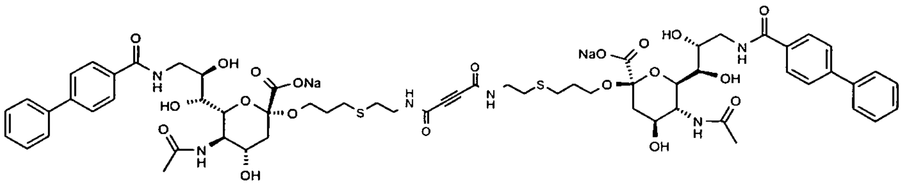 | 4944 |
| 26 | 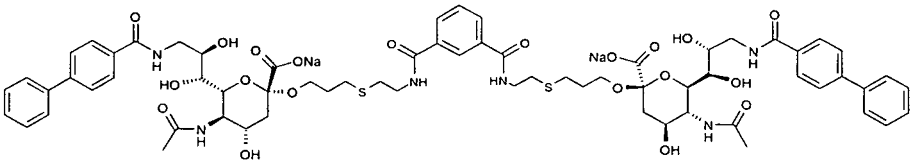 | 308 |

(fortgesetzt)

| Nr. | Struktur | rIP |
|---|---|---|
| 27 | 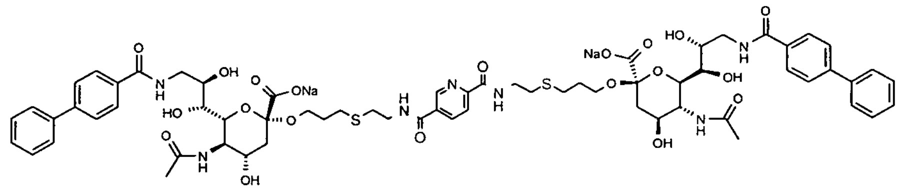 | 2990 |
| 28 | 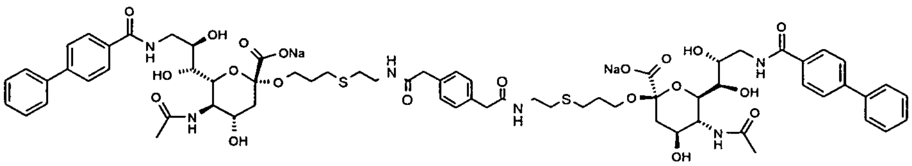 | 129 |
| 29 | 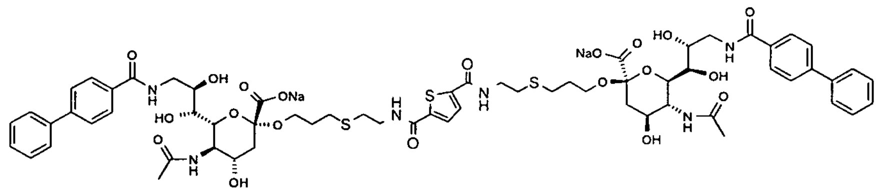 | 2061 |
| 30 | 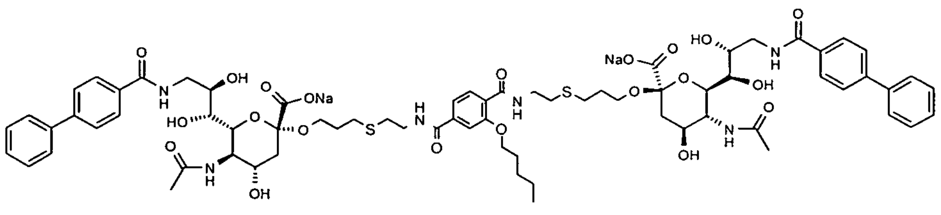 | 6998 |
| 31 | 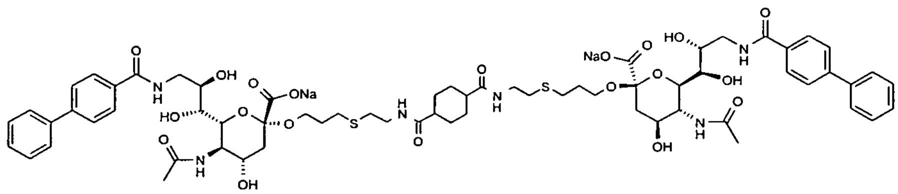 | 643 |
| 32 | 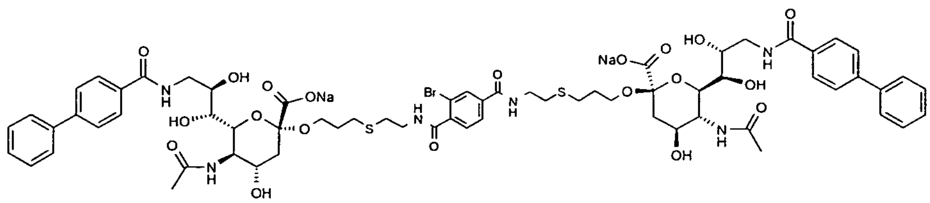 | 14260 |
| 33 | 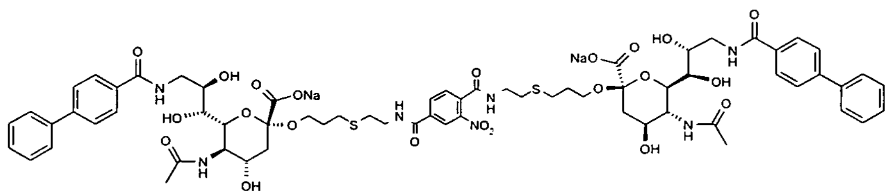 | 4476 |

(fortgesetzt)

| Nr. | Struktur | rIP |
|---|---|---|
| 34 | 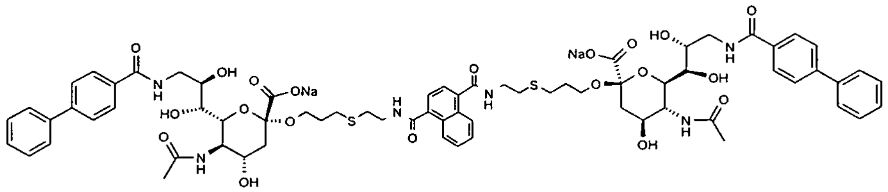 | 899 |
| 35 | 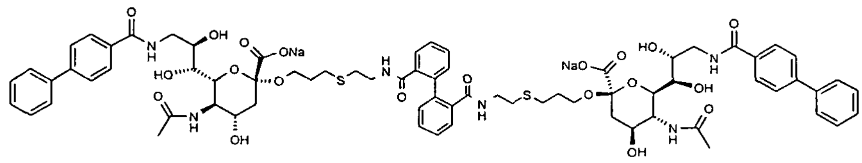 | 717 |
| 36 | 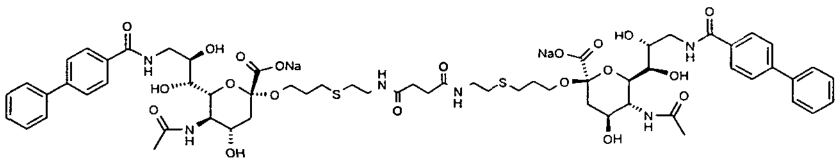 | 23 |
| 37 | 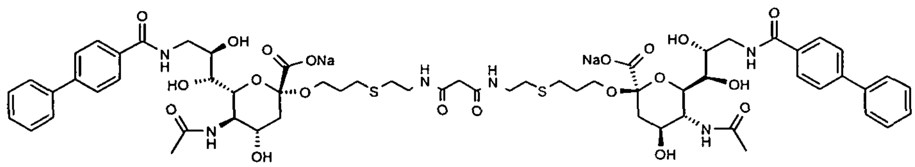 | 177 |
| 39 | 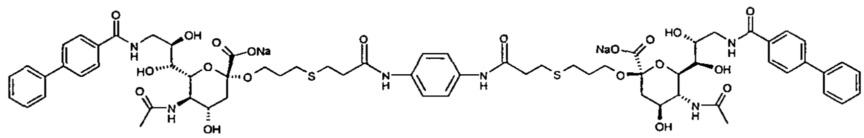 | 10760 |
| 40 | 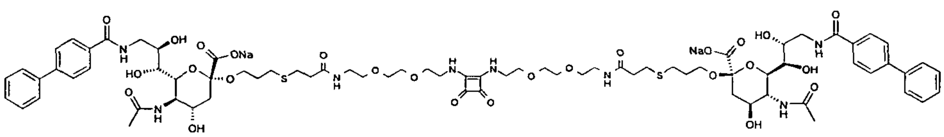 | 433 |
| 41 | 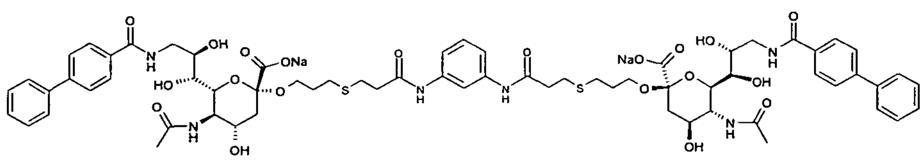 | 423 |
| 42 | 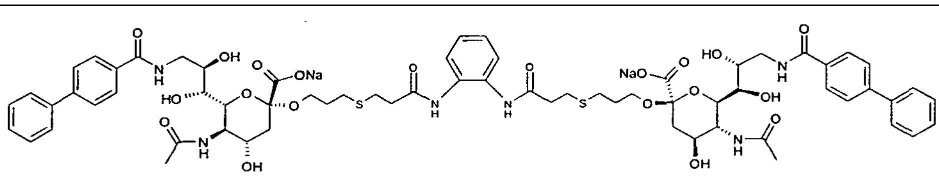 | 359 |
| 43 | 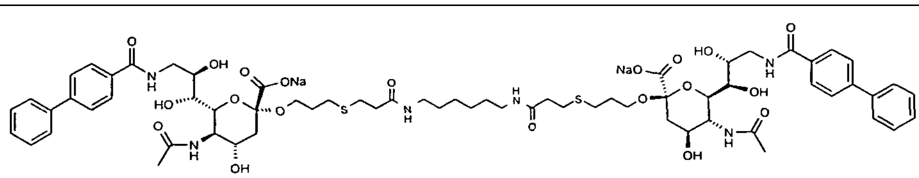 | 15 |

(fortgesetzt)

| Nr. | Struktur | rIP |
|---|---|---|
| 44 | 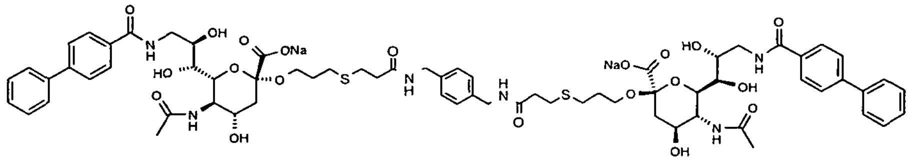 | 177 |
| 45 | 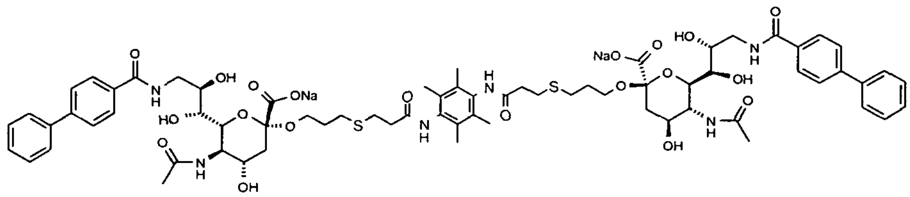 | 523 |
| 46 | 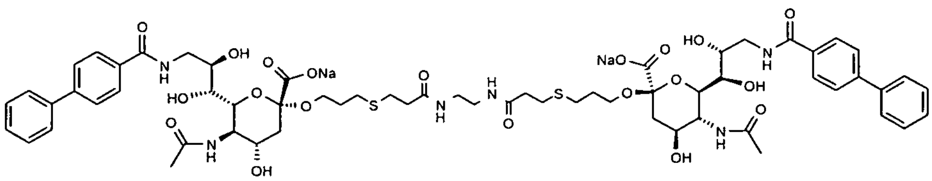 | 290 |
| 49 | 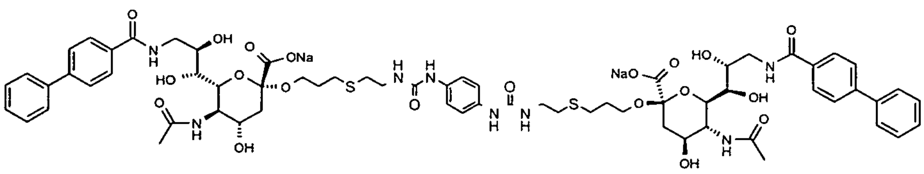 | 626 |
| 51 | 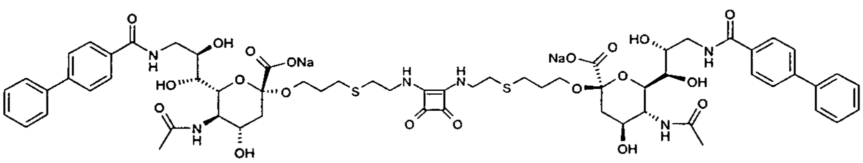 | 299 |
| 52 | 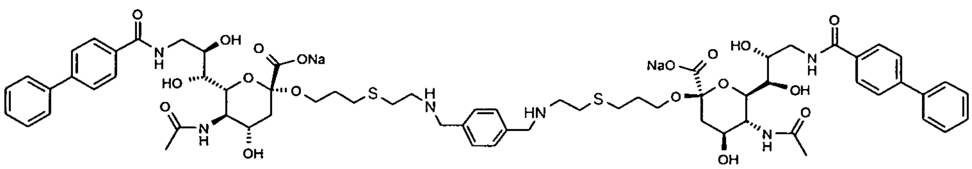 | 31 |
| 54 | 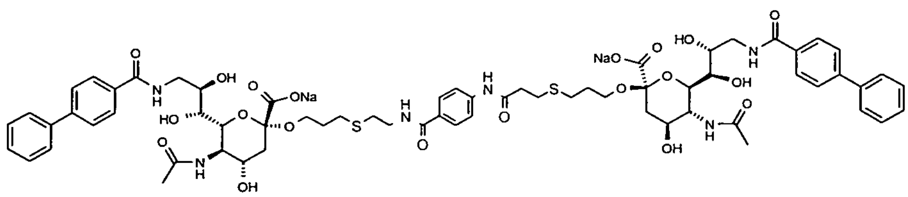 | 7092 |
| 57 | 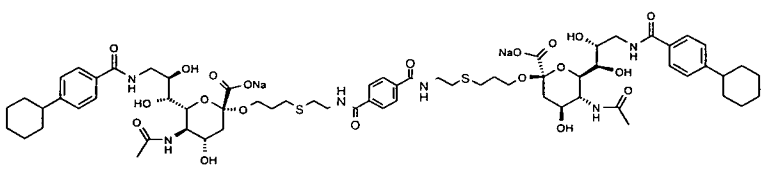 | 22 |

(fortgesetzt)

| Nr. | Struktur | rIP |
|---|---|---|
| 58 | 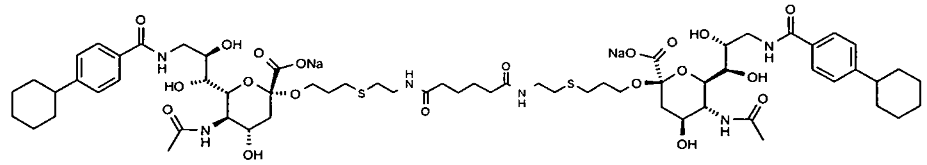 | 24 |
| 62 | 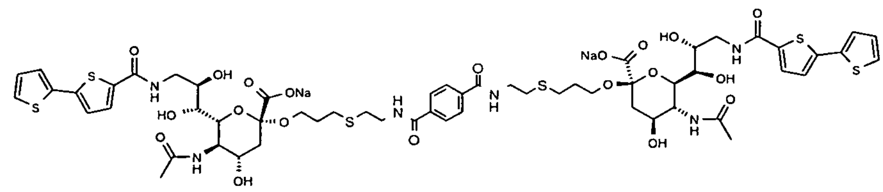 | 203 |
| 63 | 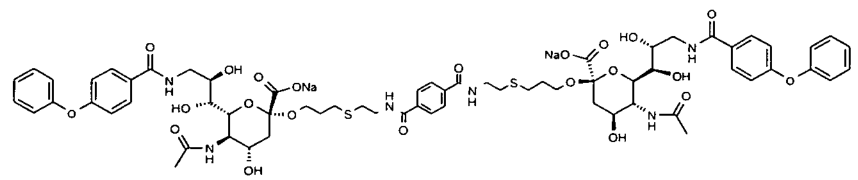 | 20 |
| 64 | 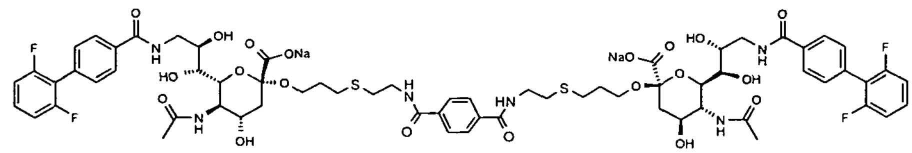 | 158 |
| 65 | 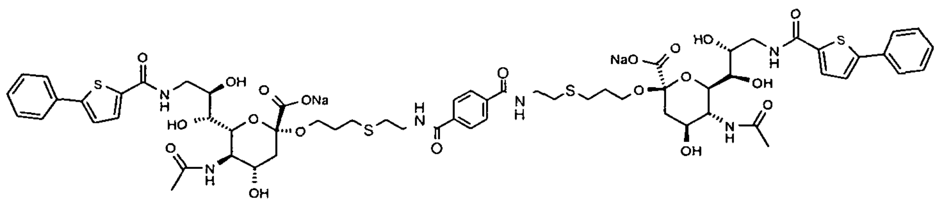 | 106 |
| 66 | 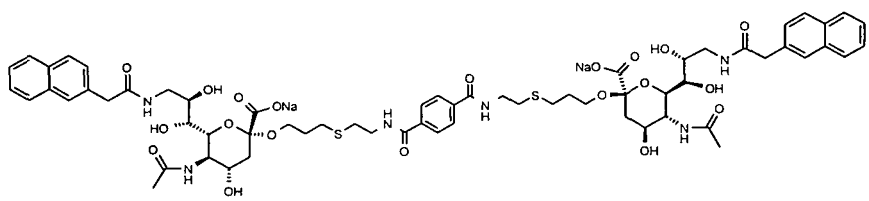 | 197 |
| 67 | 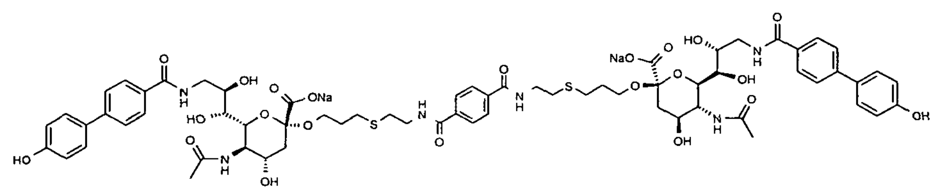 | 1959 |
| 68 | 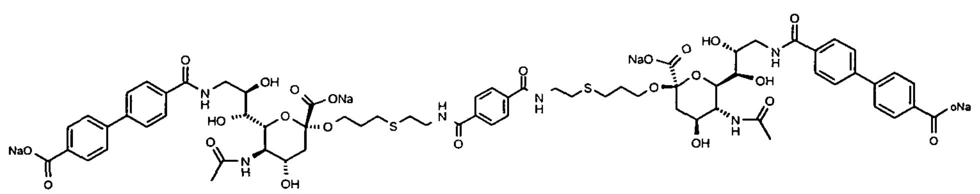 | 5 |

(fortgesetzt)

| Nr. | Struktur | rIP |
|---|---|---|
| 69 | 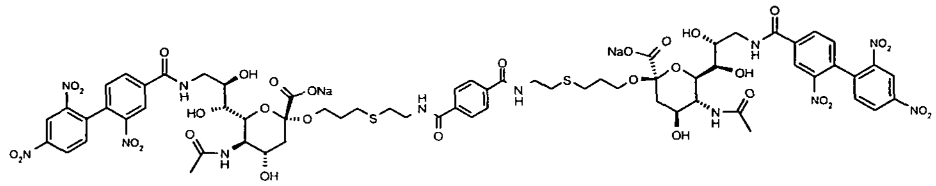 | 14 |
| 70 | 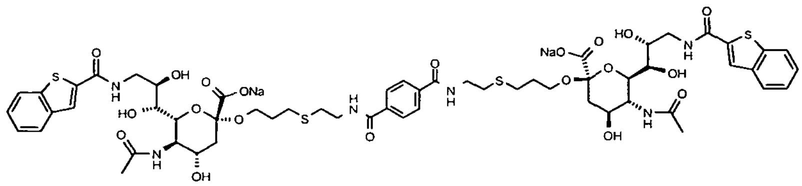 | 137 |
| 71 | 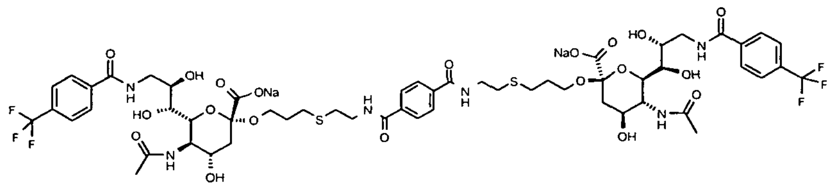 | 32 |
| 72 | 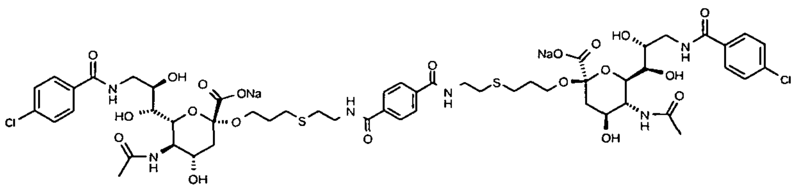 | 38 |
| 73 | 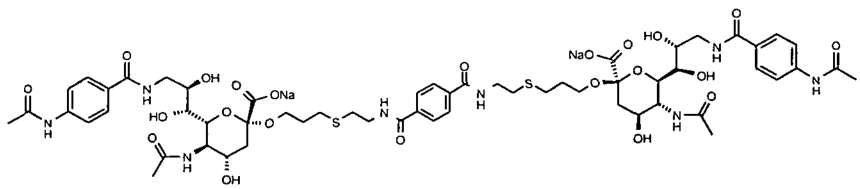 | 13 |
| 74 | 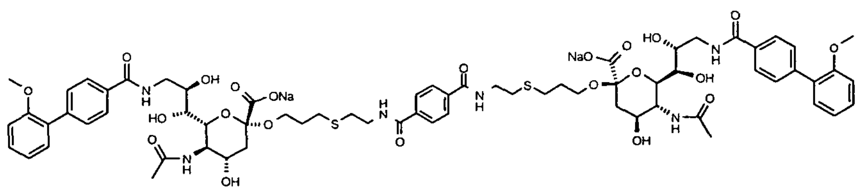 | 27 |
| 75 | 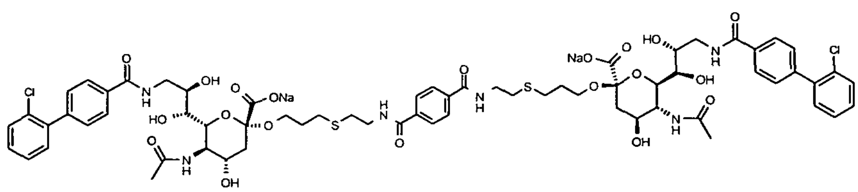 | 3210 |
| 76 | 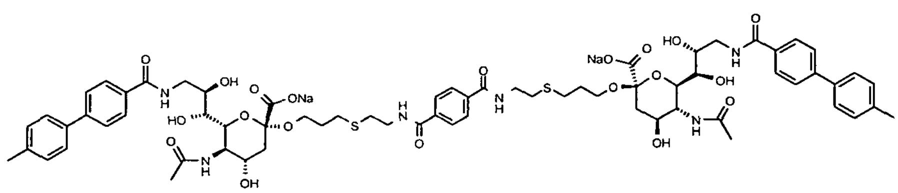 | 188 |

(fortgesetzt)

| Nr. | Struktur | rIP |
|---|---|---|
| 77 | 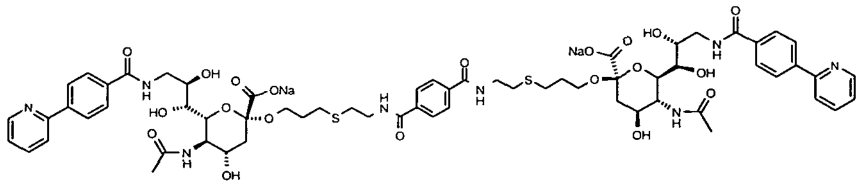 | 95 |
| 78 | 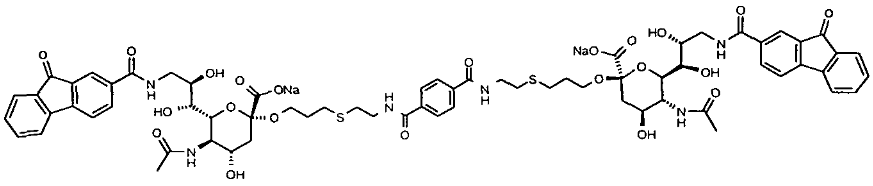 | 27 |
| 79 | 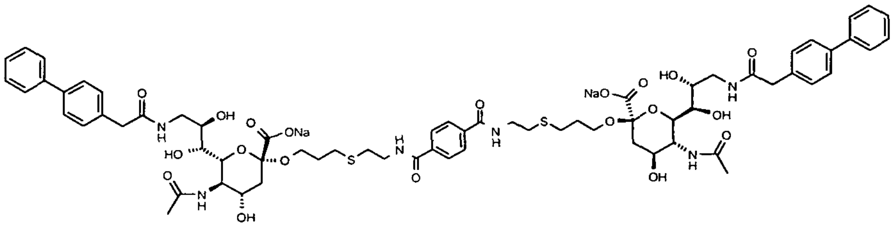 | 181 |
| 80 | 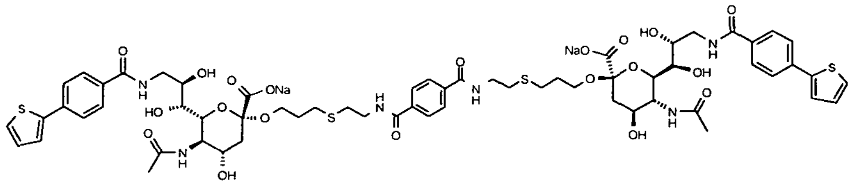 | 14342 |
| 81 | 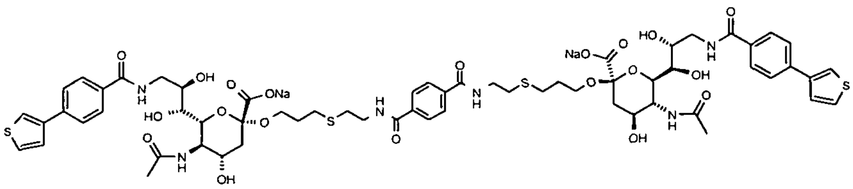 | 2068 |
| 82 | 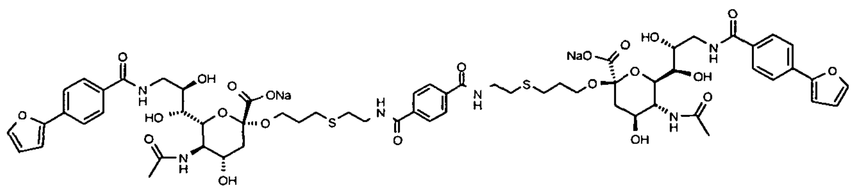 | 752 |
| 83 | 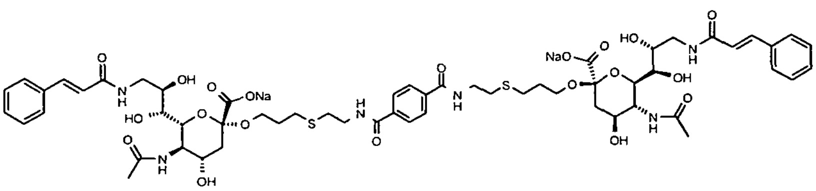 | 73 |
| 84 | 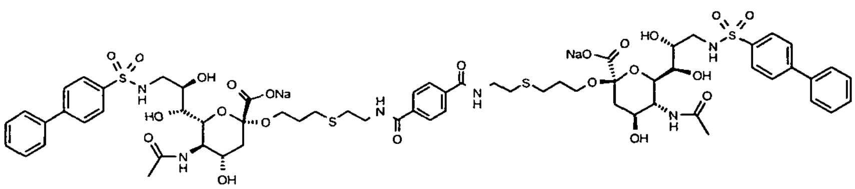 | 9 |

(fortgesetzt)

| Nr. | Struktur | rIP |
|---|---|---|
| 85 | 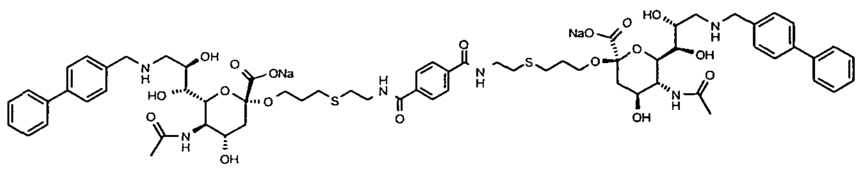 | 1 |
| 90 | 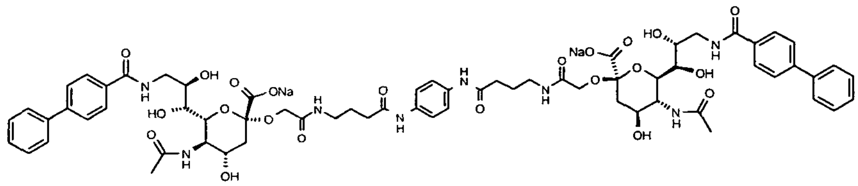 | 1445 |
| 92 | 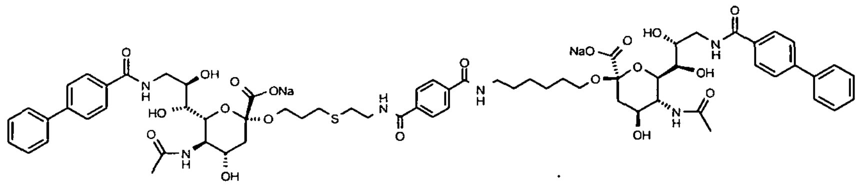 | 3788 |
| 95 | 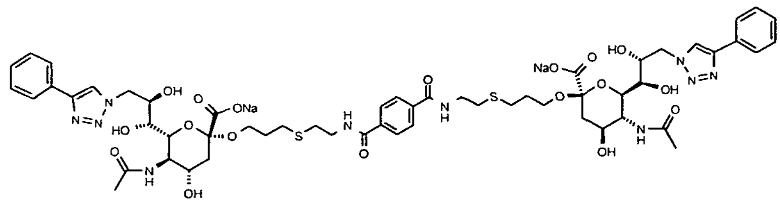 | 6 |
| 102 | 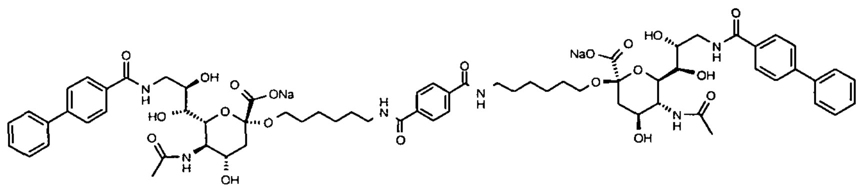 | 11771 |
| 103 | 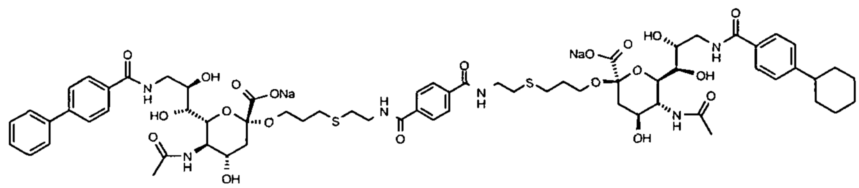 | 185 |
| 110 | 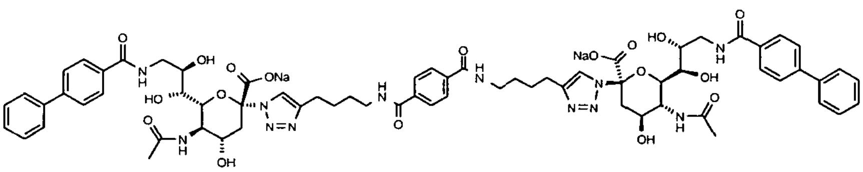 | 12 |

(fortgesetzt)

| Nr. | Struktur | rIP |
|---|---|---|
| 113 | 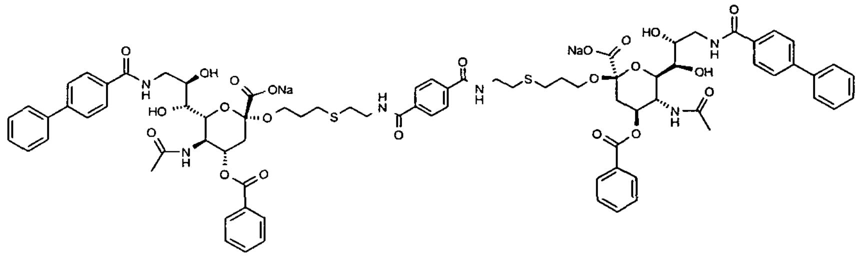 | 3522 |
| 116 | 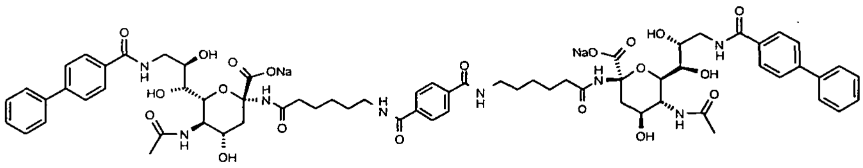 | <1 |
| 122 | 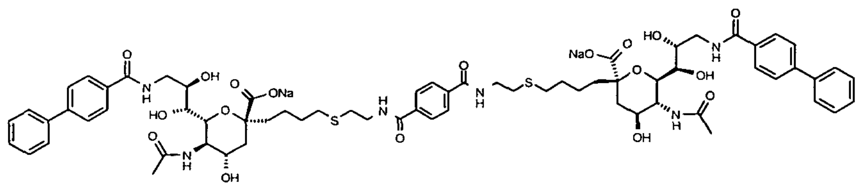 | 884 |
| 125 | 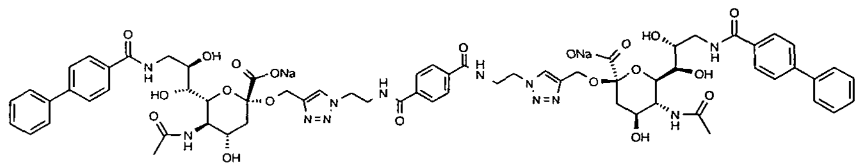 | 8 |
| 127 | 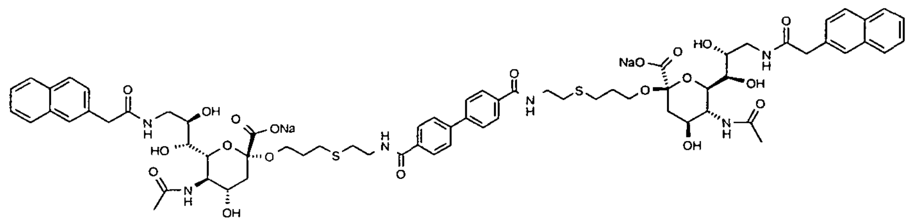 | 74 |
| 128 | 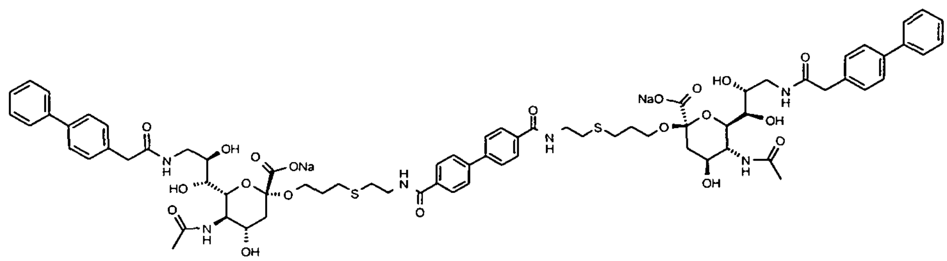 | 19 |
| 129 | 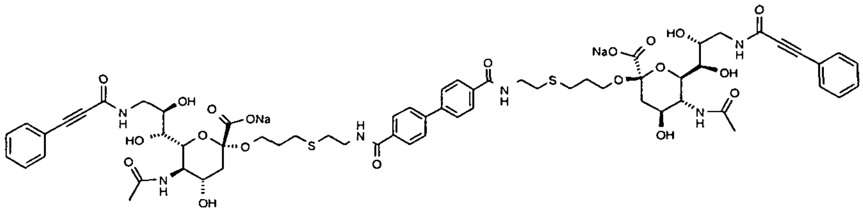 | 21 |

(fortgesetzt)

| Nr. | Struktur | rIP |
|---|---|---|
| 131 | 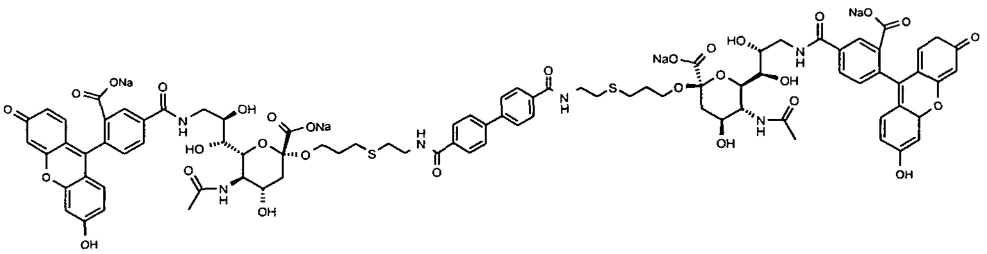 | 4 |
| 132 | 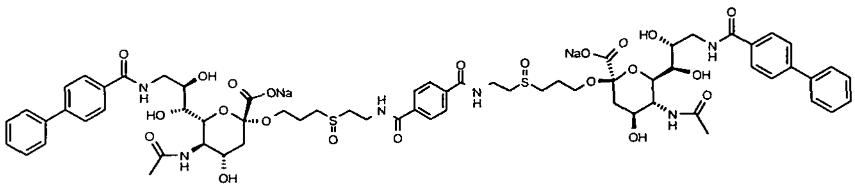 | 212 |
| 134 | 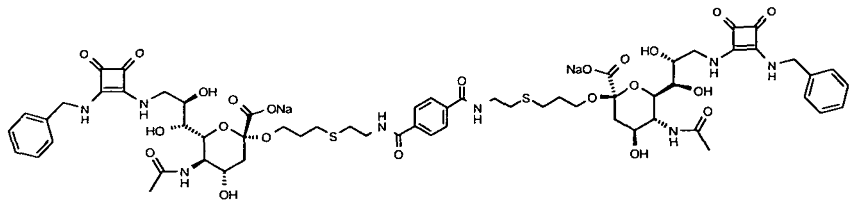 | 70 |
| 139 | 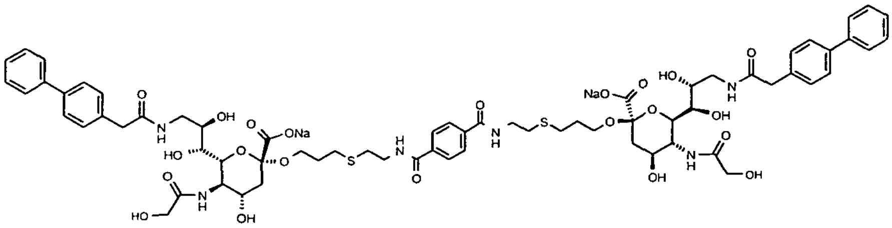 | 63 |
| 140 | 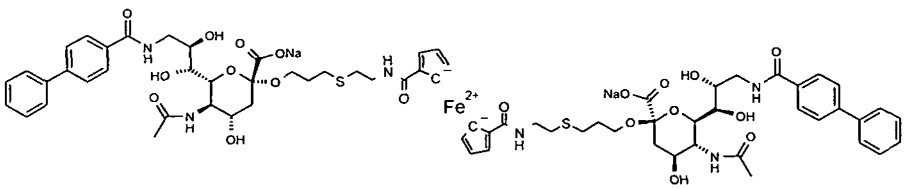 | 238 |
| 145 | 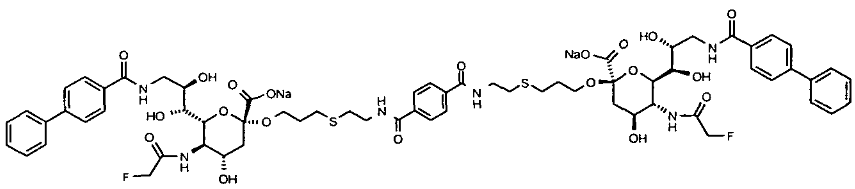 | 7003 |
| 147 | 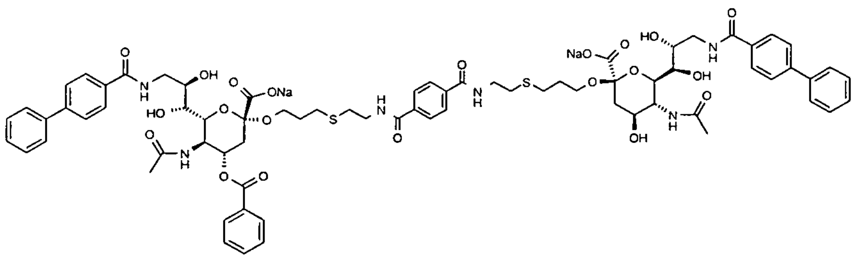 | 32069 |

(fortgesetzt)

| Nr. | Struktur | rIP |
|---|---|---|
| 149 | 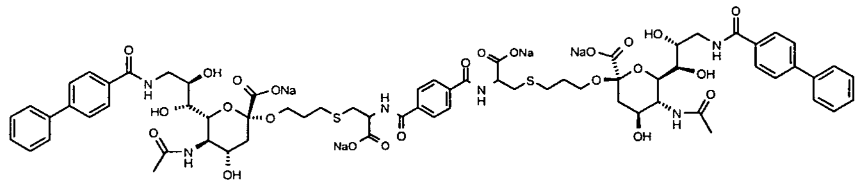 | 378 |
| 153 | 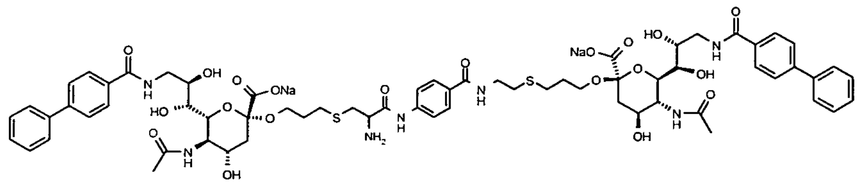 | 1597 |

In einem weiteren Test wurden einige der Substanzen auf die Fähigkeit den Calciumflux in B-Zellen zu beeinflussen untersucht. Der Test wurde wie in "J. Exp. Med. 2002, 195, 1207-1213" beschrieben durchgeführt. Die Substanzen zeigen eine starke Steigerung der Calciummobilisation auch bei niedrigen Konzentrationen. Beispielhaft ist der Calciumflux von Substanz 21 im Vergleich zu BPC Neu5Ac in Figur 1 dargestellt.

## Patentansprüche

**1.** Sialinsäurederivat der Formel (I),

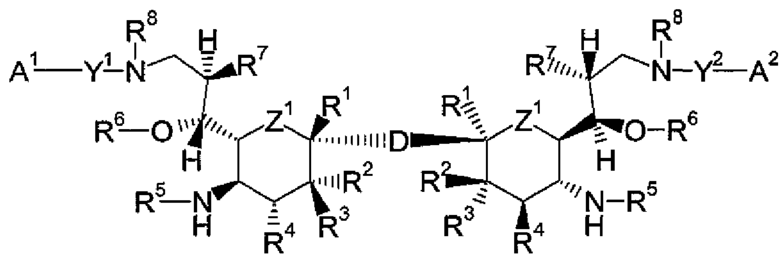

(I)

wobei die Symbole folgende Bedeutungen haben:

$A^1$, $A^2$ sind gleich oder verschieden eine Gruppe $D^1$-[$Y^3$-$D^{21}$-]$_m$-.
$D^1$, $D^2$ sind gleich oder verschieden ein mono- oder polycyclischer aromatischer, partiell ungesättigter oder gesättigter $C_3$-$C_{14}$-Kohlenwasserstoffrest oder ein aromatischer, partiell ungesättigter oder gesättigter drei- bis achtgliedriger heterocyclischer Rest, wobei die genannten Reste unsubstituiert oder ein- oder mehrfach durch eine Gruppe $X^1$ substituiert sind;
$X^1$ ist gleich oder verschieden Halogen, Cyano, Nitro, Hydroxy, Mercapto, Amino, Carboxyl, Hydroxylamino, Azido, $B(OH)_2$, SO, $SO_3M$. $OSO_3M$, $SO_2NH_2$, $SO_2CF_3$, P03M, $OPO_3M$, Cyanomethyl, Alkyl, Halogenalkyl, Alkenyl, Alkinyl, Alkyloxy, Halogenalkyloxy, Alkenyloxy, Alkinyloxy, Alkylthio, Alkylamino, Dialkylamin, Trialkylamino, Formyl, Alkylcarbonyl, Alkylsulfonyl, Alkylsulfoxyl, Alkylaminosulfoxyl, Dialkylaminosulfoxyl, Alkyloxycarbonyl, Alkylcarbonyloxy, Aminocarbonyl, Aminothiocarbonyl, Alkylaminocarbonyl, Alkylcarbonylamino, Dialkylaminocarbonyl, Alkylaminothiocarbonyl, Oxo (=O), Thioxo (=S), $C_1$-$C_8$-Alkylimino (=N-$C_1$-$C_8$-Alkyl), $C_1$-$C_8$-Alkyloximino (=N-O-$C_1$-$C_8$-Alkyl), wobei die Alkylgruppen in diesen Resten 1 bis 8 Kohlenstoffatome enthalten und die genannten Alkenyl-oder Alkinylgruppen in diesen Resten 2 bis 8 Kohlenstoffatome enthalten;
$Y^1$, $Y^2$ sind gleich oder verschieden ~($C_1$-$C_4$-Alkyl)-, ~($C_1$-$C_4$-Halogenalkyl)-, ~($C_1$-$C_4$-Alkyl)-(CO)-, ~C(O)-, ~$CH_2$-C(O)-, ~CH=CH-C(O)-, ~C≡C-C(O)-, ~$S(O)_2$-, ~$CH_2$-$S(O)_2$-, ~$NR^x$-C(O)-, ~$CH(CF_3)$-, ~$NR^x$-3-cyclobuten-1,2-dion-4-, ~$CH_2$-$NR^x$-3-cyclobuten-1,2-dion-4-, ~$NR^x$-3-2,5-Thiadiazol-1-oxid-4-, ~$NR^x$-3-2,5-Thiadiazol-1,1-dioxid-4-, wobei ~ die Bindung zur Gruppe A bezeichnet oder
$Y^1$, $Y^2$ bilden, unabhängig voneinander, zusammen mit dem Stickstoff, an den sie gebunden sind, und der

Gruppe $R^8$ eine Gruppe ~4-1H-(1,2,3)Triazol-1-yl-, ~5-1H-(1,2,3)Triazol-1-yl-, ~$CH_2$-4-1H-(1,2,3)Triazol-1-yl-oder - $CH_2$-5-1H-(1,2,3)Triazol-1-yl-, wobei ~ die Bindung zur Gruppe A bezeichnet;

$Y^3$ ist eine Bindung, O, S, S(O), $S(O)_2$, $CH_2$, C(O), $CR^x_2$ oder $NR^x$;

m ist 0, 1 oder 2;

$Z^1$ ist O, S, $CH_2$ oder $NR^x$;

D ist eine Gruppe $Z^2$-$T^1$-$Y^4$-$A^3$-$Y^5$-$T^2$-$Z^2$;

$Z^2$ ist gleich oder verschieden -O~, -S~, -$NR^x$~, -NH-C(O)~, -$CH_2$~, -$CF_2$~, -CH(OH)~" -N($R^x$)-O~, -O-$NR^x$~, -O-N-CH~, ~-1H-(1,2,3)Triazol-1 yl-, oder ~5-1H-(1,2,3)Triaaal-1-yl- wobei ~ die Bindung zur Gruppe T bezeichnet;

$T^1$, $T^2$ sind gleich oder verschieden eine geradkettige oder verzweigte Allcandiylgruppe mit 1 bis 30 C-Atomen, wobei

(i) gegebenenfalls eine oder mehrere nicht terminal $CH_2$-Gruppen durch -O-, -S-, -S(O)-, -$S(O)_2$-, -$S^+(CH_3)$-, -$P(O_2)$- und/oder -$NR^x$-ersetzt sind und/oder

(ii) gegebenenfalls ein oder mehrere H-Atome durch F, Cl, $OR^x$, $OSO_3M$, (=0), (=S), Carboxyl, $NH_2$, $NHR^y$ und/oder $NHR^z$ ersetzt sind und/oder

(iii) gegebenenfalls eine oder mehrere nicht terminale -$CH_2$-$CH_2$-Gruppen durch -5-1H-(1,2,3)Triazol-1-yl-, -$CR^x$=$CR^x$- und/oder -C≡C- ersetzt sind und/oder

(iv) gegebenfalls eine -$CH_2CH_2CH_2$-Gruppe durch -4-1H-(1,2,3)Triazol-1-yl- und /oder -O-N=CH- ersetzt ist und/oder

(v) gegebenfalls eine -$CH_2CH_2CH_2CH_2$-Gruppe durch Phenyl-1,4-diyl ersetzt ist;

$Y^4$, $Y^5$ sind gleich oder verschieden eine Bindung O, S, $NR^x$, S(O), $S(O)_2$, C(O), ~C(O)-$NR^x$-, ~$NR^x$-C(O)-, ~C(O)-O-, ~O-C(O)-, ~$NR^x$-CO-$NR^x$-, ~$NR^x$-$S(O)_2$-, ~$S(O)_2$-$NR^x$-, ~$CH_2$-$NR^x$-C(O)-, ~$CH_2$-C(O)-$NR^x$-, ~$CH_2$-$NR^x$-, ~CH($CF_3$)-$NR^x$-, ~CH=N-O- oder ~O-N=CH-, wobei ~ die Bindung zu Gruppe A bezeichnet;

$A^3$ ist

a) $C_1$-$C_8$ Alkandiyl, $C_2$-$C_8$-Alkendiyl, $C_2$-$C_8$-Alkindiyl, $C_4$-$C_8$-Alkadiendiyl, wobei in den genannten Gruppen mehrere $CH_2$-Gruppen durch O, S, S(O), $S(O)_2$, $NR^x$ und/oder C(O) ersetzt sind und wobei in den genannten Gruppen gegebenenfalls ein oder mehrere H-Atome durch eine Gruppe $X^2$ ersetzt sind,

b) eine Gruppe $A^4$-[$Z^3$-$A^5$]$_n$,

c) 1,1'-Ferrocendiyl, 1,1'-Cobaltocendiyl, 1,1'-Ruthenocen oder Dichlorplatindiaminodiyl;

$A^4$, $A^5$ sind gleich oder verschieden ein gesättigter, partiell ungesättigter oder aromatischer, mono- oder polycyclischer Kohlenwasserstoffrest mit 3 bis 14 C-Atomen oder ein drei- bis achtgliedriger, aromatischer, partiell ungesättigter oder gesättigter mono oder polycyclischer heterocyclischer Rest, wobei die genannten Gruppen jeweils gegebenenfalls durch eine oder mehrere Gruppen $X^2$ substituiert sind;

$Z^3$ ist eine Bindung, O, S, S(O), $S(O)_2$, $NR^x$, C(O) oder $CR^x_2$;

$X^2$ ist gleich oder verschieden Fluor, Chlor, Brom, Nitro, Hydroxylamino, $B(OH_2)$, $SO_3M$, $OSO_3M$, $SO_2NH_2$, $PO_3M$, $OPO_3M$, Alkyl, Halogenalkyl, Alkyloxy, Halogenalkyloxy, Alkylthio, Alkylamino, Dialkylamino, Trialkylamin, Alkylcarbonyl. Alkylsulfonyl, Alkylsulfoxyl, Alkylaminosulfoxyl, Dialkylaminosulfoxyl, Alkyloxycarbonyl, Alkylcarbonyloxy, Aminocarbonyl, Alkylaminocarbonyl, Alkylcarbonylamino, Dialkylaminocarbonyl, Alkylaminothiocarbonyl, $C_1$-$C_4$-Alkylimino (=N-$C_1$-$C_4$-Alkyl), $C_1$-$C_4$-Alkyloximino (=N-O-$C_1$-C4-Alkyl), wobei die Alkylgruppen in diesen Resten 1 bis 4 Kohlenstoffatome enthalten und die Gruppen Halogenalkyl und Halogenalkyloxy die Halogene F und/oder C1 enthalten;

n ist 0, 1.

$R^1$ ist gleich C(O)OM, O-$PO_3M_2$, O-$SO_3M$, $SO_3M$, C(O)-NH-$S(O)_2$-$R^x$, $PO_3M_2$ oder C(O)NOM;

$R^2$, $R^3$ sind gleich oder verschieden H oder F;

$R^4$, $R^7$ sind gleich oder verschieden H, OH, $OR^z$, OC(O)$NHR^y$ oder $NR^x$;

$R^6$ ist gleich oder verschieden H oder $R^z$;

$R^5$ ist gleich oder verschieden H, $R^x$, $R^x_2$, C(O)H, C(O)$CH_2$OH oder C(O)-Halogenalkyl;

$R^8$ ist gleich oder verschieden $R^x$;

M ist H, $C_1$-$C_6$-Alkyl oder ein Kation;

$R^x$ ist gleich oder verschieden H, $R^y$ oder $R^z$;

$R^y$ ist gleich oder verschieden $C_1$-$C_6$-Alkyl, Phenyl oder Benzyl und

$R^z$ ist gleich oder verschieden -C(O)-$C_1C_6$-Alkyl, -C(O)-Phenyl oder - C(O)-$C_1$-$C_4$-Alkyl-Phenyl,

wobei in den genannten Gruppen Alkyl für gesättigte, geradkettige, verzweigte oder cyclische Kohlenwasserstoffreste mit der jeweils angegebenen Anzahl von Kohlenstoffatomen, Alkenyl für ungesättigte, geradkettige oder verzweigte Kohlenwasserstoffreste mit der angegebenen Anzahl von Kohlenstoffatomen und ein oder zwei Doppelbindungen in beliebiger Position und Alkinyl für geradkettige oder verzweigte Kohlenwasserstoffgruppen mit der angegebenen Zahl von Kohlenstoffatomen und einer oder zwei Dreifachbindungen in beliebiger Position steht, sowie dessen pharmakologisch verträgliche Salze.

**2.** Sialinsäurederivat gemäß Anspruch 1, wobei die Symbole in der Formel (I) folgende Bedeutungen haben:

$A^1$, $A^2$ sind gleich oder verschieden eine Gruppe $D^1$-[$Y^3$-$D^2$-]$_m$-.
$D^1$ ist gleich oder verschieden

a) $C_6$-$C_{14}$-Aryl,
b) $C_3$-$C_{14}$-Cycloalkenyl oder $C_5$-$C_{14}$-Cycloalkadienyl,
c) $C_3$-$C_8$-Cycloalkyl,
d) nicht-aromatisches, gesättigtes oder teilweise ungesättigtes 5- oder 6-gliedriges Heterocyclyl, enthaltend ein bis drei Stickstoffatome und/oder ein Sauerstoff- oder Schwefelatom oder ein oder zwei Sauerstoff- und/oder Schwefelatom,
e) 5-gliedriges Heteroaryl, enthaltend ein bis vier Stickstoffatome oder ein bis drei Stickstoffatome und/oder ein Schwefel- oder Sauerstoffatom,
f) 6-gliedriges Heteroaryl, enthaltend ein bis drei bzw. ein bis vier Stickstoffatome,
g) polycyclische heterocyclische Reste

wobei die genannten Gruppen unsubstituiert oder durch eine oder mehrere Gruppen $X^1$ substituiert sind;
$D^2$ ist gleich oder verschieden

a) $C_6$-$C_{14}$-Aryldiyl,
b) $C_3$-$C_8$-Cycloalkyldiyl,
c) nicht-aromatisches, gesättigtes oder teilweise ungesättigtes 5- oder 6-gliedriges Heterocyclodiyl, enthaltend ein bis drei Stickstoffatome und/oder ein Sauerstoff- oder Schwefelatom oder ein oder zwei Sauerstoff- und/oder Schwefelatorne,
d) 5-gliedriges Heteroaryldiyl, enthaltend ein bis vier Stickstoffatome oder ein bis drei Stickstoffatome und ein Schwefel- oder Sauerstoffatom,
e) 6-gliedriges Heteroaryldiyl, enthaltend ein bis drei bzw. ein bis vier Stickstoffatome,

wobei die genannten Gruppen unsubstituiert oder durch eine oder mehrere Gruppen $X^1$ substituiert sind;

$X^1$ ist gleich oder verschieden F, Cl, Br, I, Cyano, Nitro, Hydroxy, Mercapto, Amino, Carboxyl, Hydroxylamino, Azido, $SO_3M$, $OSO_3M$, $SO_2NH_2$, $OPO_3M$, Alkyl, Halogenalkyl, Alkenyl, Alkinyl, Alkyloxy, Halogenalkyloxy, Alkenyloxy, Alkinyloxy, Alkylthio, Alkylamino, Dialkylamino, Trialkylamino, Formyl, Alkylcarbonyl, Alkylsulfonyl, Alkylsulfoxyl, Aminocarbonyl, Alkylaminocarbonyl, Alkylcarbonylamino, Oxo (=O), Thioxo (=S), wobei die Alkylgruppen in diesen Resten 1 bis 8 Kohlenstoffatome enthalten und die genannten Alkenyl- oder Alkinylgruppen in diesen Resten 2 bis 8 Kohlenstoffatome enthalten;
$Y^1$, $Y^2$ sind gleich oder verschieden $\sim CH_2$-, $\sim CH_2CH_2$-, $\sim CH_2CH_2CH_2$-, ~C(O)-, $\sim CH_2C(O)$-, $\sim CH_2CH_2C(O)$-, $\sim CH_2CH_2CH_2C(O)$-, ~CH=CH-C(O)-, ~C≡C-C(O)-, $\sim S(O)_2$-, $\sim CH_2S(O)_2$-, ~NH-C(O)-, $\sim NR^x$-3-Cyclobuten-1,2-dion-4-, $\sim CH_2$-$NR^x$-3-Cyclobuten-1,2-dion-4-, $\sim NR^x$-3-2,5-thiadiazol-1,1-dioxid-4-, wobei ~ die Bindung zur Gruppe A bezeichnet oder
$Y^1$, $Y^2$ bilden, unabhängig voneinander, zusammen mit dem Stickstoff, an den sie gebunden sind, und der Gruppe $R^8$ einen ~4-1H-(1,2,3)Triazol-1-yl-, ~5-1H-(1,2,3)Triazol-1-yl-, $\sim CH_2$-4-1H-(1,2,3)Triazol-1-yl- oder $\sim CH_2$-5-1H-(1,2,3)Triazol-1-yl- Ring, wobei ~ die Bindung zur Gruppe A bezeichnet;
$Y^3$ ist eine Bindung, O, S(O), S($O_2$), $CH_2$, $NR^x$ oder C(O);
m ist 0, 1 oder 2;
$Z^1$ ist O, S oder $CH_2$;
D ist eine Gruppe $Z^2$-$T^1$-$Y^4$-$A^3$-$Y^5$-$T^2$-$Z^2$;
$Z^2$ ist gleich oder verschieden -O~, -S~, $-NR^x\sim$, -NHC(O)~, $-CH_2\sim$, $-O-NR^x\sim$ oder ~4-1H-(1,2,3)Triazol-1-yl-, wobei ~ die Bindung zur Gruppe T bezeichnet;
$T^1$, $T^2$ sind gleich oder verschieden eine geradkettige oder verzweigte Alkandiylgruppe mit 4 bis 30 C-Atomen, wobei

(i) gegebenenfalls eine oder mehrere nicht terminale $CH_2$-Gruppen durch ~O-, -S-, -S(O)-, $-S(O)_2-$, $-P(O_2)-$ und/oder $-NR^x-$ ersetzt sind und/oder

(ii) gegebenenfalls ein oder mehrere H-Atome durch F, Cl, $OR^x$,$-OSO_3M$, (=O), Carboxyl, $NH_2$, $NHR^y$ oder $NHR^z$ ersetzt sind und/oder

(iii) gegebenenfalls eine nicht terminale $-CH_2-CH_2-$Gruppe durch -5-1H-(1,2,3)Triazol-1-yl- ersetzt ist und/oder

(iv) gegebenfalls eine nicht terminale $-CH_2CH_2CH_2-$Gruppe durch -4-1H-(1,2,3)Triazol-1-yl- oder -O-N=CH- ersetzt ist;

$Y^4$, $Y^5$ sind gleich oder verschieden O, $NR^x$, S(O), $S(O)_2$, C(O), $\sim C(O)-NR^x-$, $\sim NR^x-C(O)-$, $\sim NR^x-CO-NR^x-$, $\sim NR^x-S(O)_2-$, $\sim S(O)_2-NR^x-$, $\sim CH_2-NR^x-C(O)-$, $\sim CH2-C(O)-NR^x-$ oder $\sim CH_2-NR^x-$, wobei ~ die Bindung zu Gruppe A bezeichnet;

$A^3$ ist

a) $C_1$-$C_8$ Alkandiyl, $C_2$-$C_8$-Alkendiyl, $C_2$-$C_8$-Alkindiyl, $C_4$-$C_8$-Alkadiendiyl, wobei in den genannten Gruppen gegebenenfalls 1, 2, 3 oder 4 $CH_2$-Gruppen durch O, S(O), $S(O)_2$, $NR^x$ und/oder S ersetzt sind, und wobei in den genannten Gruppen gegebenenfalls ein oder mehrere H-Atome durch F oder Cl ersetzt sind,

b) eine Gruppe $A^4-[Z^3-A^5]_n$,

c) 1,1'-Ferrocendiyl, 1,1'-Cobaltocendiyl, 1,1'-Ruthenocen oder Dichlorplatindiamonodiyl;

$A^4$, $A^5$ sind gleich oder verschieden

a) $C_6$-$C_{14}$-Aryldiyl,

b) $C_3$-$C_8$-Cycloalkyldiyl,

c) nicht-aromatisches, gesättigtes oder teilweise ungesättigtes 5-oder 6-gliedriges Heterocyclodiyl, enthaltend ein bis drei Stickstoffatome und/oder ein Sauerstoff- oder Schwefelatom oder ein oder zwei Sauerstoff- und/oder Schwefelatome,

d) 5-gliedriges Heteroaryldiyl, enthaltend ein bis vier Stickstoffatome oder ein bis drei Stickstoffatome und/oder ein Schwefel- oder Sauerstoffatom,

e) 6-gliedriges Heteroaryldiyl, enthaltend ein bis drei bzw. ein bis vier Stickstoffatome,

f) polycyclisches Heterocyclyl aus der Gruppe wobei die genannten Reste unsubstituiert oder durch eine oder mehrere Gruppen $X^2$ substituiert sind;

$Z^3$ ist eine Bindung, O, S(O), $S(O)_2$ oder C(O);

$X^2$ ist gleich oder verschieden Fluor, Chlor, Nitro, Brom, Hydroxylamino, $SO_3M$, $OSO_3M$, $SO_2NH_2$, $PO_3M$, $OPO_3M$, Alkyl, Halogenalkyl, Alkyloxy, Halogenalkyloxy, Alkylamino, Dialkylamino, Trialkylamino, Alkylcarbonyl, Alkylsulfonyl, Alkylsulfoxyl, Alkylaminosulfoxyl, Dialkylaminosulfoxyl, Alkyloxycarbonyl, Alkylcarbonyloxy, Aminocarbortyl, Alkylaminocarbonyl, Alkylcarbonylamino, Dialkylaminocarbonyl, $C_1$-$C_4$-Alkylimino (=N-$C_1$-$C_4$-Alkyl), $C_1$-$C_4$-Alkyloximino (=N-O-$C_1$-$C_4$-Alkyl), wobei die Alkylgruppen in diesen Resten 1 bis 4 Kohlenstoffatome enthalten und die Gruppen Halogenalkyl und Halogenalkyloxy die Halogene F und/oder Cl enthalten;

n ist 0 oder 1;

$R^1$ ist gleich und ist C(O)OM, $SO_3M$, $C(O)-NH-S(O)_2-R^x$, $PO_3M_2$ oder C(O)NOM;

$R^2$, $R^3$ sind gleich oder verschieden H oder F;

$R^4$, $R^7$ sind gleich oder verschieden H, OH, $OR^2$, $OC(O)NHR^y$ oder $NR^x$;

$R^6$ ist gleich oder verschieden H oder $R^z$;

$R^5$ ist gleich oder verschieden H, $R^x$, $C(O)CH_2OH$, C(O)-Halogenalkyl oder C(O)H;

$R^8$ ist gleich oder verschieden $R^x$;

M ist H, $C_1$-$C_4$-Alkyl oder ein Kation aus der Gruppe der Alkalimetalle, Erdalkalimetalle, Mangan, Kupfer, Zink, Eisen und gegebenenfalls substituiertes Ammonium;

$R^x$ ist gleich oder verschieden H, $R^y$ oder $R^z$;

$R^y$ ist gleich oder verschieden $C_1$-$C_4$-Alkyl, Phenyl oder Benzyl und

$R^z$ ist gleich oder verschieden -C(O)-$C_1$-$C_6$-Alkyl, C(O)-Phenyl oder $C(O)CH_2$Phenyl,

sowie dessen pharmakologisch verträgliche Salze.

**3.** Sialinsäurederivat gemäß Anspruch 1 oder 2, wobei Symbole und Indizes in der Formel (I) folgende Bedeutungen haben:

$A^1$, $A^2$ sind gleich oder verschieden eine Gruppe $D^1$-[$Y^3$-$D^2$-]$_m$-.

$D^1$ ist gleich oder verschieden Phenyl, 1-Naphthyl, 2-Naphtyl, 2-Biphenylen, 2-Fluorenyl, 3-Fluorenyl, 2-Phenanthrenyl, Cyclopropyl, Cyclobutyl, Cyclopentyl, Cyclohexyl, 2-Piperidinyl, 3-Piperidinyl, 4-Piperidinyl, 1,3-Dioxan-5-yl, 4-Tetrahydropyranyl, 3-Hexahydropyridazinyl, 4-Hexahydropyridazinyl, 2-Hexahydropyrimidinyl, 4-Hexahydropyrimidinyl, 5-Hexahydropyrimidinyl, 1-Piperazinyl, 2-Piperazinyl, 4-Morpholinyl, 2-Furyl, 3-Furyl, 2-Thienyl, 3-Thienyl, 2-Pyrrolyl, 3-Pyrrolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 2-Imidazolyl, 4-imidazolyl, 1,2,3-Triazol-1-yl, 1,2,3-Triazol-4-yl, 1,2,3-Triazol-5-yl, 1,3,4-Triazol-2-yl, 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1-Benzofuran-2-yl, 1-Benzofuran-5-yl, 1-Benzofuran-6-yl, 2-Benzofuran-5-yl, 2-Benzofuran-6-yl, 2H-Chromen-3-yl, 2H-Chromen-6-yl, 2H-Chromen-7-yl, Indol-2-yl, 1-Benzothiophen-2-yl oder Benzimidazol-2-yl,

wobei die genannten Gruppen unsubstituiert oder durch eine oder mehrere Gruppen $X^1$ substituiert sind.

$D^2$ ist gleich oder verschieden Phenylen-1,4-diyl, Phenylen-1,3-diyl Biphenyl-4,4'-diyl, Naphthalin-1,4-diyl, trans-Cyclohexan-1,4-diyl, Furan-2,5-diyl, Thiophen-2,4-diyl, Thiophen-2,5-diyl, 1H(1,2,3)-Triazol-1,4-diyl oder Pyrrol-2,5-diyl, Pyridin-2,5-diyl, Pyridin-2,4-diyl, Pyridazin-3,6-diyl, Pyrirnidin-2,5-diyl, Pyrimidin-2,6-diyl, Pyrazin-2,5-diyl oder Tetrazin-3,5-diyl,

wobei die genannten Gruppen unsubstituiert oder durch eine oder eine Gruppe $X^1$ substituiert sind.

$X^1$ ist gleich oder verschieden F, Cl, Br, I, Cyano, Nitro, Hydroxy, Amino, Carboxyl, Azido, $SO_3M$, $SO_2NH_2$, Alkyl, Halogenalkyl, Alkyloxy, Halogenalkyloxy, Alkylamino, Dialkylamino, Trialkylamino, Alkylcarbonyl, Alkylsulfoxyl, Aminocarbonyl, Alkylaminocarbonyl, Alkylcarbonylamino, Oxo (=O), wobei die Alkylgruppen in diesen Resten 1 bis 6 Kohlenstoffatome.

$Y^1$, $Y^2$ sind gleich oder verschieden ~$CH_2$-, ~C(O)-, ~$CH_2$-C(O)-, ~$CH_2CH_2C$(O)-, ~CH=CH-C(O)-, ~C≡C-C(O)-, ~NH-C(O)-, ~$CH_2$-$NR^x$-3-Cyclobuten-1,2-dion-4-, ~$S(O)_2$- oder ~$CH_2S(O)_2$- wobei ~ die Bindung zur Gruppe A bezeichnet oder

$Y^1$, $Y^2$ bilden, unabhängig voneinander, zusammen mit dem Stickstoff, an den sie gebunden sind, und der Gruppe $R^8$ eine Gruppe ~4-1H-(1,2,3)Triazol-1-yl- wobei ~ die Bindung zur Gruppe A bezeichnet;

$Y^3$ ist eine Bindung, O, $S(O_2)$ $CH_2$ oder C(O).

m ist 0, 1 oder 2.

$Z^1$ ist O.

D ist eine Gruppe $Z^2$-$T^1$-$Y^4$-$A^3$-$Y^5$-$T^2$-$Z^2$.

$Z^2$ ist gleich O, S, ~4-1H-(1,2,3)Triazol-1-yl-, -NH-C(O)- oder $CH_2$, wobei ~ die Bindung zur Gruppe T bezeichnet.

$T^1$, $T^2$ sind gleich oder verschieden eine geradkettige oder verzweigte Alkandiylgruppe mit 4 bis 20 C-Atomen, wobei

(i) gegebenenfalls eine oder mehrere nicht terminale $CH_2$-Gruppen durch -S-, -S(O)-, -$S(O)_2$-, und/oder -$NR^z$- ersetzt sind und /oder

(ii) gegebenenfalls ein oder mehrere H-Atome durch Fluor, Chlor, (=O), Carboxyl, $NH_2$, $NHR^y$ oder $NHR^z$ ersetzt sind und/oder

(iv) gegebenfalls eine nicht terminale -$CH_2CH_2CH_2$-Gruppe durch -4-1H-(1,2,3)Triazol-1-yl- ersetzt ist.

$Y^4$, $Y^5$ sind gleich oder verschieden ~$NR^x$-C(O)-, ~C(O)-NRx-, ~$NR^x$-$S(O)_2$, ~$NR^x$-, ~$CH_2NR^x$-, ~$NR^x$C(O)$NR^x$-, ~$CH_2NR^x$C(O)-, ~$CH_2$C(O)$NR^x$-oder ~$S(O)_2$-$NR^x$-, wobei ~ die Bindung zu Gruppe A bezeichnet.

$A^3$ ist

a) $C_1$-$C_6$ Alkandiyl, $C_2$-$C_4$-Alkindiyl wobei in den genannten Gruppen gegebenenfalls ein oder mehrere H-Atome durch F oder Cl ersetzt sind, oder

b) eine Gruppe $A^4$

$A^4$ ist gleich Phenylen-1,4-diyl, Biphenyl-4,4'-diyl, Naphthalin-1,4-diyl, trans-Cyclohexan-1,4-diyl, Thiophen-2,5-diyl, 1H-(1,2,3)Triazol-1,4-diyl, Pyridin-2,5-diyl, Pyridin-2,4-diyl, Pyridazin-2,5-diyl, Pyrimidin-2,5-diyl, Pyrimidin-2,4-diyl, Pyrazin-2,5-diyl oder Tetrazin-2,5-diyl, wobei die genannten Reste unsubstituiert oder durch eine Gruppe $X^2$ substituiert sind;

$X^2$ ist gleich oder verschieden Fluor, Chlor, Brom, Nitro, $SO_3M$, Alkyl, Halogenalkyl, Alkyloxy, Halogenalkyloxy, Alkylamino, Dialkylamino, Trialkylamino, Alkylcarbonyl, Alkylsulfoxyl, Alkylcarbonyloxy, Aminocarbonyl, Alkylaminocarbonyl, Alkylcarbonylamino, wobei die Alkylgruppen in diesen Resten 1 bis 4 Kohlenstoffatome enthalten und die Gruppen Halogenalkyl und Halogenalkyloxy die Halogene F und/oder Cl enthalten;
$R^1$ ist gleich und ist C(O)OM oder C(O)NOM.
$R^2$, $R^3$ sind gleich H.
$R^4$, $R^7$ sind gleich OH oder $OR^z$;
$R^6$ ist gleich H oder $R^z$.
$R^5$ ist gleich $R^x$, $C(O)CH_2OH$ oder C(O)-Halogenalkyl.
$R^8$ ist gleich oder verschieden $R^x$.
M ist H, Methyl, Ethyl oder ein Kation aus der Gruppe Li, Na, K, Ca, Mg und gegebenenfalls substituiertes Ammonium.
$R^x$ ist gleich oder verschieden H, $R^y$ oder $R^z$.
$R^y$ ist gleich oder verschieden Methyl, Ethyl, 1-Methylethyl, Propyl, 1-Methylpropyl, 1,1-Dimethylpropyl oder Benzyl.
$R^z$ ist gleich oder verschieden Methylcarbonyl, Ethylcarbonyl, Propylcarbonyl, 1-Methylethylcarbonyl, 1,1-Dimethylethylcarbonyl oder Phenylcarbonyl,

sowie dessen pharmakologisch verträgliche Salze.

**4.** Sialinsäurederivat gemäß einem der Ansprüche 1 bis 3, wobei die Symbole und Indizes in der Formel (I) folgende Bedeutungen haben:

$A^1$, $A^2$ sind gleich oder verschieden eine Gruppe $D^1$-[$Y^3$-$D^2$-]$_m$-,
$D^1$ ist gleich oder verschieden Phenyl, 1-Naphthyl, 2-Naphtyl, 2-Biphenylen, 2-Fluorenyl, 3-Fluorenyl, 2-Phenanthrenyl, Cyclopropyl, Cyclobutyl, Cyclopentyl, Cyclohexyl, 2-Piperidinyl, 3-Piperidinyl, 4-Piperidinyl, 1,3-Dioxan-5-yl, 4-Tetrahydropyranyl, 3-Hexahydropyridazinyl, 4-Hexahydropyridazinyl, 2-Hexahydropyrimidinyl, 4-Hexahydropyrimidinyl, 5-Hexahydropyrimidinyl, 1-Piperazinyl, 2-Piperazinyl, 4-Morpholinyl, 2-Furyl, 3-Furyl, 2-Thienyl, 3-Thienyl, 2-Pyrrolyl, 3-Pyrrolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, 1,2,3-Triazol-1-yl, 1,2,3-Triazol-4-yl, 1,2,3-Triazol-5-yl, 1,3,4-Triazol-2-yl, 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1-Benzofuran-2-yl, 1-Benzofuran-5-yl, 1-Benzofuran-6-yl, 2-Benzofuran-5-yl, 2-Benzofuran-6-yl, 2H-Chromen-3-yl, 2H-Chromen-6-yl, 2H-Chromen-7-yl, Indol-2-yl, 1-Benzothiophen-2-yl oder Benzimidazol-2-yl,

wobei die genannten Gruppen unsubstituiert oder durch eine oder mehrere Gruppen $X^1$ substituiert sind.

$D^2$ ist gleich oder verschieden Phenylen-1,4-diyl, Phenylen-1,3-diyl Biphenyl-4,4'-diyl, Naphthalin-1,4-diyl, trans-Cyclohexan-1,4-diyl, Furan-2,5-diyl, Thiophen-2,4-diyl, Thiophen-2,5-diyl, 1H(1,2,3)-Triazol-1,4-diyl oder Pyrrol-2,5-diyl, Pyridin-2,5-diyl, Pyridin-2,4-diyl, Pyridazin-3,6-diyl, Pyrimidin-2,5-diyl, Pyrimidin-2,6-diyl, Pyrazin-2,5-diyl oder Tetrazin-3,5-diyl, wobei die genannten Gruppen unsubstituiert oder durch eine oder eine Gruppe $X^1$ substituiert sind.
$X^1$ ist gleich oder verschieden F, Cl, Br, I, Cyano, Nitro, Hydroxy, Amino, Carboxyl, Azido, $SO_3M$, $SO_2NH_2$, Alkyl, Halogenalkyl, Alkyloxy, Halogenalkyloxy, Alkylamino, Dialkylamino, Trialkylamino, Alkylcarbonyl, Alkylsulfoxyl, Aminocarbonyl, Alkylaminocarbonyl, Alkylcarbonylamino, Oxo (=O), wobei die Alkylgruppen in diesen Resten 1 bis 6 Kohlenstoffatome.
$Y^1$, $Y^2$ sind gleich oder verschieden ~CH2-, ~C(O)-, ~$CH_2$-C(O)-, ~$CH_2CH_2$C(O)-, ~CH=CH-C(O)-, ~C≡C-C(O)-, ~NH-C(O)-, ~CH2-$NR^x$-3-Cyclobuten-1,2-dion-4-, ~$S(O)_2$- oder ~$CH_2S(O)_2$- wobei ~ die Bindung zur Gruppe A bezeichnet oder
$Y^1$, $Y^2$ bilden, unabhängig voneinander, zusammen mit dem Stickstoff, an den sie gebunden sind, und der Gruppe $R^8$ eine Gruppe ~4-1H-(1,2,3)Triazol-1-yl- wobei ~ die Bindung zur Gruppe A bezeichnet;
$Y^3$ ist eine Bindung, O, $S(O_2)$, $CH_2$ oder C(O).
m ist 0, 1 oder 2.
$Z^1$ ist O.
D ist eine Gruppe $Z^2$-$T^1$-$Y^4$-$A^3$-$Y^5$-$T^2$-$Z^2$.
$Z^2$ ist gleich O, S, ~4-1H-(1,2,3)Triazal-1-yl-, -NH-C(O)~ oder $CH_2$, wobei ~ die Bindung zur Gruppe T bezeichnet
$T^1$, $T^2$ sind gleich oder verschieden eine geradkettige oder verzweigte Alkandiylgruppe mit 4 bis 20 C-Atomen, wobei

(i) gegebenenfalls eine oder mehrere nicht terminale $CH_2$-Gruppen durch -NH-, -S-, -S(O)- und/oder -$S(O)_2$-

ersetzt sind und /oder

(ii) gegebenenfalls ein oder mehrere H-Atome durch Fluor, Chlor, (=O), Carboxyl, $NH_2$, $NHR^y$ oder $NHR^z$ ersetzt sind und/oder

(iv) gegebenfalls eine nicht terminale $-CH_2CH_2CH_2$-Gruppe durch -4-1H-(1,2,3)Triazol-1-yl- ersetzt ist.

$Y^4$, $Y^5$ sind gleich oder verschieden $\sim NR^x$-C(O)-, $\sim$C(O)-$NR^x$-, $\sim NR^x$-$S(O)_2$, $\sim NR^x$-, $\sim CH_2NR^x$, $\sim NR_xC(O)NR^x$-, $\sim CH_2NR^xC(O)$-, $\sim CH_2C(O)NR^x$-oder $\sim S(O)_2$-$NR^x$-, wobei ~ die Bindung zu Gruppe A bezeichnet.
$A^3$ ist

a) $C_1C_6$ Alkandiyl, $C_2$-$C_4$-Alkindiyl wobei in den genannten Gruppen gegebenenfalls ein oder mehrere H-Atome durch F oder Cl ersetzt sind, oder

b) eine Gruppe $A^4$

$A^4$ ist gleich Phenylen-1,4-diyl, Biphenyl-4,4'-diyl, Naphthalin-1,4-diyl, trans-Cyclohexan-1,4-diyl, Thiophen-2,5-diyl oder 1H-(1,2,3)Triazol-1,4-diyl, Pyridin-2,5-diyl, Pyridin-2,4-diyl, Pyridazin-2,5-diyl, Pyrimidin-2,5-diyl, Pyrimidin-2,4-diyl, Pyrazin-2,5-diyl oder Tetrazin-2,5-diyl, wobei die genannten Reste unsubstituiert oder durch eine Gruppe $X^2$ substituiert sind.

$X^2$ ist gleich oder verschieden Fluor, Chlor, Brom, Nitro, $SO_3M$, Alkyl, Halogenalkyl, Alkyloxy, Halogenalkyloxy, Alkylamino, Dialkylamino, Trialkylamino, Alkylcarbonyl, Alkylsulfoxyl, Alkylcarbonyloxy, Aminocarbonyl, Alkylaminocarbonyl, Alkylcarbonylamino, wobei die Alkylgruppen in diesen Resten 1 bis 4 Kohlenstoffatome enthalten und die Gruppen Halogenalkyl und Halogenalkyloxy die Halogene F und/oder Cl enthalten;

$R^1$ ist gleich und ist C(O)OM oder C(O)NOM.

$R^2$, $R^3$ sind gleich H.

$R^4$, $R^7$ sind gleich OH oder $OR^z$;

$R^6$ ist gleich H oder $R^z$.

$R^5$ ist gleich $R^x$, $C(O)CH_2OH$ oder C(O)-Halogenalkyl.

$R^8$ ist gleich oder verschieden $R^x$.

M ist H, Methyl, Ethyl oder ein Kation aus der Gruppe Li, Na, K, Ca, Mg und gegebenenfalls substituiertes Ammonium.

$R^x$ ist gleich oder verschieden H, $R^y$ oder $R^z$.

$R^y$ ist gleich oder verschieden Methyl, Ethyl, 1-Methylethyl, Propyl, 1-Methylpropyl, 1,1-Dimethylpropyl oder-Benzyl.

$R^z$ ist gleich oder verschieden Methylcarbonyl, Ethylcarbonyl, Propylcarbonyl, 1-Methylethylcarbonyl, 1,1-Dimethylethylcarbonyl oder Phenylcarbonyl,

sowie dessen pharmakologisch verträgliche Salze.

**5.** Sialinsäurederivate gemäß einem der Ansprüche 1 bis 4, wobei die Symbole und Indizes in der Formel (I) folgende Bedeutungen haben:

$A^1$, $A^2$ sind gleich eine Gruppe $D^1$-[$Y^3$-$D^2$-]$_m$-.

$D^1$ ist gleich Phenyl, 2-Naphtyl, Cyclohexyl, 2-Fluorenyl, 2-Furanyl, 2-Benzothiophenyl, 2-Thienyl, 3-Thienyl, 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl oder 2-Pyrazinyl, wobei die genannten Gruppen unsubstituiert oder durch eine Gruppe $X^1$ substituiert sind.

$D^2$ ist gleich Phenylen-1,4-diyl, Naphthalin-1,4-diyl, Thiophen-2,5-diyl, Pyridin-2,5-diyl, Pyridazin-3,6-diyl, Pyrimidin-2,5-diyl oder Pyrazin-2,5-diyl, wobei die genannten Gruppen unsubstituiert oder durch eine Gruppe $X^1$ substituiert sind.

$X^1$ ist F, Cl, Br, Nitro, Hydroxy, Carboxyl, Alkyl, Halogenalkyl, Alkyloxy, Halogenalkyloxy, Alkylamino, Dialkylamino, Trialkylamino, Alkylcarbonyl, Alkylsulfoxyl, Aminocarbonyl, Alkylaminocarbonyl, Alkylcarbonylamino, wobei die Alkylgruppen in diesen Resten 1 bis 4 Kohlenstoffatome enthalten.

$Y^1$, $Y^2$ sind gleich $\sim CH_2$-, $\sim$C(O)-, $\sim CH_2$-C(O)-, $\sim$C≡C-C(O)-, $\sim$CH=CH-C(O)-, $\sim CH_2$-$NR^x$-3-Cyclobuten-1,2-dion-4- oder $\sim S(O)_2$-, wobei ~ die Bindung zur Gruppe A bezeichnet oder

$Y^1$, $Y^2$ bilden, unabhängig voneinander, zusammen mit dem Stickstoff, an den sie gebunden sind, und der Gruppe $R^8$ eine Gruppe ~4-1H-(1,2,3)Triazol-1-yl-, wobei ~ die Bindung zur Gruppe A bezeichnet.

$Y^3$ ist eine Bindung oder O.

m ist 0 oder 1.

$Z^1$ ist O.

D ist eine Gruppe $Z^2$-$T^1$-$Y^4$-$A^3$-$Y^5$-$T^2$-$Z^2$.

$Z^2$ ist gleich O, ~4-1H-(1,2,3)Triazol-1-yl-, -NH-C(O)~ oder $CH_2$, wobei ~ die Bindung zur Gruppe T bezeichnet.
$T^1$, $T^2$ sind gleich eine geradkettige Alkandiylgruppe mit 4 bis 10 C-Atomen, wobei

(i) gegebenenfalls eine oder mehrere nicht terminale $CH_2$-Gruppen durch -S- und/oder -NH- ersetzt sind und/oder
(ii) gegebenenfalls ein oder mehrere H-Atome durch F, Cl oder (=O), Carboxyl oder $NH_2$ ersetzt sind.

$Y^4$, $Y^5$ sind gleich ~$NR^x$-C(O)-, ~$NR^x$-, ~$CH_2NR^x$-, ~$NR^xC(O)NR^x$-, ~$CH_2NR^xC(O)$-, ~$CH_2C(O)NR^x$- oder ~C(O)-$NR^x$-, wobei ~ die Bindung zu Gruppe A bezeichnet.
$A^3$ ist

a) $C_1$-$C_6$ Alkandiyl wobei in den genannten Gruppen gegebenenfalls ein oder mehrere H-Atome durch F oder Cl ersetzt sind, oder
b) eine Gruppe $A^4$.

$A^4$ ist gleich Phenylen-1,4-diyl, Biphenyl-4,4'-diyl, Naphthalin-1,4-diyl, Pyridin-2,5-diyl, Pyridazin-2,5-diyl, Pyrimidin-2,5-diyl, Pyrazin-2,5-diyl, Tetrazin-2,5-diyl, Ethin-1,2-diyl, Thiophen-2,5-diyl, Cyclohexan-1,4-diyl oder -3-Cyclobuten-1,2-dion-4-; wobei die genannten Reste unsubstituiert oder durch eine Gruppe $X^2$ substituiert sind.
$X^2$ ist gleich Fluor, Chlor, Brom, Nitro, $SO_3M$, Alkyl, Halogenalkyl, Alkyloxy, Halogenalkyloxy, Alkylamino, Dialkylamino, Trialkylamino, Alkylsulfoxyl, Alkylcarbonyloxy, wobei die Alkylgruppen in diesen Resten 1 bis 4 Kohlenstoffatome enthalten und die Gruppen Halogenalkyl und Halogenalkyloxy die Halogene F und/oder Cl enthalten;
$R^3$ ist gleich und ist C(O)OM.
$R^2$, $R^3$ sind gleich H.
$R^4$, $R^7$ sind gleich OH oder $OR^z$;
$R^6$ ist gleich H oder $R^z$.
$R^5$ ist gleich $R^x$, $C(O)CH_2OH$ oder C(O)-Halogenalkyl
$R^8$ ist gleich $R^x$.
M ist H, Methyl, Ethyl oder ein Kation aus der Gruppe Li, Na und K.
$R^x$ ist gleich oder verschieden H, $R^y$ oder $R^z$.
$R^y$ ist gleich oder verschieden Methyl oder Ethyl.
$R^z$ ist gleich Ethylcarbonyl, Ethylcarbonyl oder 1-Methylethycarbonyl,

sowie deren pharmakologisch verträgliche Salze.

**6.** Sialinsäurederivat der Formel (I) gemäß einem der Ansprüche 1 bis 5, wobei die Symbole und Indizes in der Formel (I) folgende Bedeutungen haben:

$A^1$, $A^2$ sind gleich eine Gruppe $D^1$-$[Y^3$-$D^2$-$]_m$-.
$D^1$ ist gleich Phenyl, Cyclohexyl, Napht-2-yl, Fluoren-2-yl, Furan-2-yl, Benzothiophen-2-yl, Thien-2-yl, Thien-3-yl, Pyridin-2-yl, Pyridin-3-yl, Pyridin-4-yl, Pyridazin-3-yl, Pyridazin-4-yl, Pyrimidin-2-yl, Pyrimidin-4-yl, Pyrimidin-5-yl oder Pyrazin-2-yl, wobei die genannten Gruppen unsubstituiert oder durch eine Gruppe $X^1$ substituiert sind.
$D^2$ ist gleich Phenylen-1,4-diyl, Naphthalin-1,4-diyl, Thiophen-2,5-diyl, Pyridin-2,5-diyl, Pyridazin-3,6-diyl, Pyrimidin-2,5-diyl oder Pyrazin-2,5-diyl, wobei die genannten Gruppen unsubstituiert oder durch eine Gruppe $X^1$ substituiert sind.
$X^1$ ist gleich F, Cl, Nitro, Hydroxy, Carboxyl, Alkyl, Halogenalkyl, Alkyloxy, Halogenalkyloxy, Alkylamino, Dialkylamino, Trialkylamino, Alkylsulfoxyl, Aminocarbonyl, Alkylaminocarbonyl, Alkylcarbonylamino, wobei die Alkylgruppen in diesen Resten 1 bis 3 Kohlenstoffatome enthalten.
$Y^1$, $Y^2$ sind gleich ~$CH_2$-, ~C(O)-, ~$CH_2$-C(O)-, ~C≡C-C(O)-, ~CH=CH-C(O)-, ~$CH_2$-$NR^x$-3-Cyclobuten-1,2-dion-4- oder ~$S(O)_2$-, wobei ~ die Bindung zur Gruppe A bezeichnet oder
$Y^1$ $Y^2$ bilden, unabhängig voneinander, zusammen mit dem Stickstoff, an den sie gebunden sind, und der Gruppe $R^8$ eine Gruppe ~4-1H-(1,2,3)Triazol-1-yl-, wobei - die Bindung zur Gruppe A bezeichnet.
$Y^3$ ist eine Bindung.
m ist 0 oder 1.
$Z^1$ ist O.
D ist eine Gruppe $Z^2$-$T^1$-$Y^4$-$A^3$-$Y^5$-$T^2$-$Z^2$.
$Z^2$ ist gleich O, $CH_2$ oder ~4-1H-(1,2,3)Triazol-1-yl-, wobei ~ die Bindung zur Gruppe T bezeichnet.
$T^1$, $T^2$ sind gleich oder verschieden eine geradkettige Alkandiylgruppe mit 4 bis 6 C-Atomen, wobei

(i) gegebenenfalls eine oder mehrere nicht terminale $CH_2$-Gruppen durch -S-, und/oder NH ersetzt sind und/oder
(ii) gegebenenfalls ein oder mehrere H-Atome durch F, Cl, (=0), Carboxyl oder $NH_2$ ersetzt sind.

$Y^4$, $Y^5$ sind gleich oder verschieden ~C(O)-$NR^x$-, ~$NR^x$-, ~$CH_2NR^x$-, ~NHC(O)NH-, ~$CH_2$NHC(O)-, ~$CH_2$C(O)NH- oder ~$NR^x$-C(O)-, wobei ~ die Bindung zu Gruppe A bezeichnet.
$A^3$ ist

a) $C_1$-$C_6$ Alkandiyl, wobei in den genannten Gruppen gegebenenfalls ein oder mehrere H-Atome durch F oder Cl ersetzt sind, oder
b) eine Gruppe $A^4$.

$A^4$ ist gleich Phenylen-1,4-diyl, Biphenyl-4,4'-diyl, Naphthalin-1,4-diyl, Pyridin-2,5-diyl, Pyridazin-2,5-diyl, Pyrimidin-2,5-diyl, Pyrazin-2,5-diyl, Tetrazin-2,5-diyl, Ethin-1,2-diyl, Thiophen-2,5-diyl, Cyclohexan-1,4-diyl oder -3-Cyclobuten-1,2-dion-4-; wobei die genannten Reste unsubstituiert oder durch eine Gruppe $X^2$ substituiert sind.
$X^2$ ist gleich Fluor, Chlor, Brom, Nitro, $SO_3M$, Alkyl, Halogenalkyl, Alkyloxy, Halogenalkyloxy, Alkylamino, Dialkylamino, Trialkylamino, Alkylsulfoxyl, Alkylcarbonyloxy, wobei die Alkylgruppen in diesen Resten 1 bis 3 Kohlenstoffatome enthalten und die Gruppen Halogenalkyl und Halogenalkyloxy die Halogene F und/oder Cl enthalten;
$R^1$ ist C(O)OM.
$R^2$, $R^3$ sind gleich H.
$R^4$, $R^7$ sind gleich OH oder $OR^z$;
$R^6$ ist gleich H oder $R^z$.
$R^5$ ist gleich $R^x$, C(O)$CH_2$OH oder C(O)-Halogenalkyl.
$R^8$ ist gleich H.
M ist H, Methyl oder ein Kation aus der Gruppe Li, Na und K.
$R^x$ ist gleich oder verschieden H, $R^y$ oder $R^z$.
$R^y$ ist gleich oder verschieden Methyl oder Ethyl.
$R^z$ ist gleich Methylcarbonyl, Ethylcarbonyl oder 1-Methylethylcarbonyl,

sowie dessen pharmakologisch verträgliche Salze.

**7.** Sialinsäurederivat gemäß einem der Ansprüche 1 bis 6, wobei die Symbole und Indizes in der Formel (I) folgende Bedeutungen haben:

$A^1$, $A^2$ sind gleich eine Gruppe $D^1$-[$Y^3$-$D^2$-I]$_m$.
$D^1$ ist gleich Phenyl, Pyridin-2-yl, Fluoren-2-yl, Cyclohexyl, Napht-2-yl, Benzothiophen-2-yl, Furan-2-yl, Thien-2-yl oder Thien-3-yl, wobei die genannten Gruppen unsubstituiert oder durch eine Gruppe $X^1$ substituiert sind.
$D^2$ ist gleich Phenylen-1,4-diyl oder Thiophen-2,5-diyl, wobei die genannten Gruppen unsubstituiert oder durch eine Gruppe $X^1$ substituiert sind.
$X^1$ ist gleich F, Chlor, Nitro, Hydroxy, Carboxyl, Methyl, Trifluormethyl, Methyloxy.
$Y^1$, $Y^2$ sind gleich ~C(O)-, ~$CH_2$-, ~C≡C-C(O)-, ~CH=CH-C(O)-, ~$S(O)_2$-, ~CH2-NH-3-Cyclobuten-1,2-dion-4- oder ~$CH_2$-C(O)-, wobei ~ die Bindung zur Gruppe A bezeichnet oder
$Y^1$, $Y^2$ bilden, unabhängig voneinander, zusammen mit dem Stickstoff, an den sie gebunden sind, und der Gruppe $R^8$ eine Gruppe ~4-1H-(1,2,3)Triazol-1-yl-, wobei ~ die Bindung zur Gruppe A bezeichnet.
$Y^3$ ist eine Bindung.
m ist 0 oder 1.
$Z^1$ ist O.
D ist eine Gruppe $Z^2$-$T^1$-$Y^4$-$A^3$-$Y^5$-$T^2$-$Z^2$.
$Z^2$ ist gleich O, $CH_2$, ~CONH- oder ~4-1H-(1,2,3)Triazol-1-yl-, wobei ~ die Bindung zur Gruppe T bezeichnet.
$T^1$, $T^2$ sind gleich oder verschieden ~$CH_2CH_2CH_2SCH_2CH_2$-, ~$CH_2CH_2CH_2S(O)CH_2CH_2$-, ~$CH_2$C(O)NH$CH_2CH_2CH_2$-, ~$CH_2$-4-1H-(1,2,3)Triazol-1-CHz$CH_2$-, Butan-1,4-diyl, Pentan-1,5-diyl oder Hexan-1,6-diyl, wobei ~ die Bindung zur Gruppe $Z^2$ bezeichnet.
$Y^4$, $Y^5$ sind gleich ~C(O)-NH-, ~NH-, ~$CH_2$NH-, ~NHC(O)NH-, ~$CH_2$NHC(O)-, ~$CH_2$C(O)NH- oder ~NH-C(O)-, wobei ~ die Bindung zu Gruppe A bezeichnet.
$A^3$ ist Methandiyl, Ethan-1,2-diyl, Butan-1,4-diyl, Hexan-1,6-diyl, -3-Cyclobuten-1,2-dion-4-, Thiophen-2,5-diyl, Cyclohexan-1,4-diyl, Phenylen-1,4-diyl, Naphtalin-1,4-diyl, Ethin-1,2-diyl, Biphenyl-4,4'-diyl oder Pyridin-2,5-diyl, wobei die genannten Reste unsubstituiert oder durch eine oder mehrere Gruppen $X^2$ substituiert sind.

$X^2$ ist gleich Brom, Nitro, Methyl oder Pentyloxy.
$R^1$ ist C(O)ONa.
$R^2$, $R^3$ sind gleich H.
$R^4$, $R^7$ sind gleich OH.
$R^6$ ist gleich H.
$R^5$ ist gleich $C(O)CH_3$, $C(O)CH_2F$ oder $C(O)CH_2OH$.
$R^8$ ist gleich H,

sowie dessen pharmakologisch verträgliche Salze.

8. Sialinsäurederivate gemäß einem der Ansprüche 1 bis 7 mit folgenden Strukturformeln

| |
|---|
| 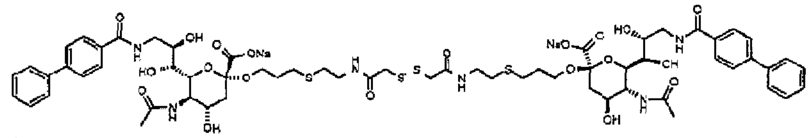 |
| 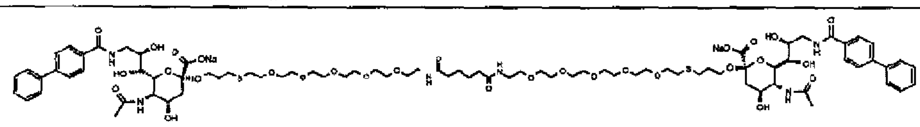 |
| 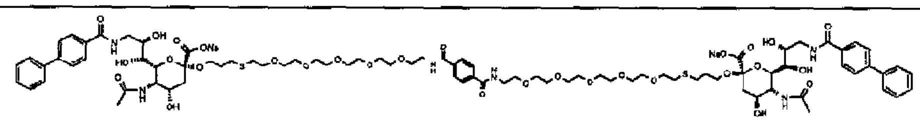 |
| 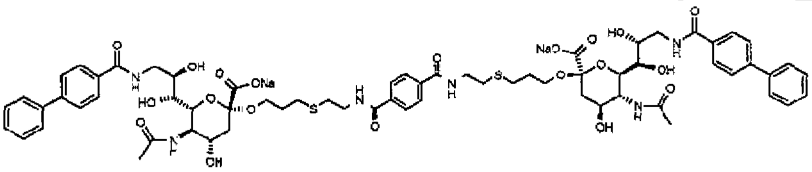 |
| 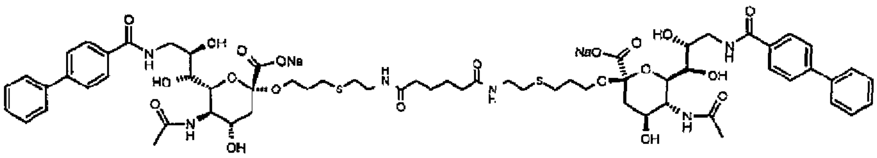 |
| 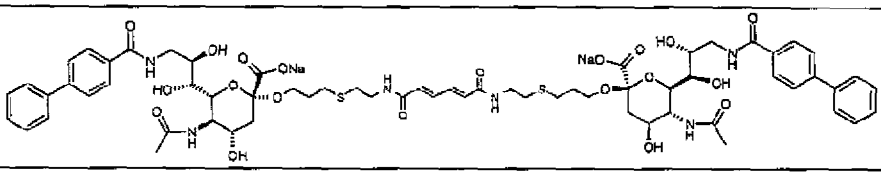 |
| 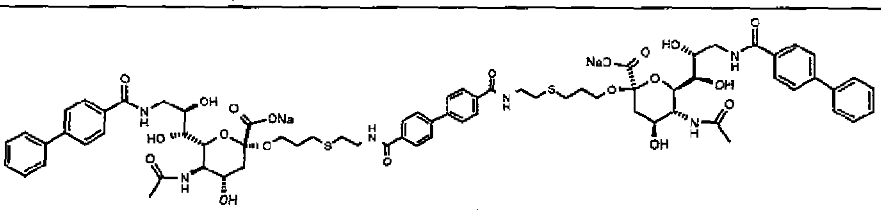 |
| 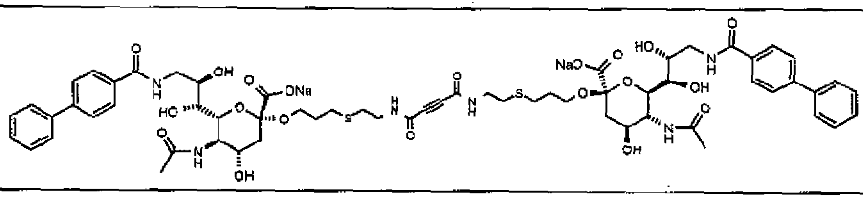 |

| |
|---|
| 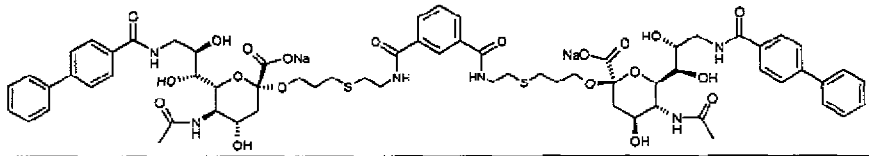 |
| 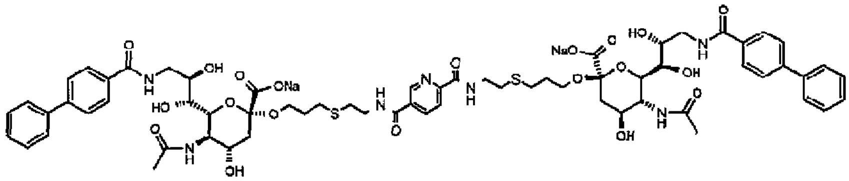 |
| 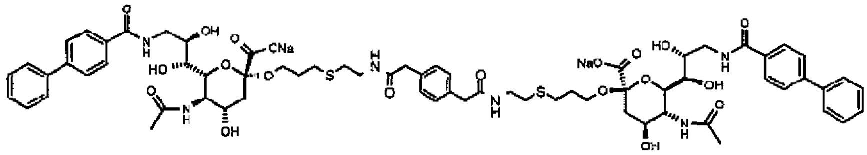 |
| 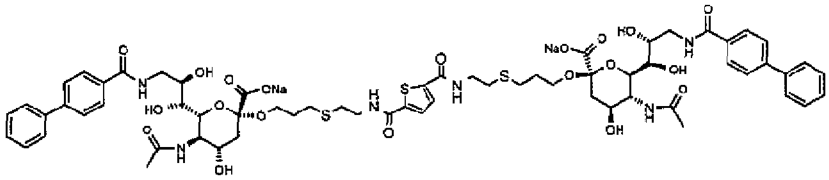 |
| 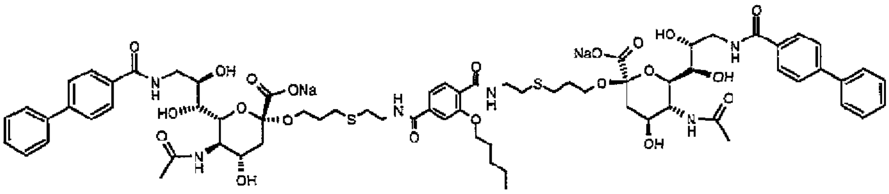 |
| 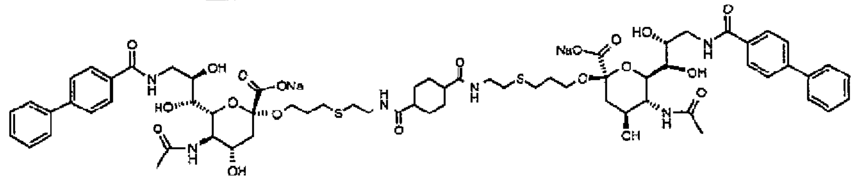 |
| 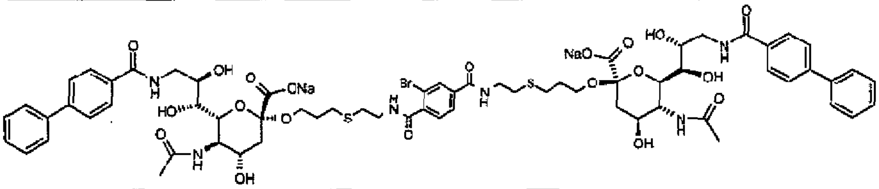 |
| 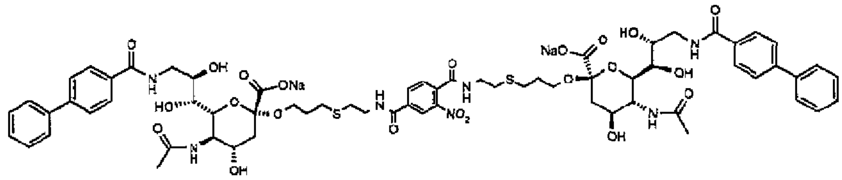 |

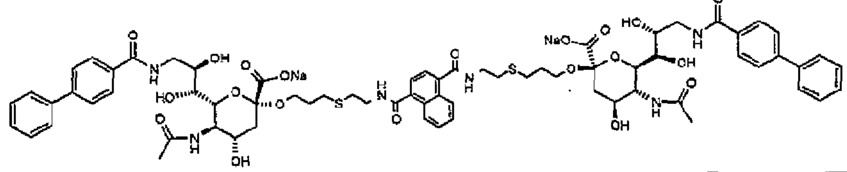

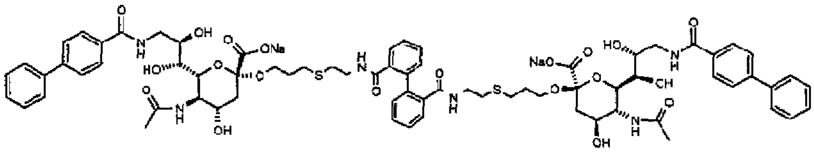

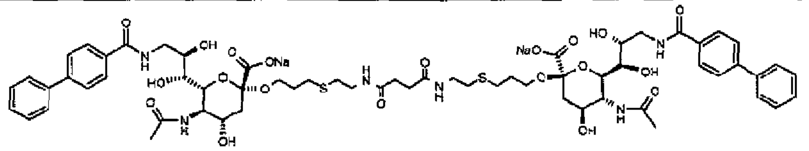

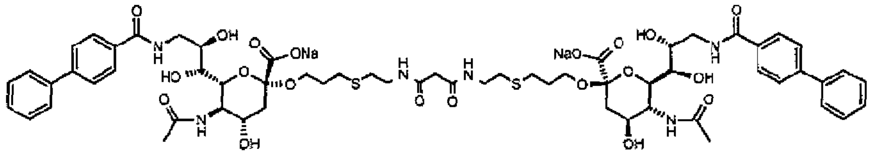

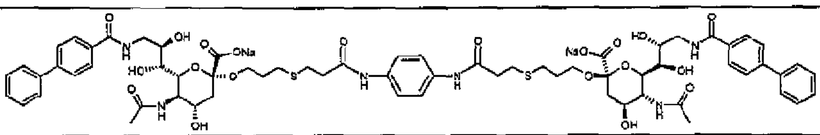

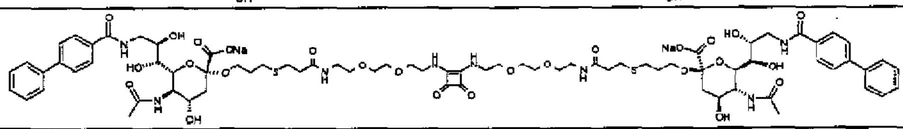

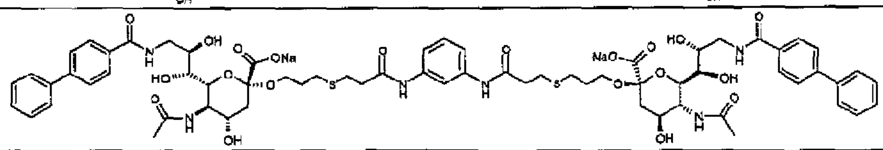

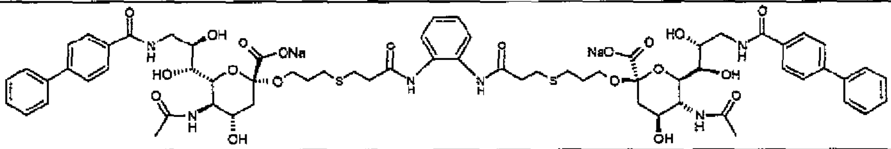

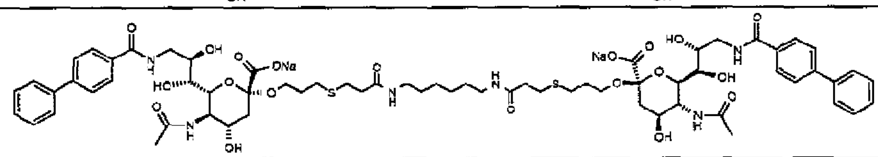

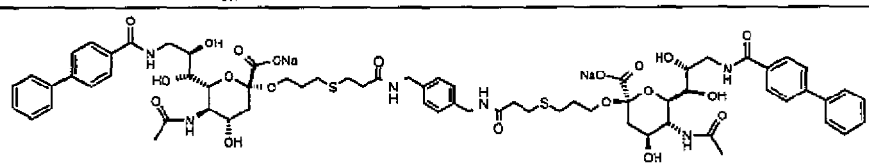

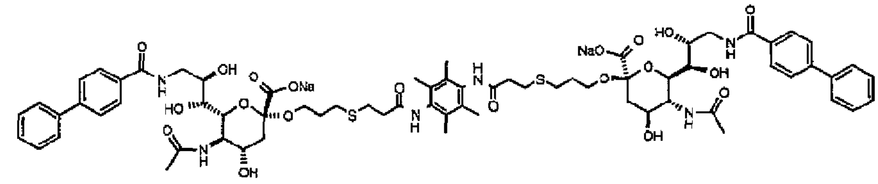

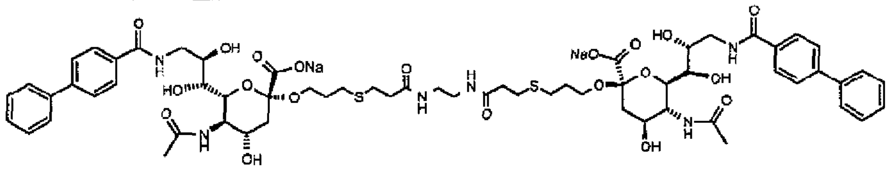

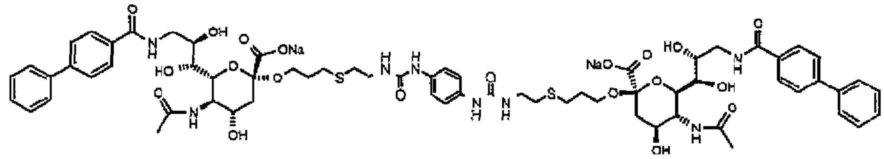

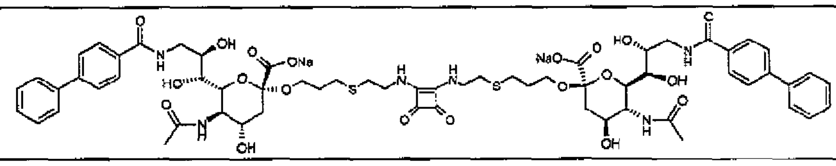

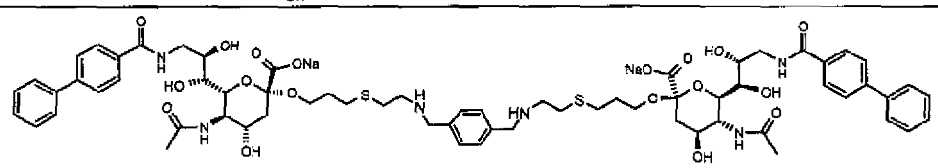

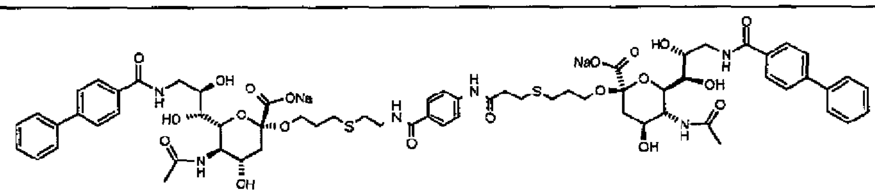

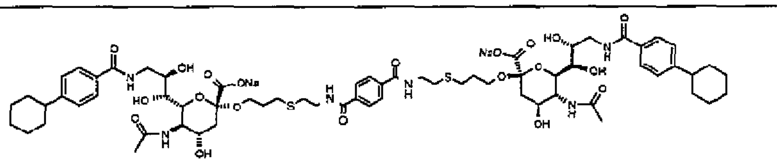

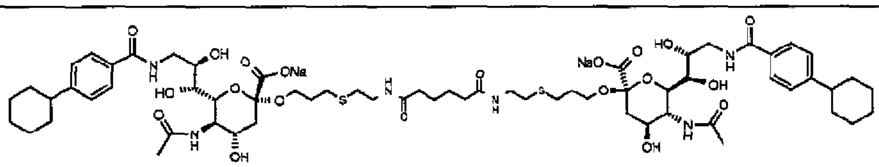

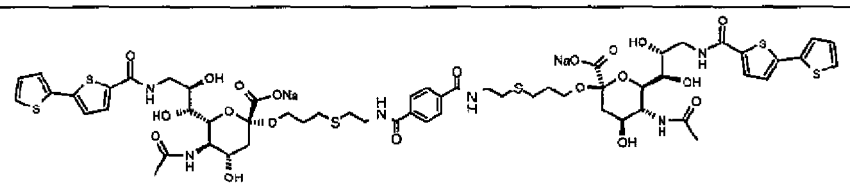

| |
|---|
| 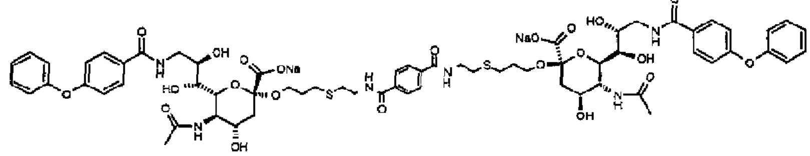 |
| 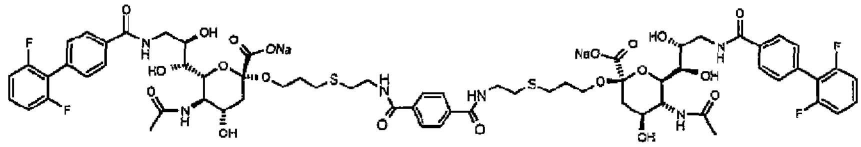 |
| 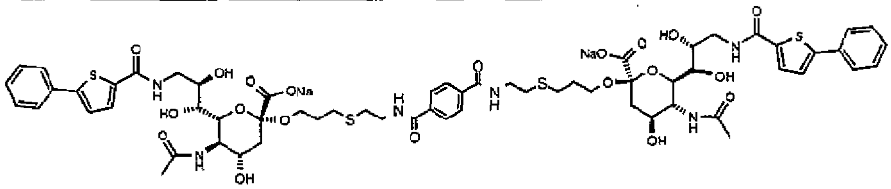 |
| 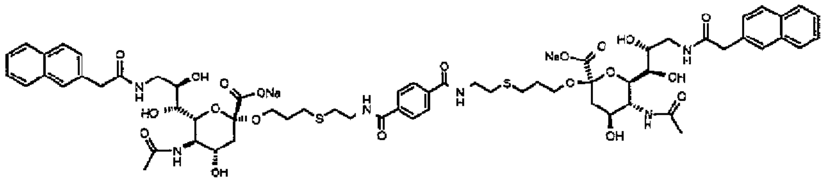 |
| 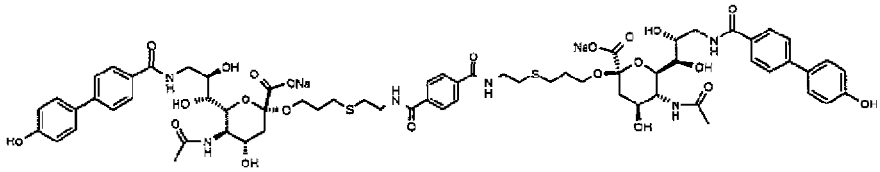 |
| 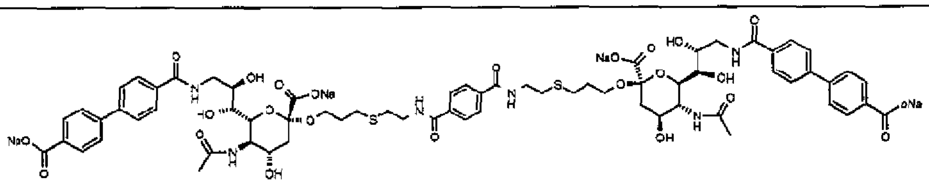 |
| 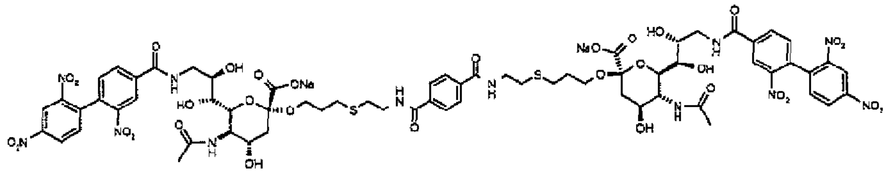 |
| 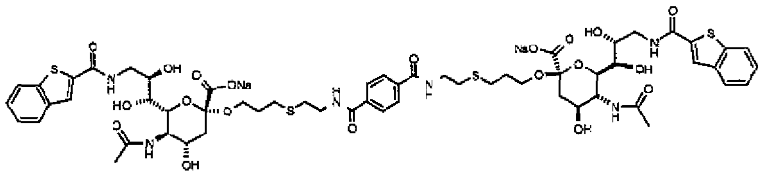 |
| 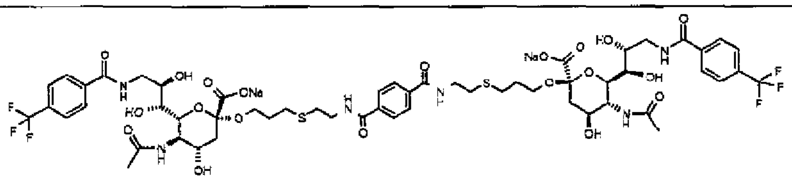 |

| |
|---|
| 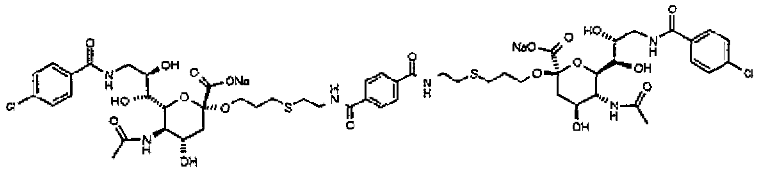 |
| 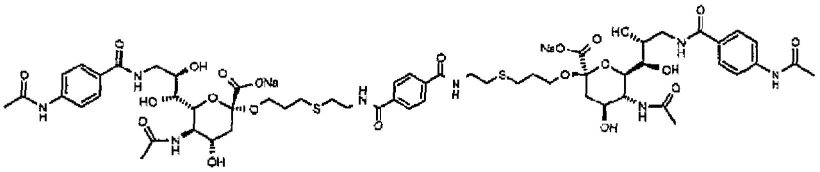 |
| 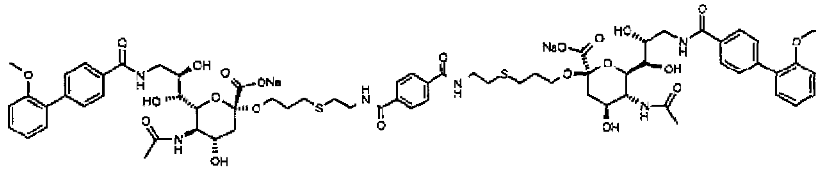 |
| 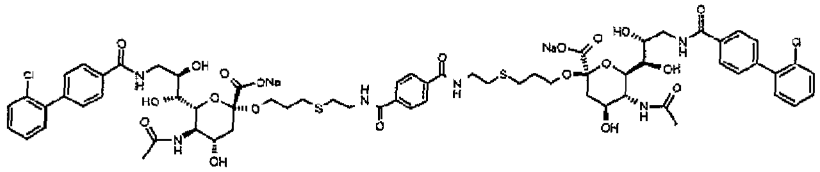 |
| 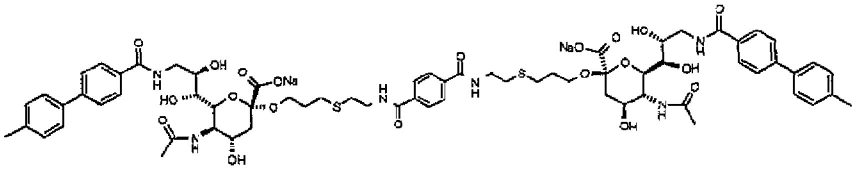 |
| 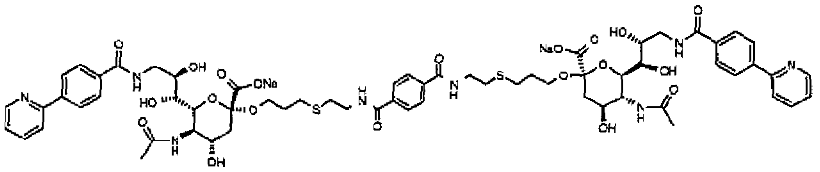 |
| 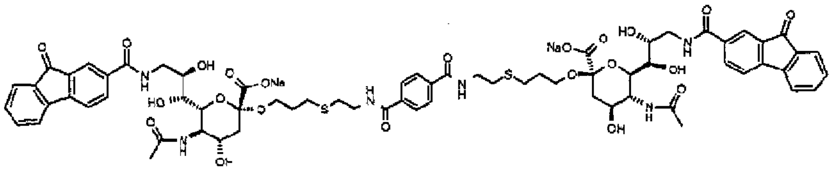 |
| 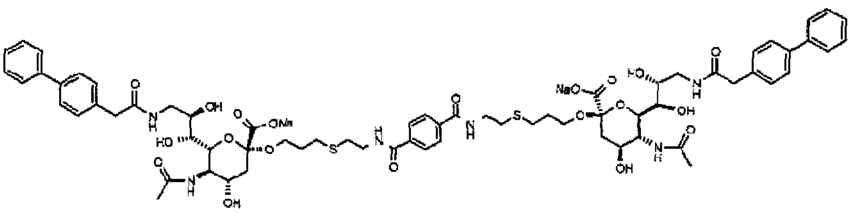 |

| |
|---|
| 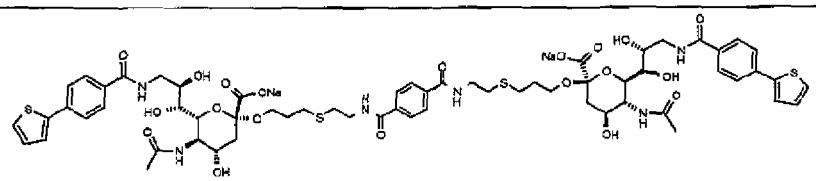 |
| 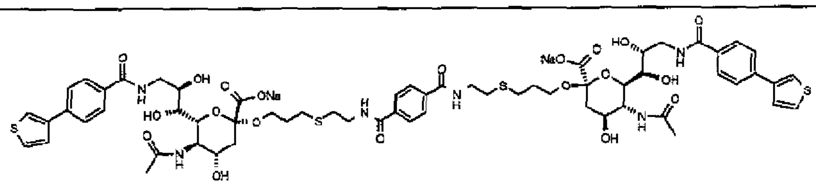 |
| 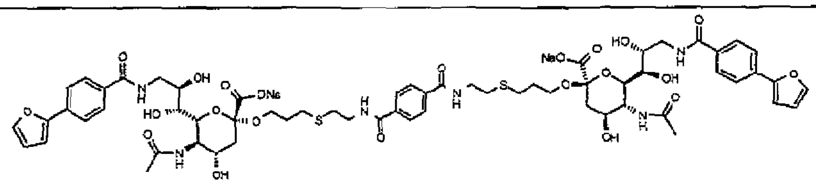 |
| 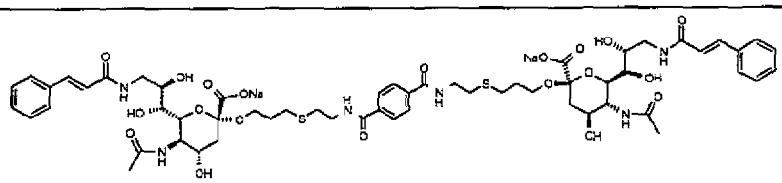 |
| 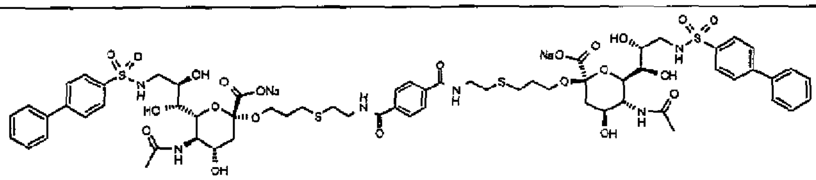 |
| 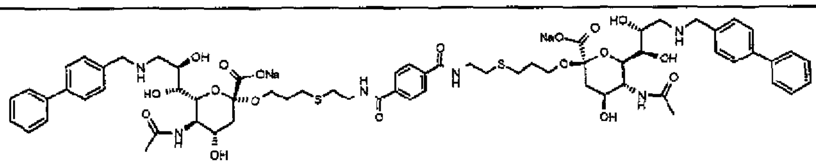 |
| 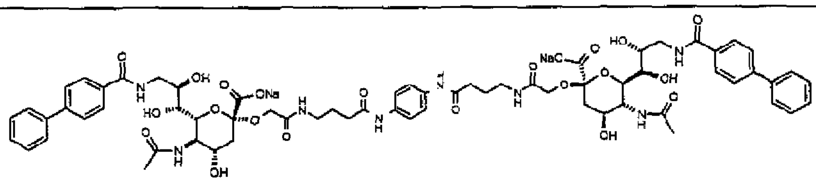 |
| 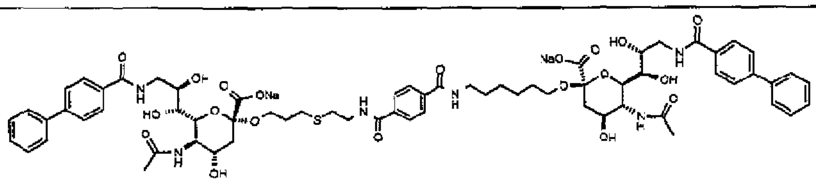 |
| 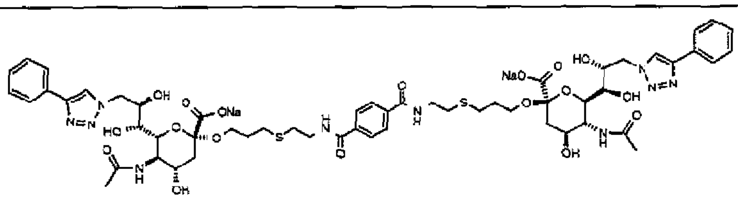 |

| |
|---|
| 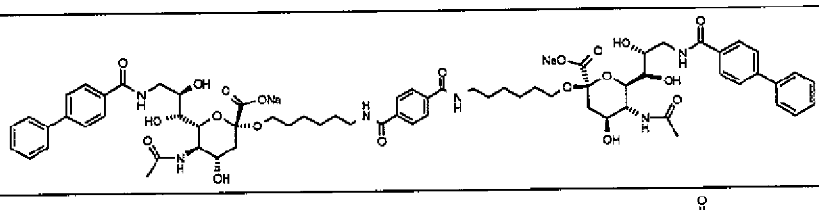 |
| 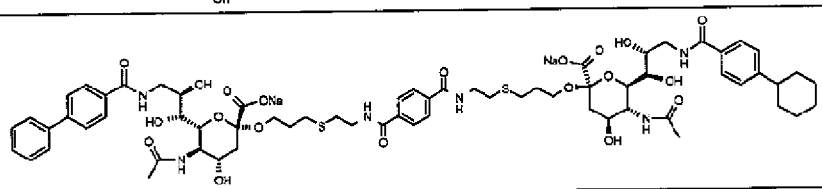 |
| 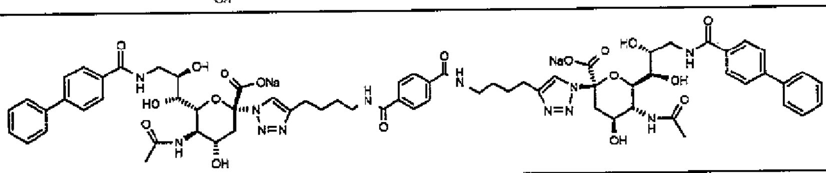 |
| 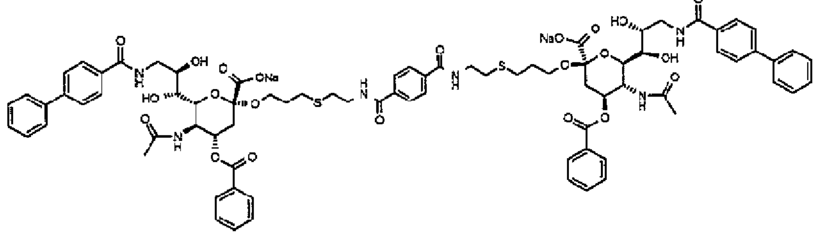 |
| 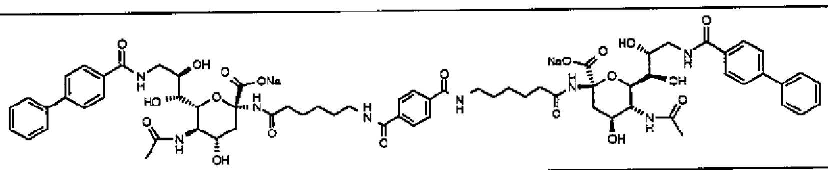 |
| 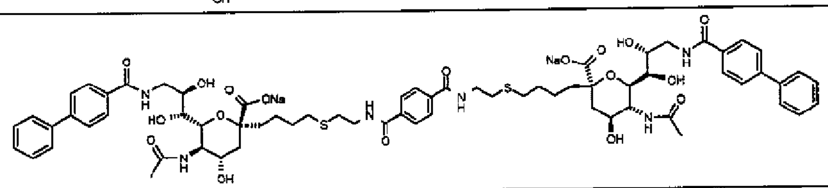 |
| 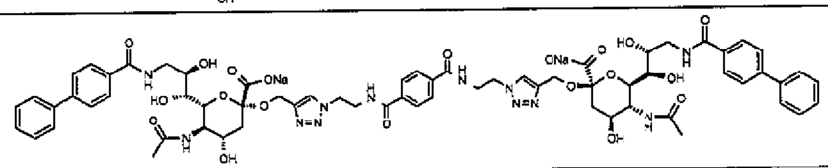 |
| 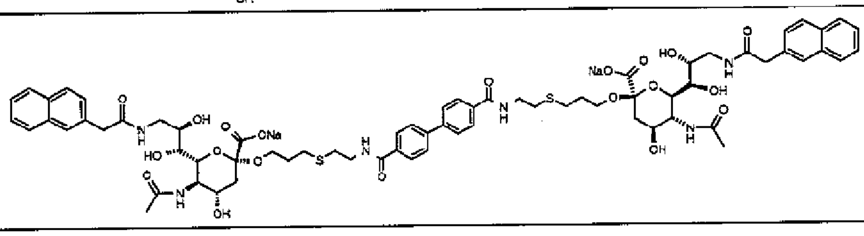 |

| |
|---|
| 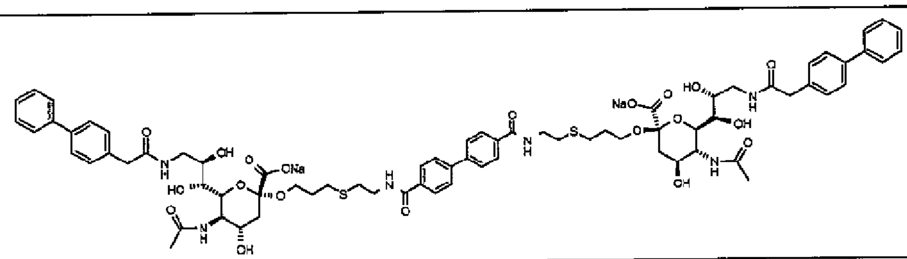 |
| 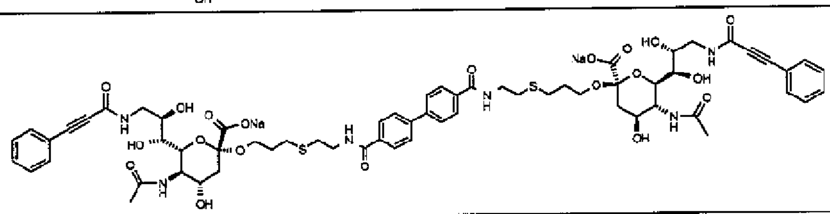 |
| 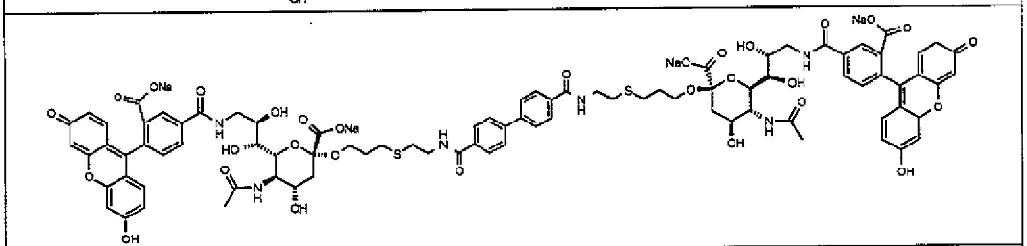 |
| 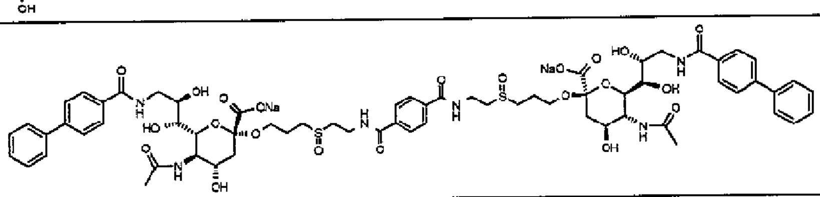 |
| 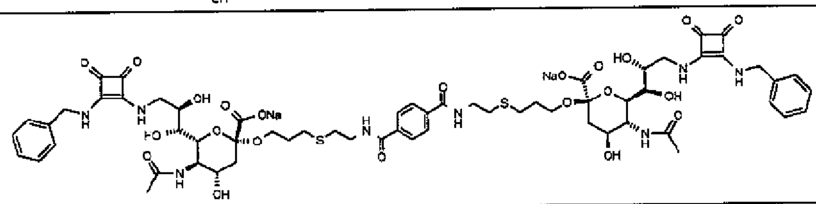 |
| 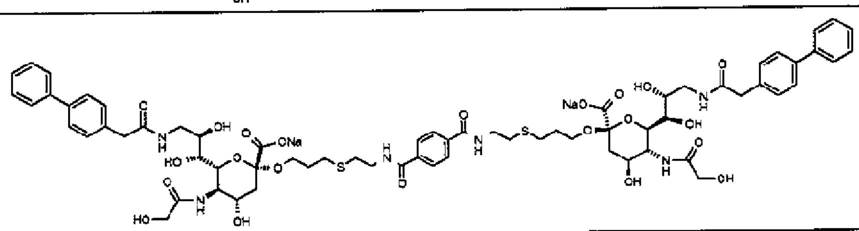 |
| 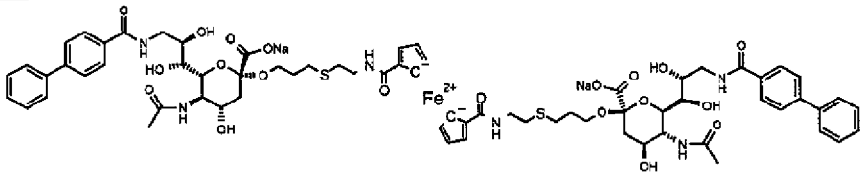 |

| |
|---|
| 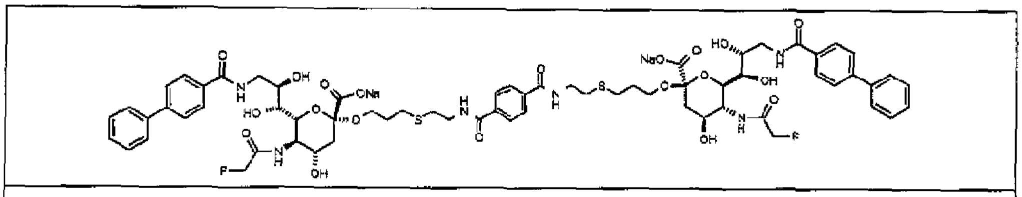 |
| 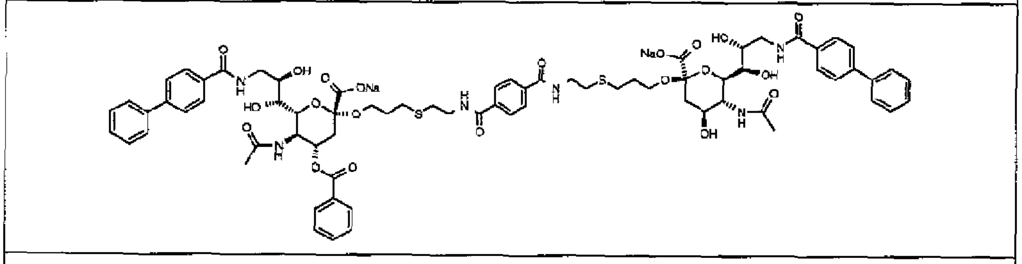 |
| 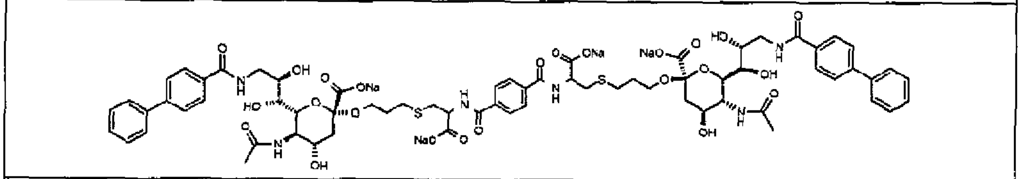 |
| 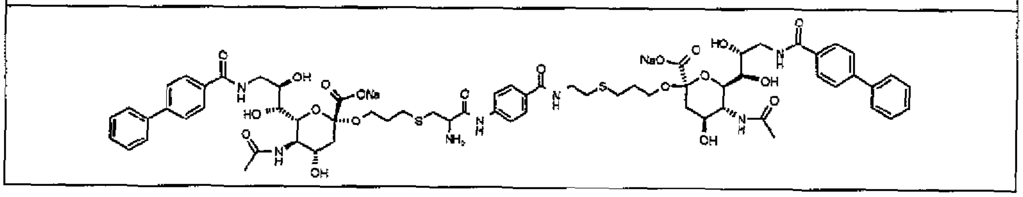 |

sowie deren pharmakologisch verträgliche Salze.

9. Pharmazeutische Zubereitung, enthaltend mindestens ein Sialinsäurederivat der Formel (I) gemäß einem der Ansprüche 1 bis 8 oder ein pharmakologisch verträgliches Salz davon und einen pharmakologisch verträglichen Träger.

10. Sialinsäurederivat der Formel (I) gemäß einem der Ansprüche 1 bis 8 oder ein pharmakologisch verträgliches Salz davon als Arzneimittel.

11. Sialinsäurederivat der Formel (I) gemäß einem der Ansprüche 1 bis 8 oder ein pharmakologisch verträgliches Salz davon zur Behandlung bakterieller Erkrankungen.

12. Sialinsäurederivat der Formel (I) gemäß einem der Ansprüche 1 bis 8 zur Verwendung zur Herstellung eines Arzneimittels für die Behandlung bakterieller, viraler, parasitärer, Autoimmun- und Immunschwäche-Krankheiten.

## Claims

1. Sialic acid derivative of the formula (I),

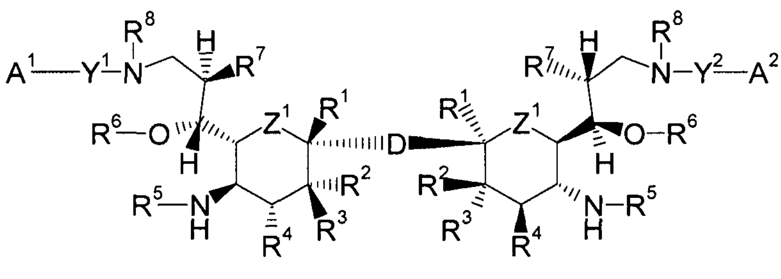

(I)

where the symbols have the following definitions:

$A^1$, $A^2$ are identically or differently a group $D^1$- [$Y^3$-$D^2$-]$_m$-;
$D^1$, $D^2$ are identically or differently a mono- or polycylic aromatic, partially unsaturated or saturated $C_3$-$C_{14}$ hydrocarbon radical or an aromatic, partially unsaturated or saturated three- to eight-membered heterocyclic radical, the stated radicals being unsubstituted or substituted one or more times by a group $X^1$;
$X^1$ is identically or differently halogen, cyano, nitro, hydroxyl, mercapto, amino, carboxyl, hydroxylamino, azido, $B(OH)_2$, SO, $SO_3M$, $OSO_3M$, $SO_2NH_2$, $SO_2CF_3$, P03M, $OPO_3M$, cyanomethyl, alkyl, haloalkyl, alkenyl, alkynyl, alkyloxy, haloalkyloxy, alkenyloxy, alkynyloxy, alkylthio, alkylamino, dialkylamino, trialkylamino, formyl, alkylcarbonyl, alkylsulphonyl, alkylsulphoxyl, alkylaminosulphoxyl, dialkylaminosulphoxyl, alkyloxycarbonyl, alkylcarbonyloxy, aminocarbonyl, aminothiocarbonyl, alkylaminocarbonyl, alkylcarbonylamino, dialkylaminocarbonyl, alkylaminothiocarbonyl, oxo (=0), thioxo (=S), $C_1$-$C_8$ alkylimino (=N-$C_1$-$C_8$ alkyl), $C_1$-$C_8$ alkyloximino (=N-O-$C_1$-$C_8$ alkyl), the alkyl groups in these radicals containing 1 to 8 carbon atoms, and the stated alkenyl or alkynyl groups in these radicals containing 2 to 8 carbon atoms;
$Y^1$, $Y^2$ are identically or differently ~($C_1$-$C_4$ alkyl)-, ~($C_1$-$C_4$ haloalkyl)-, ~($C_1$-$C_9$ alkyl)-(CO)-, ~C(O)-, ~$CH_2$-C(O)-, ~CH=CH-C(O)-, ~C≡C-C(O)-, ~$S(O)_2$-, ~$CH_2$-$S(O)_2$-, ~$NR^x$-C(O)-, ~$CH(CF_3)$-, ~$NR^x$-3-cyclobutene-1,2-dione-4-, ~$CH_2$-$NR^x$-3-cyclobutene-1,2-dione-4-, ~$NR^x$-3-2,5-thiadiazole 1-oxide-4-, ~$NR^x$-3-2,5-thiadiazole 1,1-dioxide-4-, where ~ denotes the bond to the group A, or
$Y^1$, $Y^2$ form, independently of one another, together with the nitrogen to which they are bonded, and with the group $R^8$, a group ~4-1H-(1,2,3)triazol-1-yl-, ~5-1H-(1,2,3)triazol-1-yl-, ~$CH_2$-4-1H-(1,2,3)triazol-1-yl- or -$CH_2$-5-1H-(1,2,3)triazol-1-yl-, where ~ denotes the bond to the group A;
$Y^3$ is a bond, 0, S, S(O), $S(O)_2$, $CH_2$, C(O), $CR^x_2$ or $NR^x$;
m is 0, 1 or 2;
$Z^1$ is 0, S, $CH_2$ or $NR^x$;
D is a group $Z^2$-$T^1$-$Y^4$-$A^3$-$Y^5$-$T^2$-$Z^2$;
$Z^2$ is identically or differently -O~, -S~, -$NR^x$~, -NH-C(O)~, -$CH_2$~, -$CF_2$~, -CH(OH)~, -N($R^x$)-O~, -O-$NR^x$~, -O-N=CH-, ~4-1H-(1,2,3)triazol-1-yl-, or ~5-1H-(1,2,3)triazol-1-yl-, where ~ denotes the bond to the group T;
$T^1$, $T^2$ are identically or differently a straight-chain or branched alkanediyl group having 1 to 30 C atoms, where

(i) optionally one or more non-terminal $CH_2$ groups are replaced by -0-, -S-, -S(O)-, -$S(O)_2$-, -$S^+(CH_3)$-, -$P(O_2)$- and/or -$NR^x$-, and/or
(ii) optionally one or more H atoms are replaced by F, Cl, $OR^x$, $OSO_3M$, (=O), (=S), carboxyl, $NH_2$, $NHR^y$ and/or $NHR^z$, and/or
(iii)optionally one or more non-terminal -$CH_2$-$CH_2$- groups are replaced by -5-1H-(1,2,3)triazol-1-yl-, -$CR^x$=$CR^x$- and/or -C≡C-, and/or
(iv) optionally a -$CH_2CH_2CH_2$- group is replaced by -4-1H-(1,2,3)triazol-1-yl- and/or -O-N=CH-, and/or
(v) optionally a -$CH_2CH_2CH_2CH_2$- group is replaced by phenyl-1,4-diyl;

$Y^4$, $Y^5$ are identically or differently a bond, 0, S, $NR^x$, S(O), $S(O)_2$, C(O), ~C(O)-$NR^x$-, ~$NR^x$-C(O)-, ~C(O)-O-, ~O-C(O)-, ~$NR^x$-CO-$NR^x$-, ~$NR^x$-$S(O)_2$-, ~$S(O)_2$-$NR^x$-, ~$CH_2$-$NR^x$-C(O)-, ~$CH_2$-C(O)-$NR^x$-, ~$CH_2$-$NR^x$-, ~$CH(CF_3)$-$NR^x$-, ~CH=N-O- or ~O-N=CH-, where ~ denotes the bond to group A;
$A^3$ is

a) $C_1$-$C_8$ alkanediyl, $C_2$-$C_8$ alkenediyl, $C_2$-$C_8$ alkynediyl, $C_4$-$C_8$ alkadienediyl, two or more $CH_2$ groups in the stated groups being replaced by 0, S, S(0), $S(O)_2$, $NR^x$ and/or C(0), and optionally one or more H atoms in the stated groups being replaced by a group $X^2$,
b) a group $A^4$-[$Z^3$-$A^5$]$_n$,
c) 1,1'-ferrocenediyl, 1,1'-cobaltocenediyl, 1,1'-ruthenocene or dichloroplatinumdiaminodiyl;

$A^4$, $A^5$ are identically or differently a saturated, partially unsaturated or aromatic, mono- or polycyclic hydrocarbon radical having 3 to 14 C atoms, or a three- to eight-membered aromatic, partially unsaturated or saturated mono- or polycyclic heterocyclic radical, the stated groups being each optionally substituted by one or more groups $X^2$;
$Z^3$ is a bond, 0, S, S(O), $S(O)_2$, $NR^x$, C(O) or $CR^x_2$ ;
$X^2$ is identically or differently fluoro, chloro, bromo, nitro, hydroxylamino, $B(OH_2)$, $SO_3M$, $OSO_3M$, $SO_2NH_2$, $PO_3M$, $OPO_3M$, alkyl, haloalkyl, alkyloxy, haloalkyloxy, alkylthio, alkylamino, dialkylamino, trialkylamino, alkylcarbonyl, alkylsulphonyl, alkylsulphoxyl, alkylaminosulphoxyl, dialkylaminosulphoxyl, alkyloxycarbonyl, alkylcarbonyloxy, aminocarbonyl, alkylaminocarbonyl, alkylcarbonylamino, dialkylaminocarbonyl, alkylaminothiocarbonyl, $C_1$-$C_4$ alkylimino (=N-$C_1$-$C_4$ alkyl), $C_1$-$C_4$ alkyloximino (=N-O-$C_1$-$C_4$ alkyl), the alkyl groups in these radicals containing 1 to 4 carbon atoms, and the groups haloalkyl and haloalkyloxy containing the halogens F

and/or Cl;
n is 0, 1;
$R^1$ is identically C(O)OM, O-$PO_3M_2$, O-$SO_3M$, $SO_3M$, C(O)-NH-$S(O)_2$-$R^x$, $PO_3M_2$ or C(O)NOM;
$R^2$, $R^3$ are identically or differently H or F;
$R^4$, $R^7$ are identically or differently H, OH, $OR^z$, OC(O)$NHR^y$ or $NR^x$;
$R^6$ is identically or differently H or $R^z$;
$R^5$ is identically or differently H, $R^x$, $R^x_2$, C(O)H, C(O)$CH_2$OH or C(O)-haloalkyl;
$R^8$ is identically or differently $R^x$;
M is H, $C_1$-$C_6$ alkyl or a cation;
$R^x$ is identically or differently H, $R^y$ or $R^z$;
$R^y$ is identically or differently $C_1$-$C_6$ alkyl, phenyl or benzyl, and
$R^z$ is identically or differently -C(O)-$C_1$-$C_6$ alkyl, -C(O) phenyl or -C(O)-$C_1$-$C_4$ alkyl-phenyl,

where, in the groups stated, alkyl represents saturated, straight-chain, branched or cyclic hydrocarbon radicals having the respectively specified number of carbon atoms, alkenyl represents unsaturated, straight-chain or branched hydrocarbon radicals having the specified number of carbon atoms and one or two double bonds in any position, and alkynyl represents straight-chain or branched hydrocarbon groups having the specified number of carbon atoms and one or two triple bonds in any position,
and also its pharmacologically tolerated salts.

**2.** Sialic acid derivative according to Claim 1, where the symbols in the formula (I) have the following definitions:

$A^1$, $A^2$ are identically or differently a group $D^1$-[$Y^3$-$D^2$-]$_m$-;
$D^1$ is identically or differently

a) $C_6$-$C_{14}$-aryl,
b) $C_3$-$C_{14}$ cycloalkenyl or $C_5$-$C_{14}$ cycloalkadienyl,
c) $C_3$-$C_8$ cycloalkyl,
d) non-aromatic, saturated or partially unsaturated 5- or 6-membered heterocyclyl, containing one to three nitrogen atoms and/or one oxygen or sulphur atom or one or two oxygen and/or sulphur atoms,
e) 5-membered heteroaryl, containing one to four nitrogen atoms or one to three nitrogen atoms and/or one sulphur or oxygen atom,
f) 6-membered heteroaryl, containing one to three or one to four nitrogen atoms,
g) polycyclic heterocyclic radicals, the stated groups being unsubstituted or substituted by one or more groups $X^1$;

$D^2$ is identically or differently

a) $C_6$-$C_{14}$ aryldiyl,
b) $C_3$-$C_8$ cycloalkyldiyl,
c)non-aromatic, saturated or partially unsaturated 5- or 6-membered heterocyclodiyl, containing one to three nitrogen atoms and/or one oxygen or sulphur atom or one or two oxygen and/or sulphur atoms,
d)5-membered heteroaryldiyl, containing one to four nitrogen atoms or one to three nitrogen atoms and one sulphur or oxygen atom,
e)6-membered heteroaryldiyl, containing one to three or one to four nitrogen atoms,

the stated groups being unsubstituted or substituted by one or more groups $X^1$;
$X^1$ is identically or differently F, Cl, Br, I, cyano, nitro, hydroxy, mercapto, amino, carboxyl, hydroxylamino, azido, $SO_3M$, $OSO_3M$, $SO_2NH_2$, $OPO_3M$, alkyl, haloalkyl, alkenyl, alkynyl, alkyloxy, haloalkyloxy, alkenyloxy, alkynyloxy, alkylthio, alkylamino, dialkylamino, trialkylamino, formyl, alkylcarbonyl, alkylsulphonyl, alkylsulphoxyl, aminocarbonyl, alkylaminocarbonyl, alkylcarbonylamino, oxo (=O), thioxo (=S), the alkyl groups in these radicals containing 1 to 8 carbon atoms, and the stated alkenyl or alkynyl groups in these radicals containing 2 to 8 carbon atoms;
$Y^1$, $Y^2$ are identically or differently ~CH3-, ~$CH_2CH_2$-, ~$CH_2CH_2CH_2$-, ~C(O)-, ~$CH_2$C(O)-, ~$CH_2CH_2$C(O)-, ~$CH_2CH_2CH_2$C(O)-, ~CH=CH-C(O)-, ~C≡C-C(O)-, ~$S(O)_2$-, ~$CH_2S(O)_{2-}$, ~NH-C(O)-, ~$NR^x$-3-cyclobutene-1,2-dione-4-, ~$CH_2$-$NR^x$-3-cyclobutene-1,2-dione-4-, ~$NR^x$-3-2,5-thiadiazole 1,1-dioxide-4-, where ~ denotes the bond to the group A, or

$Y^1$, $Y^2$ form, independently of one another, together with the nitrogen to which they are bonded, and with the group $R^8$, a ~4-1H-(1,2,3)triazol-1-yl-, ~5-1H-(1,2,3)triazol-1-yl-, ~$CH_2$-4-1H-(1,2,3)triazol-1-yl- or ~$CH_2$-5-1H-(1,2,3)triazol-1-yl- ring, where ~ denotes the bond to the group A;
$Y^3$ is a bond, O, S(O), $S(O_2)$, $CH_2$, $NR^x$ or C(O);
m is 0, 1 or 2;
$Z^1$ is 0, S or $CH_2$;
D is a group $Z^2$-$T^1$-$Y^4$-$A^3$-$Y^5$-$T^2$-$Z^2$;
$Z^2$ is identically or differently -O~, -S~, -$NR^x$~, -NHC(O)~, -$CH_2$~, -O-$NR^x$~ or ~4-1H-(1,2,3)triazol-1-yl-, where ~ denotes the bond to the group T;
$T^1$, $T^2$ are identically or differently a straight-chain or branched alkanediyl group having 4 to 30 C atoms, where

(i) optionally one or more non-terminal $CH_2$ groups are replaced by ~O-, -S-, -S(O)-, -$S(O)_2$-, -$P(O_2)$- and/or -$NR^x$-, and/or
(ii) optionally one or more H atoms are replaced by F, Cl, $OR^x$, -$OSO_3M$, (=O), carboxyl, $NH_2$, $NHR^y$ or $NHR^z$, and/or
(iii)optionally a non-terminal -$CH_2$-$CH_2$ group is replaced by -5-1H-(1,2,3)triazol-1-yl- and/or
(iv) optionally a non-terminal -$CH_2CH_2CH_2$- group is replaced by -4-1H-(1,2,3)triazol-1-yl- or -0-N=CH-;

$Y^4$, $Y^5$ are identically or differently O, $NR^x$, S(O), $S(O)_2$, C(O), ~C(O)-$NR^x$-. ~$NR^x$-C(O)-, ~$NR^x$-CO-$NR^x$-, ~$NR^x$-$S(O)_2$-, ~$S(O)_2$-$NR^x$-, ~$CH_2$-$NR^x$-C(O)-, ~$CH_2$-C(O)-$NR^x$- or ~$CH_2$-$NR^x$-, where ~ denotes the bond to group A;
$A^3$ is

a) $C_1$-$C_8$ alkanediyl, $C_2$-$C_8$ alkenediyl, $C_2$-$C_8$ alkynediyl, $C_4$-$C_8$ alkadiendiyl, optionally 1, 2, 3 or 4 $CH_2$ groups in the stated groups being replaced by 0, S(0), $S(O)_2$, $NR^x$ and/or S, and optionally one or more H atoms in the stated groups being replaced by F or Cl,
b) a group $A^4$-$[Z^3$-$A^5]_n$,
c) 1,1'-ferrocenediyl, 1,1'-cobaltocenediyl, 1,1'-ruthenocene or dichloroplatinum-diamonodiyl;

$A^4$, $A^5$ are identically or differently

a) $C_6$-$C_{14}$ aryldiyl,
b) $C_3$-$C_8$ cycloalkyldiyl,
c) non-aromatic, saturated or partially unsaturated 5- or 6-membered heterocyclodiyl, containing one to three nitrogen atoms and/or one oxygen or sulphur atom or one or two oxygen and/or sulphur atoms,
d) 5-membered heteroaryldiyl, containing one to four nitrogen atoms or one to three nitrogen atoms and/or one sulphur or oxygen atom,
e) 6-membered heteroaryldiyl, containing one to three or one to four nitrogen atoms,
f) polycyclic heterocyclyl from the group, the stated radicals being unsubstituted or substituted by one or more groups $X^2$;

$Z^3$ is a bond, 0, S(O), $S(O)_2$ or C(O);
$X^2$ is identically or differently fluoro, chloro, nitro, bromo, hydroxylamino, $SO_3M$, $OSO_3M$, $SO_2NH_2$, $PO_3M$, $OPO_3M$, alkyl, haloalkyl, alkyloxy, haloalkyloxy, alkylamino, dialkylamino, trialkylamino, alkylcarbonyl, alkylsulphonyl, alkylsulphoxyl, alkylaminosulphoxyl, dialkylaminosulphoxyl, alkyloxycarbonyl, alkylcarbonyloxy, aminocarbonyl, alkylaminocarbonyl, alkylcarbonylamino, dialkylaminocarbonyl, $C_1$-$C_4$ alkylimino (=N-$C_1$-$C_4$ alkyl), $C_1$-$C_4$ alkyloximino (=N-O-$C_1$-$C_4$ alkyl), the alkyl groups in these radicals containing 1 to 4 carbon atoms, and the groups haloalkyl and haloalkyloxy containing the halogens F and/or Cl;
n is 0 or 1;
$R^1$ is identical and is C(O)OM, $SO_3M$, C(O)-NH-$S(O)_2$-$R^x$, $PO_3M_2$ or C(O)NOM;
$R^2$, $R^3$ are identically or differently H or F;
$R^4$, $R^7$ are identically or differently H, OH, $OR^z$, $OC(O)NHR^y$ or $NR^x$;
$R^6$ is identically or differently H or $R^z$;
$R^5$ is identically or differently H, $R^x$, C(O)$CH_2$OH, C(O)-haloalkyl or C(O)H;
$R^8$ is identically or differently $R^x$;
M is H, $C_1$-$C_4$ alkyl or a cation from the group of the alkali metals, alkaline earth metals, manganese, copper, zinc, iron and optionally substituted ammonium;
$R^x$ is identically or differently H, $R^y$ or $R^z$;

$R^y$ is identically or differently $C_1$-$C_4$ alkyl, phenyl or benzyl and
$R^z$ is identically or differently -C(O)-$C_1$-$C_6$ alkyl, C(O) phenyl or C(O)$CH_2$ phenyl,

and also its pharmacologically tolerated salts.

**3.** Sialic acid derivative according to Claim 1 or 2, where symbols and indices in the formula (I) have the following definitions:

$A^1$, $A^2$ are identically or differently a group $D^1$-[$Y^3$-$D^2$-]$_m$-;
$D^1$ is identically or differently phenyl, 1-naphthyl, 2-naphthyl, 2-biphenylene, 2-fluorenyl, 3-fluorenyl, 2-phenanthrenyl, cyclopropyl, cyclobutyl, cyclopentyl, cyclohexyl, 2-piperidinyl, 3-piperidinyl, 4-piperidinyl, 1,3-dioxan-5-yl, 4-tetrahydropyranyl, 3-hexahydropyridazinyl, 4-hexahydropyridazinyl, 2-hexahydropyrimidinyl, 4-hexahydropyrimidinyl, 5-hexahydro-pyrimidinyl, 1-piperazinyl, 2-piperazinyl, 4-morpholinyl, 2-furyl, 3-furyl, 2-thienyl, 3-thienyl, 2-pyrrolyl, 3-pyrrolyl, 3-pyrazolyl, 4-pyrazolyl, 5-pyrazolyl, 2-oxazolyl, 4-oxazolyl, 5-oxazolyl, 2-thiazolyl, 4-thiazolyl, 5-thiazolyl, 2-imidazolyl, 4-imidazolyl, 1,2,3-triazol-1-yl, 1,2,3-triazol-4-yl, 1,2,3-triazol-5-yl, 1,3,4-triazol-2-yl, 2-pyridinyl, 3-pyridinyl, 4-pyridinyl, 3-pyridazinyl, 4-pyridazinyl, 2-pyrimidinyl, 4-pyrimidinyl, 5-pyrimidinyl, 2-pyrazinyl, 1-benzofuran-2-yl, 1-benzofuran-5-yl, 1-benzofuran-6-yl, 2-benzofuran-5-yl, 2-benzofuran-6-yl, 2H-chromen-3-yl, 2H-chromen-6-yl, 2H-chromen-7-yl, indol-2-yl, 1-benzothiophen-2-yl or benzimidazol-2-yl,

the stated groups being unsubstituted or substituted by one or more groups $X^1$;

$D^2$ is identically or differently phenylene-1,4-diyl, phenylene-1,3-diyl, biphenyl-4,4'-diyl, naphthalene-1,4-diyl, trans-cyclohexane-1,4-diyl, furan-2,5-diyl, thiophene-2,4-diyl, thiophene-2,5-diyl, 1H(1,2,3)-triazole-1,4-diyl or pyrrole-2,5-diyl, pyridine-2,5-diyl, pyridine-2,4-diyl, pyridazine-3,6-diyl, pyrimidine-2,5-diyl, pyrimidine-2,6-diyl, pyrazine-2,5-diyl or tetrazine-3,5-diyl,

the stated groups being unsubstituted or substituted by one or group $X^1$;

$X^1$ is identically or differently F, Cl, Br, I, cyano, nitro, hydroxyl, amino, carboxyl, azido, $SO_3M$, $SO_2NH_2$, alkyl, haloalkyl, alkyloxy, haloalkyloxy, alkylamino, dialkylamino, trialkylamino, alkylcarbonyl, alkylsulphoxyl, aminocarbonyl, alkylaminocarbonyl, alkylcarbonylamino, oxo (=0), the alkyl groups in these radicals containing 1 to 6 carbon atoms;
$Y^1$, $Y^2$ are identically or differently ~$CH_2$-, ~C(O)-, ~$CH_2$-C(O)-, ~$CH_2CH_2$C(O)-, ~CH=CH-C(O)-, ~C≡C-C(O)-, ~NH-C(O)-, ~$CH_2$-$NR^x$-3-cyclobutene-1,2-dione-4-, ~$S(O)_2$- or ~$CH_2S(O)_2$- where ~ denotes the bond to the group A, or
$Y^1$, $Y^2$ form, independently of one another, together with the nitrogen to which they are bonded, and with the group $R^8$, a group ~4-1H-(1,2,3)triazol-1-yl-, where ~ denotes the bond to the group A;
$Y^3$ is a bond, 0, S($O_2$), $CH_2$ or C(O);
m is 0, 1 or 2;
$Z^1$ is O;
D is a group $Z^2$-$T^1$-$Y^4$ -$A^3$-$Y^5$-$T^2$-$Z^2$
$Z^2$ is identically 0, S, ~4-1H-(1,2,3)triazol-1-yl-, -NH-C(O)~ or $CH_2$, where ~ denotes the bond to the group T;
$T^1$, $T^2$ are identically or differently a straight-chain or branched alkanediyl group having 4 to 20 C atoms, where

(i) optionally one or more non-terminal $CH_2$ groups are replaced by -S-, -S(O)-, - $S(O)_2$-, and/or -$NR^z$-, and/or
(ii) optionally one or more H atoms are replaced by fluoro, chloro, (=O), carboxyl, $NH_2$, $NHR^y$ or $NHR^z$, and/or
(iv) optionally a non-terminal -$CH_2CH_2CH_2$-group is replaced by -4-1H-(1,2,3)triazol-1-yl-;

$Y^4$, $Y^5$ are identically or differently ~$NR^x$-C(O)-, ~C(O)-$NR^x$-, ~$NR^x$-$S(O)_2$, ~$NR^x$-, ~$CH_2NR^x$-, ~$NR^x$C(O)$NR^x$-, ~$CH_2NR^x$C(O)-, ~$CH_2$C(O)$NR^x$- or ~$S(O)_2$-$NR^x$-, where ~ denotes the bond to group A;
$A^3$ is

a) $C_1$-$C_6$ alkanediyl, $C_2$-$C_4$ alkyndiyl, one or more H atoms in the stated groups being optionally replaced by F or Cl, or
b) a group $A^4$;

$A^4$ is identically phenylene-1,4-diyl, biphenyl-4,4'-diyl, naphthalene-1,4-diyl, trans-cyclohexane-1,4-diyl, thiophene-2,5-diyl, 1H-(1,2,3)triazole-1,4-diyl, pyridine-2,5-diyl, pyridine-2,4-diyl, pyridazine-2,5-diyl, pyrimidine-2,5-diyl, pyrimidine-2,4-diyl, pyrazine-2,5-diyl or tetrazine-2,5-diyl, the stated radicals being unsubstituted or substituted by a group $X^2$;

$X^2$ is identically or differently fluoro, chloro, bromo, nitro, $SO_3M$, alkyl, haloalkyl, alkyloxy, haloalkyloxy, alkylamino, dialkylamino, trialkylamino, alkylcarbonyl, alkylsulphoxyl, alkylcarbonyloxy, aminocarbonyl, alkylaminocarbonyl, alkylcarbonylamino, the alkyl groups in these radicals containing 1 to 4 carbon atoms, and the groups haloalkyl and haloalkyloxy containing the halogens F and/or Cl;

$R^1$ is identical and is C(O)OM or C(O)NOM;

$R^2$, $R^3$ are identically H;

$R^4$, $R^7$ are identically OH or $OR^z$;

$R^6$ is identically H or $R^z$;

$R^5$ is identically $R^x$, $C(O)CH_2OH$ or C(O)-haloalkyl;

$R^8$ is identically or differently $R^x$;

M is H, methyl, ethyl or a cation from the group Li, Na, K, Ca, Mg and optionally substituted ammonium;

$R^x$ is identically or differently H, $R^y$ or $R^z$;

$R^y$ is identically or differently methyl, ethyl, 1-methylethyl, propyl, 1-methylpropyl, 1,1-dimethylpropyl or benzyl;

$R^z$ is identically or differently methylcarbonyl, ethylcarbonyl, propylcarbonyl, 1-methylethylcarbonyl, 1,1-dimethylethylcarbonyl or phenylcarbonyl,

and also its pharmacologically tolerated salts.

**4.** Sialic acid derivative according to any of Claims 1 to 3, where the symbols and indices in the formula (I) have the following definitions:

$A^1$, $A^2$ are identically or differently a group $D^1$-[$Y^3$-$D^2$-]$_m$-;

$D^1$ is identically or differently phenyl, 1-naphthyl, 2-naphthyl, 2-biphenylene, 2-fluorenyl, 3-fluorenyl, 2-phenanthrenyl, cyclopropyl, cyclobutyl, cyclopentyl, cyclohexyl, 2-piperidinyl, 3-piperidinyl, 4-piperidinyl, 1,3-dioxan-5-yl, 4-tetrahydropyranyl, 3-hexahydropyridazinyl, 4-hexahydropyridazinyl, 2-hexahydropyrimidinyl, 4-hexahydropyrimidinyl, 5-hexahydropyrimidinyl, 1-piperazinyl, 2-piperazinyl, 4-morpholinyl, 2-furyl, 3-furyl, 2-thienyl, 3-thienyl, 2-pyrrolyl, 3-pyrrolyl, 3-pyrazolyl, 4-pyrazolyl, 5-pyrazolyl, 2-oxazolyl, 4-oxazolyl, 5-oxazolyl, 2-thiazolyl, 4-thiazolyl, 5-thiazolyl, 2-imidazolyl, 4-imidazolyl, 1,2,3-triazol-1-yl, 1,2,3-triazol-4-yl, 1,2,3-triazol-5-yl, 1,3,4-triazol-2-yl, 2-pyridinyl, 3-pyridinyl, 4-pyridinyl, 3-pyridazinyl, 4-pyridazinyl, 2-pyrimidinyl, 4-pyrimidinyl, 5-pyrimidinyl, 2-pyrazinyl, 1-benzofuran-2-yl, 1-benzofuran-5-yl, 1-benzofuran-6-yl, 2-benzofuran-5-yl, 2-benzofuran-6-yl, 2H-chromen-3-yl, 2H-chromen-6-yl, 2H-chromen-7-yl, indol-2-yl, 1-benzothiophen-2-yl or benzimidazol-2-yl, the stated groups being unsubstituted or substituted by one or more groups $X^1$;

$D^2$ is identically or differently phenylene-1,4-diyl, phenylene-1,3-diyl, biphenyl-4,4'-diyl, naphthalene-1,4-diyl, trans-cyclohexane-1,4-diyl, furan-2,5-diyl, thiophene-2,4-diyl, thiophene-2,5-diyl, 1H(1,2,3)-triazole-1,4-diyl or pyrrole-2,5-diyl, pyridine-2,5-diyl, pyridine-2,4-diyl, pyridazine-3,6-diyl, pyrimidine-2,5-diyl, pyrimidine-2,6-diyl, pyrazine-2,5-diyl or tetrazine-3,5-diyl, the stated groups being unsubstituted or substituted by one or one group $X^1$;

$X^1$ is identically or differently F, Cl, Br, I, cyano, nitro, hydroxyl, amino, carboxyl, azido, $SO_3M$, $SO_2NH_2$, alkyl, haloalkyl, alkyloxy, haloalkyloxy, alkylamino, dialkylamino, trialkylamino, alkylcarbonyl, alkylsulphoxyl, aminocarbonyl, alkylaminocarbonyl, alkylcarbonyl-amino, oxo (=0), the alkyl groups in these radicals 1 to 6 carbon atoms;

$Y^1$, $Y^2$ are identically or differently ~$CH_2$-, ~C(O)-, ~$CH_2$-C(O)-, ~$CH_2CH_2C(O)$-, ~CH=CH-C(O)-, ~C≡C-C(O)-, ~NH-C(O)-, ~$CH_2$-$NR^x$-3-cyclobutene-1,2-$Y^1$, $Y^2$ dione-4-, ~$S(O)_2$- or ~$CH_2S(O)_2$- where denotes the bond to the group A, or form, independently of one another, together with the nitrogen to which they are bonded, and with the group $R^8$, a group ~4-1H-(1,2,3)triazol-1-yl- where, ~ denotes thebond to the group A;

$Y^3$ is a bond, O, $S(O_2)$, $CH_2$ or C(O);

m is 0, 1 or 2;

$Z^1$ is 0;

D is a group $Z^2$-$T^1$-$Y^4$ -$A^3$-$Y^5$-$T^2$-$Z^2$;

$Z^2$ is identically 0, S, ~4-1H-(1,2,3)triazol-1-yl-, -NH-C(O)~ or $CH_2$, where ~ denotes the bond to the group T;

$T^1$, $T^2$ are identically or differently a straight-chain or branched alkanediyl group having 4 to 20 C atoms, where

(i) optionally one or more non-terminal $CH_2$ groups are replaced by -NH-, -S-, -S(O)-and/or -$S(O)_2$-, and/or
(ii) optionally one or more H atoms are replaced by fluoro, chloro, (=O), carboxyl, $NH_2$, $NHR^y$ or $NHR^z$, and/or
(iv) optionally a non-terminal -$CH_2CH_2CH_2$-group is replaced by -4-1H-(1,2,3)triazol-1-yl-;

$Y^4$, $Y^5$ are identically or differently ~$NR^x$-C(O)-, ~C(O)-$NR^x$-, ~$NR^x$-$S(O)_2$, ~$NR^x$-, ~$CH_2NR^x$, ~$NR_xC(O)NR^x$-, ~$CH_2NR^xC(O)$-, ~$CH_2C(O)NR^x$- or ~$S(O)_2$-$NR^x$-, where ~ denotes the bond to group A;
$A^3$ is

a) $C_1$-$C_6$ alkanediyl, $C_2$-$C_4$ alkynediyl, one or more H atoms in the stated groups being optionally replaced by F or Cl, or
b) a group $A^4$;

$A^4$ is identically phenylene-1,4-diyl, biphenyl-4,4'-diyl, naphthalene-1,4-diyl, trans-cyclohexane-1,4-diyl, thiophene-2,5-diyl or 1H-(1,2,3)triazole-1,4-diyl, pyridine-2,5-diyl, pyridine-2,4-diyl, pyridazine-2,5-diyl, pyrimidine-2,5-diyl, pyrimidine-2,4-diyl, pyrazine-2,5-diyl or tetrazine-2,5-diyl, the stated radicals being unsubstituted or substituted by a group $X^2$;
$X^2$ is identically or differently fluoro, chloro, bromo, nitro, $SO_3M$, alkyl, haloalkyl, alkyloxy, haloalkyloxy, alkylamino, dialkylamino, trialkylamino, alkylcarbonyl, alkylsulphoxyl, alkylcarbonyloxy, aminocarbonyl, alkylaminocarbonyl, alkylcarbonylamino, the alkyl groups in these radicals containing 1 to 4 carbon atoms, and the groups haloalkyl and haloalkyloxy containing the halogens F and/or Cl;
$R^1$ is identical and is C(O)OM or C(O)NOM;
$R^2$, $R^3$ are identically H;
$R^4$, $R^7$ are identically OH or $OR^z$;
$R^6$ is identically H or $R^z$;
$R^5$ is identically $R^x$, $C(O)CH_2OH$ or C(O)-haloalkyl;
$R^8$ is identically or differently $R^x$;
M is H, methyl, ethyl or a cation from the group Li, Na, K, Ca, Mg and optionally substituted ammonium;
$R^x$ is identically or differently H, $R^y$ or $R^z$;
$R^y$ is identically or differently methyl, ethyl, 1-methylethyl, propyl, 1-methylpropyl, 1,1-dimethylpropyl or benzyl;
$R^z$ is identically or differently methylcarbonyl, ethylcarbonyl, propylcarbonyl, 1-methylethylcarbonyl, 1,1-dimethylethylcarbonyl or phenylcarbonyl,

and also its pharmacologically tolerated salts.

**5.** Sialic acid derivatives according to any of Claims 1 to 4, where the symbols and indices in the formula (I) have the following definitions:

$A^1$, $A^2$ are identically a group $D^1$-[$Y^3$-$D^2$-]$_m$-;
$D^1$ is identically phenyl, 2-naphthyl, cyclohexyl, 2-fluorenyl, 2-furanyl, 2-benzothiophenyl, 2-thienyl, 3-thienyl, 2-pyridinyl, 3-pyridinyl, 4-pyridinyl, 3-pyridazinyl, 4-pyridazinyl, 2-pyrimidinyl, 4-pyrimidinyl, 5-pyrimidinyl or 2-pyrazinyl,

the stated groups being unsubstituted or substituted by a group $X^1$;

$D^2$ is identically phenylene-1,4-diyl, naphthalene-1,4-diyl, thiophene-2,5-diyl, pyridine-2,5-diyl, pyridazine-3,6-diyl, pyrimidine-2,5-diyl or pyrazine-2,5-diyl,

the stated groups being unsubstituted or substituted by a group $X^1$;

$X^1$ is F, Cl, Br, nitro, hydroxyl, carboxyl, alkyl, haloalkyl, alkyloxy, haloalkyloxy, alkylamino, dialkylamino, trialkylamino, alkylcarbonyl, alkylsulphoxyl, aminocarbonyl, alkylaminocarbonyl, alkylcarbonylamino, the alkyl groups in these radicals containing 1 to 4 carbon atoms;
$Y^1$, $Y^2$ are identically ~$CH_2$-, ~C(O)-, ~$CH_2$-C(O)-, ~C≡C-C(O)-, ~CH=CH-C(O)-, ~$CH_2$-$NR^x$-3-cyclobutene-1,2-dione-4- or ~$S(O)_2$-, where ~ denotes the bond to the group A, or
$Y^1$, $Y^2$ form, independently of one another, together with the nitrogen to which they are bonded, and with the group $R^8$, a group ~4-1H-(1,2,3)triazole-1-yl-, where ~ denotes the bond to the group A;
$Y^3$ is a bond or 0;
m is 0 or 1;
$Z^1$ is 0;
D is a group $Z^2$-$T^1$-$Y^4$ -$A^3$-$Y^5$-$T^2$-$Z^2$;
$Z^2$ is identically O, ~4-1H-(1,2,3)triazol-1-yl-, -NH-C(O)~ or $CH_2$, where ~ denotes the bond to the group T;
$T^1$, $T^2$ are identically a straight-chain alkanediyl group having 4 to 10 C atoms, where

(i) optionally one or more non-terminal $CH_2$ groups are replaced by -S- and/or -NH-, and/or
(ii) optionally one or more H atoms are replaced by F, Cl or (=O), carboxyl or $NH_2$;

$Y^4$, $Y^5$ are identically ~$NR^x$-C(O)-, ~$NR^x$-, ~$CH_2NR^x$-, ~$NR^xC(O)NR^x$-, ~$CH_2NR^xC(O)$-, ~$CH_2C(O)NR^x$- or ~C(O)-$NR^x$-, where ~ denotes the bond to group A;
$A^3$ is

a) $C_1$-$C_6$ alkanediyl, one or more H atoms in the stated groups being optionally replaced by F or Cl, or
b) a group $A^4$;

$A^4$ is identically phenylene-1,4-diyl, biphenyl-4,4'-diyl, naphthalene-1,4-diyl, pyridine-2,5-diyl, pyridazine-2,5-diyl, pyrimidine-2,5-diyl, pyrazine-2,5-diyl, tetrazine-2,5-diyl, ethyne-1,2-diyl, thiophene-2,5-diyl, cyclohexane-1,4-diyl or -3-cyclobutene-1,2-dione-4-; the stated radicals being unsubstituted or substituted by a group $X^2$;
$X^2$ is identically a fluoro, chloro, bromo, nitro, $SO_3M$, alkyl, haloalkyl, alkyloxy, haloalkyloxy, alkylamino, dialkylamino, trialkylamino, alkylsulphoxyl, alkylcarbonyloxy, the alkyl groups in these radicals containing 1 to 4 carbon atoms, and the groups haloalkyl and haloalkyloxy containing the halogens F and/or Cl;
$R^1$ is identical and is C(O)OM;
$R^2$, $R^3$ are identically H;
$R^4$, $R^7$ are identically OH or $OR^z$;
$R^6$ is identically H or $R^z$;
$R^5$ is identically $R^x$, $C(O)CH_2OH$ or C(O)-haloalkyl;
$R^8$ is identically $R^x$;
M is H, methyl, ethyl or a cation from the group Li, Na and K;
$R^x$ is identically or differently H, $R^y$ or $R^z$;
$R^y$ is identically or differently methyl or ethyl;
$R^z$ is identically methylcarbonyl, ethylcarbonyl or 1-methylethylcarbonyl,

and also its pharmacologically tolerated salts.

**6.** Sialic acid derivative of formula (I) according to any of Claims 1 to 5, where the symbols and indices in the formula (I) have the following definitions:

$A^1$, $A^2$ are identically a group $D^1$-[$Y^3$-$D^2$-]$_m$-;
$D^1$ is identically phenyl, cyclohexyl, naphth-2-yl, fluoren-2-yl, furan-2-yl, benzothiophen-2-yl, thien-2-yl, thien-3-yl, pyridin-2-yl, pyridin-3-yl, pyridin-4-yl, pyridazin-3-yl, pyridazin-4-yl, pyrimidin-2-yl, pyrimidin-4-yl, pyrimidin-5-yl or pyrazin-2-yl,

the stated groups being unsubstituted or substituted by a group $X^1$;

$D^2$ is identically phenylene-1,4-diyl, naphthalene-1,4-diyl, thiophene-2,5-diyl, pyridine-2,5-diyl, pyridazine-3,6-diyl, pyrimidine-2,5-diyl or pyrazine-2,5-diyl,

the stated groups being unsubstituted or substituted by a group $X^1$;

$X^1$ is identically F, Cl, nitro, hydroxy, carboxyl, alkyl, haloalkyl, alkyloxy, haloalkyloxy, alkylamino, dialkylamino, trialkylamino, alkylsulphoxyl, aminocarbonyl, alkylaminocarbonyl, alkylcarbonylamino, the alkyl groups in these radicals containing 1 to 3 carbon atoms;
$Y^1$, $Y^2$ are identically ~$CH_2$-, ~C(O)-, ~$CH_2$-C(O)-, ~C≡C-C(O)-, ~CH=CH-C(O)-, ~$CH_2$-$NR^x$-3-cyclobutene-1,2-dione-4- or ~$S(O)_2$-, where ~ denotes the bond to the group A, or
$Y^1$, $Y^2$ form, independently of one another, together with the nitrogen to which they are bonded, and with the group $R^8$, a group ~4-1H-(1,2,3)triazol-1-yl-, where ~ denotes the bond to the group A;
$Y^3$ is a bond;
m is 0 or 1;
$Z^1$ is 0;
D is a group $Z^2$-$T^1$-$Y^4$ -$A^3$-$Y^5$-$T^2$-$Z^2$;
$Z^2$ is identically 0, $CH_2$ or ~4-1H-(1,2,3)triazol-1-yl-, where ~ denotes the bond to the group T;
$T^1$, $T^2$ are identically or differently a straight-chain alkanediyl group having 4 to 6 C atoms, where

(i) optionally one or more non-terminal $CH_2$ groups are replaced by -S-, and/or NH, and/or
(ii) optionally one or more H atoms are replaced by F, Cl, (=O), carboxyl or $NH_2$;

$Y^4$, $Y^5$ are identically or differently ~C(O)-$NR^x$-, ~$NR^x$-, ~$CH_2NR^x$-, ~NHC(O)NH-, ~$CH_2$NHC(O)-, ~$CH_2$C(O)NH- or ~$NR^x$-C(O)-, where ~ denotes the bond to group A;
$A^3$ is

a) $C_1$-$C_6$ alkanediyl, one or more H atoms in the stated groups being optionally replaced by F or Cl, or
b) a group $A^4$;

$A^4$ is identically phenylene-1,4-diyl, biphenyl-4,4'-diyl, naphthalene-1,4-diyl, pyridine-2,5-diyl, pyridazine-2,5-diyl, pyrimidine-2,5-diyl, pyrazine-2,5-diyl, tetrazine-2,5-diyl, ethyne-1,2-diyl, thiophene-2,5-diyl, cyclohexane-1,4-diyl or -3-cyclobutene-1,2-dione-4-; the stated radicals being unsubstituted or substituted by a group $X^2$;
$X^2$ is identically fluoro, chloro, bromo, nitro, $SO_3M$, alkyl, haloalkyl, alkyloxy, haloalkyloxy, alkylamino, dialkylamino, trialkylamino, alkylsulphoxyl, alkylcarbonyloxy, the alkyl groups in these radicals containing 1 to 3 carbon atoms, and the groups haloalkyl and haloalkyloxy containing the halogens F and/or Cl;
$R^1$ is C (O) OM;
$R^2$, $R^3$ are identically H;
$R^4$, $R^7$ are identically OH or $OR^z$;
$R^6$ is identically H or $R^z$;
$R^5$ is identically $R^x$, C(O)$CH_2$OH or C(O)-haloalkyl;
$R^8$ is identically H;
M is H, methyl or a cation from the group Li, Na and K;
$R^x$ is identically or differently H, $R^y$ or $R^z$;
$R^y$ is identically or differently methyl or ethyl;
$R^z$ is identically methylcarbonyl, ethylcarbonyl or 1-methylethylcarbonyl,

and also its pharmacologically tolerated salts.

**7.** Sialic acid derivative according to any of Claims 1 to 6, where the symbols and indices in the formula (I) have the following definitions:

$A^1$, $A^2$ are identically a group $D^1$-[$Y^3$-$D^2$-]$_m$;
$D^1$ is identically phenyl, pyridin-2-yl, fluoren-2-yl, cyclohexyl, naphth-2-yl, benzothiophen-2-yl, furan-2-yl, thien-2-yl or thien-3-yl, the stated groups being unsubstituted or substituted by a group $X^1$;
$D^2$ is identically phenylene-1,4-diyl or thiophene-2,5-diyl, the stated groups being unsubstituted or substituted by a group $X^1$;
$X^1$ is identically F, chloro, nitro, hydroxy, carboxyl, methyl, trifluoromethyl, methyloxy;
$Y^1$, $Y^2$ are identically ~C(O)-, ~$CH_2$-, ~C≡C-C(O)-, ~CH=CH-C(O)-, ~$S(O)_2$-, ~CH2-NH-3-cyclobutene-1,2-dione-4- or ~$CH_2$-C(O)-, where ~ denotes the bond to the group A, or
$Y^1$, $Y^2$ form, independently of one another, together with the nitrogen to which they are bonded, and with the group $R^8$, a group ~4-1H-(1,2,3)triazol-1-yl-, where ~ denotes the bond to the group A;
$Y^3$ is a bond;
m is 0 or 1;
$Z^1$ is 0;
D is a group $Z^2$-$T^1$-$Y^4$ -$A^3$-$Y^5$-$T^2$-$Z^2$;
$Z^2$ is identically O, $CH_2$, ~CONH- or ~4-1H-(1,2,3)triazol-1-yl-, where ~ denotes the bond to the group T;
$T^1$, $T^2$ are identically or differently ~$CH_2CH_2CH_2SCH_2CH_2$-, ~$CH_2CH_2CH_2S(O)CH_2CH_2$-, ~$CH_2$C(O)NH$CH_2CH_2CH_2$-, ~$CH_2$-4-1H-(1,2,3)triazole-1-$CH_2CH_2$-, butane-1,4-diyl, pentane-1,5-diyl or hexane-1,6-diyl, where ~ denotes the bond to the group $Z^2$;
$Y^4$, $Y^5$ are identically ~C(O)-NH-, ~NH-, ~$CH_2$NH-, ~NHC(O)NH-, ~$CH_2$NHC(O)-, ~$CH_2$C(O)NH- or ~NH-C(O)-, where ~ denotes the bond to group A;
$A^3$ is methanediyl, ethane-1,2-diyl, butane-1,4-diyl, hexane-1,6-diyl, -3-cyclobutene-1,2-dione-4-, thiophene-2,5-diyl, cyclohexane-1,4-diyl, phenylene-1,4-diyl, naphthalene-1,4-diyl, ethyne-1,2-diyl, biphenyl-4,4'-diyl or pyridine-2,5-diyl, the stated radicals being unsubstituted or substituted by one or more groups $X^2$;
$X^2$ is identically bromo, nitro, methyl or pentyloxy;
$R^1$ is C(O)ONa;
$R^2$, $R^3$ are identically H;

$R^4$, $R^7$ are identically OH;
$R^6$ is identically H;
$R^5$ is identically $C(O)CH_3$, $C(O)CH_2F$ or $C(O)CH_2OH$;
$R^8$ is identically H,

and also its pharmacologically tolerated salts.

**8.** Sialic acid derivatives according to any of Claims 1 to 7, having the following structural formulae

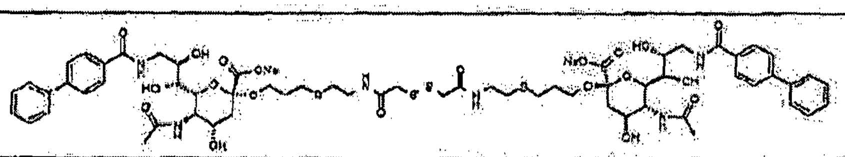

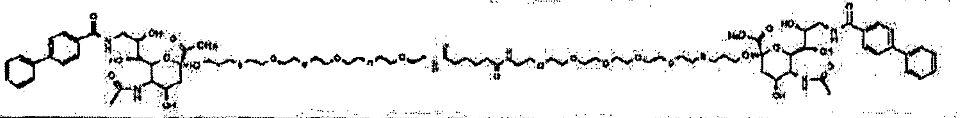

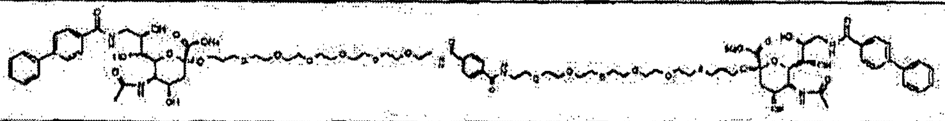

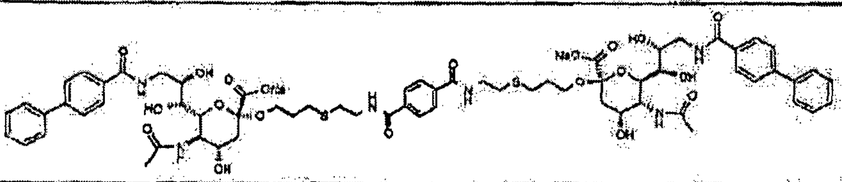

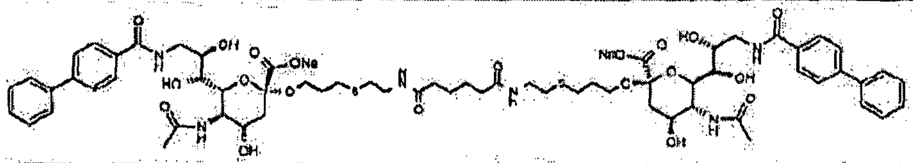

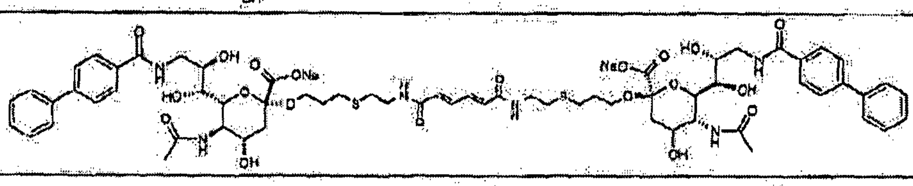

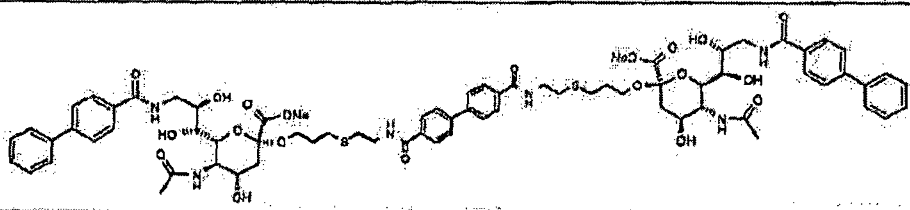

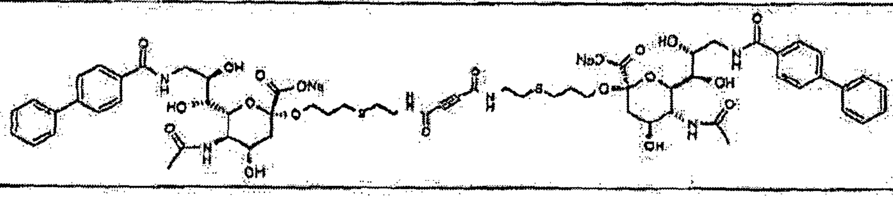

| |
|---|
| 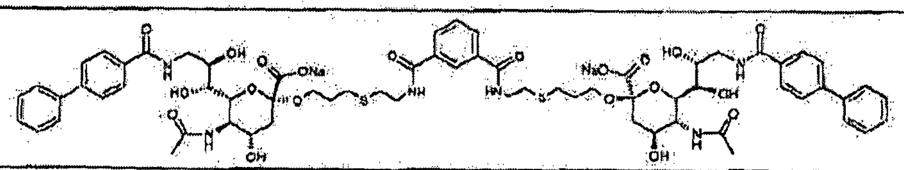 |
| 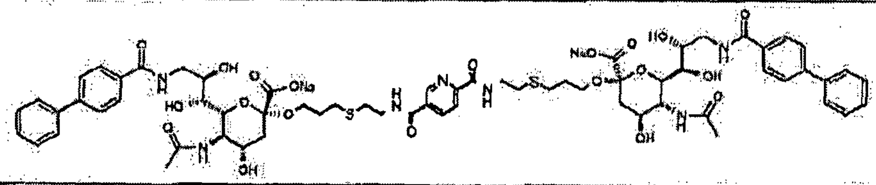 |
| 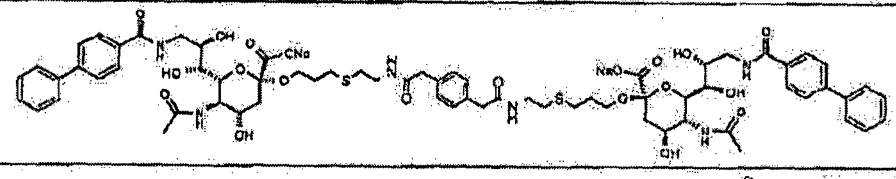 |
| 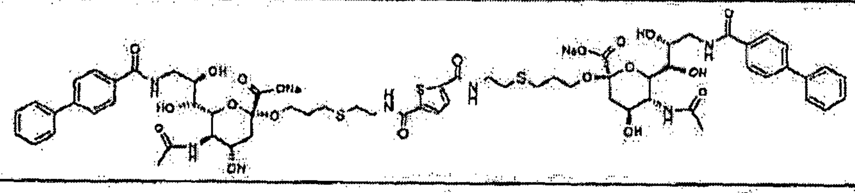 |
| 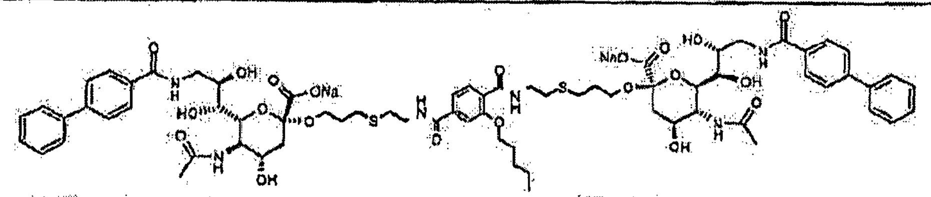 |
| 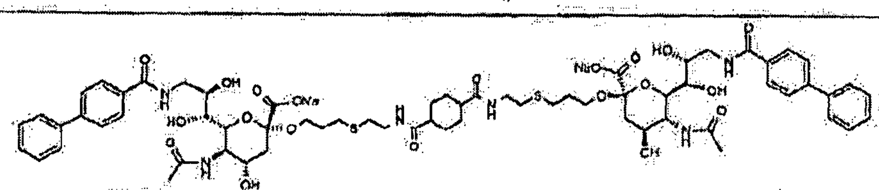 |
| 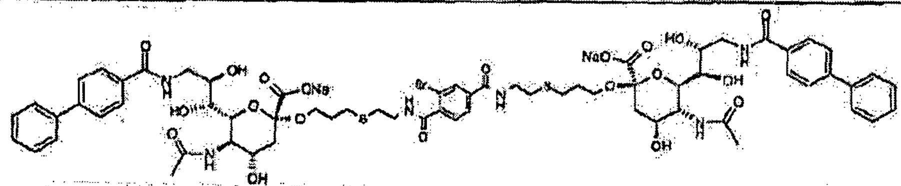 |
| 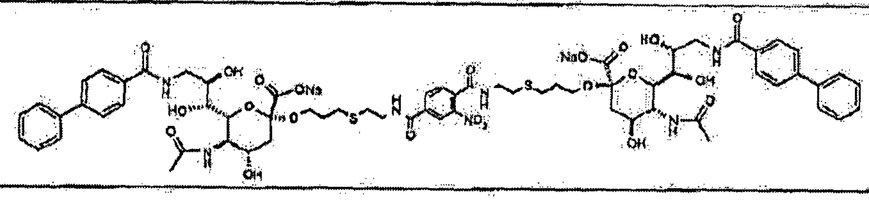 |

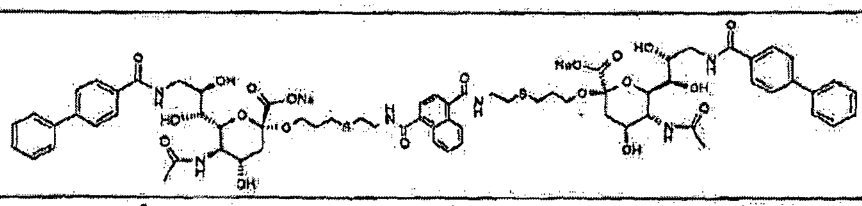

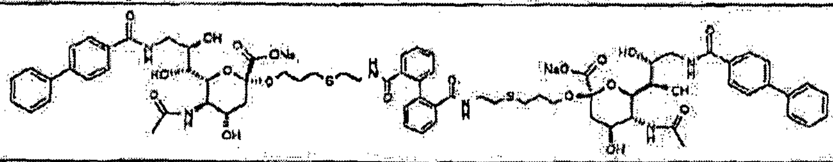

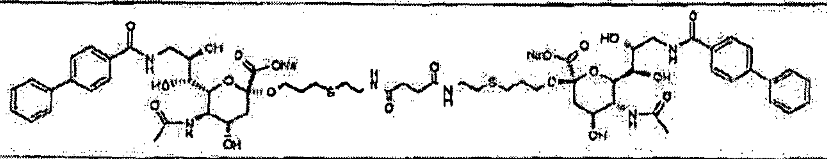

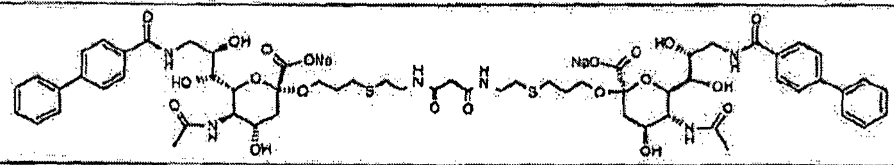

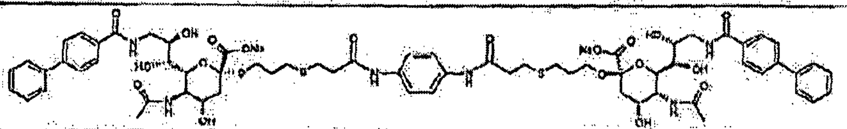

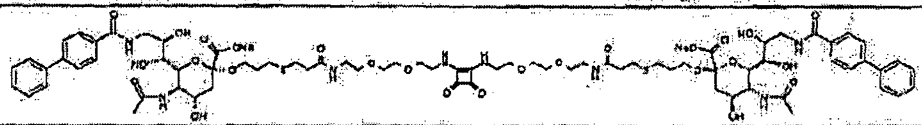

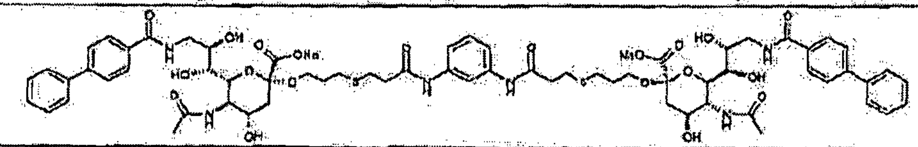

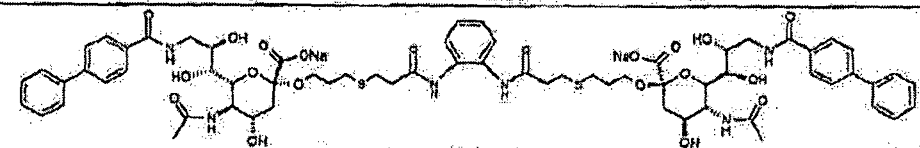

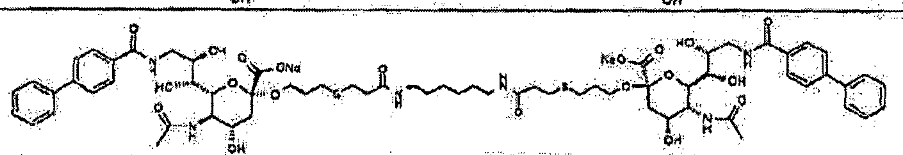

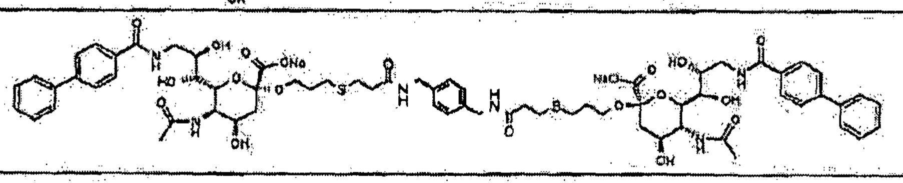

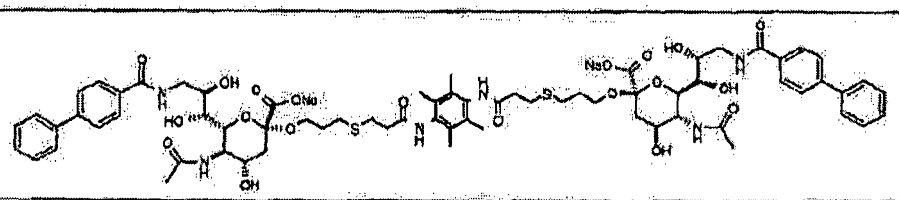

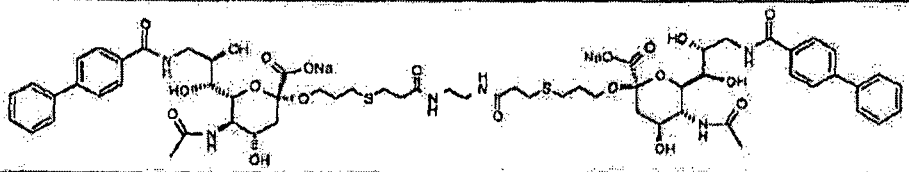

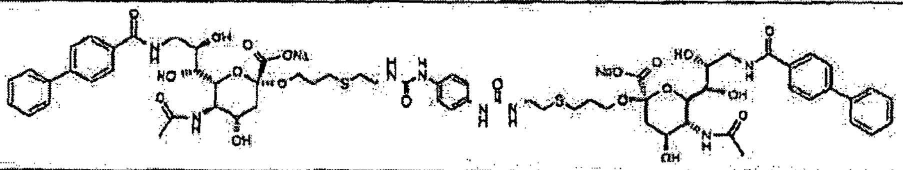

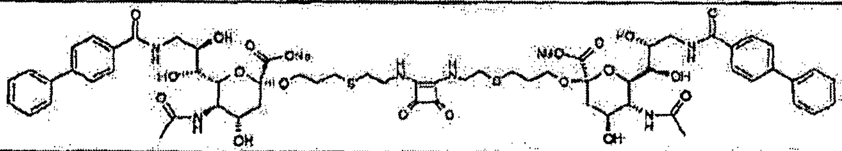

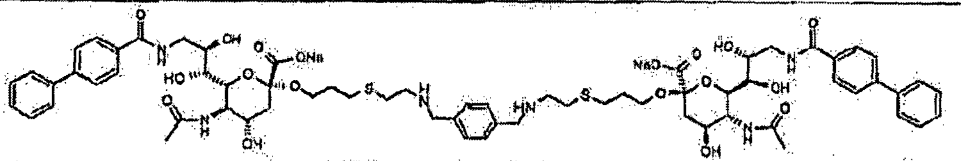

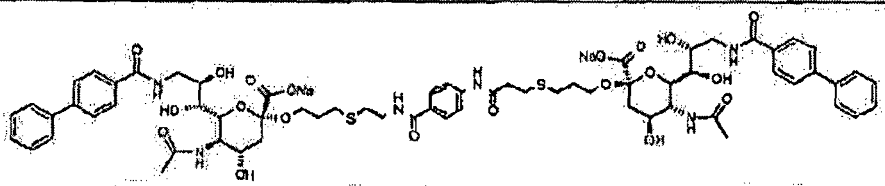

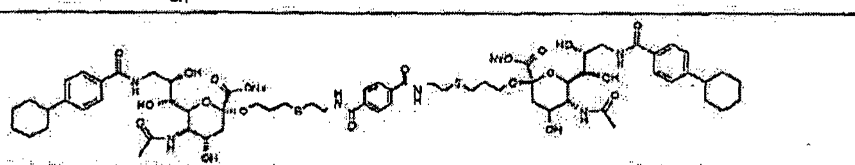

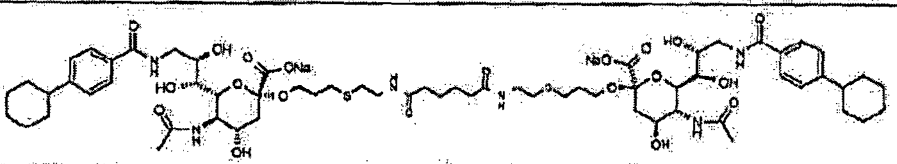

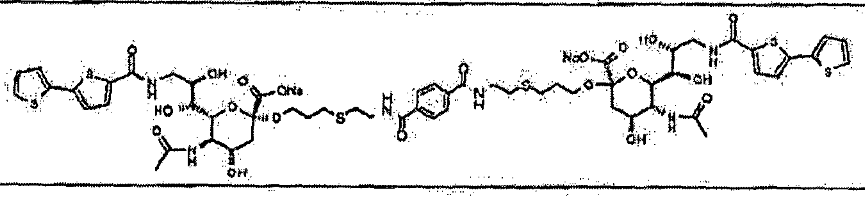

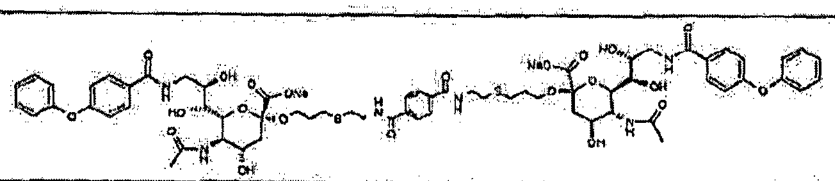

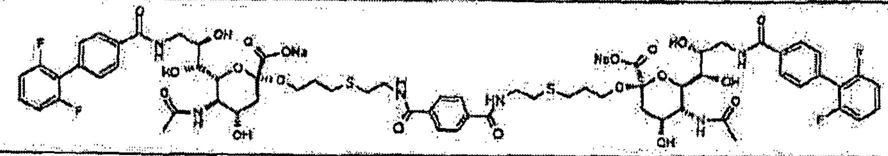

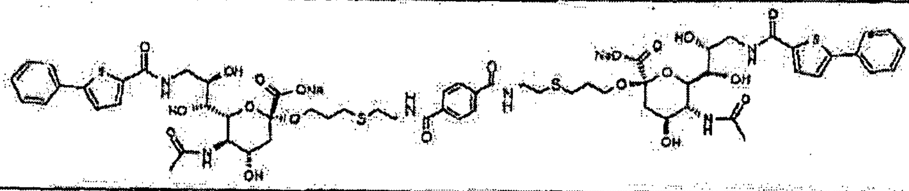

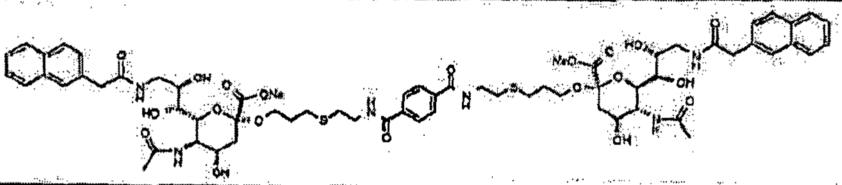

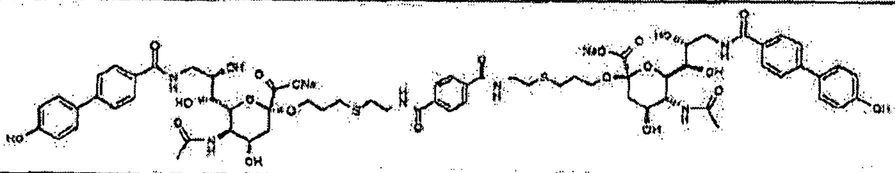

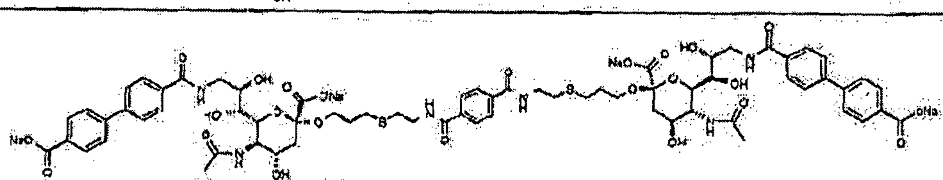

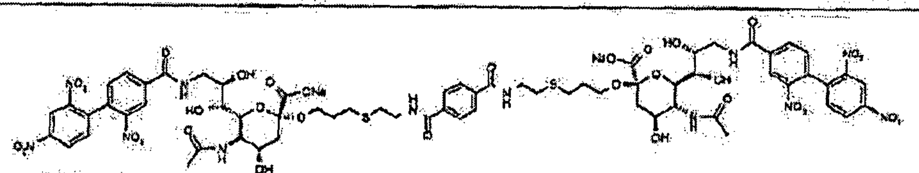

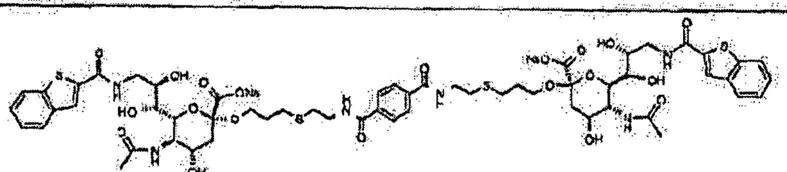

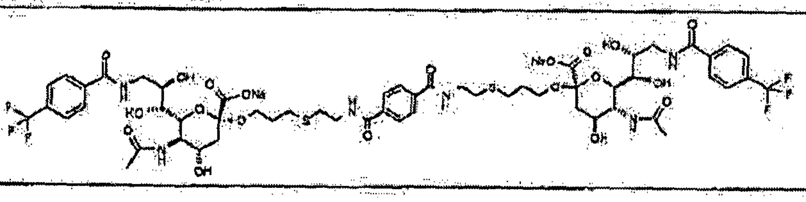

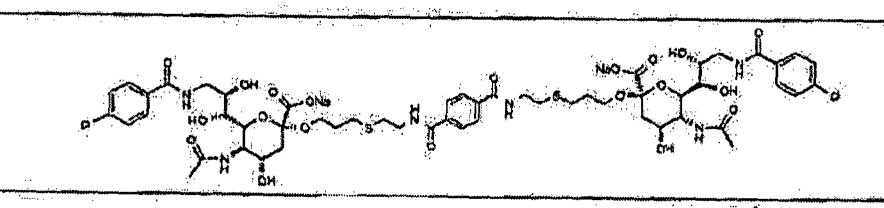

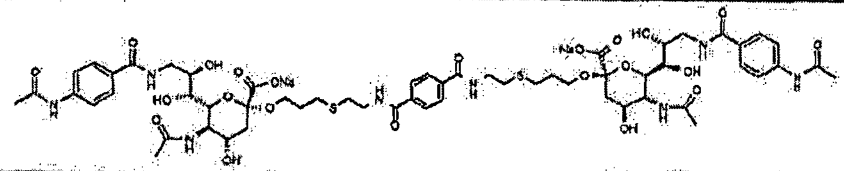

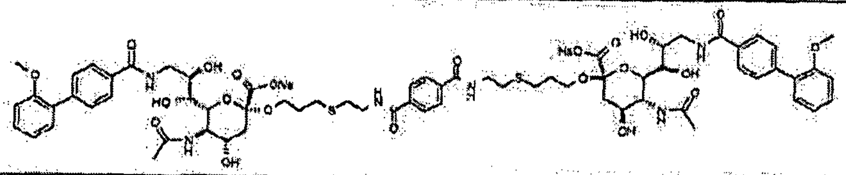

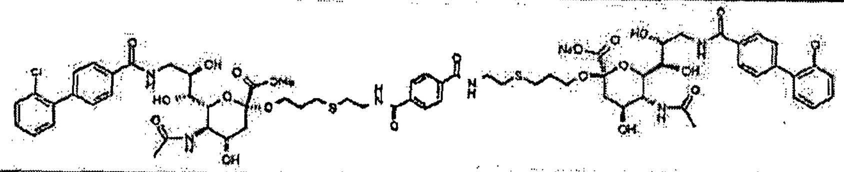

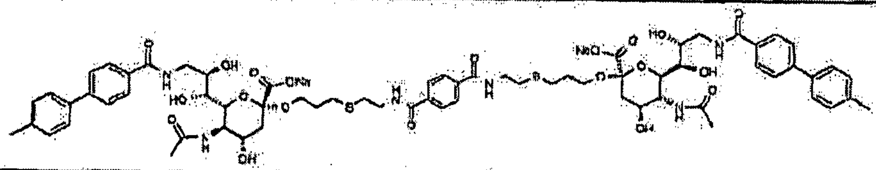

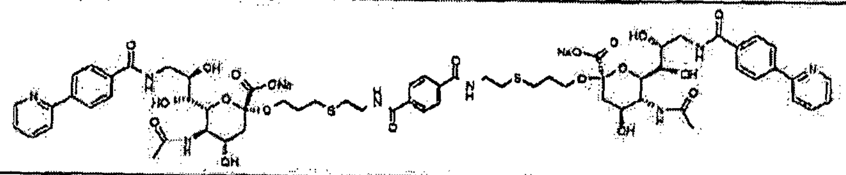

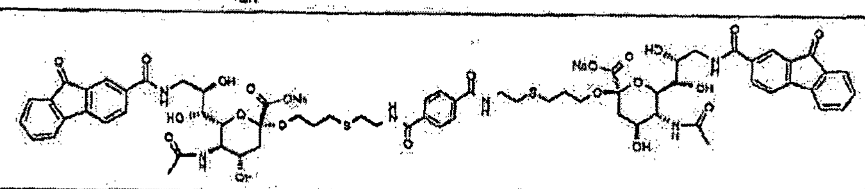

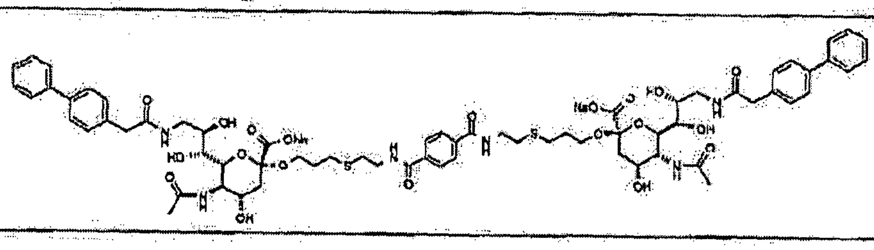

| |
|---|
| 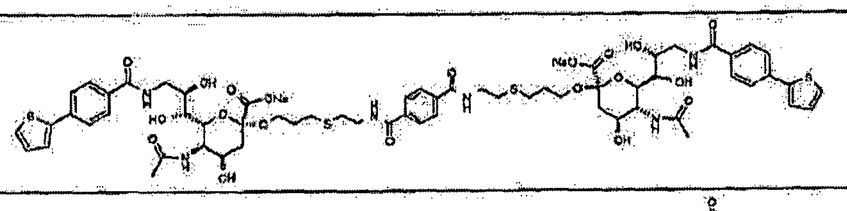 |
| 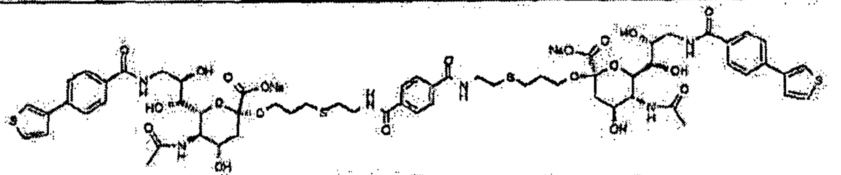 |
| 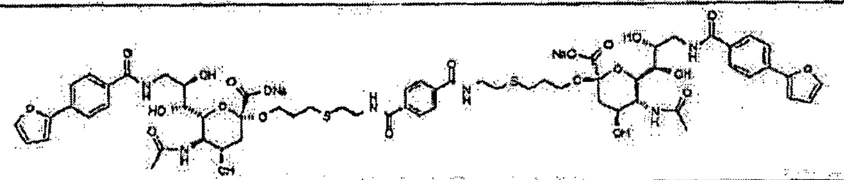 |
| 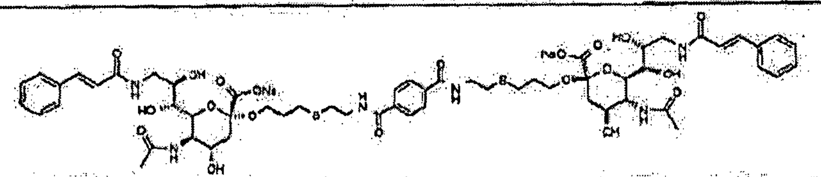 |
| 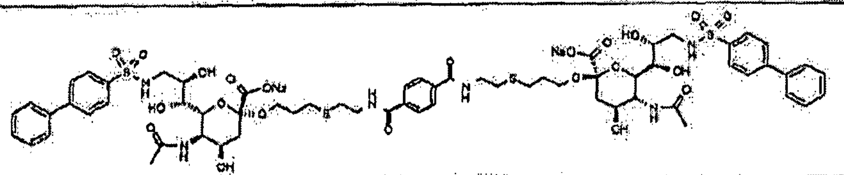 |
| 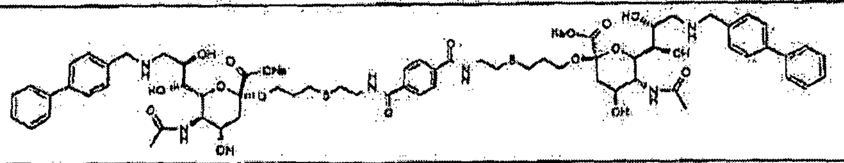 |
| 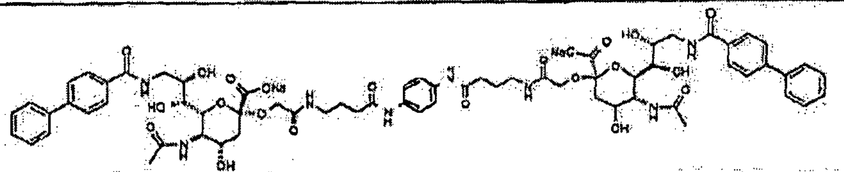 |
| 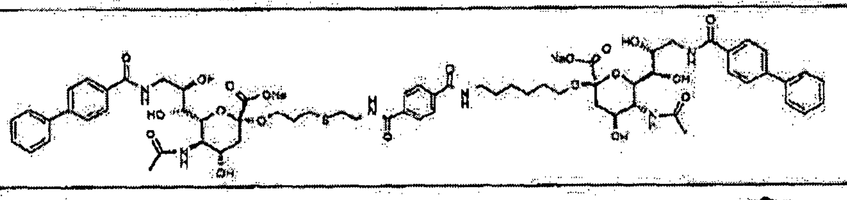 |
| 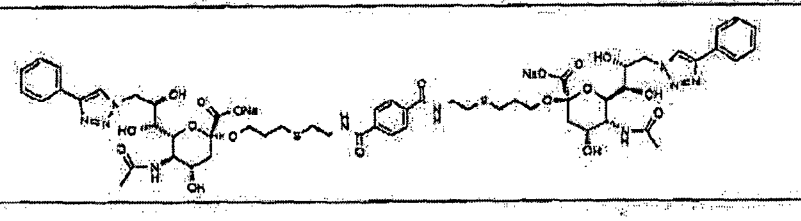 |

| |
|---|
| 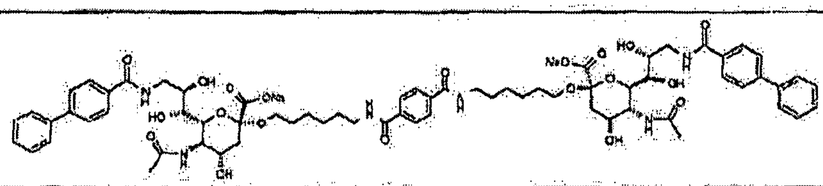 |
| 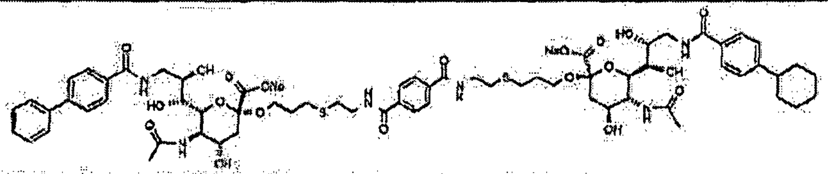 |
| 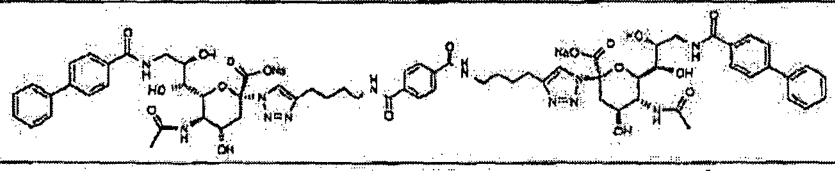 |
| 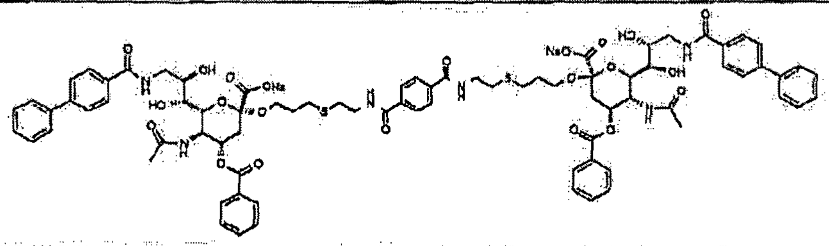 |
| 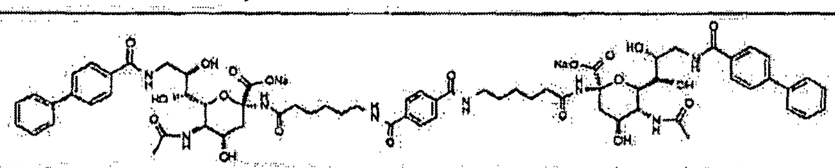 |
| 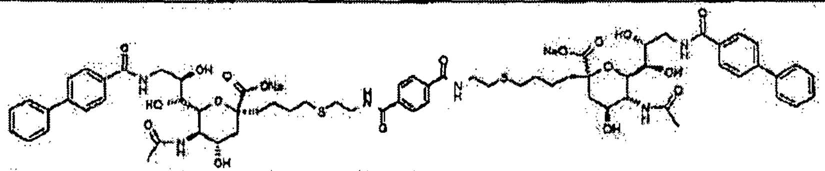 |
| 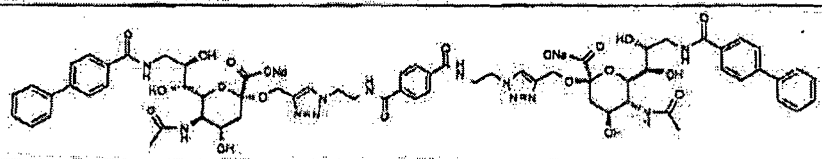 |
| 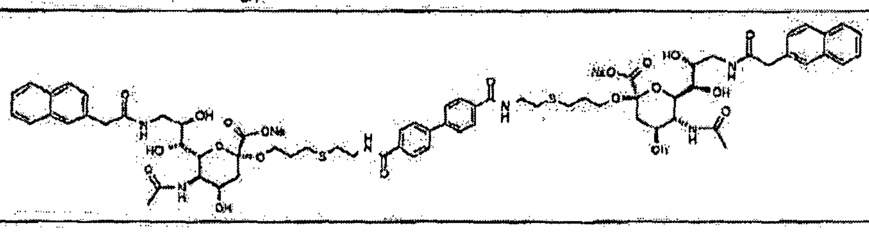 |

| |
|---|
| 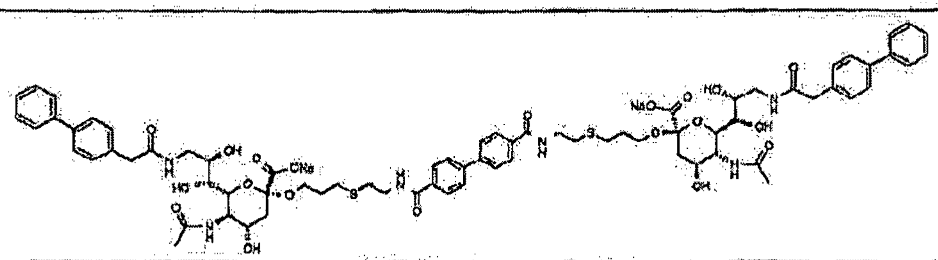 |
| 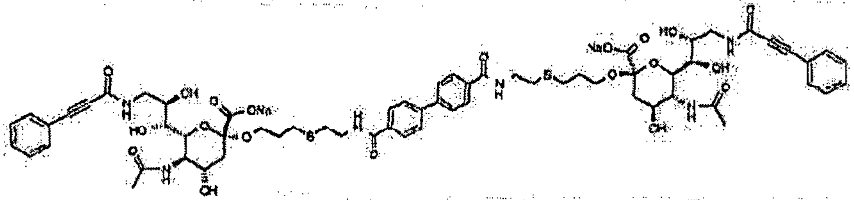 |
| 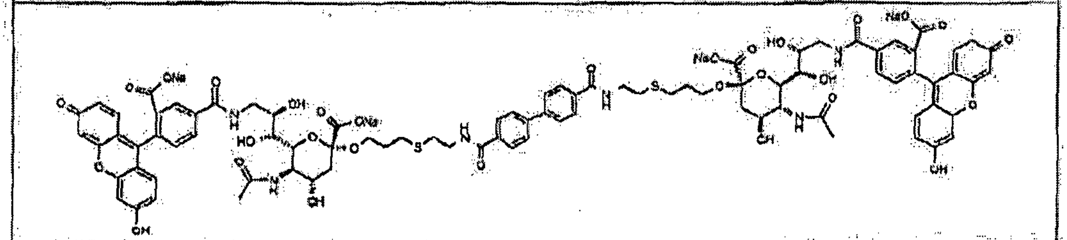 |
| 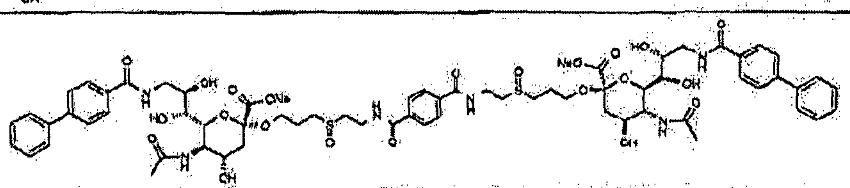 |
| 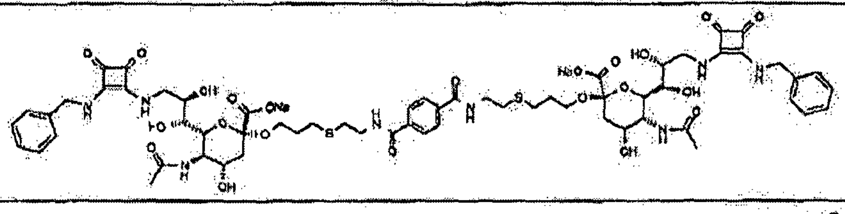 |
| 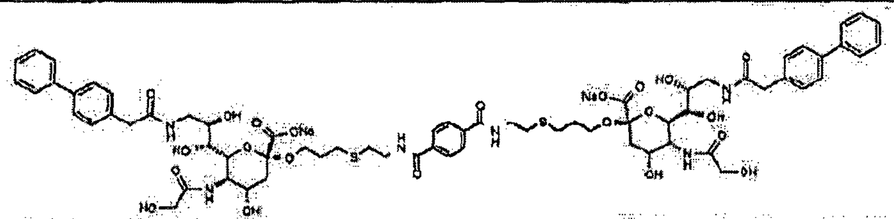 |
| 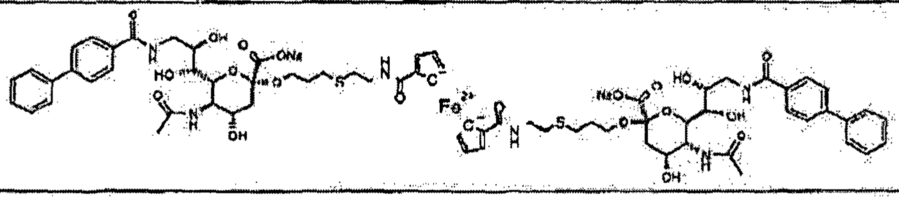 |

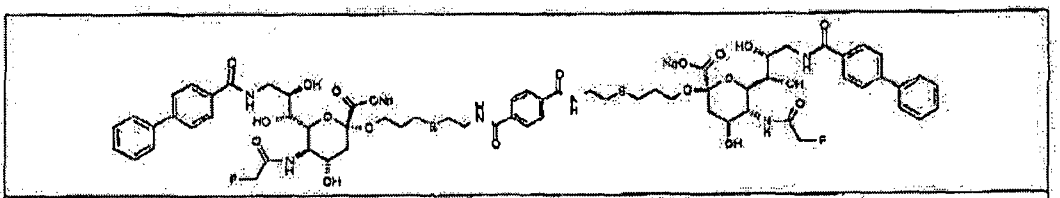

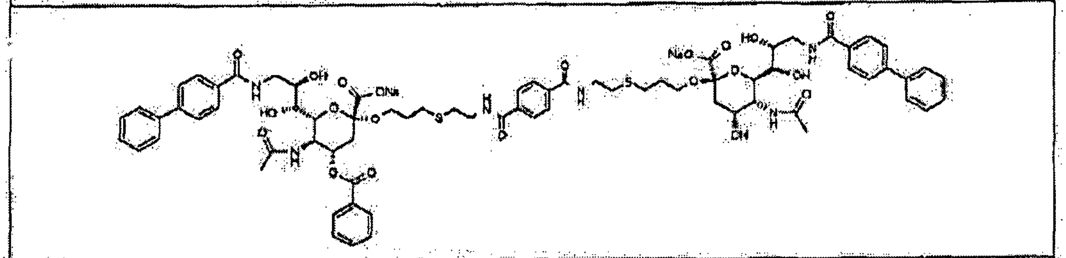

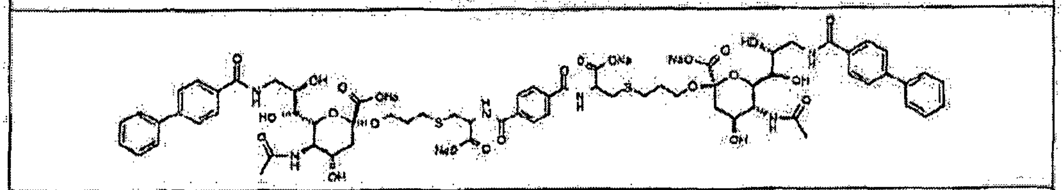

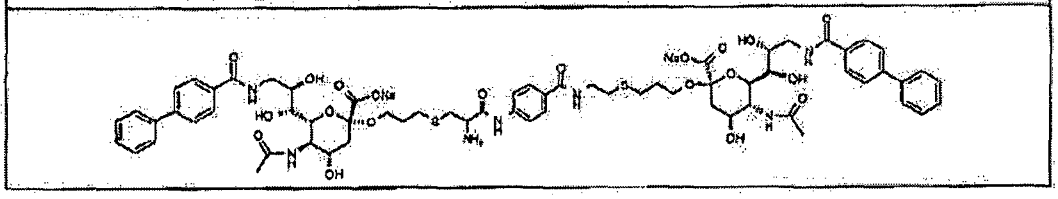

and also its pharmacologically tolerated salts.

9. Pharmaceutical preparation comprising at least one sialic acid derivative of the formula (I) according to any of Claims 1 to 8, or a pharmacologically tolerated salt thereof, and a pharmacologically tolerated carrier.

10. Sialic acid derivative of the formula (I) according to any of Claims 1 to 8, or a pharmacologically tolerated salt thereof, as medicament.

11. Sialic acid derivative of the formula (I) according to any of Claims 1 to 8, or a pharmacologically tolerated salt thereof, for the treatment of bacterial diseases.

12. Sialic acid derivative of the formula (I) according to any of Claims 1 to 8 for use in producing a medicament for the treatment of bacterial, viral, parasitic, autoimmune and immune-deficiency diseases.

**Revendications**

1. Dérivé d’acide sialique de formule (I),

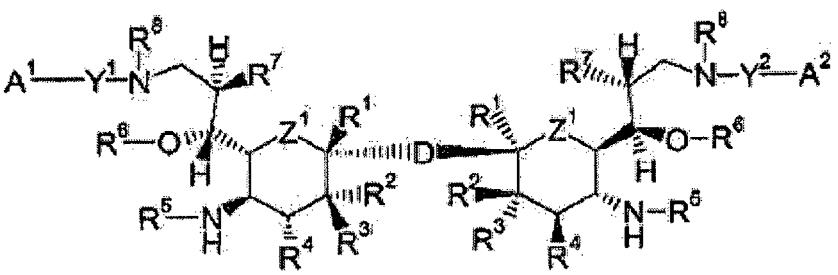

(I)

dans laquelle les symboles ont les significations suivantes :

$A^1$, $A^2$ identiques ou différents, représentent un groupe D-[$Y^3$-$D^2$-]$_m$- ;

$D^1$, $D^2$ identiques ou différents, représentent un radical hydrocarboné en $C_3$-$C_{14}$ mono- ou polycyclique aromatique, partiellement insaturé ou saturé ou un radical hétérocyclique à trois à huit chaînons aromatique, partiellement insaturé ou saturé, les radicaux nommés étant non substitués ou une ou plusieurs fois substitués par un groupe $X^1$ ;

$X^1$ est, le même ou différent, un atome d'halogène un groupe cyano, nitro, hydroxy, mercapto, amino, carboxy, hydroxylamino, azido, $B(OH)_2$, SO, $SO_3M$, $OSO_3M$, $SO_2NH_2$, $SO_2CF_3$, $PO_3M$, $OPO_3M$, cyanométhyle, alkyle, halogénoalkyle, alcényle, alcynyle, alkyloxy, halogénoalkyloxy, alcényloxy, alcynyloxy, alkylthio, alkylamino, dialkylamino, trialkylamino, formyle, alkyl-carbonyle, alkylsulfonyle, alkylsulfoxyle, alkylaminosulfoxyle, dialkylaminosulfoxyle, alkyloxycarbonyle, alkylcarbonyloxy, amino-carbonyle, aminothiocarbonyle, alkylamino-carbonyle, alkylcarbonylamino, dialkylamino-carbonyle, alkylaminothiocarbonyle, oxo (=0), thioxo (=S), alkyl($C_1$-$C_8$)imino (=N-alkyle($C_1$-$C_8$)), alkyl($C_1$-$C_8$)oximino (=N-O-alkyle($C_1$-$C_8$)), les groupes alkyle dans ces radicaux contenant de 1 à 8 atomes de carbone et lesdits groupes alcényle ou alcynyle dans ces radicaux contenant de 2 à 8 atomes de carbone ;

$Y^1$, $Y^2$ identiques ou différents, représentent un groupe ~(alkyl($C_1$-$C_4$))-, ~(halogéno-alkyl($C_1$-$C_4$))-, ~(alkyl($C_1$-$C_4$))-(CO)-, ~C(O)-, ~$CH_2$-C(O)-, ~CH=CH-C(O)-, ~C≡C-C(O)-, ~$S(O)_2$-, ~$CH_2$-$S(O)_2$-, ~$NR^x$C-(O)-, ~CH($CF_3$)-, ~$NR^x$-3-cyclobutène-1,2-dione-4-, ~$CH_2$-$NR^x$-3-cyclobutène-1,2-dione-4-, ~$NR^x$-3-2,5-thiadiazole-1-oxyde-4-, ~$NR^x$-3-2,5-thiadiazole-1,1-dioxyde-4-, ~ désignant la liaison au groupe A ou

$Y^1$, $Y^2$ forment, indépendamment l'un de l'autre, conjointement avec l'atome d'azote auquel ils sont liés et le groupe $R^8$, un groupe ~4-1H-(1,2,3)triazol-1-yl-, ~5-1H-(1,2,3)triazol-1-yl-, ~$CH_2$-4-1H-(1,2,3)triazol-1-yl- ou -$CH_2$-5-1H-(1,2,3)triazol-1-yl-, ~ désignant la liaison au groupe A ;

$Y^3$ est une liaison, O, S, S(O), $S(O)_2$, $CH_2$, C(O), $CR^x_2$ ou $NR^x$ ;

m est 0, 1 ou 2 ;

$Z^1$ est 0, S, $CH_2$ ou $NR^x$ ;

D est un groupe $Z^2$-$T^1$-$Y^4$-$A^3$-$Y^5$-$T^2$-$Z^2$

$Z^2$ le même ou différent, est -O~, -S~, -$NR^x$~, -NH-C(O)~, -$CH_2$~, -$CF_2$~, -CH(OH)~, -N($R^x$)-O~, -O-$NR^x$~, -O-N=CH-, ~4-1H-(1,2,3)triazol-1-yl-, ou ~5-1H-(1,2,3)triazol-1-yl-, ~ désignant la liaison au groupe T ;

$T^1$, $T^2$ identiques ou différents, représentent un groupe alcanediyle à chaîne droite ou ramifié, ayant de 1 à 30 atomes de carbone,

(i) éventuellement un ou plusieurs groupes $CH_2$ non terminaux étant remplacés par -O-, -S-, -S(O)-, -$S(O)_2$-, -$S^+(CH_3)$-, -$P(O_2)$- et/ou -$NR^x$- et/ou

(ii) éventuellement un ou plusieurs atomes d'hydrogène étant remplacés par F, Cl, $OR^x$, $OSO_3M$, (=O), (=S), carboxy, $NH_2$, $NHR^y$ et/ou $NHR^z$ et/ou

(iii) éventuellement un ou plusieurs groupes -$CH_2$-$CH_2$- non terminaux étant remplacés par un groupe -5-1H-(1,2,3)triazol-1-yl-, -$CR^x$=$CR^x$- et/ou -C≡C- et/ou

(iv) éventuellement un groupe -$CH_2CH_2CH_2$-étant remplacé par un groupe -4-1H-(1,2,3)triazol-1-yle et/ou -O-N=CH-et/ou

(v) éventuellement un groupe -$CH_2CH_2CH_2CH_2$-étant remplacé par un groupe phényl-1,4-diyle ;

$Y^4$, $Y^5$ identiques ou différents, représentent une liaison, O, S, $NR^x$, S(O), $S(O)_2$, C(O), ~C(O)-$NR^x$-, ~$NR^x$-C(O)-, ~C(O)-O-, ~O-C(O)-, ~$NR^x$-CO-$NR^x$-, ~$NR^x$-$S(O)_2$-, ~$S(O)_2$-$NR^x$-, ~$CH_2$-$NR^x$-C(O)-, ~$CH_2$-C(O)-$NR^x$-, ~$CH_2$-$NR^x$-, ~CH($CF_3$)-$NR^x$-, ~CH=N-O- ou ~O-N=CH-, ~ désignant la liaison au groupe A ;

$A^3$ est

a) un groupe alcane ($C_1$-$C_8$) diyle, alcène($C_2$-$C_8$)-diyle, alcyne($C_2$-$C_8$)diyle, alcadiène($C_4$-$C_8$)-diyle, dans les groupes nommés plusieurs groupes $CH_2$ étant remplacés par O, S, S(O), $S(O)_2$, $NR^x$ et/ou C(O) et dans les groupes nommés éventuellement un ou plusieurs atomes d'hydrogène étant remplacés par un groupe $X^2$,

b) un groupe $A^4$-[$Z^3$-$A^5$]$_n$,

c) le groupe 1,1'-ferrocènediyle, 1,1'-cobaltocènediyle, 1,1'-ruthénocène ou dichloroplatinediaminodiyle ;

$A^4$, $A^5$ identiques ou différents, représentent un

radical hydrocarboné mono- ou polycyclique saturé, partiellement insaturé ou aromatique, ayant de 3 à 14 atomes de carbone ou un radical hétérocyclique mono- ou polycyclique à trois à huit chaînons, aromatique, partiellement insaturé ou saturé, les groupes nommés étant éventuellement substitués chacun par un ou plu-

sieurs groupes $X^2$ ;

$Z^3$ est une liaison, O, S, S(O), $S(O)_2$, $NR^x$, C(O) ou $CR_2^x$ ;

$X^2$ le même ou différent, est un atome de fluor, chlore, brome, un groupe nitro, hydroxylamino, $B(OH_2)$, $SO_3M$, $OSO_3M$, $SO_2NH_2$, $PO_3M$, $OPO_3M$, alkyle, halogénoalkyle, alkyloxy, halogénoalkyloxy, alkylthio, alkylamino, dialkylamino, trialkylamino, alkylcarbonyle, alkylsulfonyle, alkylsulfoxyle, alkylaminosulfoxyle, dialkylaminosulfoxyle, alkyloxycarbonyle, alkylcarbonyloxy, aminocarbonyle, alkylaminocarbonyle, alkylcarbonylamino, dialkylaminocarbonyle, alkylaminothiocarbonyle, alkyl($C_1$-$C_4$)imino (=N-alkyle($C_1$-$C_4$)), alkyl($C_1$-$C_4$)oximino (=N-O-alkyle($C_1$-$C_4$)), dans ces radicaux les groupes alkyle contenant de 1 à 4 atomes de carbone et les groupes halogénoalkyle et halogénoalkyloxy contenant les halogènes F et/ou Cl ;

n est 0, 1 ;

$R^1$ le même, représente C(O)OM, O-$PO_3M_2$, O-$SO_3M$, $SO_3M$, C(O)-NH-$S(O)_2$-$R^x$, $PO_3M_2$ ou C(O)NOM ;

$R^2$, $R^3$ identiques ou différents, représentent H ou F ;

$R^4$, $R^7$ identiques ou différents, représentent H, OH, $OR^z$, OC(O)$NHR^y$ ou $NR^x$ ;

$R^6$ le même ou différent, est H ou $R^z$ ;

$R^5$ le même ou différent, est H, $R^x$, Q C(O)H, C(O)$CH_2$OH ou C(O)-halogénoalkyle ;

$R^8$ le même ou différent, est $R^x$ ;

M est H, un groupe alkyle en $C_1$-$C_6$ ou un cation ;

$R^x$ le même ou différent, est H, $R^y$ ou $R^z$ ;

$R^y$ le même ou différent, est un groupe alkyle en $C_1$-$C_6$, phényle ou benzyle et

$R^z$ le même ou différent, est un groupe-C(O)-alkyle($C_1$-$C_6$), -C(O)-phényle ou -C(O)-alkyl($C_1$-$C_4$)-phényle,

dans les groupes nommés le fragment alkyle représentant des radicaux hydrocarbonés saturés, à chaine droite, ramifiés ou cycliques ayant le nombre d'atomes de carbone indiqué dans chaque cas, le fragment alcényle représentant des radicaux hydrocarbonés insaturés, à chaîne droite ou ramifiés, ayant le nombre indiqué d'atomes de carbone et comportant une ou deux doubles liaisons en position quelconque et le fragment alcynyle représentant des groupes hydrocarbonés à chaîne droite ou ramifiés ayant le nombre indiqué d'atomes de carbone et comportant une ou deux triples liaisons en position quelconque,
ainsi que ses sels pharmacologiquement acceptables.

**2.** Dérivé d'acide sialique selon la revendication 1, dans lequel les symboles dans la formule (I) ont les significations suivantes :

$A^1$, $A^2$ identiques ou différents, représentent un groupe $D^1$-[$Y^3$-$D^2$-]$_m$- ;

$D^1$ le même ou différent, est

a) un groupe aryle en $C_6$-$C_{14}$,
b) un groupe cycloalcényle en $C_3$-$C_{14}$ ou cycloalcadiényle en $C_5$-$C_{14}$,
c) un groupe cycloalkyle en $C_3$-$C_8$,
d) un groupe hétérocyclyle non aromatique, saturé ou partiellement insaturé, à 5 ou 6 chaînons, contenant un à trois atomes d'azote et/ou un atome d'oxygène ou de soufre ou un deux atomes d'oxygène et/ou de soufre,
e) un groupe hétéroaryle à 5 chaînons, contenant un à quatre atomes d'azote ou un à trois atomes d'azote et/ou un atome de soufre ou d'oxygène,
f) un groupe hétéroaryle à 6 chaînons, contenant un à trois ou un à quatre atomes d'azote,
g) des radicaux hétérocycliques polycycliques les groupes nommés étant non substitués ou substitués par un ou plusieurs groupes $X^1$ ;

$D^2$ le même ou différent, est

a) un groupe aryl($C_6$-$C_{14}$)diyle,
b) un groupe cycloalkyl($C_3$-$C_8$)diyle,
c) un groupe hétérocyclodiyle non aromatique, saturé ou partiellement insaturé, à 5 ou 6 chaînons, contenant un à trois atomes d'azote et/ou un atome d'oxygène ou de soufre ou un deux atomes d'oxygène et/ou de soufre,
d) un groupe hétéroaryldiyle à 5 chaînons, contenant un à quatre atomes d'azote ou un à trois atomes d'azote et/ou un atome de soufre ou d'oxygène,
e) un groupe hétéroaryldiyle à 6 chaînons, contenant un à trois.ou un à quatre atomes d'azote,

les groupes nommés étant non substitués ou substitués par un ou plusieurs groupes $X^1$ ;

$X^1$ est, le même ou différent, F, Cl, Br, I, un groupe cyano, nitro, hydroxy, mercapto, amino, carboxy, hydroxylamino, azido, $SO_3M$, $OSO_3M$, $SO_2NH_2$, $OPO_3M$, alkyle, halogénoalkyle, alcényle, alcynyle, alkyloxy, halogénoalkyloxy, alcényloxy, alcynyloxy, alkylthio, alkylamino, dialkylamino, trialkylamino, formyle, alkyl-carbonyle, alkylsulfonyle, alkylsulfoxyle, aminocarbonyle, alkylaminocarbonyle, alkyl-carbonylamino, oxo (=O), thioxo (=S), les groupes alkyle dans ces radicaux contenant de 1 à 8 atomes de carbone et lesdits groupes alcényle ou alcynyle dans ces radicaux contenant de 2 à 8 atomes de carbone ;
$Y^1$, $Y^2$ identiques ou différents, représentent un groupe $\sim CH_2$-, $\sim CH_2CH_2$-, $\sim CH_2CH_2CH_2$-, ~C(O)-, $\sim CH_2C(O)$-, $\sim CH_2CH_2C(O)$-, $\sim CH_2CH_2CH_2C(O)$-, ~CH=CH-C(O)-, ~C≡C-C(O)-, $\sim S(O)_2$-, $\sim CH_2S(O)_2$-, ~NH-C(O)-, $\sim NR^x$-3-cyclobutène-1,2-dione-4-, $\sim CH_2$-$NR^x$-3-cyclobutène-1,2-dione-4-, $\sim NR^x$-3-2,5-thiadiazole-1,1-dioxyde-4-, ~ désignant la liaison au groupe A ou
$Y^1$, $Y^2$ forment, indépendamment l'un de l'autre, conjointement avec l'atome d'azote auquel ils sont liés et le groupe $R^8$, un groupe ~4-1H-(1,2,3)triazol-1-yl-, -5-1H-(1,2,3)triazol-1-yl-, $\sim CH_2$-4-1H-(1,2,3)triazol-1-yl- ou $\sim CH_2$-5-1H-(1,2,3)triazol-1-yl-, ~ désignant la liaison au groupe A ;
$Y^3$ est une liaison, O, S(O), $S(O)_2$, $CH_2$, $NR^x$ ou C(O) ;
m est 0, 1 ou 2 ;
$Z^1$ est 0, S ou $CH_2$ ;
D est un groupe $Z^2$-$T^1$-$Y^4$-$A^3$-$Y^5$-$T^2$-$Z^2$ ;
$Z^2$ le même ou différent, est -O~, -S~, $-NR^x\sim$, -NH-C(O)~, $-CH_2$-, $-O-NR^x\sim$ ou ~4-1H-(1,2,3)-triazol-1-yl-, ~ désignant la liaison au groupe T ;
$T^1$, $T^2$ identiques ou différents, représentent un groupe alcanediyle à chaîne droite ou ramifié, ayant de 4 à 30 atomes de carbone,

(i) éventuellement un ou plusieurs groupes $CH_2$ non terminaux étant remplacés par ~O-, ~S-, ~S(O)-, $-S(O)_2$-, $-P(O_2)$- et/ou $-NR^x$- et/ou
(ii) éventuellement un ou plusieurs atomes d'hydrogène étant remplacés par F, Cl, $OR^X$, $-OSO_3M$, (=O), carboxy, $NH_2$, $NHR^y$ ou $NHR^z$ et/ou
(iii) éventuellement un groupe $-CH_2$-$CH_2$- non terminal étant remplacé par un groupe -5-1H-(1,2,3)triazol-1-yl- et/ou
(iv) éventuellement un groupe $-CH_2CH_2CH_2$- non terminal étant remplacé par un groupe -4-1H-(1,2,3)triazol-1-yl- et/ou -O-N=CH- ;

$Y^4$, $Y^5$ identiques ou différents, représentent O, $NR^x$, S(O), $S(O)_2$, C(O), $\sim C(O)-NR^x$-, $\sim NR^x$-C(O)-, $\sim NR^x$-CO-$NR^x$-, $\sim NR^x-S(O)_2$-, $\sim S(O)_2-NR^x$-, $\sim CH_2-NR^x$-C(O)-, $\sim CH_2$-C(O)-$NR^x$- ou $\sim CH_2-NR^x$-, ~ désignant la liaison au groupe A ;
$A^3$ est

a) un groupe alcane ($C_1$-$C_8$) diyle, alcène ($C_2$-$C_8$) - diyle, alcyne($C_2$-$C_8$)diyle, alcadiène($C_4$-$C_8$)-diyle, dans les groupes nommés éventuellement 1, 2, 3 ou 4 groupes $CH_2$ étant remplacés par 0, S(O), $S(O)_2$, $NR^x$ et/ou S et dans les groupes nommés éventuellement un ou plusieurs atomes d'hydrogène étant remplacés par F ou Cl,
b) un groupe $A^4$-[$Z^3$-$A^5$]$_n$,
c) le groupe 1,1'-ferrocènediyle, 1,1'-cobaltocènediyle, 1,1'-ruthénocène ou dichloroplatinediamonodiyle ;

$A^4$, $A^5$ identiques ou différents, représentent

a) un groupe aryl($C_6$-$C_{14}$)diyle,
b) un groupe cycloalkyl($C_3$-$C_8$)diyle,
c) un groupe hétérocyclodiyle non aromatique, saturé ou partiellement insaturé, à 5 ou 6 chaînons, contenant un à trois atomes d'azote et/ou un atome d'oxygène ou de soufre ou un deux atomes d'oxygène et/ou de soufre,
d) un groupe hétéroaryldiyle à 5 chaînons, contenant un à quatre atomes d'azote ou un à trois atomes d'azote et/ou un atome de soufre ou d'oxygène,
e) un groupe hétéroaryldiyle à 6 chaînons, contenant un à trois ou un à quatre atomes d'azote,
f) un groupe hétérocyclyle polycyclique choisi dans le groupe
les radicaux nommés étant non substitués ou substitués par un ou plusieurs groupes $X^2$ ;

$Z^3$ est une liaison, 0, S(O), $S(O)_2$ ou C(O) ;
$X^2$ le même ou différent, est un atome de fluor, chlore, un groupe nitro, un atome de brome, un groupe hydroxylamino, $SO_3M$, $OSO_3M$, $SO_2NH_2$, $PO_3M$, $OPO_3M$, alkyle, halogénoalkyle, alkyloxy, halogénoalkyloxy, alkylamino, dialkylamino, trialkylamino, alkylcarbonyle, alkylsulfonyle, alkylsulfoxyle, alkylaminosulfoxyle, dialkylaminosulfoxyle, alkyloxycarbonyle, alkylcarbonyloxy, aminocarbonyle, alkylamino-carbonyle, alkylcarbonylamino, dialkylamino-carbonyle, alkyl($C_1$-$C_4$)imino (=N-alkyle($C_1$-$C_4$)), alkyl($C_1$-$C_4$)oximino (=N-O-alkyle($C_1$-$C_4$)), dans ces radicaux les groupes alkyle contenant de 1 à 4 atomes de carbone et les groupes halogénoalkyle et halogénoalkyloxy contenant les halogènes F et/ou Cl ;
n est 0, 1 ;
$R^1$ est le même et représente C(O)OM, $SO_3M$, C(O)-NH-$S(O)_2$-$R^x$, $PO_3M_2$ ou C(O)NOM ;
$R^2$, $R^3$ identiques ou différents, représentent H ou F ;
$R^4$, $R^7$ identiques ou différents, représentent H, OH, $OR^z$, OC(O)$NHR^y$ ou $NR^x$ ;
$R^6$ le même ou différent, est H ou $R^z$ ;
$R^5$ le même ou différent, est H, $R^x$, C(O)$CH_2$OH, C(0)-halogénoalkyle ou C(O)H ;
$R^8$ le même ou différent, est $R^x$;
M est H, un groupe alkyle en $C_1$-$C_4$ ou un cation choisi dans le groupe constitué par les métaux alcalins, les métaux alcalino-terreux, le manganèse, le cuivre, le zinc, le fer et un cation ammonium éventuellement substitué ;
$R^x$ le même ou différent, est H, $R^y$ ou $R^z$;
$R^y$ le même ou différent, est un groupe alkyle en $C_1$-$C_4$, phényle ou benzyle et
$R^z$ le même ou différent, est un groupe -C(O)-alkyle ($C_1$-$C_6$), C(O)-phényle ou C(O)$CH_2$-phényle,

ainsi que ses sels pharmacologiquement acceptables.

**3.** Dérivé d'acide sialique selon la revendication 1 ou 2, dans lequel les symboles et indices dans la formule (I) ont les significations suivantes :

$A^1$, $A^2$ identiques ou différents représentent un groupe $D^1$-[$Y^3$-$D^2$-]$_m$- ;
$D^1$ le même ou différent est le groupe phényle, 1-naphtyle, 2-naphtyle, 2-biphénylène, 2-fluorényle, 3-fluorényle, 2-phénanthrényle, cyclopropyle, cyclobutyle, cyclopentyle, cyclohexyle, 2-pipéridinyle, 3-pipéridinyle, 4-pipéridinyle, 1,3-dioxan-5-yle, 4-tétrahydropyranyle, 3-hexahydropyridazinyle, 4-hexahydropyridazinyle, 2-hexahydro-pyrimidinyle, 4-hexahydropyrimidinyle, 5-hexahydropyrimidinyle, 1-pipérazinyle, 2-pipérazinyle, 4-morpholinyle, 2-furyle, 3-furyle, 2-thiényle, 3-thiényle, 2-pyrrolyle, 3-pyrrolyle, 3-pyrazolyle, 4-pyrazolyle, 5-pyrazolyle, 2-oxazolyle, 4-oxazolyle, 5-oxazolyle, 2-thiazolyle, 4-thiazolyle, 5-thiazolyle, 2-imidazolyle, 4-imidazolyle, 1,2,3-triazol-1-yle, 1,2,3-triazol-4-yle, 1,2,3-triazol-5-yle, 1,3,4-triazol-2-yle, 2-pyridinyle, 3-pyridinyle, 4-pyridinyle, 3-pyridazinyle, 4-pyridazinyle, 2-pyrimidinyle, 4-pyrimidinyle, 5-pyrimidinyle, 2-pyrazinyle, 1-benzofuran-2-yle, 1-benzofuran-5-yle, 1-benzofuran-6-yle, 2-benzofuran-5-yle, 2-benzofuran-6-yle, 2H-chromén-3-yle, 2H-chromén-6-yle, 2H-chromén-7-yle, indol-2-yle, 1-benzothiophén-2-yle ou benzimidazol-2-yle,

les groupes nommés étant non substitués ou substitués par un ou plusieurs groupes $X^1$,

$D^2$ le même ou différent, est le groupe phénylène-1,4-diyle, phénylène-1,3-diyle, biphényle-4,4'-diyle, naphtalène-1,4-diyle, trans-cyclohexane-1,4-diyle, furane-2,5-diyle, thiophène-2,4-diyle, thiophène-2,5-diyle, 1H(1,2,3)-triazole-1,4-diyle ou pyrrole-2,5-diyle, pyridine-2,5-diyle, pyridine-2,4-diyle, pyridazine-3,6-diyle, pyrimidine-2,5-diyle, pyrimidine-2,6-diyle, pyrazine-2,5-diyle ou tétrazine-3,5-diyle,

les groupes nommés étant non substitués ou substitués par un ou un groupe $X^1$,

$X^1$ est, le même ou différent, F, Cl, Br, I, un groupe cyano, nitro, hydroxy, amino, carboxy, azido, $SO_3M$, $SO_2NH_2$, alkyle, halogénoalkyle, alkyloxy, halogénoalkyloxy, alkylamino, dialkylamino, trialkylamino, alkylcarbonyle, alkylsulfoxyle, aminocarbonyle, alkylamino-carbonyle, alkylcarbonylamino, oxo (=0), les groupes alkyle dans ces radicaux contenant de 1 à 6 atomes de carbone ;
$Y^1$, $Y^2$ identiques ou différents, représentent un groupe ~$CH_2$-, ~C(O)-, ~$CH_2$C(O)-, ~$CH_2CH_2$C(O)-, ~CH=CH-C(O)-, ~C≡C-C(O)-, ~NH-C(O)-, ~$CH_2$-$NR^x$-3-cyclobutène-1,2-dione-4-, ~$S(O)_2$- ou ~$CH_2S(O)_2$-, ~ désignant la liaison au groupe A ou
$Y^1$, $Y^2$ forment, indépendamment l'un de l'autre, conjointement avec l'atome d'azote auquel ils sont liés et le groupe $R^8$, un groupe ~4-1H-(1,2,3)triazol-1-yl-, ~ désignant la liaison au groupe A ;
$Y^3$ est une liaison, 0, $S(O)_2$, $CH_2$ ou C(O) ;

m est 0, 1 ou 2 ;
$Z^1$ est O ;
D est un groupe $Z^2$-$T^1$-$Y^4$-$A^3$-$Y^5$-$T^2$-$Z^2$;
$Z^2$ le même, est 0, S, le groupe ~4-1H-(1,2,3)-triazol-1-yl-, -NH-C(O)~ ou $CH_2$, ~ désignant la liaison au groupe T ;
$T^1$, $T^2$ identiques ou différents, représentent un groupe alcanediyle à chaîne droite ou ramifié, ayant de 4 à 20 atomes de carbone,

(i) éventuellement un ou plusieurs groupes $CH_2$ non terminaux étant remplacés par -S-, -S(O)-, -S(O)$_2$-, et/ou -$NR^z$- et/ou
(ii) éventuellement un ou plusieurs atomes d'hydrogène étant remplacés par un atome de fluor, de chlore, (=0), un groupe carboxy, $NH_2$, $NHR^y$ ou $NHR^z$ et/ou
(iv) éventuellement un groupe -$CH_2CH_2CH_2$- non terminal étant remplacé par un groupe -4-1H-(1,2,3)triazol-1-yle ;

$Y^4$, $Y^5$ identiques ou différents, représentent ~$NR^x$-C(O)-, ~C(O)-$NR^x$-, ~$NR^x$-S(O)$_2$, ~$NR^x$-, ~$CH_2$-$NR^x$-, ~$NR^x$C(O)$NR^x$-, ~$CH_2NR^x$C(O)-, ~$CH_2$C(O)$NR^x$- ou ~S(O)$_2$-$NR^x$-, ~ désignant la liaison au groupe A ;
$A^3$ est

a) un groupe alcane ($C_1$-$C_6$) diyle, alcyne ($C_2$-$C_4$)-diyle, dans les groupes nommés éventuellement un ou plusieurs atomes d'hydrogène étant remplacés par F ou Cl, ou
b) un groupe $A^4$,

$A^4$ le même, est un groupe phénylène-1,4-diyle, biphényle-4,4'-diyle, naphtalène-1,4-diyle, trans-cyclohexane-1,4-diyle, thiophène-2,5-diyle, 1H-(1,2,3)-triazole-1,4-diyle, pyridine-2,5-diyle, pyridine-2,4-diyle, pyridazine-2,5-diyle, pyrimidine-2,5-diyle, pyrimidine-2,4-diyle, pyrazine-2,5-diyle ou tétrazine-2,5-diyle, les radicaux nommés étant non substitués ou substitués par un groupe $X^2$,
$X^2$ le même ou différent, est un atome de fluor, chlore, brome, un groupe nitro, $SO_3M$, $OSO_3M$, alkyle, halogénoalkyle, alkyloxy, halogénoalkyloxy, alkylamino, dialkylamino, trialkylamino, alkylcarbonyle, alkylsulfoxyle, alkylcarbonyloxy, aminocarbonyle, alkylamino-carbonyle, alkylcarbonylamino, dans ces radicaux les groupes alkyle contenant de 1 à 4 atomes de carbone et les groupes halogénoalkyle et halogénoalkyloxy contenant les halogènes F et/ou Cl ;
$R^1$ est le même et représente C(O)OM ou C(O)NOM ;
$R^2$, $R^3$ identiques, représentent H ;
$R^4$, $R^7$ identiques, représentent OH ou $OR^z$ ;
$R^6$ le même, est H ou $R^z$ ;
$R^5$ le même, est $R^x$, C(O)$CH_2$OH ou C(O)-halogéno-alkyle ;
$R^8$ le même ou différent, est $R^x$ ;
M est H, le groupe méthyle, éthyle ou un cation choisi dans le groupe constitué par Li, Na, K, Ca, Mg et un cation ammonium éventuellement substitué ;
$R^x$ le même ou différent, est H, $R^y$ ou $R^z$ ;
$R^y$ le même ou différent, est le groupe méthyle, éthyle, 1-méthyléthyle, propyle, 1-méthyl-propyle, 1,1-diméthylpropyle ou benzyle ;
$R^z$ le même ou différent, est le groupe méthylcarbonyle, éthylcarbonyle, propyl-carbonyle, 1-méthyléthylcarbonyle, 1,1-diméthyléthylcarbonyle ou phénylcarbonyle,

ainsi que ses sels pharmacologiquement acceptables.

**4.** Dérivé d'acide sialique selon l'une quelconque des revendications 1 à 3, dans lequel les symboles et indices dans la formule (I) ont les significations suivantes :

$A^1$, $A^2$ identiques ou différents, représentent un groupe $D^1$-[$Y^3$-$D^2$-]$_m$- ;
$D^1$ le même ou différent est un groupe phényle, 1-naphtyle, 2-naphtyle, 2-biphénylène, 2-fluorényle, 3-fluorényle, 2-phénanthrényle, cyclopropyle, cyclobutyle, cyclopentyle, cyclohexyle, 2-pipéridinyle, 3-pipéridinyle, 4-pipéridinyle, 1,3-dioxan-5-yle, 4-tétrahydropyranyle, 3-hexahydropyridazinyle, 4-hexahydropyridazinyle, 2-hexahydro-pyrimidinyle, 4-hexahydropyrimidinyle, 5-hexa-hydropyrimidinyle, 1-pipérazinyle, 2-pipérazinyle, 4-morpholinyle, 2-furyle, 3-furyle, 2-thiényle, 3-thiényle, 2-pyrrolyle, 3-pyrrolyle, 3-pyrazolyle, 4-pyrazolyle, 5-pyrazolyle, 2-oxazolyle, 4-oxazolyle, 5-oxazolyle, 2-thiazolyle, 4-thiazolyle, 5-thiazolyle, 2-imidazolyle, 4-imidazolyle, 1,2,3-triazol-1-yle, 1,2,3-triazol-4-yle, 1,2,3-triazol-5-yle, 1,3,4-triazol-2-yle, 2-pyridinyle, 3-pyridinyle, 4-pyridinyle, 3-pyridazinyle, 4-pyridazinyle, 2-pyrimidinyle, 4-pyrimidinyle, 5-pyrimidinyle, 2-pyrazinyle, 1-benzofuran-2-yle, 1-benzofuran-5-yle, 1-benzofuran-6-yle, 2-benzofuran-5-yle, 2-benzofuran-6-yle, 2H-chromén-3-yle, 2H-chromén-6-yle, 2H-chromén-7-yle, indol-2-yle, 1-benzo-thiophén-2-yle ou benzimidazol-2-yle,

les groupes nommés étant non substitués ou substitués par un ou plusieurs groupes $X^1$,

$D^2$ le même ou différent, est un groupe phénylène-1,4-diyle, phénylène-1,3-diyle, biphényle-4,4'-diyle, naphtalène-1,4-diyle, trans-cyclohexane-1,4-diyle, furane-2,5-diyle, thiophène-2,4-diyle, thiophène-2,5-diyle, 1H(1,2,3)-triazole-1,4-diyle ou pyrrole-2,5-diyle, pyridine-2,5-diyle, pyridine-2,4-diyle, pyridazine-3,6-diyle, pyrimidine-2,5-diyle, pyrimidine-2,6-diyle, pyrazine-2,5-diyle ou tétrazine-3,5-diyle, les groupes nommés étant non substitués ou substitués par un ou un groupe $X^1$,
$X^1$ est, le même ou différent, F, Cl, Br, I, un groupe cyano, nitro, hydroxy, amino, carboxy, azido, $SO_3M$, $SO_2NH_2$, alkyle, halogénoalkyle, alkyloxy, halogénoalkyloxy, alkylamino, dialkylamino, trialkylamino, alkylcarbonyle, alkylsulfoxyle, aminocarbonyle, alkylamino-carbonyle, alkylcarbonylamino, oxo (=0), les groupes alkyle dans ces radicaux de 1 à 6 atomes de carbone ;
$Y^1$, $Y^2$ identiques ou différents, représentent un groupe $\sim CH_2$-, ~C(O)-, $\sim CH_2C$-(O)-, $\sim CH_2CH_2C$(O)-, ~CH=CH-C(O)-, ~C≡OC-C(O)-, ~NH-C(O)-, $\sim CH_2-NR^x$-3-cyclobutène-1,2-dione-4-, $\sim S(O)_2$- ou $\sim CH_2S(O)_2$-, ~ désignant la liaison au groupe A ou
$Y^1$, $Y^2$ forment, indépendamment l'un de l'autre, conjointement avec l'atome d'azote auquel ils sont liés et le groupe $R^8$, un groupe ~4-1H-(1,2,3)triazol-1-yl-, ~ désignant la liaison au groupe A ;
$Y^3$ est une liaison, 0, $S(O_2)$, $CH_2$ ou C(O) ;
m est 0, 1 ou 2 ;
$Z^1$ est 0 ;
D est un groupe $Z^2-T^1-Y^4-A^3-Y^5-T^2-Z^2$ ;
$Z^2$ le même, est 0, S, le groupe ~4-1H-(1,2,3)-triazol-1-yl-, -NH-C(O)~ ou $CH_2$, ~ désignant la liaison au groupe T ;
$T^1$, $T^2$ identiques ou différents, représentent un groupe alcanediyle à chaîne droite ou ramifié, ayant de 4 à 20 atomes de carbone,

(i) éventuellement un ou plusieurs groupes $CH_2$ non terminaux étant remplacés par -NH-, -S-, -S(0)- et/ou $-S(O)_2$- et/ou
(ii) éventuellement un ou plusieurs atomes d'hydrogène étant remplacés par un atome de fluor, de chlore, (=0), un groupe carboxy, $NH_2$, $NHR^y$ ou $NHR^z$ et/ou
(iv) éventuellement un groupe $-CH_2CH_2CH_2$- non terminal étant remplacé par un groupe -4-1H-(1,2,3)triazol-1-yl- ;

$Y^4$, $Y^5$ identiques ou différents, représentent $\sim NR^x$-C(O)-, $\sim C(O)-NR^x$-, $\sim NR^x-S(O)_2$-, $\sim NR^x$-, $\sim CH_2-NR^x$. $\sim NR^xC(O)NR^x$-, $\sim CH_2NR^xC(O)$-, $\sim CH_2C(O)NR^x$- ou $\sim S(O)_2-NR^x$-, ~ désignant la liaison au groupe A ;
$A^3$ est

a) un groupe alcane($C_1$-$C_6$) diyle, alcyne($C_2$-$C_4$)-diyle, dans les groupes nommés éventuellement un ou plusieurs atomes d'hydrogène étant remplacés par F ou Cl,
b) un groupe $A^4$,

$A^4$ le même, est un groupe phénylène-1,4-diyle, biphényle-4,4'-diyle, naphtalène-1,4-diyle, trans-cyclohexane-1,4-diyle, thiophène-2,5-diyle ou 1H-(1,2,3)-triazole-1,4-diyle, pyridine-2,5-diyle, pyridine-2,4-diyle, pyridazine-2,5-diyle, pyrimidine-2,5-diyle, pyrimidine-2,4-diyle, pyrazine-2,5-diyle ou tétrazine-2,5-diyle, les radicaux nommés étant non substitués ou substitués par un groupe $X^2$,
$X^2$ le même ou différent, est un atome de fluor, chlore, brome, un groupe nitro, $SO_3M$, alkyle, halogénoalkyle, alkyloxy, halogénoalkyloxy, alkylamino, dialkylamino, trialkylamino, alkylcarbonyle, alkylsulfoxyle, alkylcarbonyl-oxy, aminocarbonyle, alkylaminocarbonyle, alkylcarbonylamino, dans ces radicaux les groupes alkyle contenant de 1 à 4 atomes de carbone et les groupes halogénoalkyle et halogénoalkyloxy contenant les halogènes F et/ou Cl ;
$R^1$ est le même et représente C(O)OM ou C(O)NOM ;
$R^2$, $R^3$ identiques, représentent H ;
$R^4$, $R^7$ identiques, représentent OH ou $OR^z$ ;
$R^6$ le même, est H ou $R^z$;
$R^5$ le même, est $R^x$, $C(O)CH_2OH$ ou C(O)-halogéno-alkyle ;
$R^8$ le même ou différent, est $R^x$;

M est H, le groupe méthyle, éthyle ou un cation choisi dans le groupe constitué par Li, Na, K, Ca, Mg et un cation ammonium éventuellement substitué ;
$R^x$ le même ou différent, est H, $R^y$ ou $R^z$ ;
$R^y$ le même ou différent, est le groupe méthyle, éthyle, 1-méthyléthyle, propyle, 1-méthyl-propyle, 1,1-diméthylpropyle ou benzyle ;
$R^z$ le même ou différent, est le groupe méthylcarbonyle, éthylcarbonyle, propyl-carbonyle, 1-méthyléthylcarbonyle, 1,1-diméthyléthylcarbonyle ou phénylcarbonyle,

ainsi que ses sels pharmacologiquement acceptables.

**5.** Dérivé d'acide sialique selon l'une quelconque des revendications 1 à 4, dans lequel les symboles et indices dans la formule (I) ont les significations suivantes :

$A^1$, $A^2$ identiques ou différents, représentent un groupe $D^1$-[$Y^3$-$D^2$-]$_m$- ;
$D^1$ le même, est un groupe phényle, 2-naphtyle, cyclohexyle, 2-fluorényle, 2-furanyle, 2-benzothiophényle, 2-thiényle, 3-thiényle, 2-pyridinyle, 3-pyridinyle, 4-pyridinyle, 3-pyridazinyle, 4-pyridazinyle, 2-pyrimidinyle, 4-pyrimidinyle, 5-pyrimidinyle ou 2-pyrazinyle, les groupes nommés étant non substitués ou substitués par un groupe $X^1$,
$D^2$ le même, est un groupe phénylène-1,4-diyle, naphtalène-1,4-diyle, thiophène-2,5-diyle, pyridine-2,5-diyle, pyridazine-3,6-diyle, pyrimidine-2,5-diyle ou pyrazine-2,5-diyle, les groupes nommés étant non substitués ou substitués par un groupe $X^1$,
$X^1$ est F, Cl, Br, un groupe nitro, hydroxy, carboxy, alkyle, halogénoalkyle, alkyloxy, halogénoalkyloxy, alkylamino, dialkylamino, trialkylamino, alkylcarbonyle, alkylsulfoxyle, aminocarbonyle, alkylaminocarbonyle, alkylcarbonylamino, les groupes alkyle dans ces radicaux contenant de 1 à 4 atomes de carbone ;
$Y^1$, $Y^2$ identiques, représentent un groupe ~$CH_2$-, ~C(O)-, ~$CH_2$C(O)-, ~CO≡C-C(O)-, ~CH=CH-C(O)-, ~$CH_2$-NR$^x$-3-cyclobutène-1,2-dione-4- ou ~S(O)$_2$-, ~ désignant la liaison au groupe A ou
$Y^1$, $Y^2$ forment, indépendamment l'un de l'autre, conjointement avec l'atome d'azote auquel ils sont liés et le groupe $R^8$, un groupe ~4-1H-(1,2,3)triazol-1-yl-, ~ désignant la liaison au groupe A ;
$Y^3$ est une liaison ou O ;
m est 0 ou 1 ;
$Z^1$ est O ;
D est un groupe $Z^2$-$T^1$-$Y^4$-$A^3$-$Y^5$-$T^2$-$Z^2$ ;
$Z^2$ le même, est 0, le groupe ~4-1H-(1,2,3)triazol-1-yl-, -NH-C(O)~ ou $CH_2$, ~ désignant la liaison au groupe T ;
$T^1$, $T^2$ identiques, représentent un groupe alcanediyle à chaîne droite ayant de 4 à 10 atomes de carbone,

(i) éventuellement un ou plusieurs groupes $CH_2$ non terminaux étant remplacés par -S- et/ou -NH- et/ou
(ii) éventuellement un ou plusieurs atomes d'hydrogène étant remplacés par F, Cl ou (=0), un groupe carboxy ou $NH_2$ ;

$Y^4$, $Y^5$ identiques, représentent ~NR$^x$-C(O)-, ~NR$^x$-, ~$CH_2$-NR$^x$, ~NR$^x$C(O)NR$^x$-, ~$CH_2$NR$^x$C(O)-, ~$CH_2$C(O)NR$^x$- ou ~C(O)-NR$^x$-, ~ désignant la liaison au groupe A ;
$A^3$ est

a) un groupe alcane($C_1$-$C_6$)diyle, dans les groupes nommés éventuellement un ou plusieurs atomes d'hydrogène étant remplacés par F ou Cl, ou
b) un groupe $A^4$,

$A^4$ le même, est un groupe phénylène-1,4-diyle, biphényle-4,4'-diyle, naphtalène-1,4-diyle, pyridine-2,5-diyle, pyridazine-2,5-diyle, pyrimidine-2,5-diyle, pyrazine-2,5-diyle, tétrazine-2,5-diyle, éthyne-1,2-diyle, thiophène-2,5-diyle, cyclohexane-1,4-diyle ou -3-cyclobutène-1,2-dione-4- ; les radicaux nommés étant non substitués ou substitués par un groupe $X^2$,
$X^2$ le même, est un atome de fluor, chlore, brome, un groupe nitro, $SO_3M$, alkyle, halogénoalkyle, alkyloxy, halogénoalkyloxy, alkylamino, dialkylamino, trialkylamino, alkylsulfoxyle, alkylcarbonyloxy, dans ces radicaux les groupes alkyle contenant de 1 à 4 atomes de carbone et les groupes halogénoalkyle et halogénoalkyloxy contenant les halogènes F et/ou Cl ;
$R^1$ est le même et représente C(O)OM ;
$R^2$, $R^3$ identiques, représentent H ;
$R^4$, $R^7$ identiques, représentent OH ou OR$^z$;

$R^6$ le même, est H ou $R^z$;
$R^5$ le même, est $R^x$, $C(O)CH_2OH$ ou C(O)-halogéno-alkyle ;
$R^8$ le même, est $R^x$ ;
M est H, le groupe méthyle, éthyle ou un cation choisi dans le groupe constitué par Li, Na et K ;
$R^x$ le même ou différent, est H, $R^y$ ou $R^z$ ;
$R^y$ le même ou différent, est le groupe méthyle ou éthyle ;
$R^z$ le même, est le groupe méthylcarbonyle, éthylcarbonyle ou 1-méthyléthylcarbonyle,

ainsi que ses sels pharmacologiquement acceptables.

**6.** Dérivé d'acide sialique de formule (I) selon l'une quelconque des revendications 1 à 5, dans lequel les symboles et indices dans la formule (I) ont les significations suivantes :

$A^1$, $A^2$ identiques, représentent un groupe $D^1$-[$Y^3$-$D^2$-]$_m$- ;
$D^1$ le même, est un groupe phényle, cyclohexyle, napht-2-yle, fluorén-2-yle, furan-2-yle, benzothiophén-2-yle, thién-2-yle, thién-3-yle, pyridin-2-yle, pyridin-3-yle, pyridin-4-yle, pyridazin-3-yle, pyridazin-4-yle, pyrimidin-2-yle, pyrimidin-4-yle, pyrimidin-5-yle ou pyrazin-2-yle, les groupes nommés étant non substitués ou substitués par un groupe $X^1$,
$D^2$ le même, est un groupe phénylène-1,4-diyle, naphtalène-1,4-diyle, thiophène-2,5-diyle, pyridine-2,5-diyle, pyridazine-3,6-diyle, pyrimidine-2,5-diyle ou pyrazine-2,5-diyle, les groupes nommés étant non substitués ou substitués par un groupe $X^1$,
$X^1$ le même, est F, Cl, un groupe nitro, hydroxy, carboxy, alkyle, halogénoalkyle, alkyloxy, halogénoalkyloxy, alkylamino, dialkylamino, trialkylamino, alkylsulfoxyle, aminocarbonyle, alkylaminocarbonyle, alkylcarbonylamino, les groupes alkyle dans ces radicaux contenant de 1 à 3 atomes de carbone ;
$Y^1$, $Y^2$ identiques, représentent un groupe ~$CH_2$-, ~C(O)-, ~$CH_2$-C(O)-, ~C≡OC-C(O)-, ~CH=CH-C(O)-, ~$CH_2$-$NR^x$-3-cyclobutène-1,2-dione-4- ou ~$S(O)_2$-, ~ désignant la liaison au groupe A ou
$Y^1$, $Y^2$ forment, indépendamment l'un de l'autre, conjointement avec l'atome d'azote auquel ils sont liés et le groupe $R^8$, un groupe ~4-1H-(1,2,3)triazol-1-yl-, ~ désignant la liaison au groupe A ;
$Y^3$ est une liaison ;
m est 0 ou 1 ;
$Z^1$ est O ;
D est un groupe $Z^2$-$T^1$-$Y^4$-$A^3$-$Y^5$-$T^2$-$Z^2$ ;
$Z^2$ le même, est 0, $CH_2$ ou le groupe ~4-1H-(1,2,3)-triazol-1-yl-, ~ désignant la liaison au groupe T ;
$T^1$, $T^2$ identiques ou différents, représentent un groupe alcanediyle à chaîne droite ayant de 4 à 6 atomes de carbone,

(i) éventuellement un ou plusieurs groupes $CH_2$ non terminaux étant remplacés par -S- et/ou NH et/ou
(ii) éventuellement un ou plusieurs atomes d'hydrogène étant remplacés par F, Cl, (=O), un groupe carboxy ou $NH_2$ ;

$Y^4$, $Y^5$ identiques ou différents, représentent ~C(O)-$NR^x$-, ~$NR^x$-, ~$CH_2$-$NR^x$, ~NHC(O)NH-, ~$CH_2$NHC(O)-, ~$CH_2$C(O)NH- ou ~$NR^x$-C(O)-, ~ désignant la liaison au groupe A ;
$A^3$ est

a) un groupe alcane($C_1$-$C_6$)diyle, dans les groupes nommés éventuellement un ou plusieurs atomes d'hydrogène étant remplacés par F ou Cl, ou
b) un groupe $A^4$,

$A^4$ le même, est un groupe phénylène-1,4-diyle, biphényle-4,4'-diyle, naphtalène-1,4-diyle, pyridine-2,5-diyle, pyridazine-2,5-diyle, pyrimidine-2,5-diyle, pyrazine-2,5-diyle, tétrazine-2,5-diyle, éthyne-1,2-diyle, thiophène-2,5-diyle, cyclohexane-1,4-diyle ou -3-cyclobutène-1,2-dione-4- ; les radicaux nommés étant non substitués ou substitués par un groupe $X^2$,
$X^2$ le même, est un atome de fluor, chlore, brome, un groupe nitro, $SO_3M$, alkyle, halogénoalkyle, alkyloxy, halogénoalkyloxy, alkylamino, dialkylamino, trialkylamino, alkylsulfoxyle, alkylcarbonyloxy, dans ces radicaux les groupes alkyle contenant de 1 à 3 atomes de carbone et les groupes halogénoalkyle et halogénoalkyloxy contenant les halogènes F et/ou Cl ;
$R^1$ est C(O)OM ;
$R^2$, $R^3$ identiques, représentent H ;

$R^4$, $R^7$ identiques, représentent OH ou $OR^z$ ;
$R^6$ le même, est H ou $R^z$ ;
$R^5$ le même, est $R^x$, $C(O)CH_2OH$ ou C(O)-halogéno-alkyle ;
$R^8$ le même, est H ;
M est H, le groupe méthyle ou un cation choisi dans le groupe constitué par Li, Na et K ;
$R^x$ le même ou différent, est H, $R^y$ ou $R^z$ ;
$R^y$ le même ou différent, est le groupe méthyle ou éthyle ;
$R^z$ le même, est le groupe méthylcarbonyle, éthylcarbonyle ou 1-méthyléthylcarbonyle,

ainsi que ses sels pharmacologiquement acceptables.

**7.** Dérivé d'acide sialique selon l'une quelconque des revendications 1 à 6, dans lequel les symboles et indices dans la formule (I) ont les significations suivantes :

$A^1$, $A^2$ identiques, représentent un groupe $D^1$-[$Y^3$-$D^2$-]$_m$ ;
$D^1$ le même, est un groupe phényle, pyridin-2-yle, fluorén-2-yle, cyclohexyle, napht-2-yle, benzothiophén-2-yle, furan-2-yle, thién-2-yle ou thién-3-yle, les groupes nommés étant non substitués ou substitués par un groupe $X^1$,
$D^2$ le même, est un groupe phénylène-1,4-diyle ou thiophène-2,5-diyle, les groupes nommés étant non substitués ou substitués par un groupe $X^1$,
$X^1$ le même, est F, un atome de chlore, un groupe nitro, hydroxy, carboxy, méthyle, trifluoro-méthyle, méthyloxy ;
$Y^1$, $Y^2$ identiques, représentent un groupe ~C(O)-, ~$CH_2$-, ~CO≡C-C(O)-, ~CH=CH-C(O)-, ~$S(O)_2$-, ~$CH_2$-NH-3-cyclobutène-1,2-dione-4- ou ~$CH_2$-C(O)-, ~ désignant la liaison au groupe A ou
$Y^1$, $Y^2$ forment, indépendamment l'un de l'autre, conjointement avec l'atome d'azote auquel ils sont liés et le groupe $R^8$, un groupe ~4-1H-(1,2,3)triazol-1-yl-, ~ désignant la liaison au groupe A ;
$Y^3$ est une liaison ;
m est 0 ou 1 ;
$Z^1$ est 0 ;
D est un groupe $Z^2$-$T^1$-$Y^4$-$A^3$-$Y^5$-$T^2$-$Z^2$ ;
$Z^2$ le même, est 0, $CH_2$, ~CONH- ou le groupe ~4-1H-(1,2,3)triazol-1-yl-, ~ désignant la liaison au groupe T ;
$T^1$, $T^2$ identiques ou différents, représentent un groupe ~$CH_2CH_2CH_2SCH_2CH_2$-, ~$CH_2CH_2CH_2S(O)CH_2CH_2$-, ~$CH_2C$ (0) $NHCH_2CH_2CH_2$-, ~$CH_2$-4-1H-(1,2,3)triazole-1-$CH_2CH_2$-, butane-1,4-diyle, pentane-1,5-diyle ou hexane-1,6-diyle, ~ désignant la liaison au groupe $Z^2$ ;
$Y^4$, $Y^5$ identiques, représentent ~C(O)-NH-, ~NH-, ~$CH_2NH$-, ~NHC(O)NH-, ~$CH_2NHC(O)$-, ~$CH_2C(O)NH$- ou ~NH-C(O)-, ~ désignant la liaison au groupe A ;
$A^3$ est un radical méthanediyle, éthane-1,2-diyle, butane-1,4-diyle, hexane-1,6-diyle, -3-cyclobutène-1,2-dione-4-, thiophène-2,5-diyle, cyclohexane-1,4-diyle, phénylène-1,4-diyle, naphtalène-1,4-diyle, éthyne-1,2-diyle, biphényle-4,4'-diyle ou pyridine-2,5-diyle, les radicaux nommés étant non substitués ou substitués par un ou plusieurs groupes $X^2$ ;
$X^2$ le même, est un atome de brome, le groupe nitro, méthyle ou pentyloxy ;
$R^1$ est C(O)ONa ;
$R^2$, $R^3$ identiques, représentent H ;
$R^4$, $R^7$ identiques, représentent OH ;
$R^6$ le même, est H ;
$R^5$ le même, est $C(O)CH_3$, $C(O)CH_2F$ ou $C(O)CH_2OH$ ;
$R^8$ le même, est H ;

ainsi que ses sels pharmacologiquement acceptables.

**8.** Dérivés d'acide sialique selon l'une quelconque des revendications 1 à 7, correspondant aux formules développées suivantes

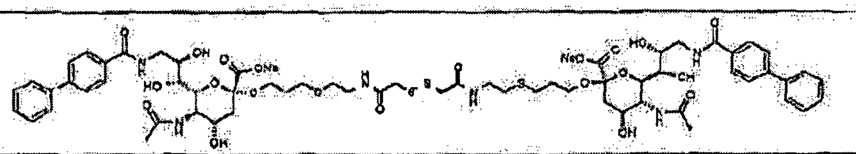

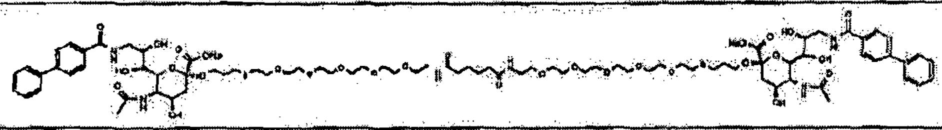

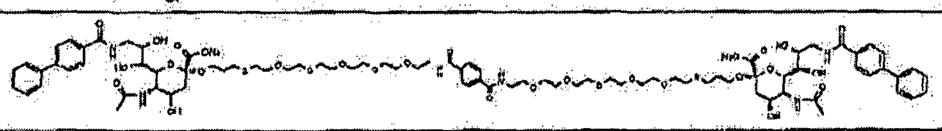

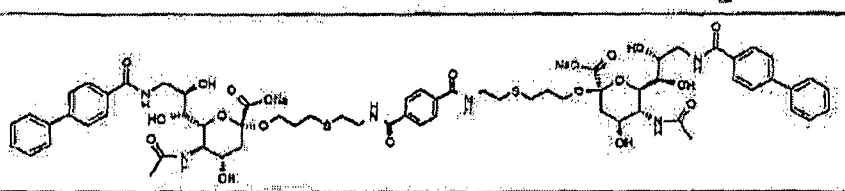

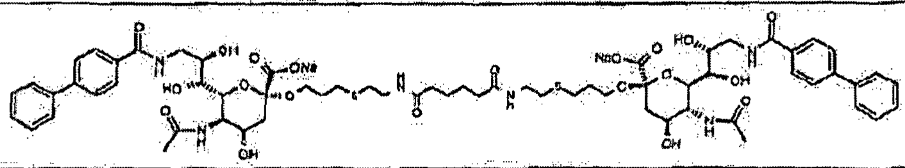

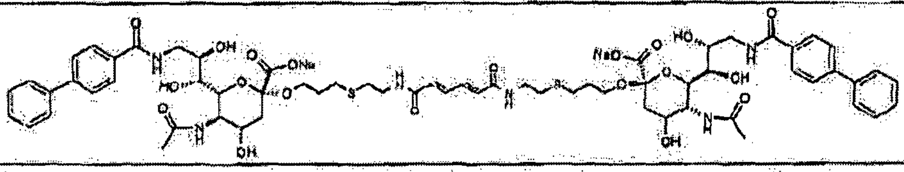

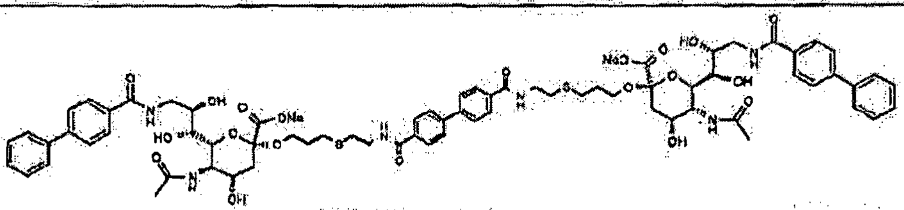

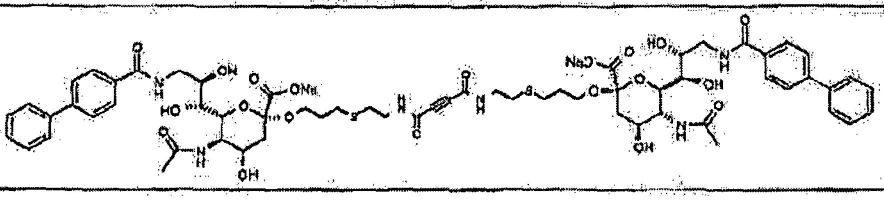

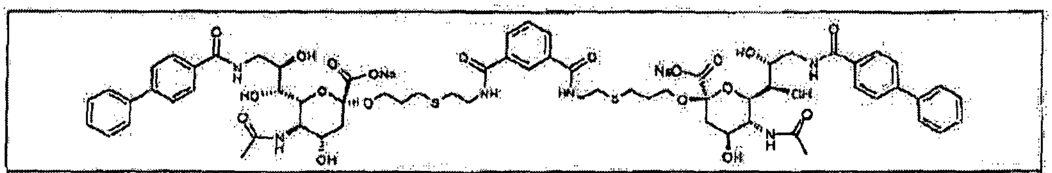

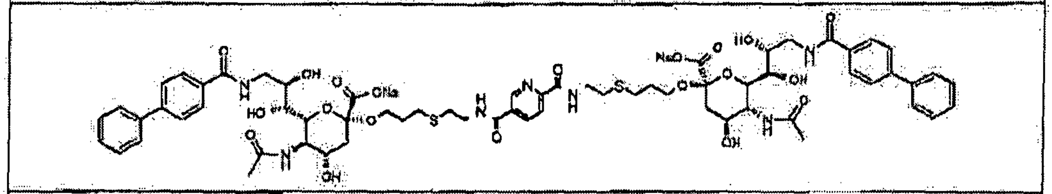

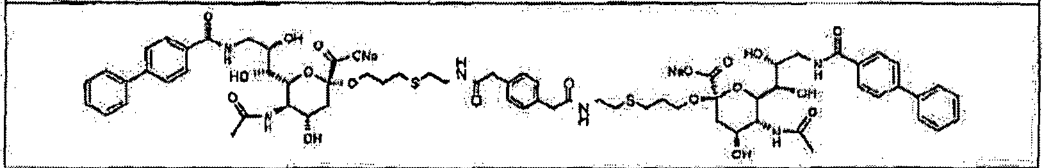

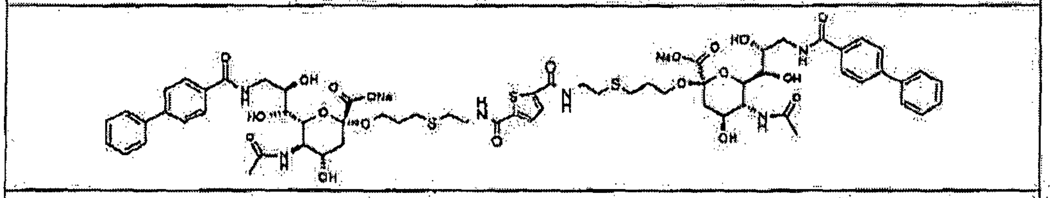

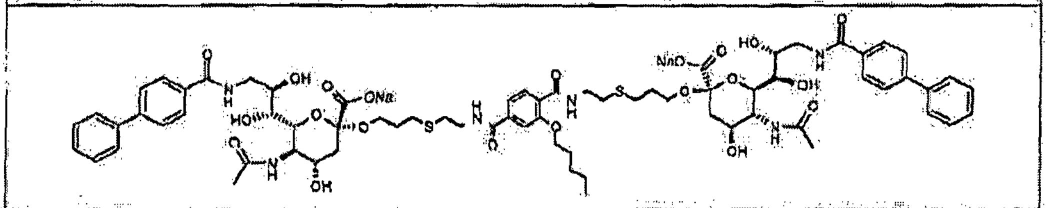

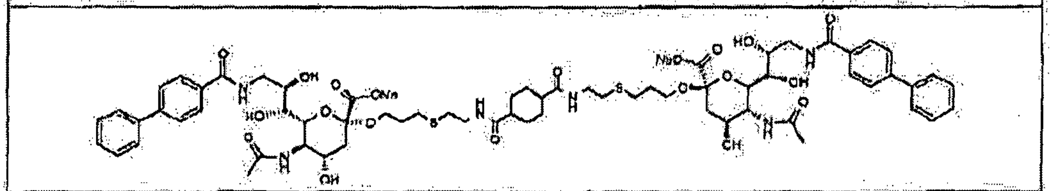

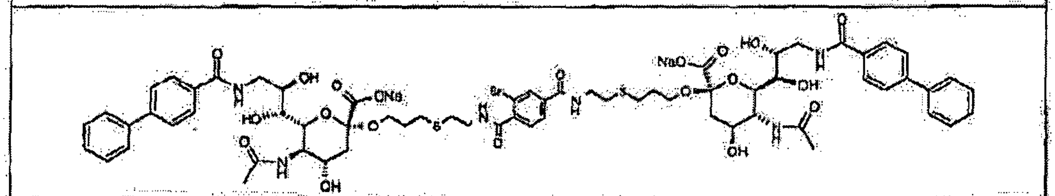

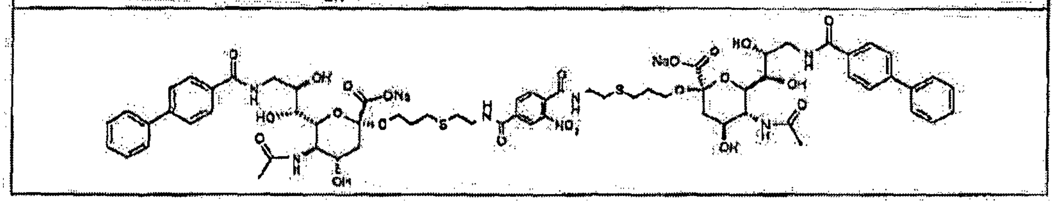

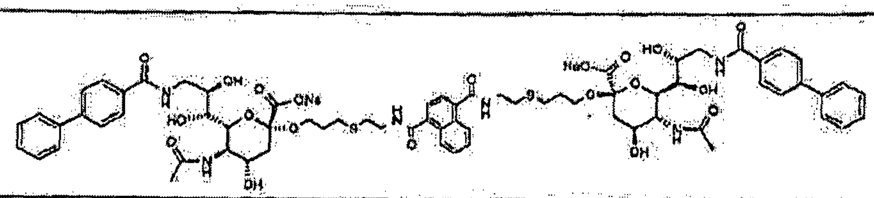

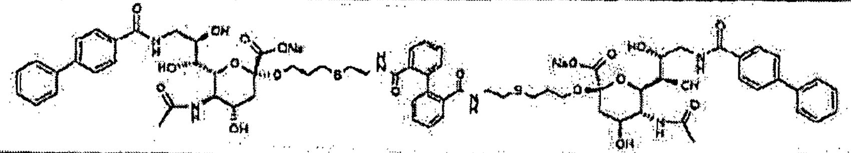

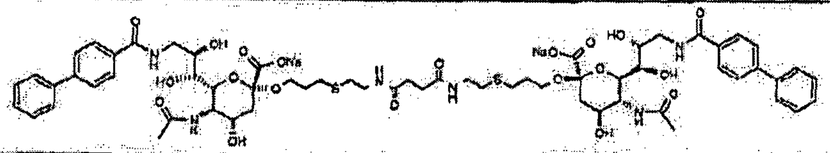

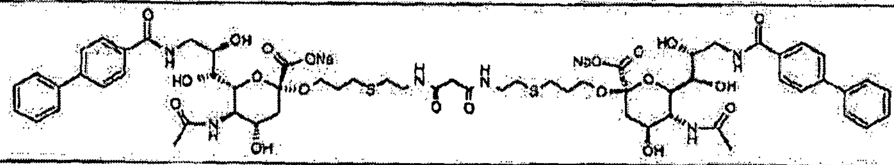

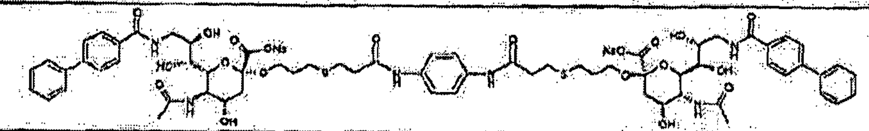

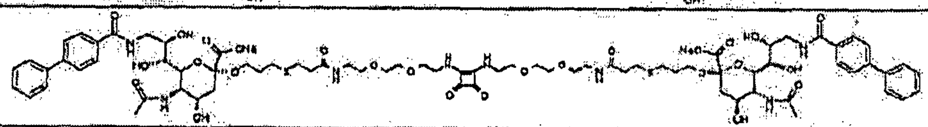

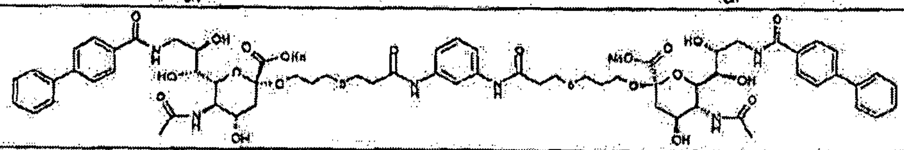

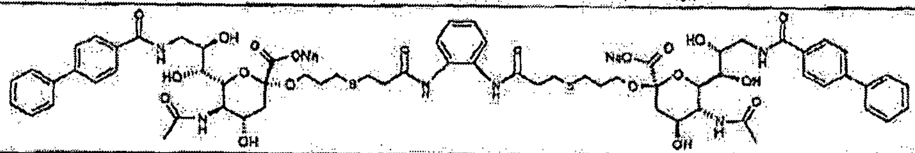

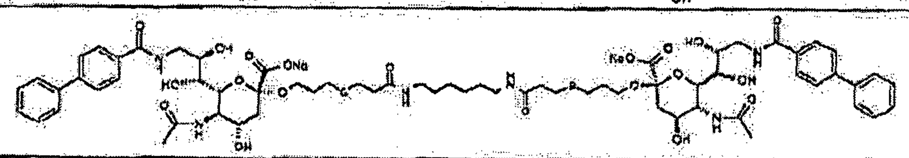

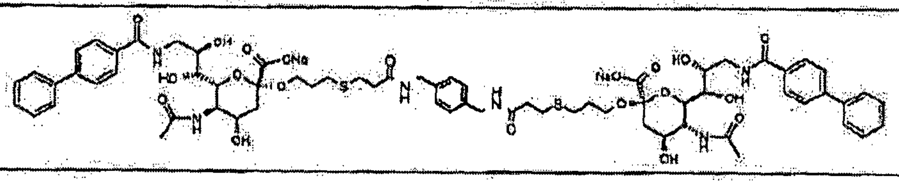

| |
|---|
| 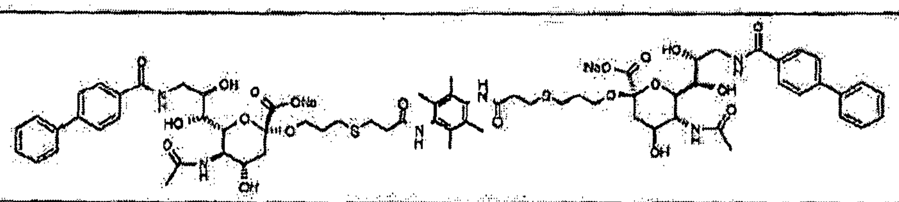 |
| 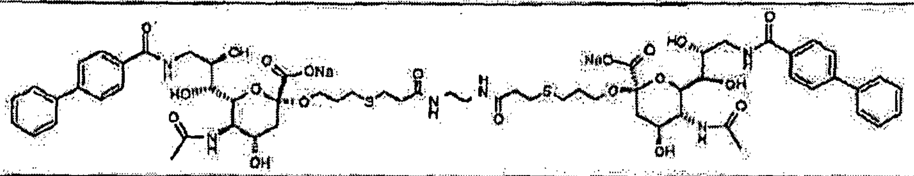 |
| 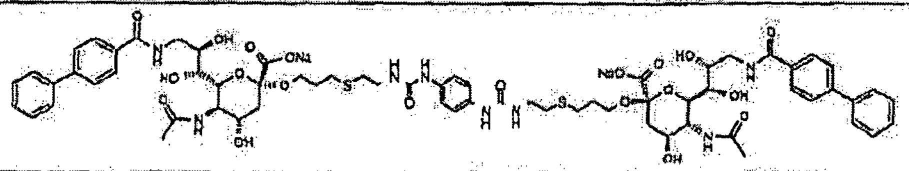 |
| 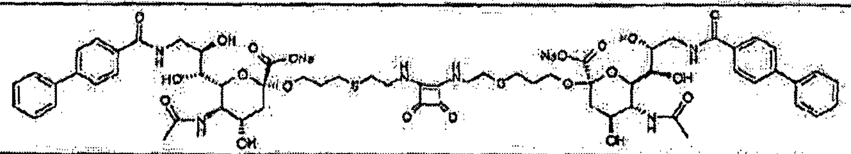 |
| 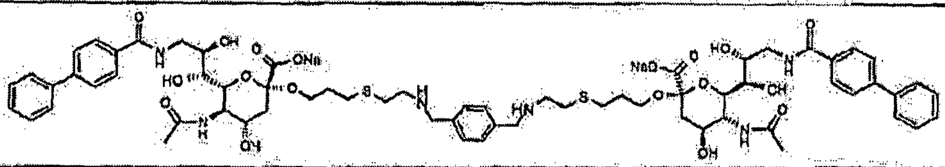 |
| 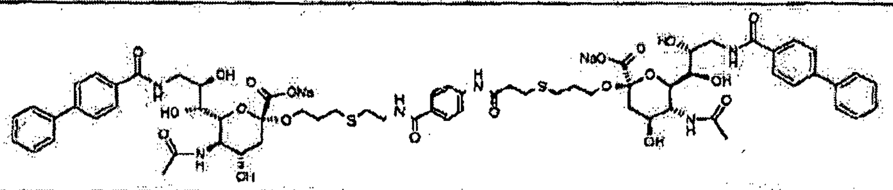 |
| 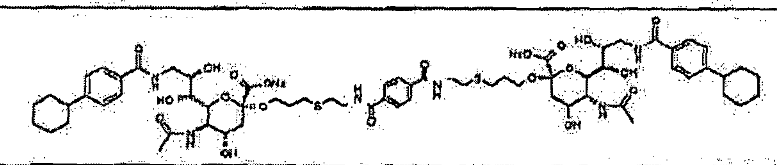 |
| 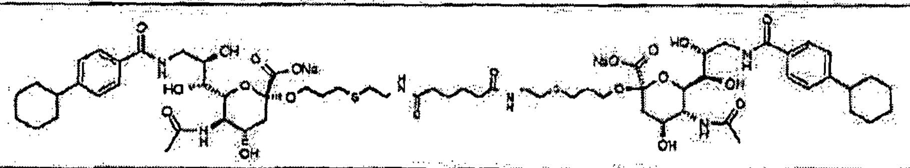 |
| 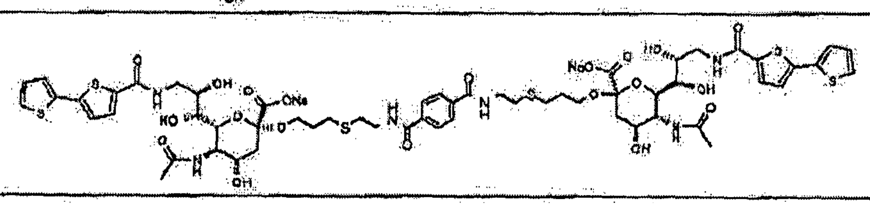 |

| |
|---|
| 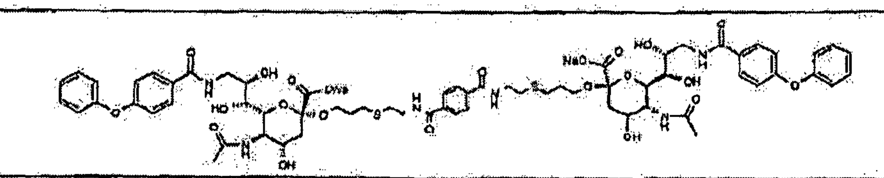 |
| 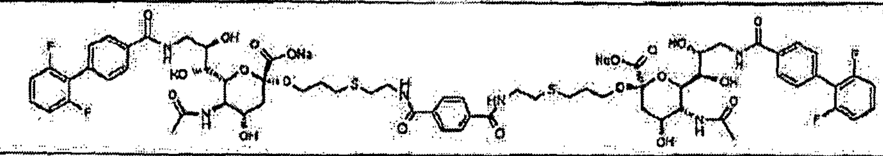 |
| 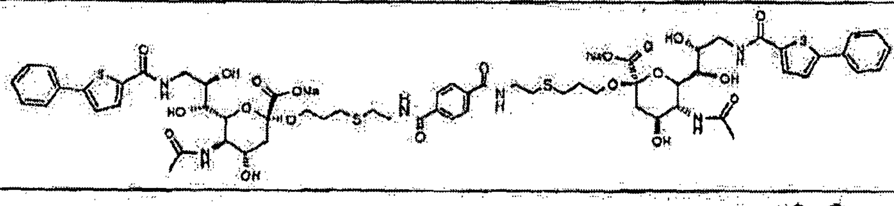 |
| 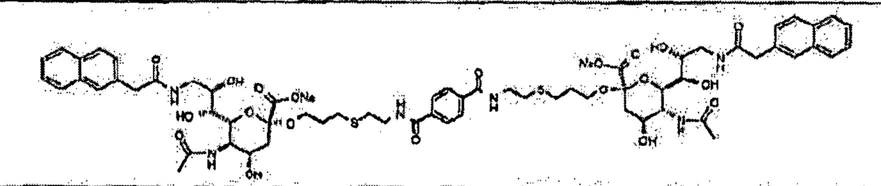 |
| 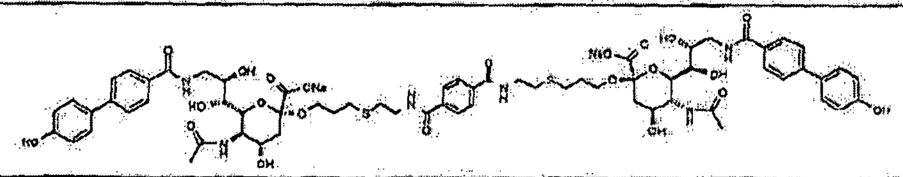 |
| 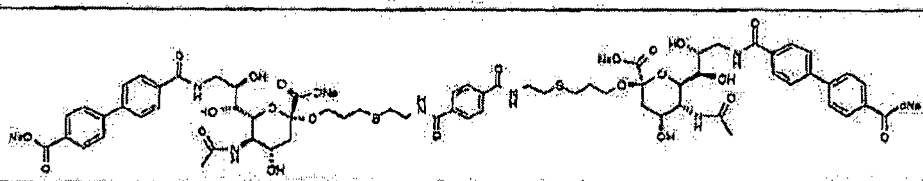 |
| 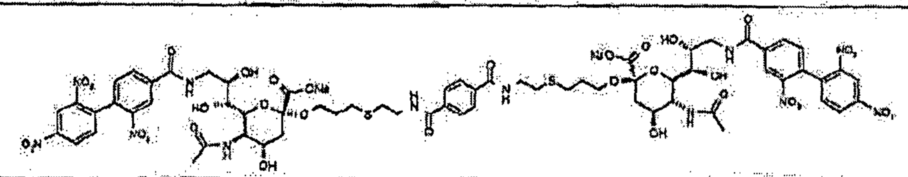 |
| 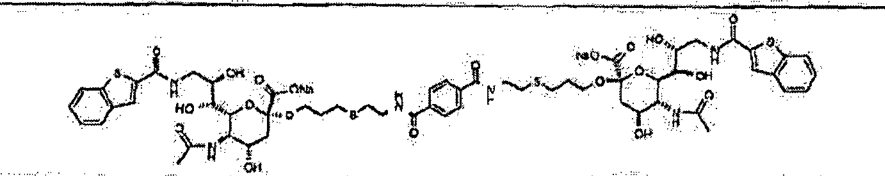 |
| 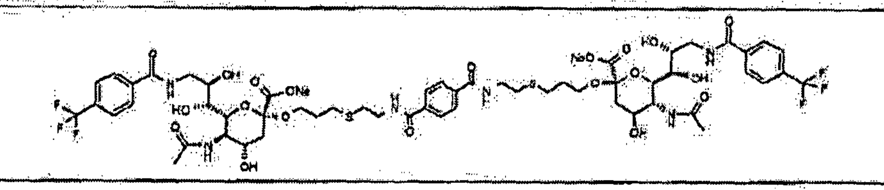 |

| |
|---|
| 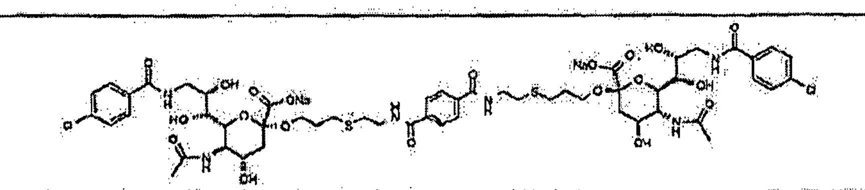 |
| 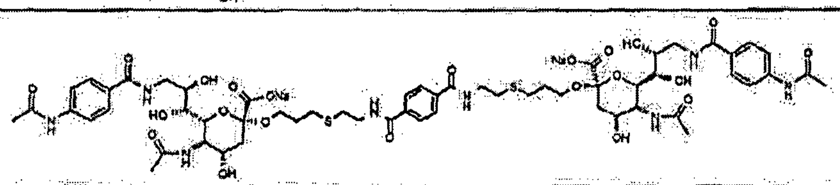 |
| 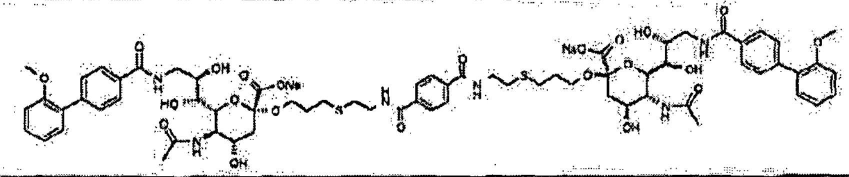 |
| 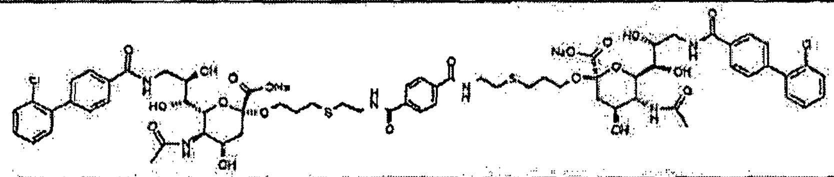 |
| 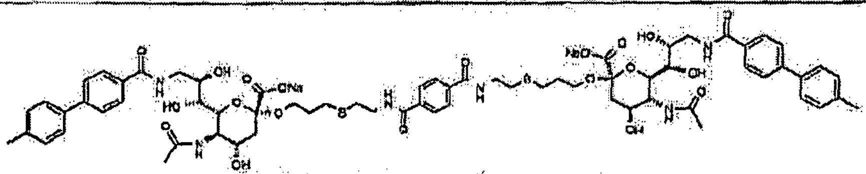 |
| 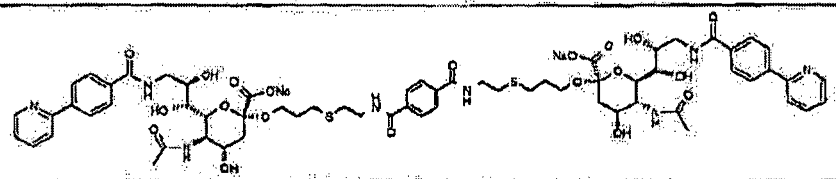 |
| 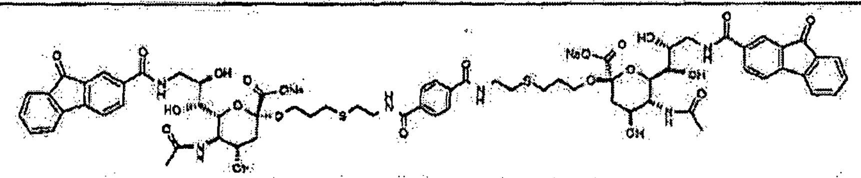 |
| 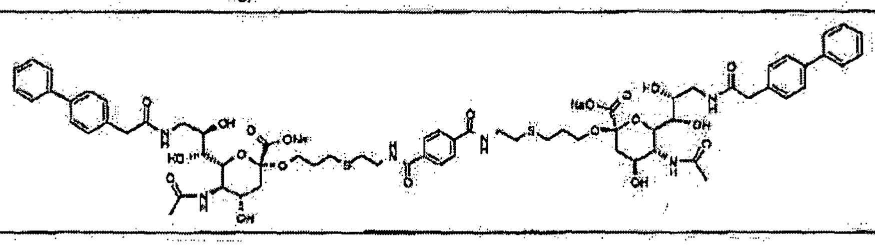 |

| |
|---|
| 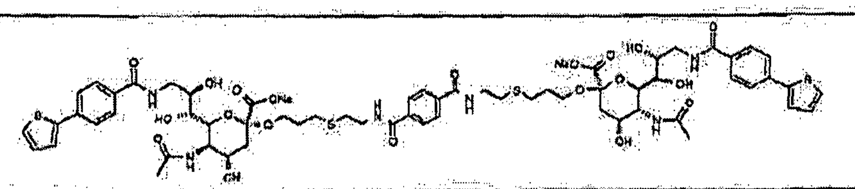 |
| 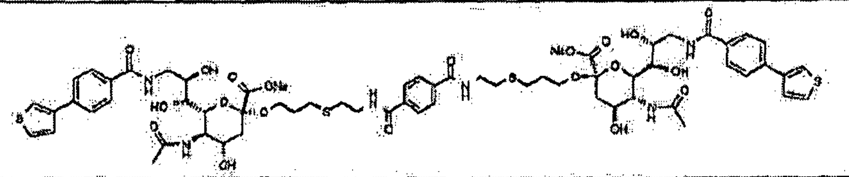 |
| 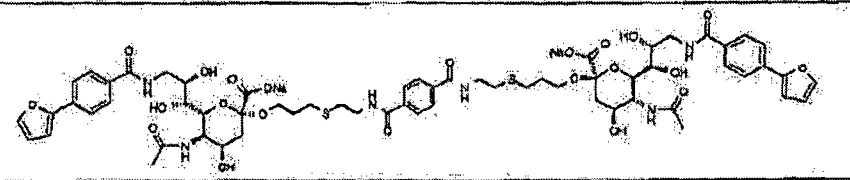 |
| 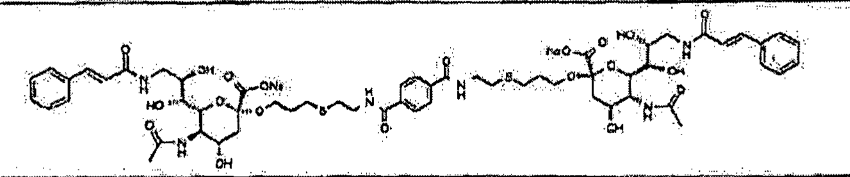 |
| 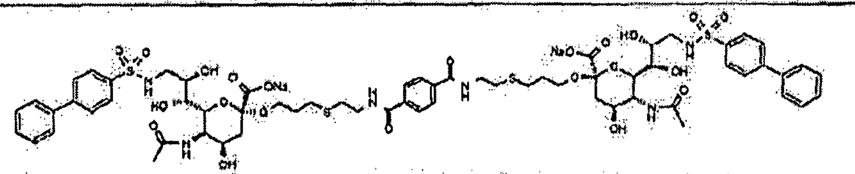 |
| 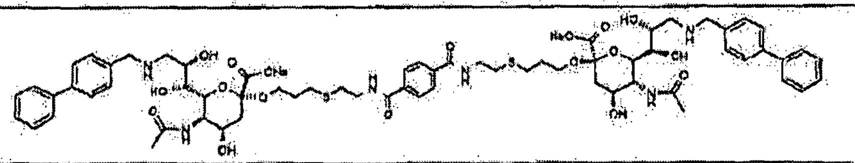 |
| 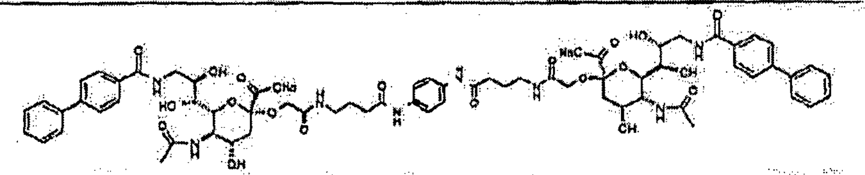 |
| 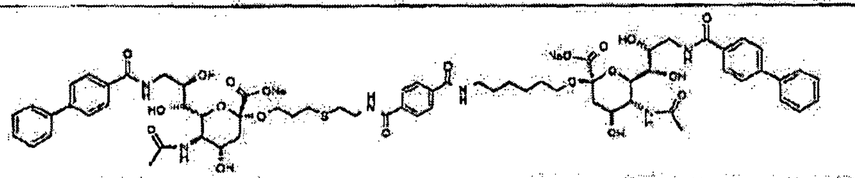 |
| 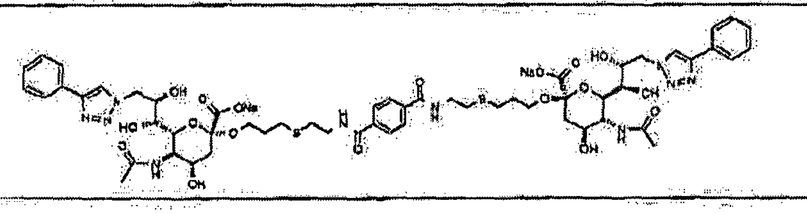 |

| |
|---|
| 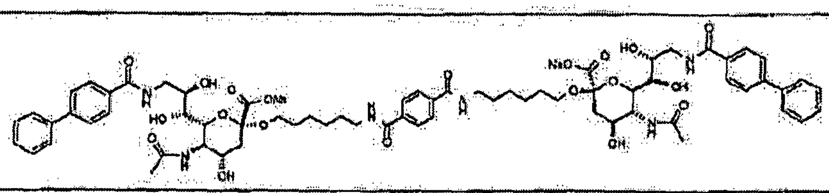 |
| 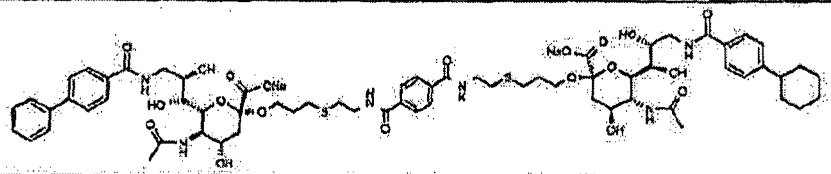 |
| 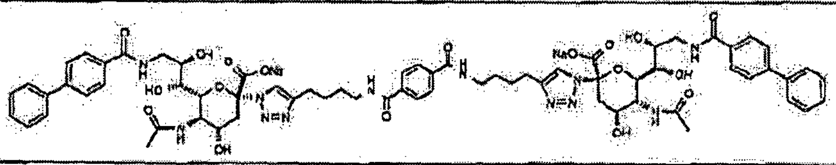 |
| 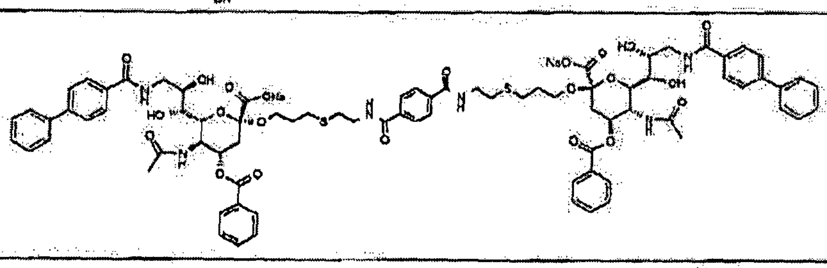 |
| 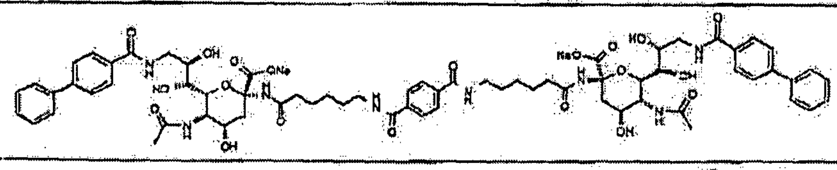 |
| 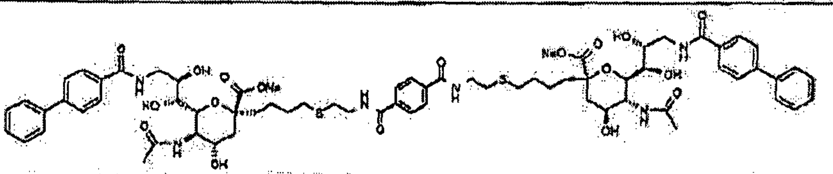 |
| 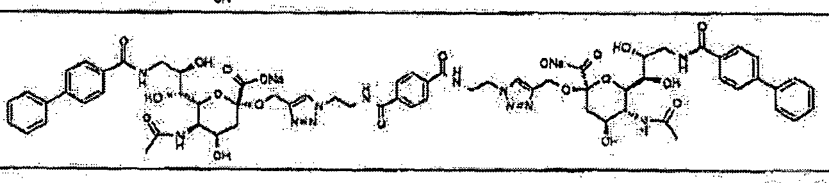 |
| 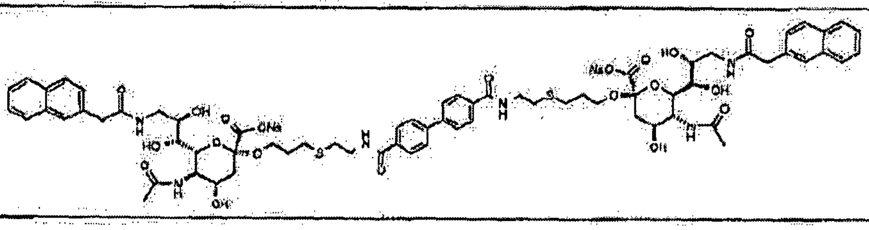 |

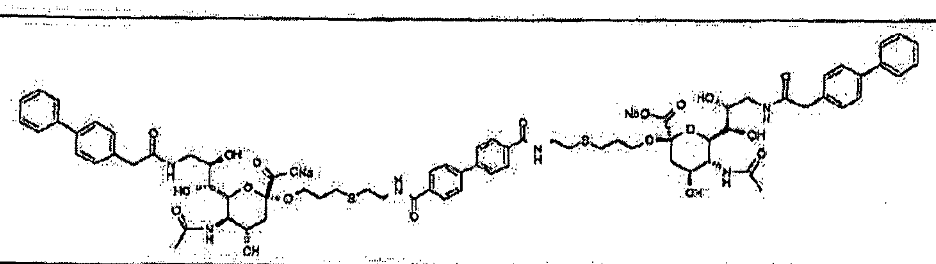

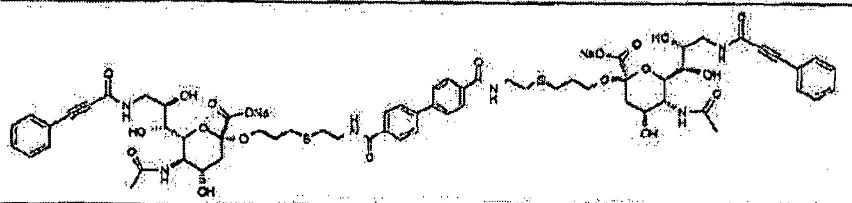

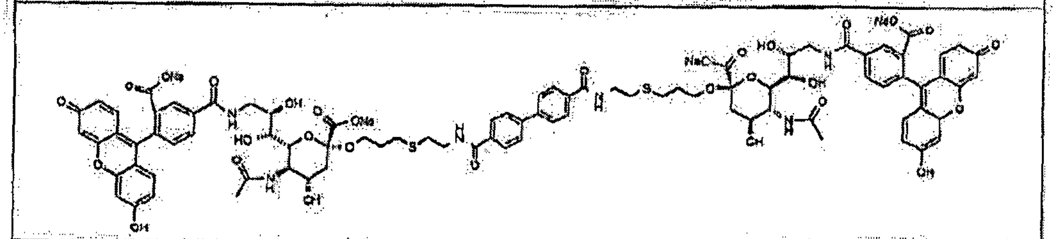

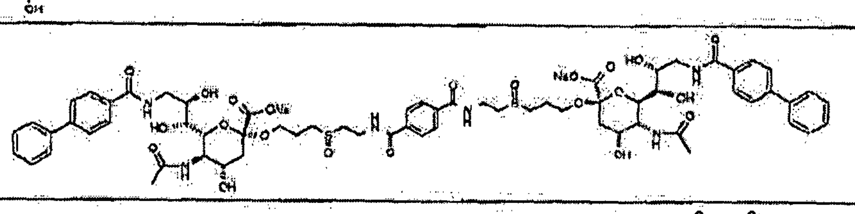

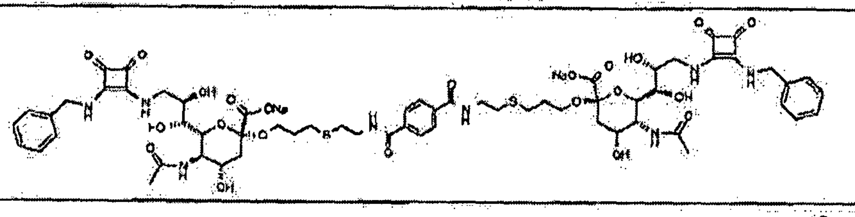

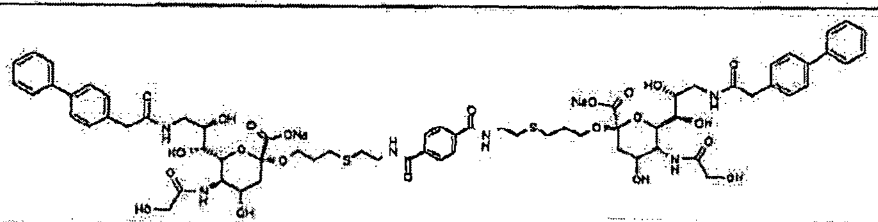

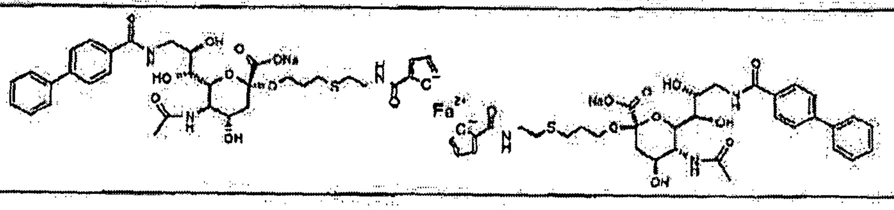

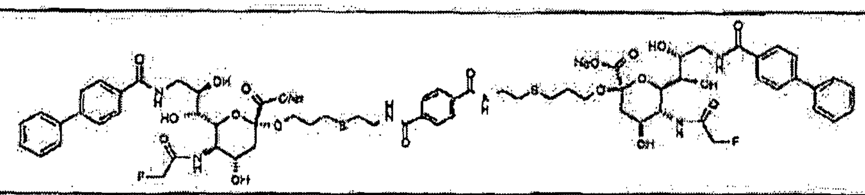

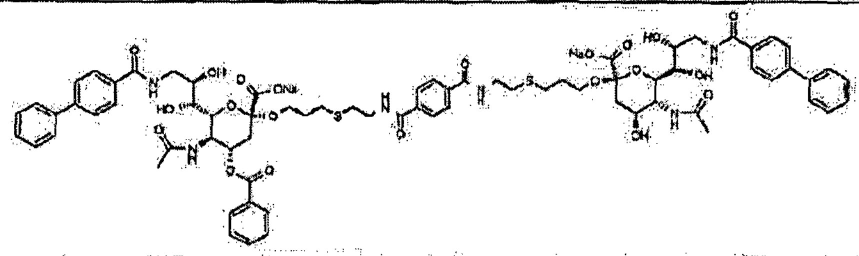

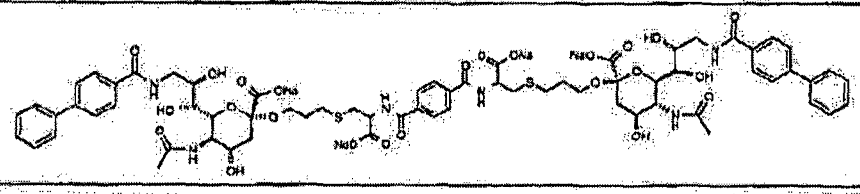

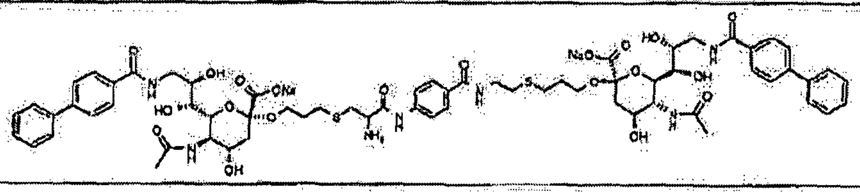

ainsi que leurs sels pharmacologiquement acceptables.

**9.** Préparation pharmaceutique, contenant au moins un dérivé d'acide sialique de formule (I) selon l'une quelconque des revendications 1 à 8 ou un sel pharmacologiquement acceptable d'un tel dérivé et un véhicule pharmacologiquement acceptable.

**10.** Dérivé d'acide sialique de formule (I) selon l'une quelconque des revendications 1 à 8 ou sel pharmacologiquement acceptable d'un tel dérivé, en tant que médicament.

**11.** Dérivé d'acide sialique de formule (I) selon l'une quelconque des revendications 1 à 8 ou sel pharmacologiquement acceptable d'un tel dérivé, destiné au traitement de maladies bactériennes.

**12.** Dérivé d'acide sialique de formule (I) selon l'une quelconque des revendications 1 à 8, pour utilisation dans la fabrication d'un médicament pour le traitement de maladies bactériennes, virales, parasitaires, auto-immunes et d'immunodéficience.

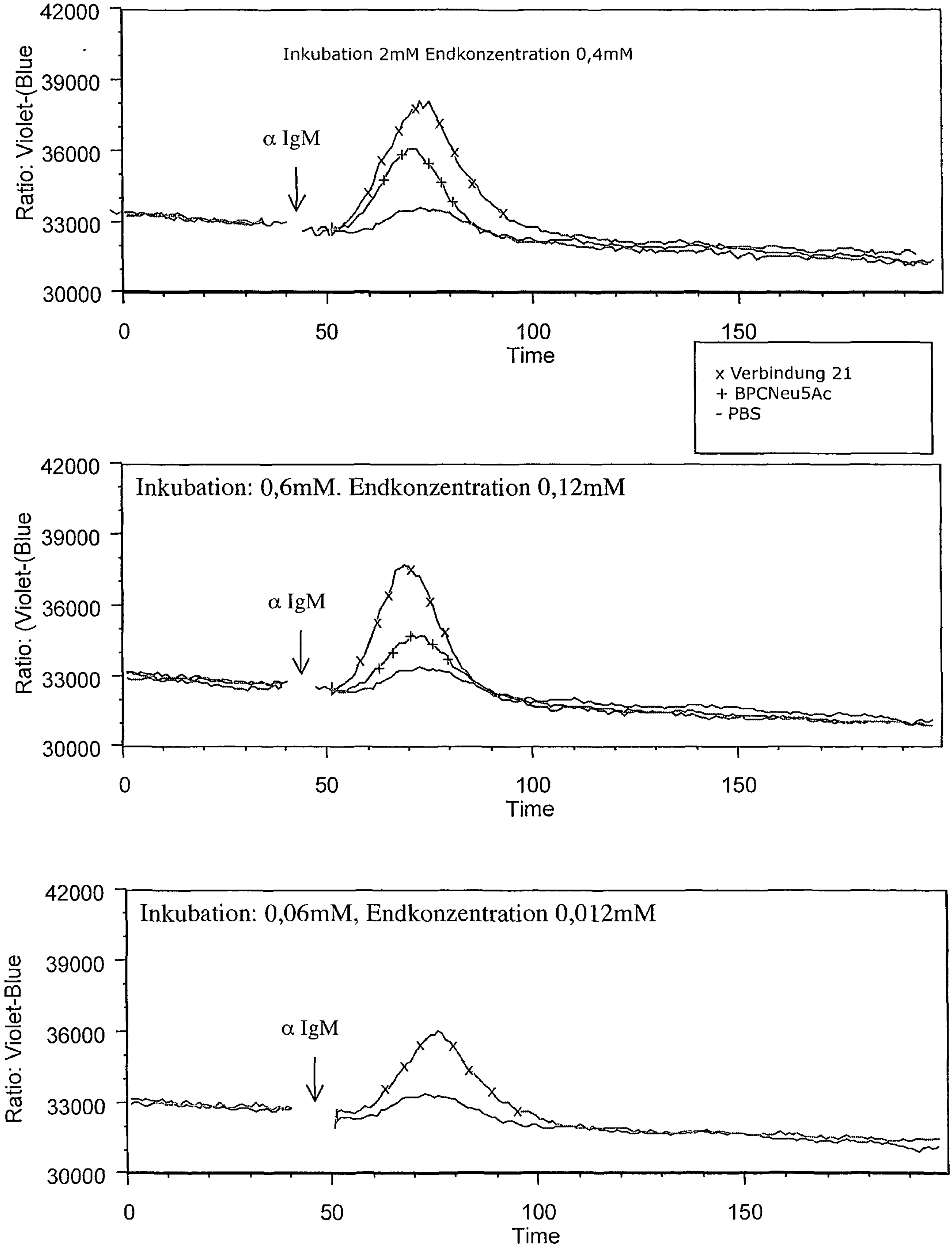
Inkubation 2mM Endkonzentration 0,4mM
α IgM
Ratio: Violet-(Blue
42000
39000
36000
33000
30000
0
50
100
150
Time
x Verbindung 21
+ BPCNeu5Ac
- PBS
Inkubation: 0,6mM. Endkonzentration 0,12mM
α IgM
Ratio: (Violet-(Blue
42000
39000
36000
33000
30000
0
50
100
150
Time
Inkubation: 0,06mM, Endkonzentration 0,012mM
α IgM
Ratio: Violet-Blue
42000
39000
36000
33000
30000
0
50
100
150
Time

Fig. 1

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 03000709 A


### In der Beschreibung aufgeführte Nicht-Patentliteratur


- **SCHAUER.** *Zoology,* 2004, vol. 107, 49-64
- **VARKI.** *Trends in Mol Med,* 2008, vol. 14 (8), 351-360
- *Glycoconjugate J.,* 2006, vol. 23
- **LEHMANN et al.** *Cell. Mol. Life Sci.,* 2006, vol. 63, 1331-1354
- *Trends in Pharmacological Sciences,* 2009, vol. 30 (5), 240-248
- **TEDDER et al.** *Advances in Immunology,* 2005, vol. 88, 1-50
- **COURTNEY et al.** *PNAS,* 2009, vol. 106 (8), 2500-505
- **COLLINS et al.** *Journal of Immunology,* 2006, vol. 177, 2994-3003
- **STEIRER et al.** *J. Biol. Chem.,* 2009, vol. 284 (6), 3777-3783
- **PHILIP J. KOCIENSKI.** Protecting Groups. Thieme, 2005
- **H.M.I. OSBORN.** Carbohydrates: Best synthetic methods. Academic Press, 2003
- *Angewandte Chemie,* 2005, vol. 117, 5320-5374
- *Chem Rev,* 1988, vol. 88 (2), 297-368
- *Medicinal Research Reviews,* 2008, vol. 28, 278-308
- *J. Am. Chem. Soc.,* 2003, vol. 125, 7754-7755
- *Carbohydrate Res.,* 2008, vol. 343, 1636-1643
- *Tetrahedron,* 2005, vol. 61, 10827-10852
- *Chem. Rev.,* 2009, vol. 109, 2455-2504
- **H.M.I. OSBORN.** Carbohydrate: Best synthetic methods. Academic Press, 2003
- *J. Org. Chem.,* 2000, vol. 65, 958-963
- *Chem. Eur. J.,* 2009, vol. 15, 9404-9416
- *Chem. Rev.,* 2009, vol. 109, 2455-2504
- **MERCK.** *Anfärbereagenzien für Dünnschicht- und Papierchromatographie,* 1970
- *Synthesis,* 2001, vol. 7, 1049-1052
- *J. Carbohydrate Chemistry,* 1987, vol. 6 (1), 161-165
- *Eur. J. Org.Chem.,* 2009, vol. 16, 2611-2620
- *Liebigs Ann. Chem.,* 1991, 487-495
- *Bioorganic & Med. Chem. Letters,* 1995, vol. 5 (23), 2809-2814
- **BOCK et al.** *Methods Mol. Biol.,* 2006, vol. 347, 359-375
- *J. Exp. Med.,* 2002, vol. 195, 1207-1213